# EUROPEAN PATENT APPLICATION

(11) **EP 4 186 921 A1**
(43) Date of publication of application: **31.05.2023**
(21) Application number: 22204543.7
(22) Date of filing: 25.03.2019
(51) Int. Cl.: C07K 14/705, C12N 9/16, C12N 9/22, C12N 15/86, C12N 15/113

(54) **GENE EDITING FOR AUTOSOMAL DOMINANT DISEASES**

(30) Priority: 23.03.2018 US 201862647415 P
(62) Divisional of application: 19770270.7
(71) Applicant: The Trustees of Columbia University in the City of New York, New York, NY 10027 (US)
(72) Inventor: WU, Wen-Hsuan, New York, 10032 (US); TSAI, Yi-Ting, New York, 10032 (US); TSANG, Stephen, H., New York, 10032 (US)
(74) Representative: Elkington and Fife LLP

(57) **Abstract**

The present disclosure provides methods for treating autosomal dominant diseases in a subject. In some aspects, the methods involve the use of a gene editing enzyme with a pair of unique guide RNA sequences that targets both mutant and wildtype forms of autosomal dominant disease-related gene for destruction in cells, and then supplying the cells with wildtype autosomal dominant disease-related gene cDNA which is codon modified to evade recognition by the guide RNAs. These methods are broadly applicable to any autosomal dominant disease.

## Description

### CROSS REFERENCE TO RELATED APPLICATION

This application claims priority to U.S. Provisional Application No. 62/647,415 filed on March 23, 2018, which is incorporated by reference herein in its entirety.

### GOVERNMENT LICENSE RIGHTS

This invention was made with government support under RO1EY024698 awarded by the National Institutes of Health (NIH). The government has certain rights in the invention.

### SEQUENCE LISTING

The instant application contains a Sequence Listing which has been submitted electronically in ASCII format and is hereby incorporated by reference in its entirety. Said ASCII copy, created on March 21, 2019, is named 01001_006660-WO0_ST25.txt and is 37 KB in size.

### FIELD OF THE INVENTION

The present disclosure relates to using CRISPR-based methods to perform gene editing in patients in order to treat autosomal dominant diseases.

### BACKGROUND OF THE DISCLOSURE

There are currently no cures for numerous autosomal dominant or recessive diseases that have a profoundly negative impact on quality of life. Dominant forms of retinitis pigmentosa (RP), cone-rod dystrophies and juvenile macular degenerations are prime examples of dominant autosomal diseases that affect the eye. Retinal degenerative diseases affect at least 9 million Americans (Friedman DS et al. Arch Ophthalmol. 2004 Apr;122(4):564-72; Schmier JK et al. Pharmacoeconomics. 2006; 24(4): 319-34). Among the most devastating retinal degenerative disease is retinitis pigmentosa (RP), a common form of inherited neurodegeneration, which affects 1.5 million people worldwide and for which treatment is inadequate. RP is a degenerative eye disease that results in retinal degeneration and vision loss. Hereditary mutations in the rhodopsin gene *(RHO)* are the most common cause of autosomal dominant RP, accounting for 20-30% of the cases. Currently, there is no cure for RP.

These autosomal dominant diseases are characterized by the presence of a mutant gene expressing a defective protein. These diseases are not, therefore, readily amenable to therapies that simply add a normal, healthy gene (so called "gene supplementation" or "gene addition"), since the disease-causing gene and protein are still present. Gene therapy for RP was tested in proof-of-concept animal models, and later used as clinical treatment, where it improved vision in up-to half of the patients. In these trials, patients' genetic abnormalities were corrected by a gene supplementation approach (i.e., rescue via overexpression of a wild type (wt) gene). Initially, the gene supplementation appeared to work because patients experienced a functional rescue, but follow-up examination showed that degeneration of photoreceptors continued, and vision loss progressed in 3 years (Cideciyan et al. Proc Natl Acad Sci USA. 2013 Feb 5;110(6):E517-25; Bainbridge et al. N Engl J Med. 2015 May 14;372(20):1887-97; Jacobson et al. N Engl J Med. 2015 May 14;372(20):1920-6). Current gene therapy trials for other RP genes are also taking a gene supplementation approach and are likely to face similar hurdles unless the reasons for failure are addressed. Since supplementation with a wt gene leaves the patient's mutant gene intact, the presence of the mutant gene can continuously trigger ongoing damage despite the presence of a wt gene.

Best vitelliform macular dystrophy (VMD) is a blinding macular disorder that arises from over 250 autosomal dominant point mutations in Bestrophin-1 (BEST1), which encodes a calcium-dependent chloride (Cl⁻) channel. BEST1 mutations decrease or extinguish the channel's ability to efflux Cl⁻ ions, causing fluid to accumulate in the subretinal space. Devising treatment for this condition is complicated by two major obstacles: 1) most VMD is autosomal dominant; and 2) BEST1 mutations are vastly heterogeneous. Thus, traditional patient-specific therapy, which targets specific mutations, is not a practical treatment option.

Dominant disorders are much more difficult to treat than recessive disorders. In fact, there are currently no effective treatments for dominant inherited disorders. The present method addresses the unmet need and can be generally applied to all patients with the mutations of a disease-related gene. The method overcomes the limitations of traditional patient-specific gene therapy to treat only autosomal recessive diseases and can be broadly applicable to any dominant disease.

### SUMMARY

In one embodiment, the disclosure is related to methods for modifying an autosomal dominant disease-related gene in a cell, the method comprising, or consisting essentially of, or yet further consisting of: contacting the cell with at least one type of vector encodinga CRISPR-Cas system directed to a mutant allele of the autosomal dominant disease-related gene, wherein the autosomal dominant disease-related gene relates to or corrects an ocular disease. In one aspect, the CRISPR-Cas system is comprised within the at least one type of vector comprises: (i) a first sequence encoding a first guide RNA that hybridizes to a variant of single nucleotide polymorphism (SNP) in the autosomal dominant disease-related gene ; (ii) a second sequence encoding a second guide RNA that hybridizes to an intron of the autosomal dominant disease-related gene; and, (iii) a third sequence encoding a Cas nuclease, wherein the Cas nuclease cleaves or targets only the mutant allele that encodes the autosomal dominant disease-related gene that comprises a single nucleotide polymorphism (SNP). Non-limiting examples of cells include animal cell, mammalian cells, canine cells, feline cells, equine cells and human cells.

In one embodiment, the first guide RNA comprises a nucleotide sequence selected from SEQ ID NO: 40, SEQ. ID NO. 41, SEQ ID NO: 42, SEQ ID NO: 43, SEQ ID NO: 44, SEQ ID NO: 45, SEQ ID NO: 46, SEQ ID NO: 47, SEQ ID NO: 48, or SEQ ID NO: 49 or an equivalent of each thereof.

In another aspect, the disclosure provides methods for modifying an autosomal dominant disease-related gene in a cell, the method comprising, or alternatively consisting essentially of, or yet further consisting of: contacting the cell with at least one type of vector encoding a CRISPR-Cas system directed to a mutant allele of the autosomal dominant disease-related gene, wherein the autosomal dominant disease-related gene relates to or corrects an ocular disease. In one aspect, the CRISPR-Cas system is comprised within at least one type of vector that comprises: (i) a first sequence encoding a first guide RNA that hybridizes to a variaent of a single nucleotide polymorphism (SNP) in the autosomal dominant disease-related gene that and, (ii) a second sequence encoding a catalytically defective Cas nuclease (dCas). Non-limiting examples of cells include animal cell, mammalian cells, canine cells, feline cells, equine cells and human cells.

In one embodiment, the second guide RNA comprises a nucleotide sequence selected from SEQ ID NO: 34, SEQ ID NO: 35, SEQ ID NO: 36, SEQ ID NO: 37, SEQ ID NO: 38 or SEQ ID NO: 39 or an equivalent of each thereof.

In one aspect, the disclosure provides method for treating an autosomal dominant ocular disease in a subject, the method comprising, or consisting essentially of, or yet further consisting of: administering to the subject a therapeutically effective amount of at least one type of vector encoding a CRISPR-Cas system directed to, or to correct a mutant allele of an autosomal dominant disease-related gene in the subject. In one aspect, the CRISPR-Cas system is comprised within at least one type of vector that comprises: (i) a first sequence encoding a first guide RNA that hybridizes to a variant of a single nucleotide polymorphism (SNP) in the autosomal dominant disease-related gene that comprises a single nucleotide polymorphism (SNP); (ii) a second sequence encoding a second guide RNA that hybridizes to an intron of the autosomal dominant disease-related gene; and, (iii) a third sequence encoding a Cas nuclease, wherein the Cas nuclease cleaves or targets only the mutant allele that encodes the autosomal dominant disease-related gene that comprises a single nucleotide polymorphism (SNP). Non-limiting examples of subjects include animals, mammalians, canines, felines, equines and human patients.

The disclosure further relates to methods for treating an autosomal dominant ocular disease in a subject, the method comprising, or consisting essentially of, or yet further consists of: administering to the subject a therapeutically effective amount of at least one type of vector encoding a CRISPR-Cas system directed to a mutant allele of an autosomal dominant disease-related gene in the subject. In one aspect, the CRISPR-Cas system comprisesat least one type of vector that comprises: (i) a first sequence encoding a first guide RNA that hybridizes to a variant of a single nucleotide polymorphism (SNP) in the autosomal dominant disease-related gene that comprises a single nucleotide polymorphism (SNP); and, (ii) a second sequence encoding a catalytically defective Cas nuclease (dCas), wherein the Cas nuclease cleaves or targets only the mutant allele that encodes the autosomal dominant disease-related gene that comprises a single nucleotide polymorphism (SNP). Non-limiting examples of subjects include animals, mammalians, canines, felines, equines and human patients.

In one embodiment, the autosomal dominant disease-related gene comprises a sequence selected from SEQ ID NO: 28, SEQ ID NO: 29, SEQ ID NO: 30, SEQ ID NO: 32, or SEQ ID NO: 33, or an equivalent of each thereof. In another embodiment, the first guide RNA that hybridizes to the autosomal dominant disease-related gene comprises a sequence of SEQ ID NO: 31 or an equivalent thereof. In one embodiment, the Cas nuclease does not cleave or target the wild-type allele that encodes the autosomal dominant disease-related gene.

In another embodiment, the first guide RNA hybridizes to the autosomal dominant disease-related gene comprises a sequence of SEQ ID NO: 31.

In one embodiment, the autosomal dominant disease-related gene is *RHO.* In one embodiment, the variant of the SNP is rs7984:G SNP, rs7984:A SNP, rs2855558:G SNP, or rs2855558:A SNP.

### BRIEF DESCRIPTION OF THE DRAWINGS

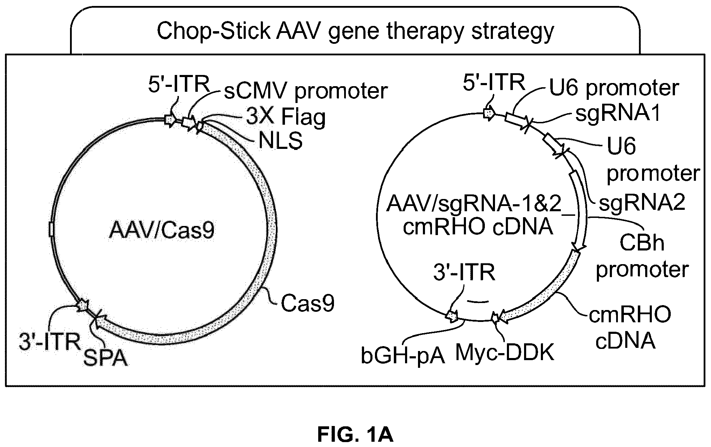

**FIG. 1A** is schematic representations of the ChopStick AAV vectors. The left side shows a schematic representation of the AAV/Cas9 vector. Cas9 from S. *pyogenes* is driven by a 173-bp short CMV promoter (sCMV, SEQ ID NO: 14) and is terminated by a 50-bp synthetic poly-A signal (SPA) (SEQ ID NO: 19). The right side shows a schematic representation of the *RHO* sgRNAs and codon-modified cDNA (cm*RHO*, (SEQ ID NO: 9) expression vector. sgRNA1 and sgRNA2 are driven by U6 promoter (SEQ ID NO: 12). cm*RHO* cDNA with c-terminal tagged c-Myc is driven by CBh promoter (SEQ ID NO: 10) and terminated by bGH poly-A signal (SEQ ID NO: 11) Arrows indicate the direction of transcription. 5'-and 3'-ITR, inverted terminal repeats of AAV.

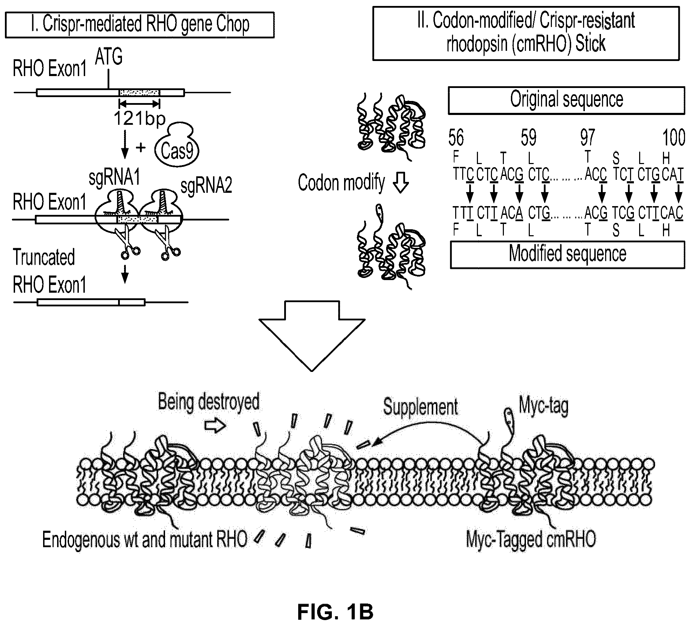

**FIG. 1B** is a schematic representation of the ChopStick AAV gene therapy strategy. The left side schematic representation (I) shows that following co-infection of AAV/Cas9 and AAV/sgRNA1&2_cm*RHO*, the co-expression of Cas9 protein and two h*RHO* exon 1-specific sgRNAs, sgRNA1 and sgRNA2, will lead to a 121- bp deletion in the host *RHO* Exon1. The right side of the FIG. (II) shows the original and codon-modified rhodopsin sequence.

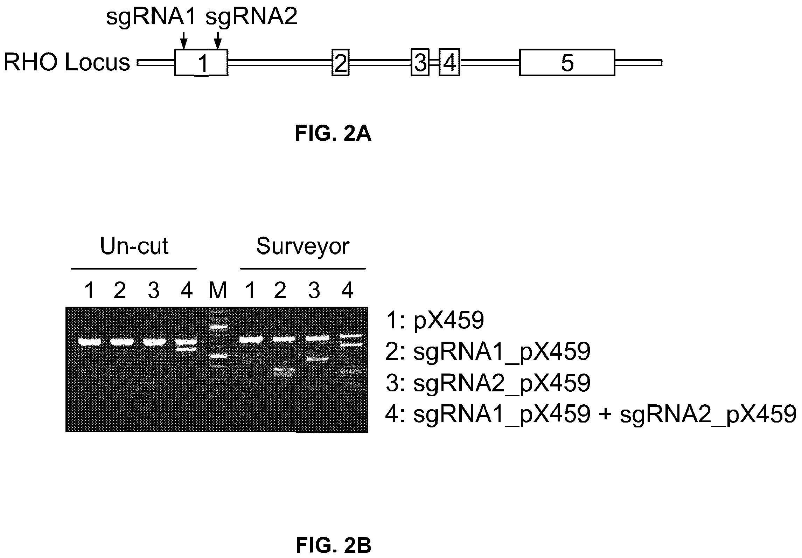

**FIGS. 2A-2B** show that dual sgRNA provides more efficient "Chop" of *RHO* than single sgRNA. FIG. 2A is a schematic representation of the target sites of sgRNA1 and sgRNA2 on RHO. The two sgRNAs both target *RHO* exon1, which is the beginning of the translation. Once the gene editing occurs, independent of whether one or two sgRNAs sites are targeted, most of the coding region will be affected. This design ensures the greatest disruption of gene expression and can be applied to many different types of *RHO* mutations. FIG. 2B shows improved efficiency in truncating genes by the "Chop" strategy in human kidney cell line compared to using only one sgRNA. HEK293FT cells were transfected with Cas9 vector (pX459) carrying either no sgRNA, single sgRNA1, single sgRNA2, or both. Ninety-six hours later, DNA was extracted, and the RHO locus was amplified and analyzed by mismatch detection SURVEYOR assay. Applying two sgRNAs together resulted in gene deletion of approximately 30-40%, which indicated that "Chop" (gene deletion/disruption) strategy works efficiently in mammalian cells (lane 4). Using one sgRNA (lanes 2 and 3) at a time in contrast does not result in change in size of the RHO gene. Approximately 30% of the genomic DNA underwent non-homologous end joining (NHEJ) by one sgRNA. In contrast, up to 80% was edited (deletion and NHEJ) when two sgRNAs were used. AS a control, equal amounts of plasmid DNA (1 µg / 1 × 10⁵ 293FT cells) were used in each group.

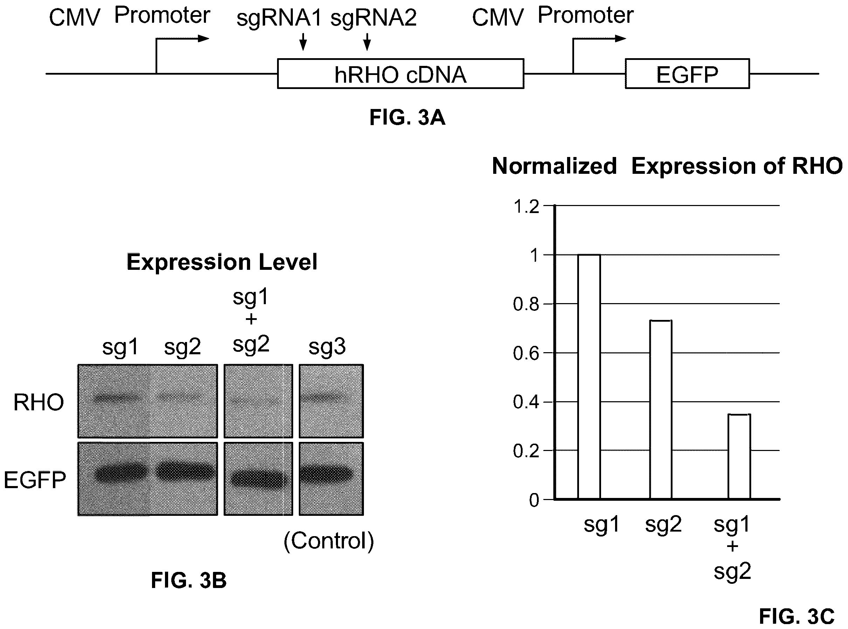

**FIGS. 3A-3C** show improved efficacy of inactivating a gene by dual sgRNA ("Chop" or gene deletion/disruption) when compared with a single sgRNA. FIG. 3A is schematic representation of the target sites of sgRNA1 and sgRNA2 on a *RHO* expression vector. The two sgRNAs target the 5' end of *RHO* cDNA as indicated. Wt *RHO* cDNA was driven by a CMV promoter. EGFP driven by CMV promoter was used as an internal control in immunoblot assay, which normalizes the difference in transfection efficiency and protein loading. FIG. 3B shows protein levels as measured by immunoblot when the HEK293FT cells were co-transfected with *RHO* expression vector and another vector expressing Cas9 machinery (pX459) carrying either sgRNA1, sgRNA2, or both. The sg3 group is a non-specific control sgRNA. FIG. 3C indicates that, after normalization with EGFP, two sgRNAs together lower *RHO* expression by 70%, while using single sgRNA reduced expression only by 0-30% (compared to the control group (sg3)). This result indicated that "Chop" strategy can be used to significantly reduce or inactivate protein expression.

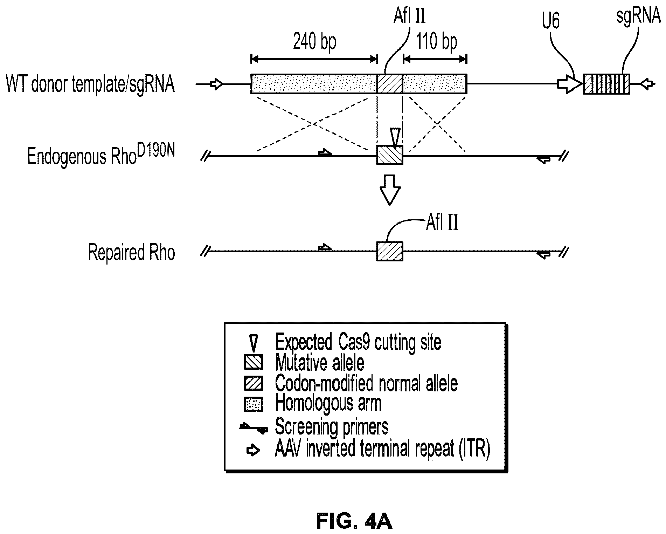

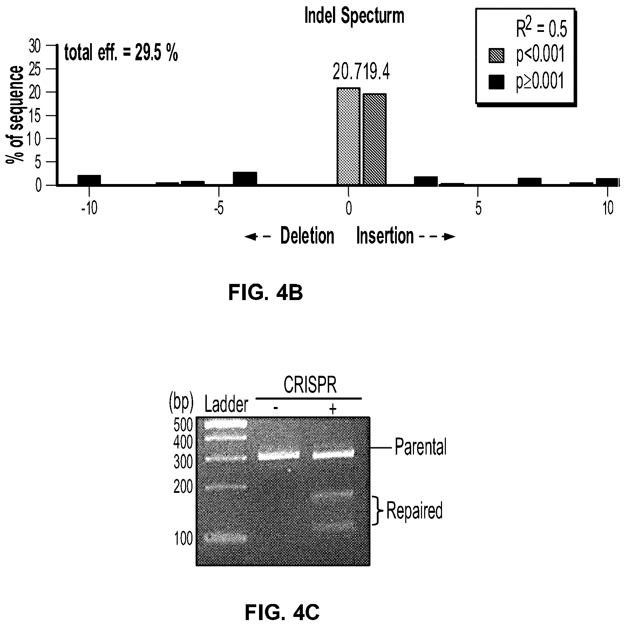

**FIGS. 4A-4C** show that "Chop" (gene deletion or disruption strategy) has a potential to create a double strand break in order to facilitate precise repair through mechanism like homologous recombination. FIG. 4A is schematic representation of the AAV-mediated CRISPR editing in *Rho*^{D190N} mouse RP model. Dual virus treatment of AAV/Cas9 vector and a bicistronic AAV vector containing wt donor template and an sgRNA targeting D190N mutation would result in mutation-specific repair. Donor template construct contains wild-type Rho sequence with two modifications: 1) creation of an additional *Afl*II site upstream of the D190 codon for the identification of DNA replacement following CRISPR-induced homologous recombination and 2) introduction of 5 wobble base pairs (bps) to render the donor template unrecognizable by sgRNA and thus, Cas9-resistant. FIG. 4B shows editing efficiency evaluated using tracking of indels (insertions and deletions) by decomposition (TIDE) analysis (publicly available at http://tide-calculator.nki.nl: retrieved April 30, 2016) in mouse retina DNA treated with aforementioned AAV viruses, which showed that -50% of photoreceptors underwent NHEJ. FIG. 4C is a representative *Afl*II digestion of retinal DNA from a *Rho*^{D190N/+} mouse showing a large portion of photoreceptors being repaired through homologous recombination (lane 2). *Rho*^{D190N/+} mice were treated with the Cas9 vector with (lane 2) or without (lane 1) the wild-type donor template, and retinal DNA was extracted and amplified with indicated screening primers.

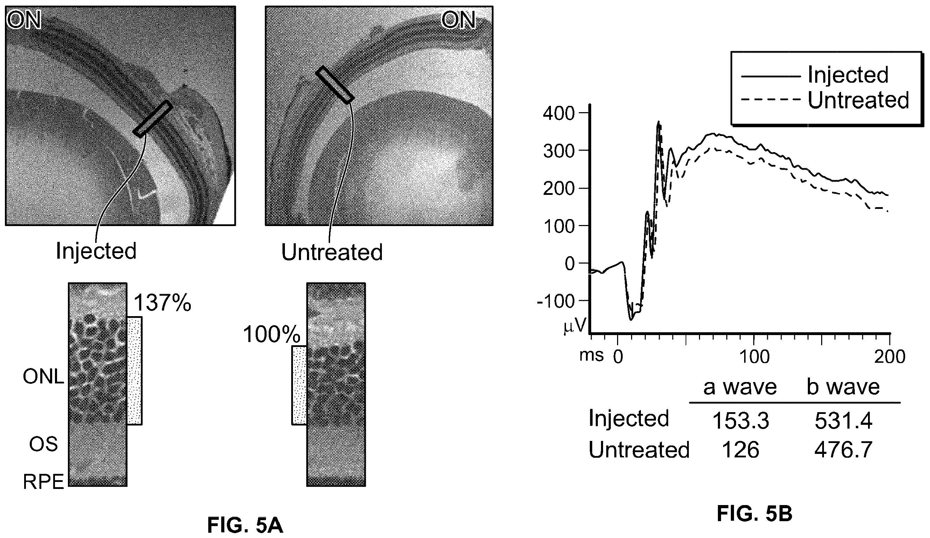

**FIGS. 5A-5B** show the histological and functional rescue by CRISPR/donor template-mediated repair. *Rho*^{D190N/+} heterozygote mice were treated with dual virus treatment described in FIGS. 4A-C by subretinal injection at postnatal day 3. FIG. 5B shows a visual function of mice evaluated by ERG following the treatment. FIG. 5A shows a histological evaluation of the retinal tissue section. The H&E staining of retinal section shows the increase of photoreceptors (outernuclear layer, ONL) survival at 137%, as compared to the untreated eye (FIG. 5A). The rectangular bars show an enlarged cross-sectional area of an ONL of photoreceptors in CRISPR/Cas9 (injected) and control eyes (untreated). The electroretinograms (ERGs) indicate a noticeable improvement in both a wave and b wave, of gene specific CRISPR-mediated therapy of 3-month old Rho^{D190N/+} heterozygote (FIG. 5B).

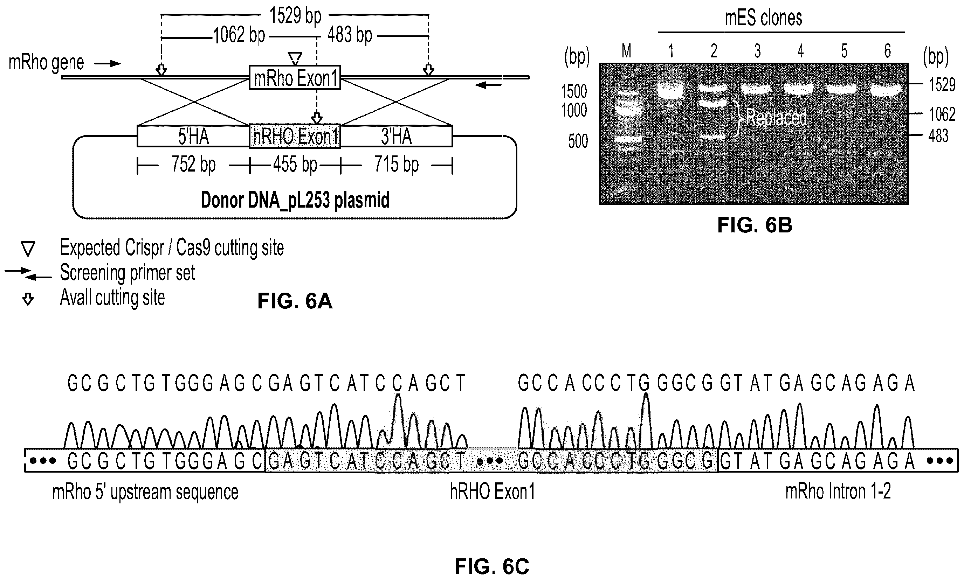

**FIGS. 6A-6C** describe the generation of RHO-humanized animal model by CRISPR-mediated exon 1 replacement at the mouse *Rho* locus. This system enables the researchers to test CRISPR components *in vivo.* FIG. 6A is an illustration of the strategy of replacing mouse (m) *Rho* exon 1 with either wild-type (wt) or mutant human (h) *RHO* exon 1. By co-electroporation of plasmid pX459 encoding Cas9 and *Rho* exon 1-specific sgRNA, a double strand break can be created in mouse exon1 that facilitates the homologous recombination in ES cells. FIG. 6B shows restriction fragment length polymorphism (RFLP) assay results of ES cell DNA featuring additional *Ava*II site indicating the replacement of mouse *Rho* exon 1 with human *RHO* exon 1 (lane 1 and 2). FIG. 6C: Sequence electropherogram of PCR amplicons reveals fusion of human and mouse sequence from one targeted ES clone.

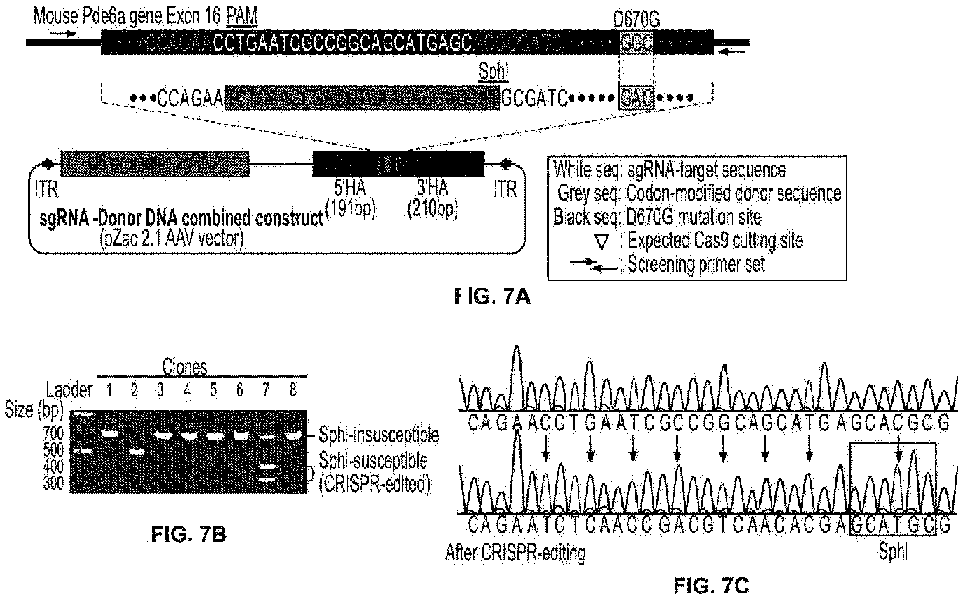

**FIGS. 7A-7C** show the successful gene replacement of the D670G allele in the gene encoding *Pde6a* by CRISPR in mouse embryonic stem cells. FIG. 7A is a schematic of donor construct which contains *Pde6a* with two changes: (1) a *Pde6a*-codon modification was introduced which creates an additional *SphI* site upstream from the D670G codon; and (2) eight wobble base pairs were introduced, making the donor template resistant to sgRNA targeting. FIG. 7B shows PCR amplicons generated from ES cells that underwent homologous recombination. FIG. 7C shows sequencing electropherogram data of target ES clone DNA, featuring an expected replacement of the D670G allele with donor template.

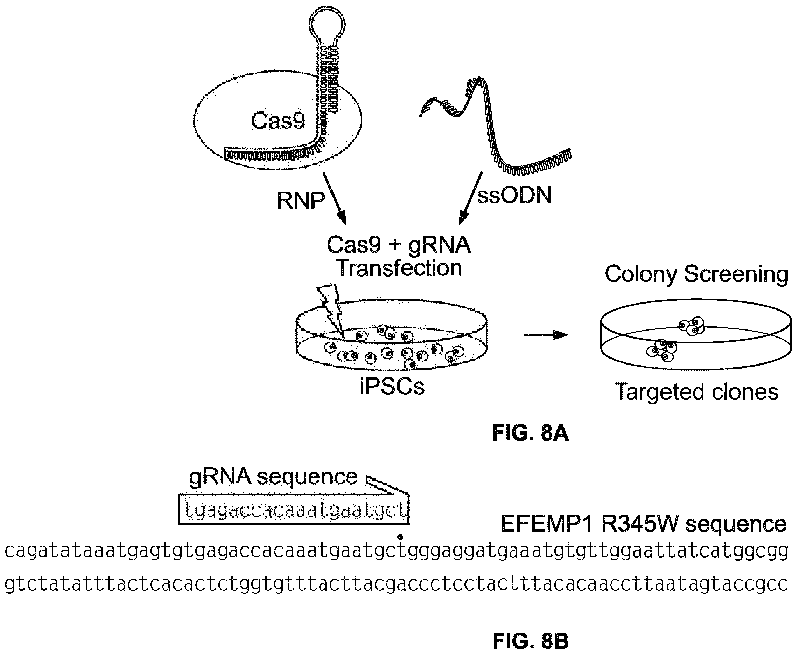

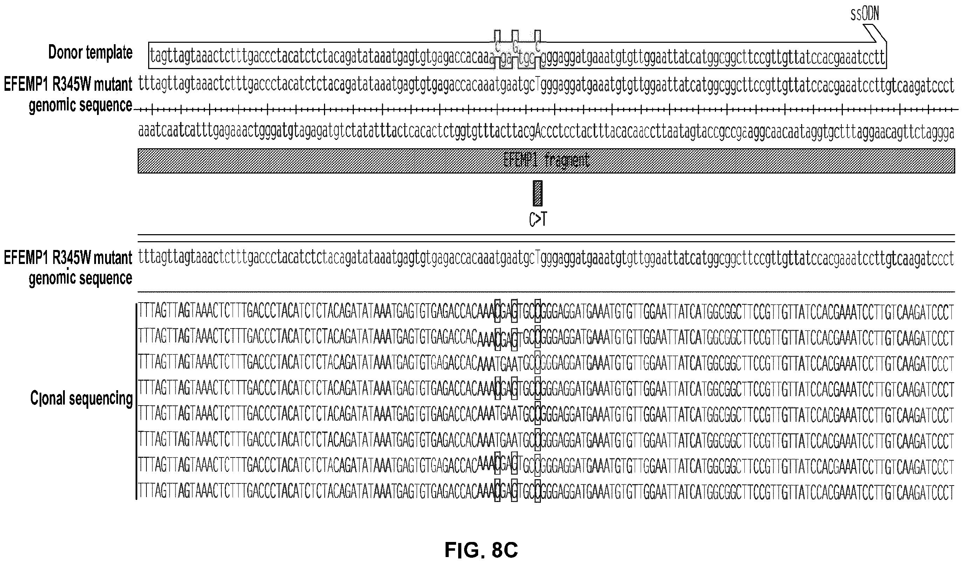

**FIGS. 8A-8C** show that "ChopStick" (gene deletion or gene disruption) can be used to efficiently delete and correct a gene region of interest, such as one containing a mutation, in induced pluripotent stem (iPS) cell from a patient with juvenile macular degenerations (OMIM #126600). FIG. 8A is a schematic illustration of the introduction of CRISPR components into human iPSCs (iPS cells). Cas9 protein/sgRNA complex (RNP) was co-nucleofected into human iPS cells with single strand donor template (ssODN). The clones were further selected and screened by restriction fragment length polymorphism (RFLP) assay. FIG. 8B is a schematic representation of sgRNA targeting site in this case. The nucleotide marked with a dot corresponds to the mutation site. FIG. 8C is the sequencing result of colony PCR, indicating replacement of donor template.

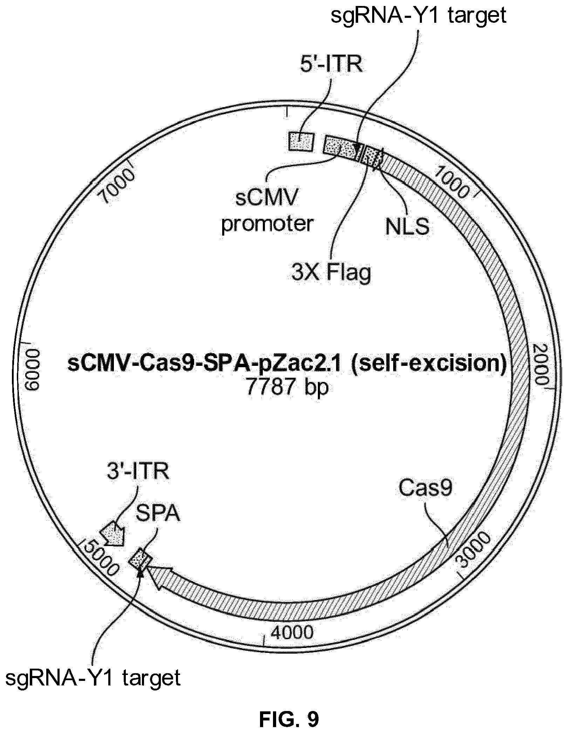

**FIG. 9** is a schematic representation of the self-excisional AAV/Cas9 vector. Cas9 from S. *pyogenes,* which is driven by a 173-bp short CMV promoter (sCMV) and terminated by a 50-bp synthetic poly-A signal (SPA), is flanked by sgRNA-Y1 (SEQ ID NO: 7) target sequences (GGTTTTGGACAATGGAACCGTGG, originated from Drosophila). Once the cell expresses Cas9 protein and sgRNA-Y1 simultaneously, this AAV/Cas9 vector is destroyed by Cas9 itself.

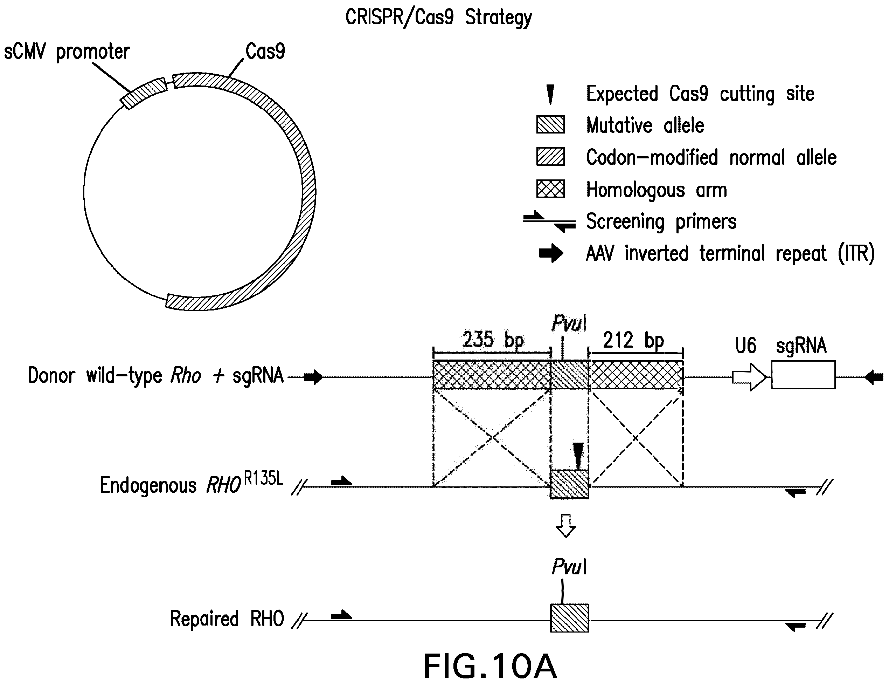

**FIGS. 10A-10D****:** CRISPRr correction of several genes in patient-derived iPS cells. (A) Diagram of the Cas9 expression vector and a schematic of the CRISPR/Cas9 repair strategy for targeting RHO^{R135L}. The same design was used in B-D. (B) A restriction fragment length polymorphism (RFLP) assay at the Pvul restriction site reveals that Cas9 cut and integrated the donor template. (C) An *in vitro* Surveyor mismatch cleavage assays show cutting of RPGR by g59 gRNA (arrows on the right of the left panel). The DNA sequencing chromatogram shows a corrected point mutation (arrow in the bottom chromatogram of the right panel) and the uncorrected, untreated control (arrow in the top chromatogram of the right panel). (D) HTRA1 DNA sequencing chromatogram shows a corrected point mutation (arrow in the bottom chromatogram of the right panel) and the uncorrected, untreated control (arrow in the top chromatogram of the right panel).

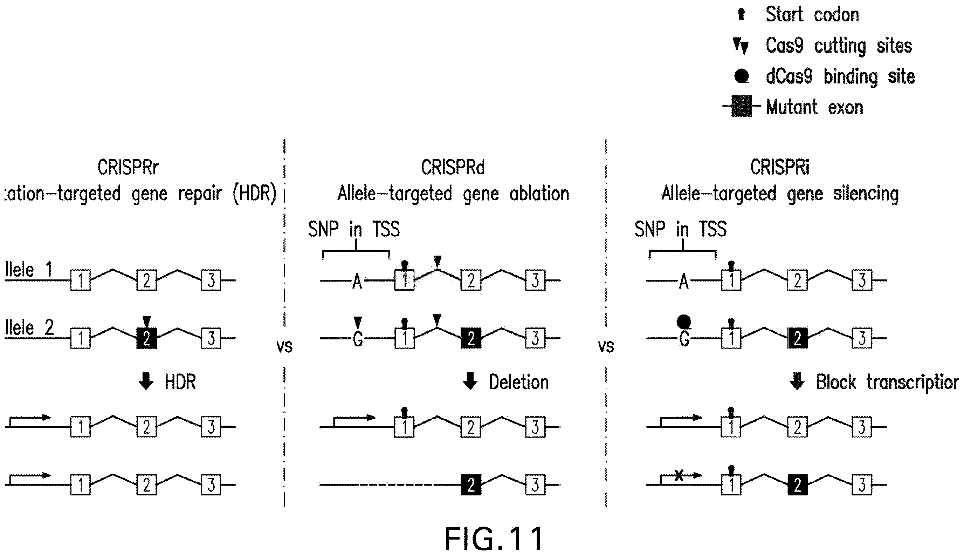

**FIG. 11****.** Comparison of CRISPRr, CRISPRd, and CRISPRi. HDR: homology-directed repair; SNP: single-nucleotide polymorphism; TSS: transcription start site. CRISPRr: Pros: impeccable gene correction; cons: low frequency of HDR; requires mutation-specific design (>250 mutations found in *BEST1*). CRISPRd: one design treats all mutations; efficient gene ablation. CRISPRi: one design treats all mutations; reversible.

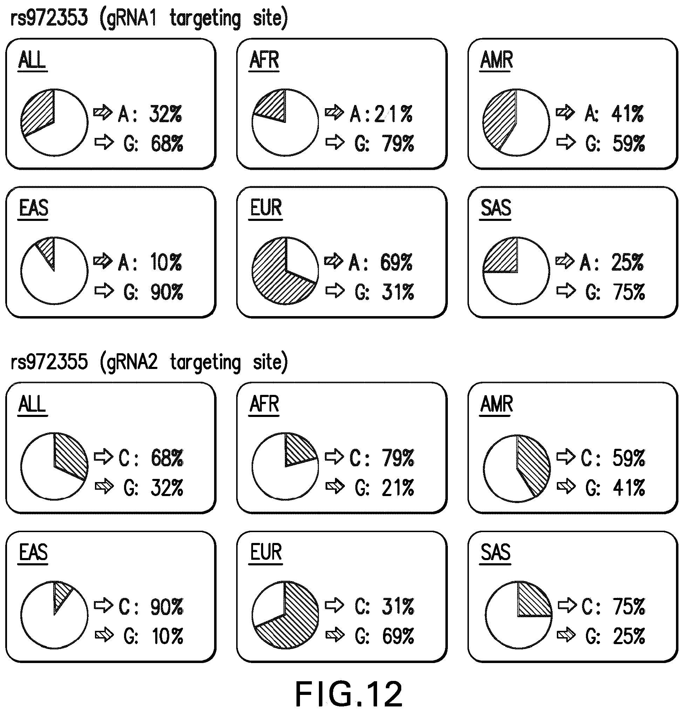

**FIG. 12****.** Two gRNA targeting SNPs with high heterozygosity. Levels of the variants are indicated by region. ALL, worldwide prevalence; AFR, Africa; AMR, America; EAS, East Asia; EUR, Europe; SAS, South Asia.

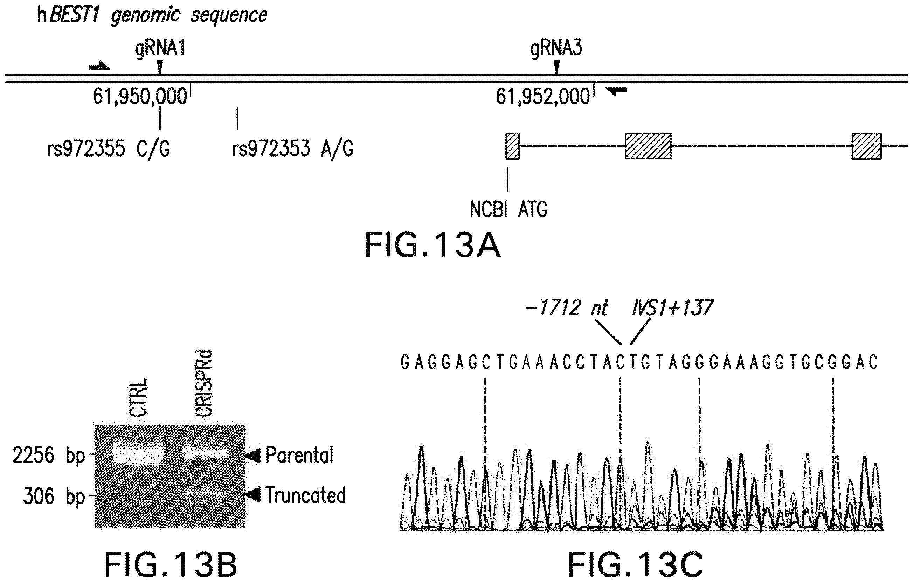

**FIGS. 13A-13C****.** CRISPRd mediates selective deletion of *BEST1* exon 1. **(A)** Schematic of CRISPRd cutting sites. gRNA1 targets SNP rs972355 and gRNA3 targets the center of intron 1. Horizontal arrows indicate the screening primer set. **(B)** Plasmid px459 bearing gRNA1 and gRNA3 was transfected into HEK293 cells, which are heterozygous for SNP rs972355. After 48 h incubation, genomic DNA was screened for CRISPRd-mediated deletion. A 306-bp truncated fragment was detected (compare with the 2,256-bp parental sequence). **(C)** The gross DNA was analyzed by Sanger sequencing, confirming that the targeted sequence was deleted.

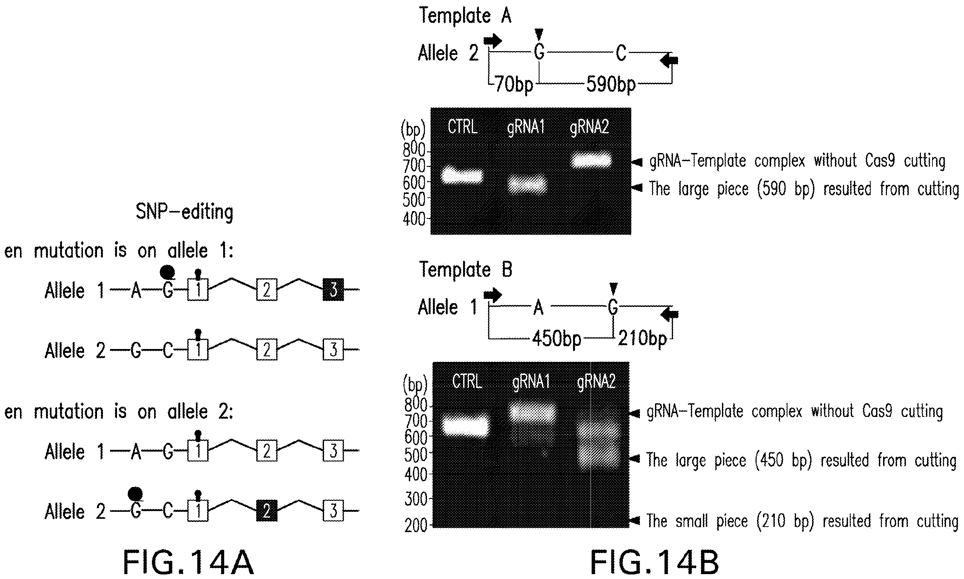

**FIGS. 14A-14B****.** *In vitro* SNP-specific *BEST1* targeting validates the allele-specific SNP strategy. **(A)** CRISPR cutting templates A and B were generated by PCR amplification of the *BEST1* TSS region from a patient with heterozygous SNP variants at rs972353 and rs972355. In the case of both SNPs, only variant form G is CRISPR-competent. **(B)** Templates A or B (650 bp) were exposed to Cas9 and either gRNA1 or gRNA2 (CTRL = no gRNA). gRNA1 but not gRNA2 efficiently directs the cutting on patient template A, resulting in 590-bp + 70-bp bands. (Note that the 70-bp band is too faint to see here, and that Cas9/gRNA2 binding - but with no cutting due to no adequate PAM site - to template A results in a shift of molecular weight of DNA). When the same experiment is carried out using template B, only gRNA2 (but not gRNA1) can mediate the cutting.

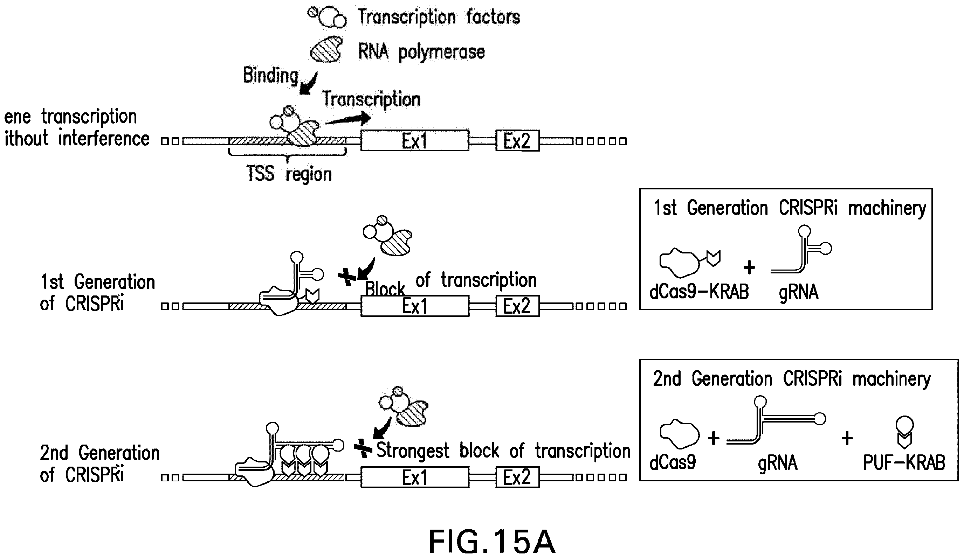

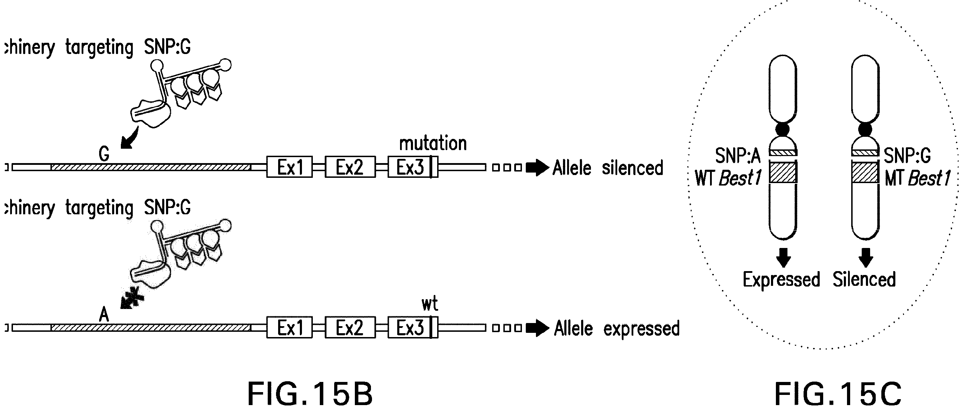

**FIGS. 15A-15C****.** CRISPRi selectively silences a mutant *BEST1* allele. **(A)** CRISPRi does not create a double-stranded DNA break but instead silences genes by blocking TSS binding by transcription factors. Unlike CRISPRr, CRISPRi uses a catalytically deficient Cas9 (dCas9) that does not cleave DNA but targets it via gRNAs. When the CRISPRi machinery binds the TSS, the Kruppel-associated box (KRAB) gene repressor, which is fused with dCas9, silences a downstream gene (firstgeneration). Second generation CRISPRi more strongly represses via PUF-KRAB repressors. The PUF adaptor protein binds gRNA modified with a PUF-binding sequence. **(B, C)** Two consecutive SNPs (rs972353 or rs972355) in the TSS enable allele-specific targeting. For ex ample, gRNA2 targets the rs972353:G SNP near the TSS to knock down MT *BEST1* expression.

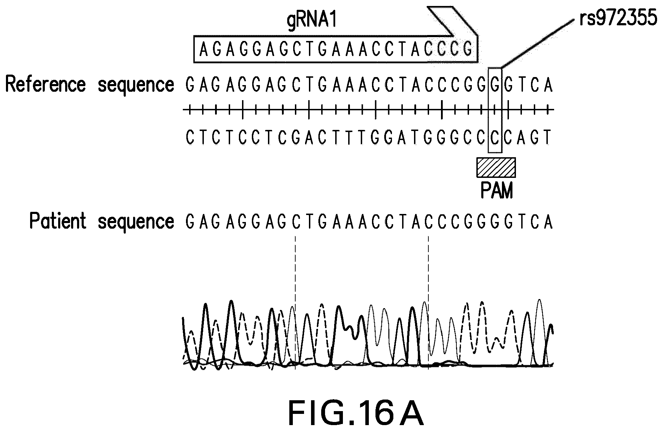

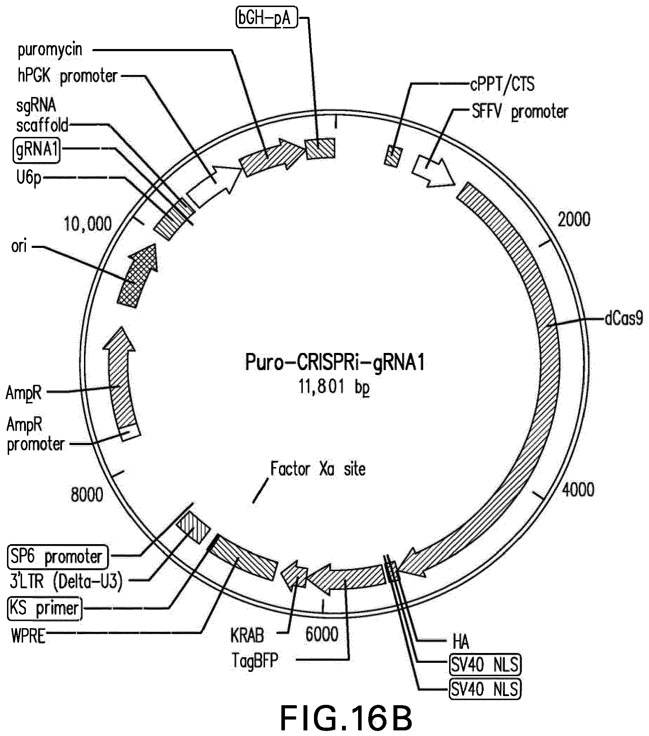

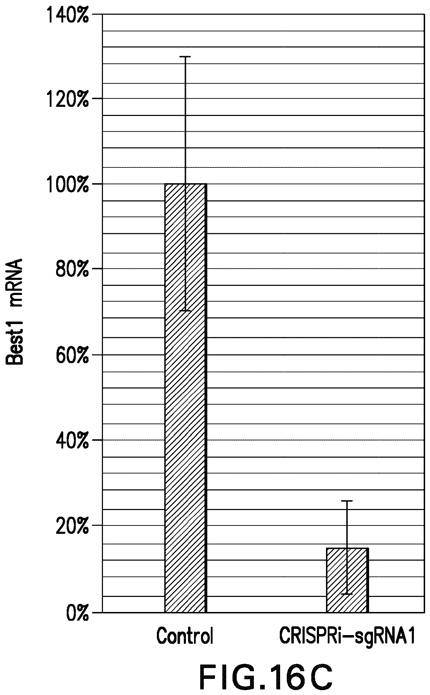

**FIGS. 16A-16C****.** CRISPRi knockdown of *BEST1* mRNA expression in iRPE cells. The iPS-derived RPE cells from a VMD patient with GGG variant at SNP rs972355. **(A)** The design of gRNA1 targeting site. **(B)** The cells were transfected by plasmid Puro-CRISPRi-sgRNA1, which was selected for by puromycin over the span of two weeks. **(C)** The *BEST1* mRNA level was evaluated by qPCR.

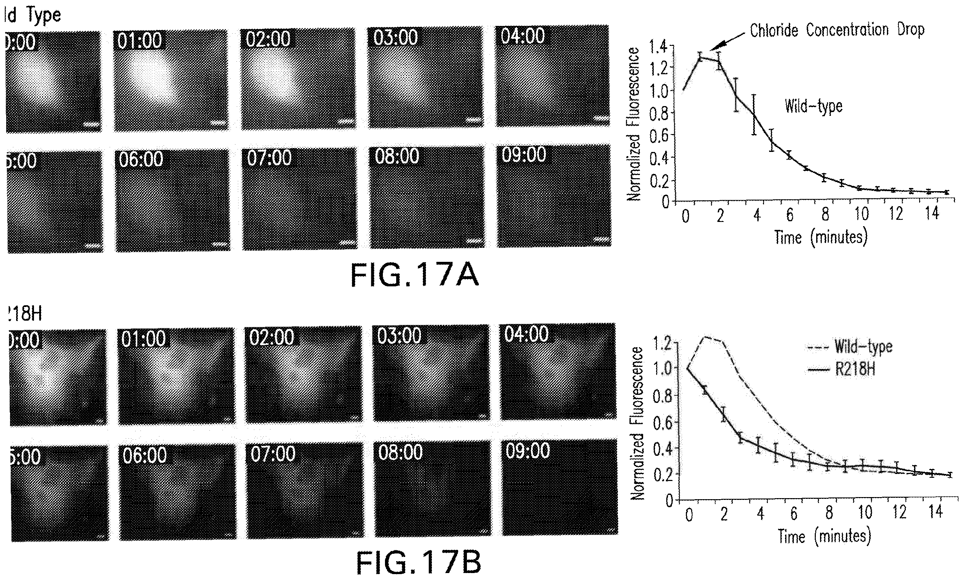

**FIGS. 17A-17B****.** Representative still images of wild-type **(A)** and pArg218H **(B)** patient-derived RPE cells expressing the Cl- biosensor and stimulated with 5µM A23187 (left panels). Images were acquired at the indicated times (min) after stimulation. Graphs show quantification of mean whole-cell biosensor emission intensity, normalized to the t = 0 time point (Moshfegh, Velez et al. 2016).

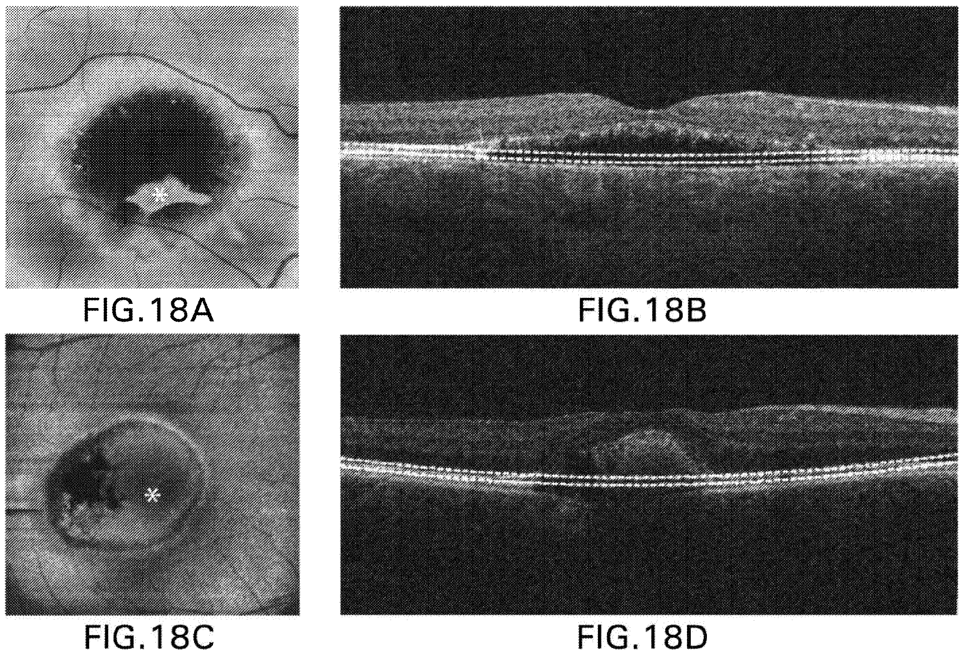

**FIGS. 18A-18D****.** En face OCT of a 45-year-old male **(A)** and 31-year-old female **(C)** with VMD acquired with a Cirrus HDOCT (Zeiss; Oberkochen, Germany), and the corresponding 37µm slice in cross section **(B, D)** at the approximate level of the IS/OS band. The central vitelliform lesion can be viewed at high resolution with this technique and supplement traditional FAF and SD-OCT imaging when assessing the structural constituents of the vitelliform lesion. A pseudohypopyon is visualized in each patient (white asterisk), but is more advanced in the 31 year old female **(C).**

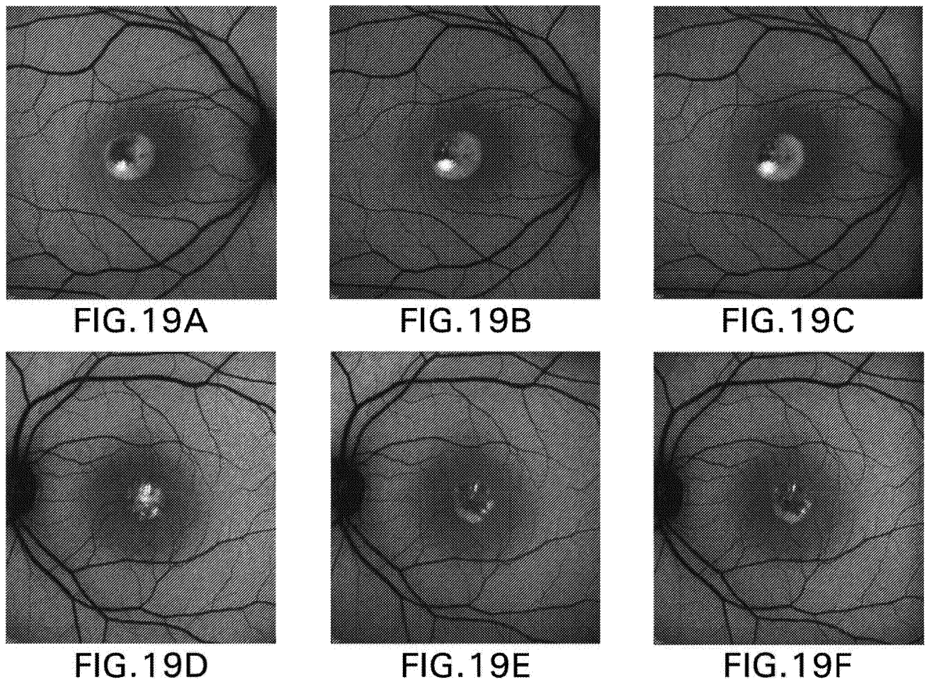

**FIGS. 19A-19F****.** SW-AF in 31-year-old woman with VMD due to p.R218H:c.653G>A. **(A)** and **(D),** OD and OS respectively, taken on November 4, 2015 at age 30. **(B**b**)** and **(E),** OD and OS respectively, taken on October 26, 2016 at age 31, 12 months after. **(C)** and **(F),** OD and OS respectively, taken on December 14, 2016 at age 31, 13 months after.

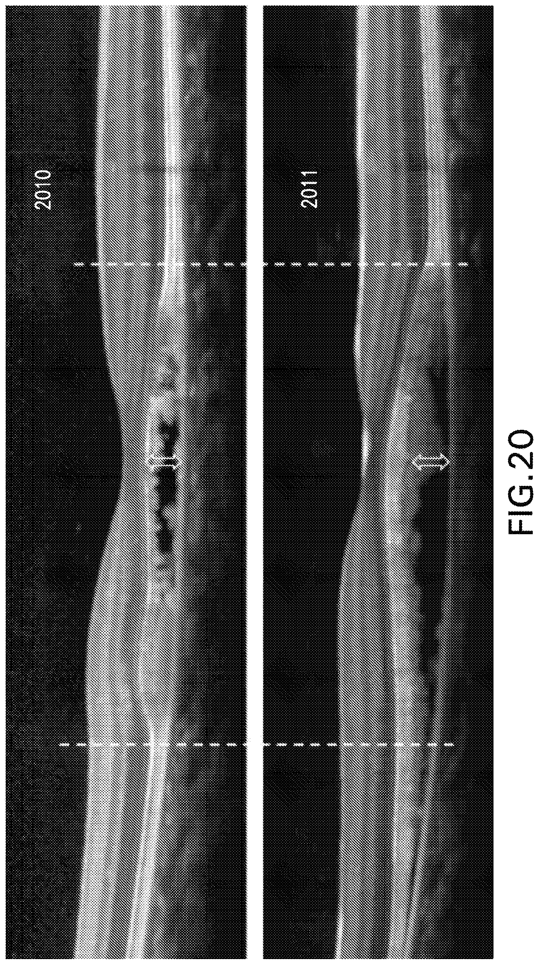

**FIG. 20****.** Optical coherence tomography (OCT) can be a reliable endpoint. OCT images monitor progression within 1 year. Progression seen in 11-year-old girl with autosomal dominant VMD due to p.Ala10Thr. Dashed lines indicate the initial horizontal diameter of lesion. Two-sided arrows indicate the initial elevation of lesion.

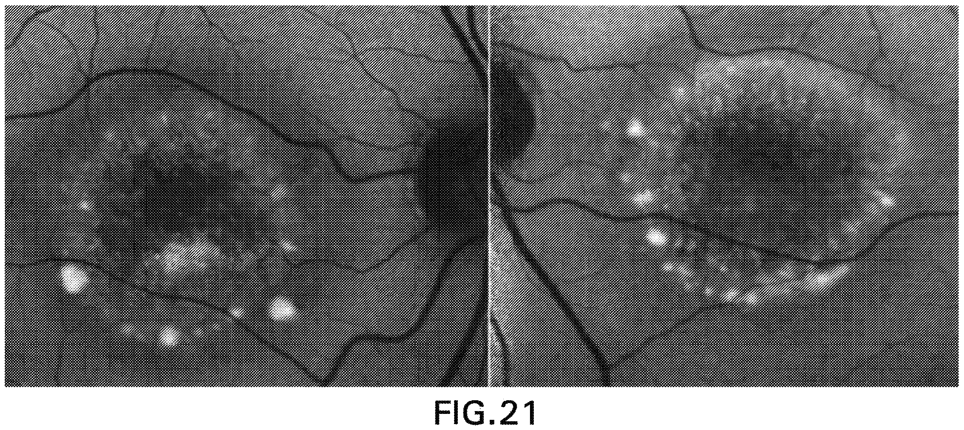

**FIG. 21****.** Symmetry between OD and OS seen in 44-year-old man with VMD due to p.Gln293Lys. FA images detect bisretinoid lipofuscin accumulation and pigment epithelium atrophy.

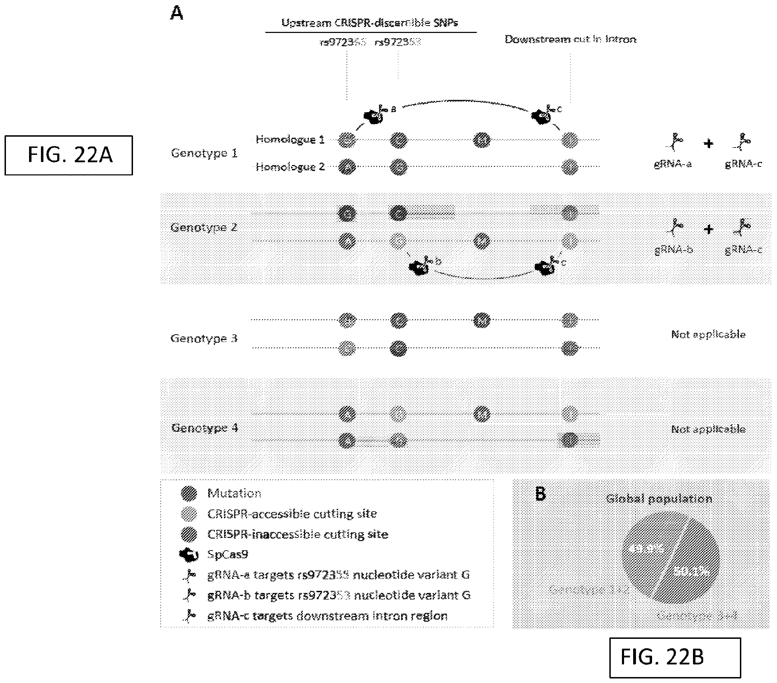

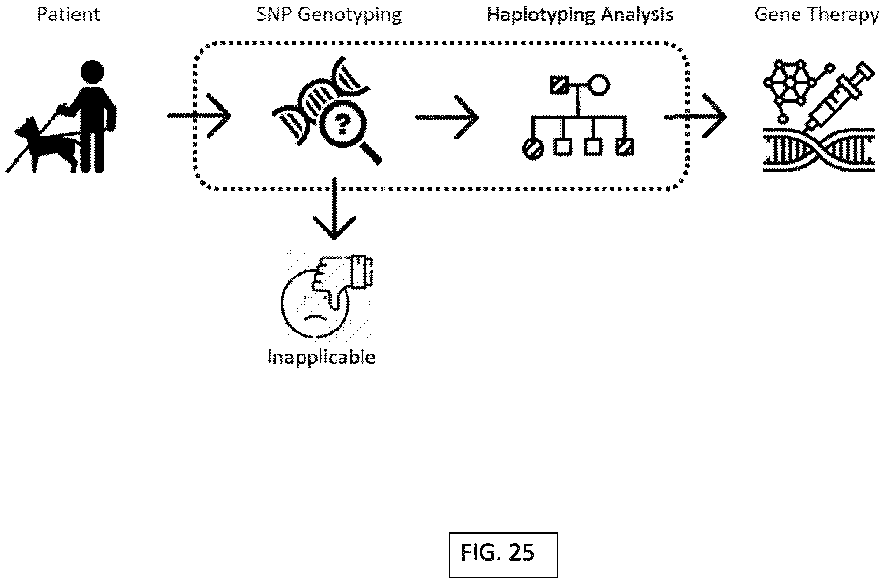

**FIGS. 22A and 22B****.** CRISPR3.0: a treatment for patients with Best VMD patients carrying different dominant mutations. **(A)** A pair of closely-linked SNPs, r972355 and rs972353, are utilized in CRISPR3.0 **(FIG. 22C)** to mark the identity of each homologue in a pair of homologous. All four possible combinations of nucleotide variants of both SNPs on chromosomes are shown. Three gRNAs were designed in order to achieve CRISPR3.0: gRNA-a targets rs972355 nucleotide variant G (and not A); gRNA-b targets rs972353 nucleotide variant G (and not C); gRNA-c targets a downstream intron region. When mutation is present in homologue 1 (Genotype 1), gRNA-a + gRNA-c are used to specifically destroy expression of the mutant *BEST1* gene from homologue 1. If the mutation is instead on homologue 2 (Genotype 2), gRNA-b + gRNA-c will be used. Because these two SNPs do not distinguish between the two homologues in genotypes 3 and 4, these genotypes would require the use of other SNPs and gRNAs. **(B)** The gRNA sets designed based on rs972355 and rs972353 can treat -50% of the Best VMD patient population (Genotypes 1 + 2, based on TOPMED database). DNA from each patient can be analyzed to determine the genotype before treatment (See Fig. 25, workflow).

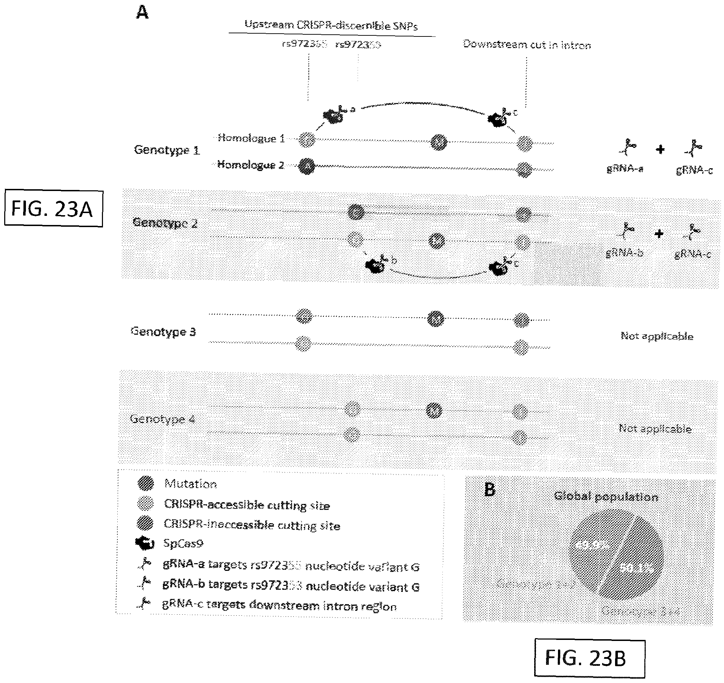

**FIGS. 23A and 23B****.** CRISPR3.0: a potential treatment for patients with Best VMD patients carrying different dominant mutations. **(A)** In CRISPR3.0, a pair of closely-linked SNPs, r972355 and rs972353, are utilized to mark the identity of each homologue in a pair of homologous. All four possible combinations of nucleotide variants of both SNPs on chromosomes are shown. Three gRNAs were designed in order to achieve CRISPR3.0: gRNA-a targets rs972355 nucleotide variant G (and not A); gRNA-b targets rs972353 nucleotide variant G (and not C); gRNA-c targets a downstream intron region. When a mutation is present in homologue 1 (Genotype 1), gRNA-a + gRNA-c are used to specifically destroy expression of the *BEST1* gene from homologue 1. If the mutation is instead on homologue 2 (Genotype 2), gRNA-b + gRNA-c are used. Because these two SNPs do not distinguish between the two homologues in genotypes 3 and 4, these genotypes require the use of other SNPs and gRNAs. **(B)** The gRNA sets are designed based on rs972355 and rs972353 can treat ~50% of the total Best VMD patient population (Genotypes 1 + 2, based on TOPMED database). DNA from each patient are analyzed to determine the genotype before treatment (See **FIG. 22D,** workflow).

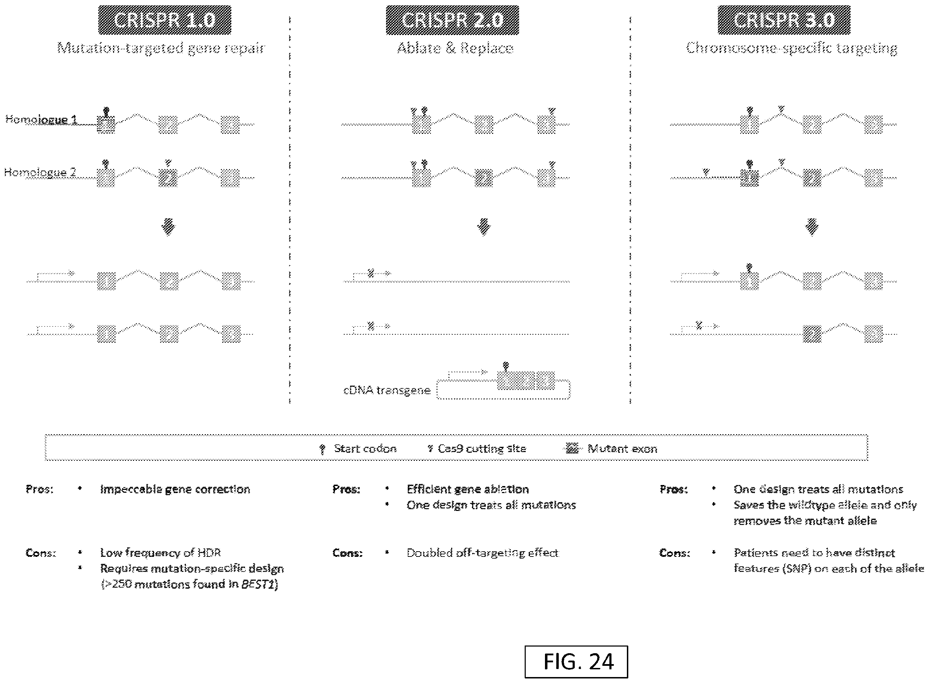

**FIG. 24** is an overview of CRISPR 1.0, CRISPR 2.0 and CRISPR 3.0
**FIG. 25** summarizes a workflow for CRISPR 3.0 treatment.

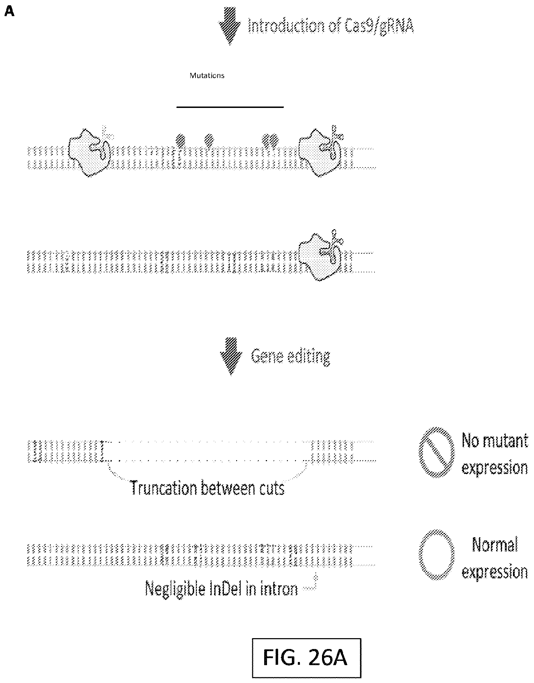

**FIGS. 26A-26C****.** CSGT (CRISPR3.0) - a treatment for roughly half of Best VMD patients. The BEST1 gene was sequenced using blood samples from 35 Best VMD patients to reveal all four possible combinations of nucleotide variants of both upstream SNPs, rs972355 and rs972353 (note that these two SNPs are genetically linked). CRISPR3.0, which exploits differences in TSS SNPs between homologous chromosomes, mediates selective gene truncation when the targeted TSS SNP is heterozygous. Thus, genotypes 1 and 2 (SNP heterozygotes in cis with the mutation), which represent 49.9% of a patient population, are treatable by CRISPR3.0, and genotypes 3 and 4 (SNP homozygotes) are can be treated by CRISPR2.0.

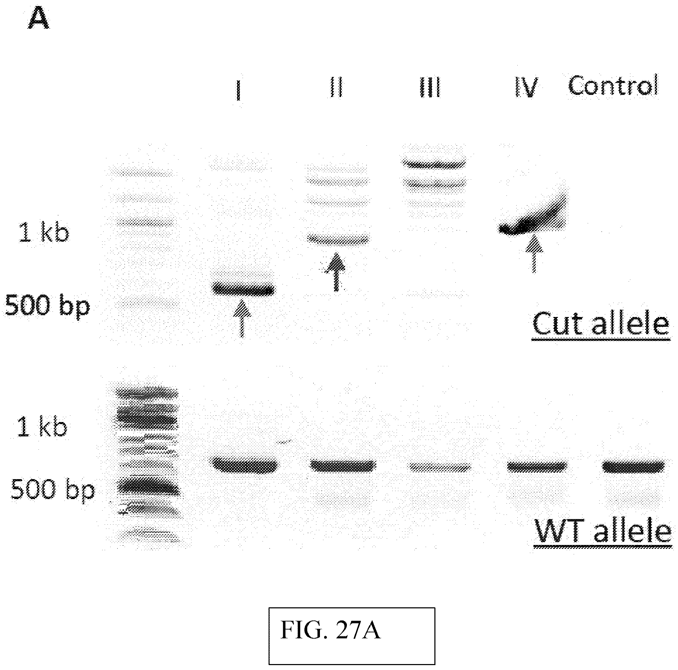

**FIGS. 27A-27B****.** Allele-specific excision of genomic *BEST1* by dual gRNA and measurement of *BEST1* expression upon SNP editing in RPE cells in vitro. **(A)** Genomic *BEST1* was truncated in 293FT cells in vitro by different dual-gRNA systems (CRISPR 3.0). Of the four groups. the truncated *BEST1* alleles of different sizes (arrows) were detected in three groups (I, II and IV). **(B)** *BEST1* expression is robust in CRISPR2.0- and 3.0-edited VMD iRPE. iRPE derived from VMD patient fibroblasts was transduced with lentiviral dual-gRNA vectors for CRISPR2.0 or 3.0 editing of BEST1 alleles; vectors carried WT BEST1 cDNA (+ WT) or not; WT iRPE was not transduced. After two weeks, RNA was collected, cDNA generated, and expression of BEST1 transcript determined by qPCR. PCR product of *BEST1* is ~260 bp. WT, WT RPE homozygous for SNP rs972355; Control, dual vector carrying WT *BEST1* cDNA only (no gRNA); GAPDH, internal control. No lentiviral vector was employed in the control group; GAPDH was used as the internal control.

### DETAILED DESCRIPTION

Embodiments according to the present disclosure will be described more fully hereinafter. Aspects of the disclosure may, however, be embodied in different forms and should not be construed as limited to the embodiments set forth herein. Rather, these embodiments are provided so that this disclosure will be thorough and complete, and will fully convey the scope of the invention to those skilled in the art. The terminology used in the description herein is for the purpose of describing particular embodiments only and is not intended to be limiting.

Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of the present application and relevant art and should not be interpreted in an idealized or overly formal sense unless expressly so defined herein. While not explicitly defined below, such terms should be interpreted according to their common meaning.

The terminology used in the description herein is for the purpose of describing particular embodiments only and is not intended to be limiting of the invention. All publications, patent applications, patents and other references mentioned herein are incorporated by reference in their entirety.

The practice of the present technology will employ, unless otherwise indicated, conventional techniques of tissue culture, immunology, molecular biology, microbiology, cell biology, and recombinant DNA, which are within the skill of the art.

Unless the context indicates otherwise, it is specifically intended that the various features of the invention described herein can be used in any combination. Moreover, the disclosure also contemplates that in some embodiments, any feature or combination of features set forth herein can be excluded or omitted. To illustrate, if the specification states that a complex comprises components A, B and C, it is specifically intended that any of A, B or C, or a combination thereof, can be omitted and disclaimed singularly or in any combination.

Unless explicitly indicated otherwise, all specified embodiments, features, and terms intend to include both the recited embodiment, feature, or term and biological equivalents thereof.

All numerical designations, e.g., pH, temperature, time, concentration, and molecular weight, including ranges, are approximations which are varied (+) or (-) by increments of 1.0 or 0.1, as appropriate, or alternatively by a variation of +/- 15 %, or alternatively 10%, or alternatively 5%, or alternatively 2% and such ranges are included. It is to be understood, although not always explicitly stated, that all numerical designations are preceded by the term "about". It also is to be understood, although not always explicitly stated, that the reagents described herein are merely exemplary and that equivalents of such are known in the art.

Throughout this disclosure, various publications, patents and published patent specifications are referenced by an identifying citation or by an Arabic numeral. The full citation for the publications identified by an Arabic numeral are found immediately preceding the claims. The disclosures of these publications, patents and published patent specifications are hereby incorporated by reference into the present disclosure in their entirety to more fully describe the state of the art to which this invention pertains.

### Definitions

The practice of the present technology will employ, unless otherwise indicated, conventional techniques of organic chemistry, pharmacology, immunology, molecular biology, microbiology, cell biology and recombinant DNA, which are within the skill of the art. See, e.g., Sambrook, Fritsch and Maniatis, Molecular Cloning: A Laboratory Manual, 2nd edition (1989); Current Protocols In Molecular Biology (F. M. Ausubel, et al. eds., (1987)); the series Methods in Enzymology (Academic Press, Inc.): PCR 2: A Practical Approach (M.J. MacPherson, B.D. Hames and G.R. Taylor eds. (1995)), Harlow and Lane, eds. (1988) Antibodies, a Laboratory Manual, and Animal Cell Culture (R.I. Freshney, ed. (1987)).

As used in the description of the invention and the appended claims, the singular forms "a," "an" and "the" are intended to include the plural forms as well, unless the context clearly indicates otherwise.

As used herein, the term "comprising" is intended to mean that the compositions and methods include the recited elements, but do not exclude others. As used herein, the transitional phrase consisting essentially of (and grammatical variants) is to be interpreted as encompassing the recited materials or steps and those that do not materially affect the basic and novel characteristic(s) of the recited embodiment. Thus, the term "consisting essentially of" as used herein should not be interpreted as equivalent to "comprising." "Consisting of" shall mean excluding more than trace elements of other ingredients and substantial method steps for administering the compositions disclosed herein. Aspects defined by each of these transition terms are within the scope of the present disclosure.

The term "about," as used herein when referring to a measurable value such as an amount or concentration and the like, is meant to encompass variations of 20%, 10%, 5%, 1 %, 0.5%, or even 0.1 % of the specified amount.

The terms or "acceptable," "effective," or "sufficient" when used to describe the selection of any components, ranges, dose forms, etc. disclosed herein intend that said component, range, dose form, etc. is suitable for the disclosed purpose.

Also as used herein, "and/or" refers to and encompasses any and all possible combinations of one or more of the associated listed items, as well as the lack of combinations when interpreted in the alternative ("or").

The term "cell" as used herein may refer to either a prokaryotic or eukaryotic cell, optionally obtained from a subject or a commercially available source.

"Eukaryotic cells" comprise all of the life kingdoms except monera. They can be easily distinguished through a membrane-bound nucleus. Animals, plants, fungi, and protists are eukaryotes or organisms whose cells are organized into complex structures by internal membranes and a cytoskeleton. The most characteristic membrane-bound structure is the nucleus. Unless specifically recited, the term "host" includes a eukaryotic host, including, for example, yeast, higher plant, insect and mammalian cells. Non-limiting examples of eukaryotic cells or hosts include simian, bovine, porcine, murine, rat, avian, reptilian and human, e.g., HEK293 cells and 293T cells.

"Prokaryotic cells" that usually lack a nucleus or any other membrane-bound organelles and are divided into two domains, bacteria and archaea. In addition to chromosomal DNA, these cells can also contain genetic information in a circular loop called on episome. Bacterial cells are very small, roughly the size of an animal mitochondrion (about 1 -2 µm in diameter and 10 µm long). Prokaryotic cells feature three major shapes: rod shaped, spherical, and spiral. Instead of going through elaborate replication processes like eukaryotes, bacterial cells divide by binary fission. Examples include but are not limited to *Bacillus* bacteria, E. *coli* bacterium, and *Salmonella* bacterium.

The term "encode" as it is applied to nucleic acid sequences refers to a polynucleotide which is said to "encode" a polypeptide if, in its native state or when manipulated by methods well known to those skilled in the art, can be transcribed and/or translated to produce the mRNA for the polypeptide and/or a fragment thereof. The antisense strand is the complement of such a nucleic acid, and the encoding sequence can be deduced therefrom.

The terms "equivalent" or "biological equivalent" are used interchangeably when referring to a particular molecule, biological, or cellular material and intend those having minimal homology while still maintaining desired structure or functionality. Non-limiting examples of equivalent polypeptides, include a polypeptide having at least 60%, or alternatively at least 65%, or alternatively at least 70%, or alternatively at least 75%, or alternatively 80%, or alternatively at least 85%, or alternatively at least 90%, or alternatively at least 95% identity thereto or for polypeptide sequences, or a polypeptide which is encoded by a polynucleotide or its complement that hybridizes under conditions of high stringency to a polynucleotide encoding such polypeptide sequences. Conditions of high stringency are described herein and incorporated herein by reference. Alternatively, an equivalent thereof is a polypeptide encoded by a polynucleotide or a complement thereto, having at least 70%, or alternatively at least 75%, or alternatively 80%, or alternatively at least 85%, or alternatively at least 90%, or alternatively at least 95% identity, or at least 97% sequence identity to the reference polynucleotide, e.g., the wild-type polynucleotide.

Non-limiting examples of equivalent polypeptides, include a polynucleotide having at least 60%, or alternatively at least 65%, or alternatively at least 70%, or alternatively at least 75%, or alternatively 80%, or alternatively at least 85%, or alternatively at least 90%, or alternatively at least 95%, or alternatively at least 97%, identity to a reference polynucleotide. An equivalent also intends a polynucleotide or its complement that hybridizes under conditions of high stringency to a reference polynucleotide.

A polynucleotide or polynucleotide region (or a polypeptide or polypeptide region) having a certain percentage (for example, 80%, 85%, 90%, or 95%) of "sequence identity" or homology (equivalence or equivalents) to another sequence means that, when aligned, that percentage of bases (or amino acids) are the same in comparing the two sequences. The alignment and the percent homology or sequence identity can be determined using software programs known in the art, for example those described in Current Protocols in Molecular Biology (Ausubel et al., eds. 1987) Supplement 30, section 7.7.18, Table 7.7.1. In certain embodiments, default parameters are used for alignment. A non-limiting exemplary alignment program is BLAST, using default parameters. In particular, exemplary programs include BLASTN and BLASTP, using the following default parameters: Genetic code=standard; filter=none; strand=both; cutoff=60; expect=10; Matrix=BLOSUM62; Descriptions=50 sequences; sort by=HIGH SCORE; Databases=non-redundant, GenBank+EMBL+DDBJ+PDB+GenBank CDS translations+SwissProtein+SPupdate+PIR. Details of these programs can be found at the following Internet address: ncbi.nlm.nih.gov/cgi-bin/BLAST. Sequence identity and percent identity can be determined by incorporating them into clustalW (available at the web address: genome.jp/tools/clustalw/, last accessed on Jan. 13, 2017).

"Homology" or "identity" or "similarity" refers to sequence similarity between two peptides or between two nucleic acid molecules. Homology can be determined by comparing a position in each sequence that may be aligned for purposes of comparison. When a position in the compared sequence is occupied by the same base or amino acid, then the molecules are homologous at that position. A degree of homology between sequences is a function of the number of matching or homologous positions shared by the sequences. An "unrelated" or "non-homologous" sequence shares less than 40% identity, or alternatively less than 25% identity, with one of the sequences of the present disclosure.

"Homology" or "identity" or "similarity" can also refer to two nucleic acid molecules that hybridize under stringent conditions.

"Hybridization" refers to a reaction in which one or more polynucleotides react to form a complex that is stabilized via hydrogen bonding between the bases of the nucleotide residues. The hydrogen bonding may occur by Watson-Crick base pairing, Hoogstein binding, or in any other sequence-specific manner. The complex may comprise two strands forming a duplex structure, three or more strands forming a multi-stranded complex, a single self-hybridizing strand, or any combination of these. A hybridization reaction may constitute a step in a more extensive process, such as the initiation of a PCR reaction, or the enzymatic cleavage of a polynucleotide by a ribozyme.

Examples of stringent hybridization conditions include: incubation temperatures of about 25° C. to about 37° C.; hybridization buffer concentrations of about 6×SSC to about 10×SSC; formamide concentrations of about 0% to about 25%; and wash solutions from about 4×SSC to about 8×SSC. Examples of moderate hybridization conditions include: incubation temperatures of about 40° C. to about 50° C.; buffer concentrations of about 9×SSC to about 2×SSC; formamide concentrations of about 30% to about 50%; and wash solutions of about 5×SSC to about 2×SSC. Examples of high stringency conditions include: incubation temperatures of about 55° C. to about 68° C.; buffer concentrations of about 1 ×SSC to about 0.1×SSC; formamide concentrations of about 55% to about 75%; and wash solutions of about 1×SSC, 0.1×SSC, or deionized water. In general, hybridization incubation times are from 5 minutes to 24 hours, with 1, 2, or more washing steps, and wash incubation times are about 1, 2, or 15 minutes. SSC is 0.15 M NaCl and 15 mM citrate buffer. It is understood that equivalents of SSC using other buffer systems can be employed.

As used herein, "expression" refers to the process by which polynucleotides are transcribed into mRNA and/or the process by which the transcribed mRNA is subsequently being translated into peptides, polypeptides, or proteins. If the polynucleotide is derived from genomic DNA, expression may include splicing of the mRNA in an eukaryotic cell.

The term "isolated" as used herein refers to molecules or biologicals or cellular materials being substantially free from other materials.

As used herein, the term "functional" may be used to modify any molecule, biological, or cellular material to intend that it accomplishes a particular, specified effect.

As used herein, the terms "nucleic acid sequence" and "polynucleotide" are used interchangeably to refer to a polymeric form of nucleotides of any length, either ribonucleotides or deoxyribonucleotides. Thus, this term includes, but is not limited to, single-, double-, or multi-stranded DNA or RNA, genomic DNA, cDNA, DNA-RNA hybrids, or a polymer comprising purine and pyrimidine bases or other natural, chemically or biochemically modified, non-natural, or derivatized nucleotide bases.

The term "protein", "peptide" and "polypeptide" are used interchangeably and in their broadest sense to refer to a compound of two or more subunits of amino acids, amino acid analogs or peptidomimetics. The subunits may be linked by peptide bonds. In another aspect, the subunit may be linked by other bonds, e.g., ester, ether, etc. A protein or peptide must contain at least two amino acids and no limitation is placed on the maximum number of amino acids which may comprise a protein's or peptide's sequence. As used herein the term "amino acid" refers to either natural and/or unnatural or synthetic amino acids, including glycine and both the D and L optical isomers, amino acid analogs and peptidomimetics.

As used herein, the term "recombinant expression system" refers to a genetic construct or constructs for the expression of certain genetic material formed by recombination.

A "gene delivery vehicle" is defined as any molecule that can carry inserted polynucleotides into a host cell. Examples of gene delivery vehicles are liposomes, micelles biocompatible polymers, including natural polymers and synthetic polymers; lipoproteins; polypeptides; polysaccharides; lipopolysaccharides; artificial viral envelopes; metal particles; and bacteria, or viruses, such as baculovirus, adenovirus and retrovirus, bacteriophage, cosmid, plasmid, fungal vectors and other recombination vehicles typically used in the art which have been described for expression in a variety of eukaryotic and prokaryotic hosts, and may be used for gene therapy as well as for simple protein expression.

A polynucleotide disclosed herein can be delivered to a cell or tissue using a gene delivery vehicle. "Gene delivery," "gene transfer," "transducing," and the like as used herein, are terms referring to the introduction of an exogenous polynucleotide (sometimes referred to as a "transgene") into a host cell, irrespective of the method used for the introduction. Such methods include a variety of well-known techniques such as vector-mediated gene transfer (by, e.g., viral infection/transfection, or various other protein-based or lipid-based gene delivery complexes) as well as techniques facilitating the delivery of "naked" polynucleotides (such as electroporation, "gene gun" delivery and various other techniques used for the introduction of polynucleotides). The introduced polynucleotide may be stably or transiently maintained in the host cell. Stable maintenance typically requires that the introduced polynucleotide either contains an origin of replication compatible with the host cell or integrates into a replicon of the host cell such as an extrachromosomal replicon (e.g., a plasmid) or a nuclear or mitochondrial chromosome. A number of vectors are known to be capable of mediating transfer of genes to mammalian cells, as is known in the art and described herein.

A "plasmid" is an extra-chromosomal DNA molecule separate from the chromosomal DNA which is capable of replicating independently of the chromosomal DNA. In many cases, it is circular and double-stranded. Plasmids provide a mechanism for horizontal gene transfer within a population of microbes and typically provide a selective advantage under a given environmental state. Plasmids may carry genes that provide resistance to naturally occurring antibiotics in a competitive environmental niche, or alternatively the proteins produced may act as toxins under similar circumstances.

"Plasmids" used in genetic engineering are called "plasmid vectors". Many plasmids are commercially available for such uses. The gene to be replicated is inserted into copies of a plasmid containing genes that make cells resistant to particular antibiotics and a multiple cloning site (MCS, or polylinker), which is a short region containing several commonly used restriction sites allowing the easy insertion of DNA fragments at this location. Another major use of plasmids is to make large amounts of proteins. In this case, researchers grow bacteria containing a plasmid harboring the gene of interest. Just as the bacterium produces proteins to confer its antibiotic resistance, it can also be induced to produce large amounts of proteins from the inserted gene.

As used herein, "target", "targets" or "targeting" refers to partial or no breakage of the covalent backbone of polynucleotide. In one embodiment, a deactivated Cas protein (or dCas) targets a nucleotide sequence after forming a DNA-bound complex with a guide RNA. Because the nuclease activity of the dCas is entirely or partially deactivated, the dCas binds to the sequence without cleaving or fully cleaving the sequence. In some embodiment, targeting a gene sequence or its promoter with a dCas can inhibit or prevent transcription and/or expression of a polynucleotide or gene.

A "yeast artificial chromosome" or "YAC" refers to a vector used to clone large DNA fragments (larger than 100 kb and up to 3000 kb). It is an artificially constructed chromosome and contains the telomeric, centromeric, and replication origin sequences needed for replication and preservation in yeast cells. Built using an initial circular plasmid, they are linearized by using restriction enzymes, and then DNA ligase can add a sequence or gene of interest within the linear molecule by the use of cohesive ends. Yeast expression vectors, such as YACs, YIps (yeast integrating plasmid), and YEps (yeast episomal plasmid), are extremely useful as one can get eukaryotic protein products with posttranslational modifications as yeasts are themselves eukaryotic cells, however YACs have been found to be more unstable than BACs, producing chimeric effects.

A "viral vector" is defined as a recombinantly produced virus or viral particle that comprises a polynucleotide to be delivered into a host cell, either *in vivo, ex vivo* or *in vitro.*

Examples of viral vectors include retroviral vectors, adenovirus vectors, adeno-associated virus vectors, alphavirus vectors and the like. Infectious tobacco mosaic virus (TMV)-based vectors can be used to manufacturer proteins and have been reported to express Griffithsin in tobacco leaves (O'Keefe et al. (2009) Proc. Nat. Acad. Sci. USA 106(15):6099-6104). Alphavirus vectors, such as Semliki Forest virus-based vectors and Sindbis virus-based vectors, have also been developed for use in gene therapy and immunotherapy. See, Schlesinger & Dubensky (1999) Curr. Opin. Biotechnol. 5:434-439 and Ying et al. (1999) Nat. Med. 5(7):823-827. In aspects where gene transfer is mediated by a retroviral vector, a vector construct refers to the polynucleotide comprising the retroviral genome or part thereof, and a therapeutic gene. Further details as to modern methods of vectors for use in gene transfer may be found in, for example, Kotterman et al. (2015) Viral Vectors for Gene Therapy: Translational and Clinical Outlook Annual Review of Biomedical Engineering 17.

As used herein, "retroviral mediated gene transfer" or "retroviral transduction" carries the same meaning and refers to the process by which a gene or nucleic acid sequences are stably transferred into the host cell by virtue of the virus entering the cell and integrating its genome into the host cell genome. The virus can enter the host cell via its normal mechanism of infection or be modified such that it binds to a different host cell surface receptor or ligand to enter the cell. As used herein, retroviral vector refers to a viral particle capable of introducing exogenous nucleic acid into a cell through a viral or viral-like entry mechanism.

Retroviruses carry their genetic information in the form of RNA; however, once the virus infects a cell, the RNA is reverse-transcribed into the DNA form which integrates into the genomic DNA of the infected cell. The integrated DNA form is called a provirus.

In aspects where gene transfer is mediated by a DNA viral vector, such as an adenovirus (Ad) or adeno-associated virus (AAV), a vector construct refers to the polynucleotide comprising the viral genome or part thereof, and a transgene. Adenoviruses (Ads) are a relatively well characterized, homogenous group of viruses, including over 50 serotypes. Ads do not require integration into the host cell genome. Recombinant Ad derived vectors, particularly those that reduce the potential for recombination and generation of wild-type virus, have also been constructed. Such vectors are commercially available from sources such as Takara Bio USA (Mountain View, CA), Vector Biolabs (Philadelphia, PA), and Creative Biogene (Shirley, NY). Wild-type AAV has high infectivity and specificity integrating into the host cell's genome. See, Wold and Toth (2013) Curr. Gene. Ther. 13(6):421-433, Hermonat & Muzyczka (1984) Proc. Natl. Acad. Sci. USA 81:6466-6470, and Lebkowski et al. (1988) Mol. Cell. Biol. 8:3988-3996.

Vectors that contain both a promoter and a cloning site into which a polynucleotide can be operatively linked are well known in the art. Such vectors are capable of transcribing RNA *in vitro* or *in vivo,* and are commercially available from sources such as Agilent Technologies (Santa Clara, Calif.) and Promega Biotech (Madison, Wis.). In order to optimize expression and/or *in vitro* transcription, it may be necessary to remove, add or alter 5' and/or 3' untranslated portions of the clones to eliminate extra, potential inappropriate alternative translation initiation codons or other sequences that may interfere with or reduce expression, either at the level of transcription or translation. Alternatively, consensus ribosome binding sites can be inserted immediately 5' of the start codon to enhance expression.

Gene delivery vehicles also include DNA/liposome complexes, micelles and targeted viral protein-DNA complexes. Liposomes that also comprise a targeting antibody or fragment thereof can be used in the methods disclosed herein. In addition to the delivery of polynucleotides to a cell or cell population, direct introduction of the proteins described herein to the cell or cell population can be done by the non-limiting technique of protein transfection, alternatively culturing conditions that can enhance the expression and/or promote the activity of the proteins disclosed herein are other non-limiting techniques.

As used herein, the term "viral capsid" or "capsid" refers to the proteinaceous shell or coat of a viral particle. Capsids function to encapsidate, protect, transport, and release into host cell a viral genome. Capsids are generally comprised of oligomeric structural subunits of protein ("capsid proteins"). As used herein, the term "encapsidated" means enclosed within a viral capsid.

As used herein, the term "helper" in reference to a virus or plasmid refers to a virus or plasmid used to provide the additional components necessary for replication and packaging of a viral particle or recombinant viral particle, such as the modified AAV disclosed herein. The components encoded by a helper virus may include any genes required for virion assembly, encapsidation, genome replication, and/or packaging. For example, the helper virus may encode necessary enzymes for the replication of the viral genome. Non-limiting examples of helper viruses and plasmids suitable for use with AAV constructs include pHELP (plasmid), adenovirus (virus), or herpesvirus (virus).

As used herein, the term "AAV" is a standard abbreviation for adeno-associated virus. Adeno-associated virus is a single-stranded DNA parvovirus that grows only in cells in which certain functions are provided by a co-infecting helper virus. General information and reviews of AAV can be found in, for example, Carter, 1989, Handbook of Parvoviruses, Vol. 1, pp. 169-228, and Berns, 1990, Virology, pp. 1743-1764, Raven Press, (New York). It is fully expected that the same principles described in these reviews will be applicable to additional AAV serotypes characterized after the publication dates of the reviews because it is well known that the various serotypes are quite closely related, both structurally and functionally, even at the genetic level. (See, for example, Blacklowe, 1988, pp. 165-174 of Parvoviruses and Human Disease, J. R. Pattison, ed.; and Rose, Comprehensive Virology 3: 1-61 (1974)). For example, all AAV serotypes apparently exhibit very similar replication properties mediated by homologous rep genes; and all bear three related capsid proteins such as those expressed in AAV2. The degree of relatedness is further suggested by heteroduplex analysis which reveals extensive cross -hybridization between serotypes along the length of the genome; and the presence of analogous self-annealing segments at the termini that correspond to "inverted terminal repeat sequences" (ITRs). The similar infectivity patterns also suggest that the replication functions in each serotype are under similar regulatory control.

An "AAV vector" as used herein refers to a vector comprising one or more polynucleotides of interest (or transgenes) that are flanked by AAV terminal repeat sequences (ITRs). Such AAV vectors can be replicated and packaged into infectious viral particles when present in a host cell that has been transfected with a vector encoding and expressing rep and cap gene products.

An "AAV virion" or "AAV viral particle" or "AAV vector particle" refers to a viral particle composed of at least one AAV capsid protein and an encapsidated polynucleotide AAV vector. If the particle comprises a heterologous polynucleotide (i.e. a polynucleotide other than a wild-type AAV genome such as a transgene to be delivered to a mammalian cell), it is typically referred to as an "AAV vector particle" or simply an "AAV vector." Thus, production of AAV vector particle necessarily includes production of AAV vector, as such a vector is contained within an AAV vector particle.

Adeno-associated virus (AAV) is a replication-deficient parvovirus, the single-stranded DNA genome of which is about 4.7 kb in length including two 145 nucleotide inverted terminal repeat (ITRs). There are multiple serotypes of AAV. The nucleotide sequences of the genomes of the AAV serotypes are known. For example, the complete genome of AAV-1 is provided in GenBank Accession No. NC_002077; the complete genome of AAV-2 is provided in GenBank Accession No. NC_001401 and Srivastava et al., J. Virol., 45: 555-564 { 1983); the complete genome of AAV-3 is provided in GenBank Accession No. NC_1829; the complete genome of AAV-4 is provided in GenBank Accession No. NC_001829; the AAV-5 genome is provided in GenBank Accession No. AF085716; the complete genome of AAV-6 is provided in GenBank Accession No. NC_00 1862; at least portions of AAV-7 and AAV-8 genomes are provided in GenBank Accession Nos. AX753246 and AX753249, respectively; the AAV-9 genome is provided in Gao et al., J. Virol., 78: 6381-6388 (2004); the AAV-10 genome is provided in Mol. Ther., 13(1): 67-76 (2006); and the AAV-11 genome is provided in Virology, 330(2): 375-383 (2004). The sequence of the AAV rh.74 genome is provided in U.S. Patent 9,434,928, incorporated herein by reference. Cis-acting sequences directing viral DNA replication (rep), encapsidation/packaging and host cell chromosome integration are contained within the AAV ITRs. Three AAV promoters (named p5, pl9, and p40 for their relative map locations) drive the expression of the two AAV internal open reading frames encoding rep and cap genes. The two rep promoters (p5 and pi 9), coupled with the differential splicing of the single AAV intron (at nucleotides 2107 and 2227), result in the production of four rep proteins (rep 78, rep 68, rep 52, and rep 40) from the rep gene. Rep proteins possess multiple enzymatic properties that are ultimately responsible for replicating the viral genome. The cap gene is expressed from the p40 promoter and it encodes the three capsid proteins VP1, VP2, and VP3. Alternative splicing and non-consensus translational start sites are responsible for the production of the three related capsid proteins. A single consensus polyadenylation site is located at map position 95 of the AAV genome. The life cycle and genetics of AAV are reviewed in Muzyczka, Current Topics in Microbiology and Immunology, 158: 97-129 (1992).

AAV possesses unique features that make it attractive as a vector for delivering foreign DNA to cells, for example, in gene therapy. AAV infection of cells in culture is noncytopathic, and natural infection of humans and other animals is silent and asymptomatic. Moreover, AAV infects many mammalian cells allowing the possibility of targeting many different tissues in vivo. Moreover, AAV transduces slowly dividing and non-dividing cells, and can persist essentially for the lifetime of those cells as a transcriptionally active nuclear episome (extrachromosomal element). The AAV proviral genome is inserted as cloned DNA in plasmids, which makes construction of recombinant genomes feasible. Furthermore, because the signals directing AAV replication and genome encapsidation are contained within the ITRs of the AAV genome, some or all of the internal approximately 4.3 kb of the genome (encoding replication and structural capsid proteins, rep-cap) may be replaced with foreign DNA. To generate AAV vectors, the rep and cap proteins may be provided in trans. Another significant feature of AAV is that it is an extremely stable and hearty virus. It easily withstands the conditions used to inactivate adenovirus (56° to 65°C for several hours), making cold preservation of AAV less critical. AAV may even be lyophilized. Finally, AAV-infected cells are not resistant to superinfection.

Multiple studies have demonstrated long-term (> 1.5 years) recombinant AAV-mediated protein expression in muscle. See, Clark et al., Hum Gene Ther, 8: 659-669 (1997); Kessler et al., Proc Nat. Acad Sc. USA, 93: 14082-14087 (1996); and Xiao et al., J Virol, 70: 8098-8108 (1996). See also, Chao et al., Mol Ther, 2:619-623 (2000) and Chao et al., Mol Ther, 4:217-222 (2001). Moreover, because muscle is highly vascularized, recombinant AAV transduction has resulted in the appearance of transgene products in the systemic circulation following intramuscular injection as described in Herzog et al., Proc Natl Acad Sci USA, 94: 5804-5809 (1997) and Murphy et al., Proc Natl Acad Sci USA, 94: 13921- 13926 (1997). Moreover, Lewis et al., J Virol, 76: 8769-8775 (2002) demonstrated that skeletal myofibers possess the necessary cellular factors for correct antibody glycosylation, folding, and secretion, indicating that muscle is capable of stable expression of secreted protein therapeutics. AAV DNA in the rAAV genomes may be from any AAV serotype for which a recombinant virus can be derived including, but not limited to, AAV serotypes AAV-1, AAV-2, AAV-3, AAV-4, AAV-5, AAV-6, AAV-7, AAV-8, AAV-9, AAV- 10, AAV-11, AAV- 12, AAV-13 and AAV rh74. Production of pseudotyped rAAV is disclosed in, for example, WO 01/83692. Other types of rAAV variants, for example rAAV with capsid mutations, are also contemplated. See, for example, Marsic et al., Molecular Therapy, 22(11): 1900-1909 (2014). The nucleotide sequences of the genomes of various AAV serotypes are known in the art.

The term "Cas9" refers to a CRISPR associated endonuclease referred to by this name. Non-limiting exemplary Cas9s are provided herein, e.g. the Cas9 provided for in UniProtKB G3ECR1 (CAS9_STRTR) or the *Staphylococcus aureus* Cas9, as well as the nuclease dead Cas9, orthologs and biological equivalents each thereof. Orthologs include but are not limited to *Streptococcus pyogenes* Cas9 ("spCas9"); Cas 9 from *Streptococcus thermophiles, Legionella pneumophilia*, *Neisseria lactamica*, *Neisseria meningitides, Francisella novicida*; and Cpfl (which performs cutting functions analogous to Cas9) from various bacterial species including *Acidaminococcus* spp. and *Francisella novicida U112.*

As used herein, the term "CRISPR" refers to a technique of sequence specific genetic manipulation relying on the clustered regularly interspaced short palindromic repeats pathway. CRISPR can be used to perform gene editing and/or gene regulation, as well as to simply target proteins to a specific genomic location. Gene editing refers to a type of genetic engineering in which the nucleotide sequence of a target polynucleotide is changed through introduction of deletions, insertions, or base substitutions to the polynucleotide sequence. In some aspects, CRISPR-mediated gene editing utilizes the pathways of non-homologous end-joining (NHEJ) or homologous recombination to perform the edits. Gene regulation refers to increasing or decreasing the production of specific gene products such as protein or RNA.

The term "gRNA" or "guide RNA" as used herein refers to the guide RNA sequences used to target specific genes for correction employing the CRISPR technique. Techniques of designing gRNAs and donor therapeutic polynucleotides for target specificity are well known in the art. For example, Doench, J., et al. Nature biotechnology 2014; 32(12): 1262-7, Mohr, S. et al. (2016) FEBS Journal 283: 3232-38, and Graham, D., et al. Genome Biol. 2015; 16: 260. gRNA comprises or alternatively consists essentially of, or yet further consists of a fusion polynucleotide comprising CRISPR RNA (crRNA) and trans-activating CRIPSPR RNA (tracrRNA); or a polynucleotide comprising CRISPR RNA (crRNA) and trans-activating CRIPSPR RNA (tracrRNA). In some aspects, a gRNA is synthetic (Kelley, M. et al. (2016) J of Biotechnology 233 (2016) 74-83). As used herein, a biological equivalent of a gRNA includes but is not limited to polynucleotides or targeting molecules that can guide a Cas9 or equivalent thereof to a specific nucleotide sequence such as a specific region of a cell's genome.

A "composition" is intended to mean a combination of active polypeptide, polynucleotide or antibody and another compound or composition, inert (e.g., a detectable label) or active (e.g., a gene delivery vehicle).

A "pharmaceutical composition" is intended to include the combination of an active polypeptide, polynucleotide or antibody with a carrier, inert or active such as a solid support, making the composition suitable for diagnostic or therapeutic use *in vitro, in vivo* or *ex vivo.*

As used herein, the term "pharmaceutically acceptable carrier" encompasses any of the standard pharmaceutical carriers, such as a phosphate buffered saline solution, water, and emulsions, such as an oil/water or water/oil emulsion, and various types of wetting agents. The compositions also can include stabilizers and preservatives. For examples of carriers, stabilizers and adjuvants, see Martin (1975) Remington's Pharm. Sci., 15th Ed. (Mack Publ. Co., Easton).

A "subject" of diagnosis or treatment is a cell or an animal such as a mammal, or a human. A subject is not limited to a specific species and includes non-human animals subject to diagnosis or treatment and are those subject to infections or animal models, for example, simians, murines, such as, rats, mice, chinchilla, canine, such as dogs, leporids, such as rabbits, livestock, sport animals, and pets. Human patients are included within the term as well.

The term "tissue" is used herein to refer to tissue of a living or deceased organism or any tissue derived from or designed to mimic a living or deceased organism. The tissue may be healthy, diseased, and/or have genetic mutations. The biological tissue may include any single tissue (e.g., a collection of cells that may be interconnected) or a group of tissues making up an organ or part or region of the body of an organism. The tissue may comprise a homogeneous cellular material or it may be a composite structure such as that found in regions of the body including the thorax which for instance can include lung tissue, skeletal tissue, and/or muscle tissue. Exemplary tissues include, but are not limited to those derived from liver, lung, thyroid, skin, pancreas, blood vessels, bladder, kidneys, brain, biliary tree, duodenum, abdominal aorta, iliac vein, heart and intestines, including any combination thereof.

As used herein, "treating" or "treatment" of a disease in a subject refers to (1) preventing the symptoms or disease from occurring in a subject that is predisposed or does not yet display symptoms of the disease; (2) inhibiting the disease or arresting its development; or (3) ameliorating or causing regression of the disease or the symptoms of the disease. As understood in the art, "treatment" is an approach for obtaining beneficial or desired results, including clinical results. For the purposes of the present technology, beneficial or desired results can include one or more, but are not limited to, alleviation or amelioration of one or more symptoms, diminishment of extent of a condition (including a disease), stabilized (i.e., not worsening) state of a condition (including disease), delay or slowing of condition (including disease), progression, amelioration or palliation of the condition (including disease), states and remission (whether partial or total), whether detectable or undetectable. In one aspect, the term "treatment" excludes prevention.

To treat dominantly-inherited diseases, conventional CRISPR-based gene therapies focus on a mutation-specific gene repair approach. In contrast, the present method employs a more generalizable strategy which enables allele-specific targeting. For example, the present method can capitalize on single nucleotide polymorphism (SNP) heterozygosity to create a system applicable to multiple mutations. In one embodiment, SNPs in the BEST1 transcription start site (TSS) can be used to enable a CRISPR gRNA to discriminate between mutant and wildtype alleles. The disclosure involves mapping these mutations (e.g., SNPs) to a pathological mutation and designing a gRNA to direct a Cas nuclease to cleave only the mutant allele. The method ablates mutant genes in a manner that is allele-specific so that the wildtype allele is left intact to support normal function. In one embodiment, the Cas nuclease does not cleave or target the wild-type allele that encodes the autosomal dominant disease-related gene. This approach will allow targeting and editing of all mutations of an autosomal dominant ocular disease-related gene (e.g., BEST1), rather than just one mutation. The present method may be used to treat other macular degenerative and dominantly-inherited conditions.

The present disclosure provides for a method for modifying an autosomal dominant disease-related gene (e.g., an autosomal dominant ocular disease-related gene) in a cell. The method may comprise contacting the cell with at least one type of vector encoding a CRISPR-Cas system directed to a mutant allele of the autosomal dominant disease-related gene (e.g., the autosomal dominant ocular disease-related gene). The at least one type of vector may comprise: (i) a first sequence encoding a first guide RNA that hybridizes to a variant of a single nucleotide polymorphism (SNP) in the autosomal dominant disease-related gene; (ii) a second sequence encoding a second guide RNA that hybridizes to an intron of the autosomal dominant disease-related gene; and, (iii) a third sequence encoding a Cas nuclease.

The present disclosure provides for a method for modifying an autosomal dominant disease-related gene (e.g., an autosomal dominant ocular disease-related gene) in a cell. The method may comprise contacting the cell with at least one type of vector encoding a CRISPR-Cas system directed to a mutant allele of the autosomal dominant disease-related gene. The at least one type of vector may comprise: (i) a first sequence encoding a first guide RNA that hybridizes to a variant of a single nucleotide polymorphism (SNP) in the autosomal dominant disease-related gene; and, (ii) a second sequence encoding a catalytically defective Cas nuclease (dCas).

The cell may be from a subject having a dominant disease, such as an autosomal dominant ocular disease. The cell may be derived from a cell from a subject having a dominant disease condition, such as an autosomal dominant ocular disease.

The cell may be an induced pluripotent stem cell (iPSC), e.g., derived from a fibroblast of a subject. In another embodiment, the cell can be a fibroblast.

The method may further comprise culturing the iPSC to differentiate into a retinal pigment epithelium (RPE) cell. The method may further comprise administering the RPE cell to a subject. In one embodiment, the RPE cell is administered via subretinal transplantation.

The RPE cell may be autologous or allogeneic to the subject.

The cell may be heterozygous or homozygous for the SNP.
The cell may be heterozygous for the autosomal dominant disease-related gene having a mutant allele and a wildtype allele. The cell may be homozygous for the autosomal dominant disease-related gene having two mutant alleles.

The present disclosure provides for a method for modifying an autosomal dominant disease-related gene in a cell, the method comprising: contacting the cell with at least one type of vector encoding a CRISPR-Cas system directed to a mutant allele of the autosomal dominant disease-related gene, wherein the autosomal dominant disease-related gene relates to an ocular disease, wherein the at least one type of vector comprises: (i) a first sequence encoding a first guide RNA that hybridizes to the autosomal dominant disease-related gene that comprises a single nucleotide polymorphism (SNP); (ii) a second sequence encoding a second guide RNA that hybridizes to an intron of the autosomal dominant disease-related gene; and, (iii) a third sequence encoding a Cas nuclease, wherein the Cas nuclease cleaves or targets only the mutant allele that encodes the autosomal dominant disease-related gene that comprises a single nucleotide polymorphism (SNP). In some emboidments, the first guide RNA comprises a nucleotide sequence selected from SEQ ID NO: 40, SEQ. ID NO. 41, SEQ ID NO: 42, SEQ ID NO: 43, SEQ ID NO: 44, SEQ ID NO: 45, SEQ ID NO: 46, SEQ ID NO: 47, SEQ ID NO: 48, or SEQ ID NO: 49 or an equivalent of each thereof. In one aspect, the autosomal dominant disease-related gene comprises a sequence selected from SEQ ID NOS: 28-33, or an equivalent of each thereof. In one aspect, the first guide RNA hybridizes to the autosomal dominant disease-related gene that comprises a sequence of SEQ ID NO: 31, or an equivalent of each thereof.

In another embodiment, the present disclosure provides a method for modifying an autosomal dominant disease-related gene in a cell, the method comprising: contacting the cell with at least one type of vector encoding a CRISPR-Cas system directed to a mutant allele of the autosomal dominant disease-related gene, wherein the autosomal dominant disease-related gene relates to an ocular disease, wherein the at least one type of vector comprises: (i) a first sequence encoding a first guide RNA that hybridizes to the autosomal dominant disease-related gene that comprises a single nucleotide polymorphism (SNP); and, (ii) a second sequence encoding a catalytically defective Cas nuclease (dCas); wherein the Cas nuclease cleaves or targets only the mutant allele that encodes the autosomal dominant disease-related gene comprising a single nucleotide polymorphism (SNP). In some embodiments, the second guide RNA comprises a nucleotide sequence selected from SEQ ID NO: 34, SEQ ID NO: 35, SEQ ID NO: 36, SEQ ID NO: 37, SEQ ID NO: 38 or SEQ ID NO: 39 or an equivalent of each thereof. In one aspect, the autosomal dominant disease-related gene comprises a sequence selected from SEQ ID NOS: 28-33, or an equivalent of each thereof. In one aspect, the first guide RNA hybridizes to the autosomal dominant disease-related gene that comprises a sequence of SEQ ID NO: 31, or an equivalent thereof.

The present disclosures provides for a system comprising at least one type of vector: (i) a first sequence encoding a first guide RNA that hybridizes to a variant of a single nucleotide polymorphism (SNP) in an autosomal dominant disease-related gene; (ii) a second sequence encoding a second guide RNA that hybridizes to an intron of the autosomal dominant disease-related gene; and, (iii) a third sequence encoding a Cas nuclease, wherein the Cas nuclease cleaves or targets only the mutant allele that encodes the autosomal dominant disease-related gene that comprises a single nucleotide polymorphism (SNP). The first guide RNA can comprise a nucleotide sequence selected from SEQ ID NO: 40, SEQ. ID NO. 41, SEQ ID NO: 42, SEQ ID NO: 43, SEQ ID NO: 44, SEQ ID NO: 45, SEQ ID NO: 46, SEQ ID NO: 47, SEQ ID NO: 48, or SEQ ID NO: 49 or an equivalent of each thereof. In some embodiments, the Cas nuclease is a Cas nickase. In another embodiment, the Cas nuclease is Cas9. In one aspect, the Cas system is used to modify or edit an autosomal dominant disease-related gene that comprises a sequence selected from SEQ ID NOS: 28-33, or an equivalent of each thereof. In one aspect, the first guide RNA hybridizes to the autosomal dominant disease-related gene that comprises a sequence of SEQ ID NO: 31, or an equivalent thereof.

The present disclosure also provides for a system comprising at least one type of vector: (i) a first sequence encoding a first guide RNA that hybridizes to the autosomal dominant disease-related gene that comprises a single nucleotide polymorphism (SNP); and, (ii) a second sequence encoding a catalytically defective Cas nuclease (dCas); wherein the Cas nuclease cleaves or targets only the mutant allele that encodes the autosomal dominant disease-related gene comprising a single nucleotide polymorphism (SNP). The second guide RNA can comprise a nucleotide sequence selected from SEQ ID NO: 34, SEQ ID NO: 35, SEQ ID NO: 36, SEQ ID NO: 37, SEQ ID NO: 38 or SEQ ID NO: 39 or an equivalent of each thereof. In some embodiments, the dCas is fused to a repressor domain. In another embodiment, the repressor domain is a Krüppel-associated Box (KRAB) domain. In one embodiment, the first guide RNA comprises at least one PUF (Pumilio mRNA binding factor) binding sequence and in a further embodiment, the at least one PUF binding sequence binds to PUF-KRAB.

The present disclosure provides for a method for treating an autosomal dominant disease (e.g., an autosomal dominant ocular disease) in a subject. The method may comprise administering to the subject a therapeutically effective amount of at least one type of vector encoding a CRISPR-Cas system directed to a mutant allele of an autosomal dominant disease-related gene in the subject. The at least one type of vector may comprise: (i) a first sequence encoding a first guide RNA that hybridizes to a variant of a single nucleotide polymorphism (SNP) in the autosomal dominant disease-related gene; (ii) a second sequence encoding a second guide RNA that hybridizes to an intron of the autosomal dominant disease-related gene; and, (iii) a third sequence encoding a Cas nuclease.

Also encompassed by the present disclosure is a method for treating an autosomal dominant disease (e.g., an autosomal dominant ocular disease) in a subject. The method may comprise administering to the subject a therapeutically effective amount of at least one type of vector encoding a CRISPR-Cas system directed to a mutant allele of an autosomal dominant disease-related gene in the subject. The at least one type of vector may comprise: (i) a first sequence encoding a first guide RNA that hybridizes to a variant of a single nucleotide polymorphism (SNP) in the autosomal dominant disease-related gene; and, (ii) a second sequence encoding a catalytically defective Cas nuclease (dCas).

The subject may be heterozygous or homozygous for the SNP.

The subject may be heterozygous for the autosomal dominant disease-related gene having a mutant allele and a wildtype allele. The subject may be homozygous for the autosomal dominant disease-related gene having two mutant alleles.

The at least one type of vector may be administered by injection into an eye of the subject.

The variant of the SNP and the mutant allele of the autosomal dominant disease-related gene may be on the same chromosome. The variant of the SNP and the mutant allele of the autosomal dominant disease-related gene may be on different chromosomes.

The SNP may be in a non-coding region, in a coding region, or in an intergenic region of the autosomal dominant disease-related gene. In one embodiment, the SNP is in a transcription start site (TSS) region of the autosomal dominant disease-related gene.

The Cas protein or enzyme/nuclease may be a wildtype (wt) Cas (e.g., a wildtype (wt) Cas9), a Cas nickase (e.g., a Cas9 nickase), or a dCas (e.g., a dCas9).

In one embodiment, the dCas is fused to a repressor domain, such as a Krüppel-associated Box (KRAB) domain, or any other repressor domain as described herein including combinations thereof.

In another embodiment, the first guide RNA comprises at least one PUF (Pumilio mRNA binding factor) binding sequence, which may bind to, e.g., PUF or the PUF-KRAB fusion protein.

The autosomal dominant disease-related gene may be BEST1, RHO, PRDM13, RGR, TEAD1, AIPL1, CRX, GUCA1A, GUCY2D, PITPNM3, PROM1, PRPH2, RIMS1, SEMA4A, UNC119, GNAT1, PDE6B, WSF1, IMPDH1, OTX2, C1QTNF5, CTNNA1, EFEMP1, ELOVL4, FSCN2,GUCA1B, HMCN1, IMPG1, RP1L1, TIMP3, VCAN, MFN2, NR2F1, OPA1, ARL3, CA4, HK1, KLHL7, NR2E3, NRL, PRPF3, PRPF4, PRPF6, PRPF8, PRPF31, RDH12, ROM1, RP1, RP9, RPE65, SNRNP200, SPP2, TOPORS, ABCC6, ATXN7, COL11A1, COL2A1, JAG1, KCNJ13, KIF11, OPA3, PAX2, TREX1, CAPN5, CRB1, FZD4, ITM2B, LRP5, MAPKAPK3, MIR204, OPN1SW, RB1, TSPAN12, or ZNF408.

In certain embodiments, the autosomal dominant disease-related gene is *BEST1.* In certain embodiments, the variant of the SNP is rs972353:G SNP, rs972355:G SNP, or rs2668899 SNP.

In certain embodiments, the second guide RNA hybridizes to intron 1 of *BEST1.*

The mutations of the disease-related gene may be single base mutations, missense mutations (including single missense mutations), deletions, etc.

The mutations of the disease-related gene may be in one or more coding regions, in one or more non-coding regions, in one or more intergenic regions (regions between genes), or in combinations of one or more coding regions, and/or one or more non-coding regions, and/or intergenic regions. The mutations may be in one or more introns, in one or more exons, or in a combination of one or more introns and one or more exons.

In one aspect, the vector is an adeno-associated viral vector. Adeno-associated virus (AAV) vectors are replication defective viruses that are engineered to deliver genetic cargo efficiently to cells. They are non-enveloped viruses that in their vector form only possess the inverted terminal repeats (ITR) of the original virus. The structural and enzymatic AAV proteins are supplied "in trans" by additional plasmids and are transfected together into a cell to generate the engineered particles for gene delivery. AAVs have been widely utilized for genetic therapy - and more specifically with CRISPR/Cas9 systems - due to their safety and efficiency. AAV efficiently infects a variety of cells and during the infection process the capsid binds to and enters the nucleus where the vector genome is delivered.

The AAV structural particle is composed of 60 protein molecules made up of VP1, VP2 and VP3. Each particle contains approximately 5 VP1 proteins, 5 VP2 proteins and 50 VP3 proteins ordered into an icosahedral structure. It has been shown that AAV2 particles can support the insertion of peptides and proteins at various sites within the capsid structure. The ability to introduce unique peptides into the capsid has led to the development of AAV particles with altered tropism, which allows the virus to bind and infect cells and tissues that may normally be refractory to infection. In addition, large peptides and even functional proteins have been introduced into the capsid of AAV2 vectors with varying levels of success.

One of the constraints with AAV vectors for gene delivery is the size limitation of the genetic insert that can be efficiently packaged into particles. For example, the size of the wild-type AAV2 genome is 4679 bases of single stranded DNA. Packaging even one of the new smaller variants of Cas9 (staphylococcus aureus Cas9, SaCas9, 130 kD MW) requires approximately 3255 bp just for the coding region. Adding a ubiquitous or tissue specific promoter to the construct may add another 500-800 bp. Include another 500 bp for a poly A addition sequence and the ITR's and the vector is close to the packaging capacity of an AAV particle. To achieve functional CRISPR/Cas9 gene correction a guide RNA (gRNA) with the target sequence must also be included. To have this RNA expressed further requires a minimal polIII promoter and termination sequence. Together these elements are too large to be combined into an AAV vector that is efficiently packaged. One can choose to package the Cas9 construct and guide RNA expression cassettes into separate vectors, but, for them to be functional, both viruses must infect the same target cells.

Further aspects of the disclosure relate to a recombinant expression system for the generation of such a modified AAV. In some embodiments the recombinant expression system comprises a plurality of plasmids; the plurality encoding all of the AAV viral proteins - VP1, VP2, and VP3. In some embodiments, each viral protein is encoded in a different plasmid. In some embodiments, one or more viral proteins is encoded in the same plasmid. In some embodiments, at least one viral protein is encoded as a fusion protein with Cas9. In one embodiment, the AAV vector is a AAV8 vector. In another embodiment, the AAV vector is a AAV2 vector.

The at least one type of vector may be at least one type of recombinant adeno-associated viral (AAV) vector. For example, the at least one type of recombinant AAV vector is at least one type of AAV2 vector, or at least one type of AAV8 vector. The recombinant AAV vector can be packaged using a viral packaging system such as a retroviral, adenoviral, herpes virus, or baculovirus packaging system, and this disclosure provides the systems and elements to produce the vector used in the methods of this disclosure. In some embodiments, packaging is achieved by using a helper virus or helper plasmid and a cell line. The helper virus or helper plasmid contains elements and sequences that facilitate the delivery of genetic materials into cells. In another aspect, the helper plasmid or a polynucleotide comprising the helper plasmid is stably incorporated into the genome of a packaging cell line, such that the packaging cell line does not require additional transfection with a helper plasmid.

A helper plasmid may comprise, for example, at least one viral helper DNA sequence derived from a replication-incompetent viral genome encoding in trans all virion proteins required to package a replication incompetent AAV, and for producing virion proteins capable of packaging the replication-incompetent AAV at high titer, without the production of replication-competent AAV. The viral DNA sequence lacks the region encoding the native enhancer and/or promoter of the viral 5' LTR of the virus, and lacks both the psi function sequence responsible for packaging helper genome and the 3' LTR, but encodes a foreign polyadenylation site, for example the SV40 polyadenylation site, and a foreign enhancer and/or promoter which directs efficient transcription in a cell type where virus production is desired. The virus is a leukemia virus such as a Moloney Murine Leukemia Virus (MMLV), the Human Immunodeficiency Virus (HIV), or the Gibbon Ape Leukemia virus (GALV). The foreign enhancer and promoter may be the human cytomegalovirus (HCMV) immediate early (IE) enhancer and promoter, the enhancer and promoter (U3 region) of the Moloney Murine Sarcoma Virus (MMSV), the U3 region of Rous Sarcoma Virus (RSV), the U3 region of Spleen Focus Forming Virus (SFFV), or the HCMV IE enhancer joined to the native Moloney Murine Leukemia Virus (MMLV) promoter. The helper plasmid may consist of two retroviral helper DNA sequences encoded by plasmid-based expression vectors, for example where a first helper sequence contains a cDNA encoding the gag and pol proteins of ecotropic MMLV or GALV and a second helper sequence contains a cDNA encoding the env protein. The Env gene, which determines the host range, may be derived from the genes encoding xenotropic, amphotropic, ecotropic, polytropic (mink focus forming) or 10A1 murine leukemia virus env proteins, or the Gibbon Ape Leukemia Virus (GALV env protein, the Human Immunodeficiency Virus env (gp160) protein, the Vesicular Stomatitus Virus (VSV) G protein, the Human T cell leukemia (HTLV) type I and II env gene products, chimeric envelope gene derived from combinations of one or more of the aforementioned env genes or chimeric envelope genes encoding the cytoplasmic and transmembrane of the aforementioned env gene products.

In the packaging process, the helper plasmids and the plasmids encoding the AAV viral proteins are transiently co-transfected into a first population of mammalian cells that are capable of producing virus, such as human embryonic kidney cells, for example 293 cells (ATCC No. CRL1573, ATCC, Rockville, Md.) to produce high titer recombinant retrovirus-containing supernatants. In another method of the invention this transiently transfected first population of cells is then co-cultivated with mammalian target cells, for example human lymphocytes, to transduce the target cells with the foreign gene at high efficiencies.

In one embodiment, two types of recombinant AAV vectors are administered to the subject, wherein a first type of recombinant AAV vector comprises the first sequence and the second sequence, and wherein a second type of recombinant AAV vector comprises the third sequence that encodes a Cas nuclease.

The present system may comprise (or consist essentially of) at least one type of vector encoding a CRISPR-Cas system directed to a mutant allele of the autosomal dominant disease-related gene (e.g., the autosomal dominant ocular disease-related gene). The at least one type of vector may comprise: (i) a first sequence encoding a first guide RNA that hybridizes to a variant of a single nucleotide polymorphism (SNP) in the autosomal dominant disease-related gene; (ii) a second sequence encoding a second guide RNA that hybridizes to an intron of the autosomal dominant disease-related gene; and, (iii) a third sequence encoding a Cas nuclease.

The present system may comprise (or consist essentially of) at least one type of vector encoding a CRISPR-Cas system directed to a mutant allele of the autosomal dominant disease-related gene. The at least one type of vector may comprise: (i) a first sequence encoding a first guide RNA that hybridizes to a variant of a single nucleotide polymorphism (SNP) in the autosomal dominant disease-related gene; and, (ii) a second sequence encoding a catalytically defective Cas nuclease (dCas).

In one embodiment, the present system comprises at least one nucleic acid sequence comprising a CRISPR system polynucleotide sequence, wherein the polynucleotide sequence comprises: (i) one or more guide RNA sequences that hybridize to an autosomal dominant disease-related gene sequence; (ii) a second sequence encoding a codon-modified autosomal dominant disease-related gene or fragment, wherein at least one disease related mutation in the modified autosomal dominant disease-related gene or fragment has been corrected and the codon-modified autosomal dominant disease related gene or fragment cannot be recognized by one or more sg RNA sequences that hybridize to an unmodified autosomal dominant disease-related gene sequence; and (iii) a sequence encoding a Cas family enzyme.

The present method and system may treat or prevent a dominantly-inherited condition (such as an ocular disease) in a subject, or modify a gene in a cell from a subject (or derived from a cell from a subject) having a dominantly-inherited condition (such as an ocular disease). The ocular diseases include, but are not limited to, vitelliform macular dystrophy (VMD), Best vitelliform macular dystrophy, autosomal dominant chorioretinal atrophy or degeneration, autosomal dominant cone or cone-rod dystrophy, autosomal dominant congenital stationary night blindness, autosomal dominant Leber congenital amaurosis, autosomal dominant macular degeneration, autosomal dominant ocular-retinal developmental disease, autosomal dominant optic atrophy, autosomal dominant retinitis pigmentosa, autosomal dominant syndromic/systemic diseases with retinopathy, sorsby macular dystrophy, age-related macular degeneration, doyne honeycomb macular disease, and juvenile macular degeneration. As is apparent to the skilled artisan, the gene to be delivered for correction of the disease or condition will vary with the disease to be treated.

In one embodiment, the ocular disease is age-related macular degeneration. In another embodiment, the ocular disease is juvenile macular degeneration.

The present system (e.g., nucleases, polynucleotides encoding these nucleases, donor polynucleotides and compositions comprising the proteins and/or polynucleotides described herein) may be delivered by any suitable means. In certain embodiments, the system is delivered *in vivo.* In other embodiments, the system is delivered to isolated/cultured cells (e.g., autologous iPS cells) *in vitro* to provide modified cells useful for *in vivo* delivery to patients afflicted with an autosomal dominant disease (e.g., an ocular autosomal dominant disease).

As an alternative to injection of viral particles encoding CRISPR components described in the present disclosure (e.g., sgRNA(s), codon-modified donor template gene of fragment sequences, and Cas family nuclease), cell replacement therapy can be used to prevent, correct or treat diseases, where the methods of the present disclosure are applied to isolated patient's cells *(ex vivo),* which is then followed by the injection of "corrected" cells back into the patient.

In one embodiment, the disclosure provides for introducing one or more vectors encoding CRISPR-Cas into a eukaryotic cell. The cell may be a stem cell. Examples of stem cells include pluripotent, multipotent and unipotent stem cells. Examples of pluripotent stem cells include embryonic stem cells, embryonic germ cells, embryonic carcinoma cells and induced pluripotent stem cells (iPSCs). In one aspect, the iPSC is derived from a fibroblast cell.

For the treatment of ocular diseases, patient's iPS cells can be isolated and differentiated into retinal pigment epithelium (RPE) cells *ex vivo.* Patient's iPS cells or RPE cells characterized by the mutation in autosomal dominant disease-related gene may be manipulated using methods of the present disclosure in a manner that results in the ablation (e.g., deletion) or silencing (e.g., transcription blocked) of a mutant allele of the autosomal dominant disease-related gene.

Thus, the present disclosure provides methods for correcting autosomal dominant ocular disease in a subject, wherein the method results in the ablation of a mutant allele of the autosomal dominant ocular disease-related gene. The method may comprise administering to the subject a therapeutically effective amount of autologous or allogeneic retinal pigment RPE cells with the ablated mutant allele of the autosomal dominant ocular disease-related gene. Administration of the pharmaceutical preparations comprising RPE cells with the ablated mutant allele of the autosomal dominant ocular disease-related gene may be effective to reduce the severity of symptoms and/or to prevent further deterioration in the patient's condition. Such administration may be effective to fully restore any vision loss or other symptoms.

"Induced pluripotent stem cells," commonly abbreviated as iPS cells or iPSCs, refer to a type of pluripotent stem cell artificially prepared from a non-pluripotent cell, typically an adult somatic cell, or terminally differentiated cell, such as a fibroblast, a hematopoietic cell, a myocyte, a neuron, an epidermal cell, or the like, by introducing certain factors, referred to as reprogramming factors.

The present methods may further comprise differentiating the iPS cell to a differentiated cell, for example, an ocular cell.

For example, patient fibroblast cells can be collected from the skin biopsy and transformed into iPS cells. Dimos JT et al. (2008) Induced pluripotent stem cells generated from patients with ALS can be differentiated into motor neurons. Science 321: 1218-1221; Nature Reviews Neurology 4, 582-583 (November 2008). Luo et al., Generation of induced pluripotent stem cells from skin fibroblasts of a patient with olivopontocerebellar atrophy, Tohoku J. Exp. Med. 2012, 226(2): 151-9. The CRISPR-mediated modification can be done at this stage. The corrected cell clone can be screened and selected by RFLP assay. The corrected cell clone is then differentiated into RPE cells and tested for its RPE-specific markers (e.g., Bestrophin1, RPE65, Cellular Retinaldehyde-binding Protein, and MFRP). Well-differentiated RPE cells can be transplanted autologously back to the donor patient.

The cell may be autologous or allogeneic to the subject who is administered the cell.

The term "autologous" refers to any material derived from the same individual to whom it is later to be re-introduced into the same individual.

The term "allogeneic" refers to any material derived from a different animal of the same species as the individual to whom the material is introduced. Two or more individuals of the same species are said to be allogeneic to one another.

The corrected cells for cell therapy to be administered to a subject (e.g., RPE cells) described in the present disclosure may be formulated with a pharmaceutically acceptable carrier. For example, cells can be administered alone or as a component of a pharmaceutical formulation. The cells (e.g., RPE cells) can be administered in combination with one or more pharmaceutically acceptable sterile isotonic aqueous or nonaqueous solutions (e.g., balanced salt solution (BSS)), dispersions, suspensions or emulsions, or sterile powders which may be reconstituted into sterile injectable solutions or dispersions just prior to use, which may contain antioxidants, buffers, bacteriostats, solutes or suspending or thickening agents.

The present system may be delivered into the retina of a subject. The present system may be administered through injections, such as subretinal or intravitreal injections.

The corrected cells (e.g., RPE cells) may be delivered in a pharmaceutically acceptable ophthalmic formulation by intraocular injection. Concentrations for injections may be at any amount that is effective and nontoxic. The pharmaceutical preparations of the cells of the present disclosure for treatment of a patient may be formulated at doses of at least about 10⁴ cells/mL. The cell preparations for treatment of a patient can be formulated at doses of at least or about 10³, 10⁴, 10⁵, 10⁶, 10⁷, 10⁸, 10⁹, or 10¹⁰ cells/mL.

Subjects, which may be treated according to the present disclosure, include all animals which may benefit from the present invention. Such subjects include mammals, preferably humans (infants, children, adolescents and/or adults), but can also be an animal such as dogs and cats, farm animals such as cows, pigs, sheep, horses, goats and the like, and laboratory animals (e.g., rats, mice, guinea pigs, and the like).

The term "nuclease" is used to generally refer to any enzyme that hydrolyzes nucleic acid sequences.

The term "ocular cells" refers to any cell in, or associated with the function of, the eye. The term may refer to any one or more of photoreceptor cells, including rod, cone and photosensitive ganglion cells, retinal pigment epithelium (RPE) cells, Müeller cells, bipolar cells, horizontal cells, or amacrine cells. In one embodiment, the ocular cells are bipolar cells. In another embodiment, the ocular cells are horizontal cells. In yet a third embodiment, the ocular cells include ganglion cells.

The terms "polynucleotide", "nucleotide", "nucleotide sequence", "nucleic acid" and "oligonucleotide" are used interchangeably. These terms refer to a polymeric form of nucleotides of any length, either deoxyribonucleotides or ribonucleotides, or analogs. Examples of polynucleotides include, but are not limited to, coding or non-coding regions of a gene or gene fragment, exons, introns, messenger RNA (mRNA), transfer RNA, ribosomal RNA, short interfering RNA (siRNA), short-hairpin RNA (shRNA), micro-RNA (miRNA), ribozymes, cDNA, recombinant polynucleotides, branched polynucleotides, plasmids, vectors, isolated DNA of any sequence, isolated RNA of any sequence, nucleic acid probes, and primers. One or more nucleotides within a polynucleotide sequence can further be modified. The sequence of nucleotides may be interrupted by non-nucleotide components. A polynucleotide may also be modified after polymerization, such as by conjugation with a labeling agent.

### Single-nucleotide Polymorphism (SNP)

A single-nucleotide polymorphism, often abbreviated to SNP, is a substitution of a single nucleotide that occurs at a specific position in the genome, where each variation is present to some appreciable degree within a population (e.g., > 1%). In one embodiment, at a specific base position in the human genome, the A nucleotide may appear in most individuals, but in a minority of individuals, the position is occupied by a G. This means that there is a SNP at this specific position, and the two possible nucleotide variations - A or G - are said to be alleles for this position. The term SNP refers to a difference of one base at the same relative site when two alleles are aligned and compared; herein, the term is also used in some contexts to mean a single base change.

A single-nucleotide variant (SNV) is a variation in a single nucleotide without any limitations of frequency and may arise in somatic cells.

The variant of the SNP may be a G variant, a C variant, an A variant, or a T variant.

The one or more SNPs may be in one or more coding regions, one or more non-coding regions, one or more intergenic regions (regions between genes), or combinations of one or more coding regions, and/or one or more non-coding regions, and/or one or more intergenic regions. The one or more SNPs may be in one or more introns, one or more exons, a combination of one or more introns and one or more exons. SNPs within a coding region may or may not change the amino acid sequence of the protein that is produced. SNPs may be synonymous SNPs or nonsynonymous SNPs. Synonymous SNPs do not affect the protein sequence, while nonsynonymous SNPs change the amino acid sequence of protein. The nonsynonymous SNPs may be missense SNPs or nonsense SNPs. SNPs that are not in protein-coding regions may affect gene splicing, transcription factor binding, messenger RNA degradation, and/or the sequence of noncoding RNA. Gene expression affected by this type of SNP may be upstream or downstream from the gene.

Genotyping of polymorphic variants can be carried out using any suitable methodology known in the art. Techniques which may be used for genotyping single nucleotide polymorphisms (SNPs) include DNA sequencing; capillary electrophoresis; ligation detection reaction (Day et al., Genomics 29, 152 62 (1995)); mass spectrometry, such as matrix-assisted laser desorption/ionization time-of-flight mass spectrometry (MALDI-TOF-MS); single-strand conformation polymorphism (SSCP); single-base extension; electrochemical analysis; denaturating HPLC and gel electrophoresis; restriction fragment length polymorphism; hybridization analysis; single nucleotide primer extension and DNA chips or microarrays (see review by Schafer et al., Nature Biotechnology, Vol 16, pp33-39 (1998)). The use of DNA chips or microarrays may enable simultaneous genotyping at many different polymorphic loci in a single individual or the simultaneous genotyping of a single polymorphic locus in multiple individuals. SNPs may also be scored by DNA sequencing.

In addition to the above, SNPs may be scored using PCR-based techniques, such as PCR-SSP using allele-specific primers (Bunce et al., Tissue Antigens, 1995; 50: 23-31). This method generally involves performing DNA amplification reactions using genomic DNA as the template and two different primer pairs, the first primer pair comprising an allele-specific primer which under appropriate conditions is capable of hybridizing selectively to the wild type allele and a non allele-specific primer which binds to a complementary sequence elsewhere within the gene in question, the second primer pair comprising an allele-specific primer which under appropriate conditions is capable of hybridizing selectively to the variant allele and the same non allele-specific primer. Further suitable techniques for scoring SNPs include PCR ELISA and denaturing high performance liquid chromatography (DHPLC).

If the SNP results in the abolition or creation of a restriction site, genotyping can be carried out by performing PCR using non-allele specific primers spanning the polymorphic site and digesting the resultant PCR product using the appropriate restriction enzyme (also known as PCR-RFLP). Restriction fragment length polymorphisms, including those resulting from the presence of a single nucleotide polymorphism, may be scored by digesting genomic DNA with an appropriate enzyme then performing a Southern blot using a labelled probe corresponding to the polymorphic region (Molecular Cloning: A Laboratory Manual, Sambrook, Fritsch and Maniatis, Cold Spring Harbor Laboratory, Cold Spring Harbor, N.Y.).

"Genotyping" of any given polymorphic variant may comprise screening for the presence or absence in the genome of the subject of both the normal or wild type allele and the variant or mutant allele, or may comprise screening for the presence or absence of either individual allele, it generally being possible to draw conclusions about the genotype of an individual at a polymorphic locus having two alternative allelic forms just by screening for one or other of the specific alleles.

It is within the scope of the present disclosure to perform genotyping of polymorphisms or polymorphic variants within multiple genes. Such a panel screen of multiple genes may be used to simultaneously analyze multiple polymorphisms in the same subject. In one embodiment, genotyping of multiple polymorphisms in a single subject sample may be carried out simultaneously, for example with the use of a microarray or gene chip.

"Multiple" should be taken to mean two or more, three or more, four or more, five or more, six or more etc.

Genotyping may be carried out *in vitro,* and can be performed on an isolated sample containing genomic DNA prepared from a suitable sample obtained from the subject under test. For example, genomic DNA is prepared from a sample of whole blood or tissue, or any suitable sample as described herein, according to standard procedures which are well known in the art. If genomic sequence data for the individual under test in the region containing the SNP is available, for example in a genomic sequence database as a result of a prior genomic sequencing exercise, then genotyping of the SNP may be accomplished by searching the available sequence data.

In the case of genetic variants which have a detectable effect on the mRNA transcripts transcribed from a given gene, for example variants which cause altered splicing or which affect transcript termination or which affect the level or mRNA expression, then as an alternative to detecting the presence of the variant at the genomic DNA level, the presence of the variant may be inferred by evaluating the mRNA expression pattern using any suitable technique. Similarly, in the case of genetic variants which have a detectable effect on the protein products encoded by a gene, for example variants which cause a change in primary amino acid sequence, structure or properties of the encoded protein, the presence of the variant may be inferred by evaluating the sequence, structure or properties of the protein using any convenient technique.

In one embodiment, the autosomal dominant disease-related gene comprises a sequence selected from SEQ ID NO: 28, SEQ ID NO: 29, SEQ ID NO: 30, SEQ ID NO: 32, or SEQ ID NO: 33, or an equivalent of each thereof. In another embodiment, the first guide RNA that hybridizes to the autosomal dominant disease-related gene comprises a sequence of SEQ ID NO: 31 or an equivalent thereof.

### Bestrophin-1 (BEST1)

Bestrophin-1 (Best1) is a protein that is encoded by the BEST1 gene, in humans (RPD ID - 5T5N/4RDQ). The bestrophin family of proteins comprises four evolutionary related genes (BEST1, BEST2, BEST3, and BEST4) that code for integral membrane proteins. This gene family is characterized by proteins with a highly conserved N-terminus with four to six transmembrane domains. Specifically, the BEST1 gene on chromosome 1 1q12.3 encodes the Bestrophin-1 protein in humans whose expression is highest in the retina. Bestrophins may form chloride ion channels or may regulate voltage-gated L-type calcium-ion channels. Bestrophins are generally believed to form calcium-activated chloride-ion channels in epithelial cells but they have also been shown to be highly permeable to bicarbonate ion transport in retinal tissue. Mutations in this gene are responsible for juvenile-onset vitelliform macular dystrophy (VMD2), also known as Best macular dystrophy, in addition to adult-onset vitelliform macular dystrophy (AVMD) and other retinopathies. Alternative splicing results in multiple variants encoding distinct isoforms. In humans, BEST1 mutations have been linked with Best vitelliform macular dystrophy (BVMD). Mutations in the BEST1 gene have been identified as the primary cause for at least five different degenerative retinal diseases.

The NCBI Reference Sequence (RefSeq) accession numbers for human BEST1 mRNA may include NM_001139443, NM_001300786, NM _001300787, NM_004183 and NM _001363591. The NCBI Reference Sequence (RefSeq) accession numbers for human BEST1 protein may include NP_001132915, NP_001287715, NP_001287716, NP_004174, and NP_001350520. The NCBI Reference Sequence (RefSeq) accession numbers for murine BEST1 mRNA may include NM_011913. The NCBI Reference Sequence (RefSeq) accession numbers for murine BEST1 protein may include NP_036043.

The single-nucleotide polymorphisms (SNPs) of BEST1 include, but are not limited to, rs972353, rs972355, rs2668899, rs972354, rs1800007, rs1801393, rs1109748, rs199529046, rs1805140, rs17156602, rs2668897, rs2668898, rs2736597, rs195156, rs195157, rs195158, rs195160, rs195162, rs741886, rs760306, rs1801621, and rs195165.

The mutants of BEST1 include, but are not limited to, pR218H, pL234P, and pA243T.

### CRISPR/Cas and Other Nucleases

The Cas protein or enzyme/nuclease may be a wildtype (wt) Cas (e.g., a wildtype (wt) Cas9), a Cas nickase (e.g., a Cas9 nickase), or a dCas (e.g., a dCas9).

A nuclease-defective or nuclease-deficient Cas protein (e.g., dCas9) with one or more mutations on its nuclease domains retains DNA binding activity when complexed with gRNA. dCas protein can tether and localize effector domains or protein tags by means of protein fusions to sites matched by gRNA, thus constituting an RNA-guided DNA binding enzyme. dCas can be fused to transcriptional activation domain (e.g., VP64) or repressor domain (e.g., KRAB), and be guided by gRNA to activate or repress target genes, respectively. dCas can also be fused with fluorescent proteins and achieve live-cell fluorescent labeling of chromosomal regions.

It is appreciated by those skilled in the art that gRNAs can be generated for target specificity to target a specific gene, optionally a gene associated with a disease, disorder, or condition. Thus, in combination with Cas9, the guide RNAs facilitate the target specificity of the CRISPR/Cas9 system. Further aspects such as promoter choice, as discussed above, may provide additional mechanisms of achieving target specificity - e.g., selecting a promoter for the guide RNA encoding polynucleotide that facilitates expression in a particular organ or tissue. Accordingly, the selection of suitable gRNAs for the particular disease, disorder, or condition is contemplated herein. In one embodiment, the gRNA hybridizes to the autosomal dominant disease-related gene that comprises a single nucleotide polymorphism (SNP).

Administration of the modified AAV or compositions can be effected in one dose, continuously or intermittently throughout the course of treatment. Administration may be through any suitable mode of administration, including but not limited to: intravenous, intraarterial, intramuscular, intracardiac, intrathecal, subventricular, epidural, intracerebral, intracerebroventricular, sub-retinal, intravitreal, intraarticular, intraocular, intraperitoneal, intrauterine, intradermal, subcutaneous, transdermal, transmuccosal, and inhalation.

Methods of determining the most effective means and dosage of administration are known to those of skill in the art and will vary with the composition used for therapy, the purpose of the therapy and the subject being treated. Single or multiple administrations can be carried out with the dose level and pattern being selected by the treating physician. It is noted that dosage may be impacted by the route of administration. Suitable dosage formulations and methods of administering the agents are known in the art. Non-limiting examples of such suitable dosages may be as low as 1E+9 vector genomes to as much as 1E+17 vector genomes per administration.

In a further aspect, the viral particle and compositions of the invention can be administered in combination with other treatments, e.g., those approved treatments suitable for the particular disease, disorder, or condition. A non-limiting example includes the treatment of muscular dystrophy with a combination of the modified viral particle and one or more steroids. One can determine if the treatment has been successful by monitoring for clinical or sub-clinical evidence of gene modification, as determined by the treating physician.

This administration of the modified viral particle or compositions of the invention can be done to generate an animal model of the desired disease, disorder, or condition for experimental and screening assays.

In some embodiments, the nucleotide sequence encoding the Cas (e.g., Cas9) nuclease is modified to alter the activity of the protein. In some embodiments, the Cas (e.g., Cas9) nuclease is a catalytically inactive Cas (e.g., Cas9) (or a catalytically deactivated/defective Cas9 or dCas9). In one embodiment, dCas (e.g., dCas9) is a Cas protein (e.g., Cas9) that lacks endonuclease activity due to point mutations at one or both endonuclease catalytic sites (RuvC and HNH) of wild type Cas (e.g., Cas9). For example, dCas9 contains mutations of catalytically active residues (D10 and H840) and does not have nuclease activity. In some cases, the dCas has a reduced ability to cleave both the complementary and the non-complementary strands of the target DNA. As a non-limiting example, in some cases, the dCas9 harbors both D10A and H840A mutations of the amino acid sequence of S. pyogenes Cas9. In some embodiments when a dCas9 has reduced or defective catalytic activity (e.g., when a Cas9 protein has a D10, G12, G17, E762, H840, N854, N863, H982, H983, A984, D986, and/or a A987 mutation, e.g., D10A, G12A, G17A, E762A, H840A, N854A, N863A, H982A, H983A, A984A, and/or D986A), the Cas protein can still bind to target DNA in a site-specific manner, because it is still guided to a target polynucleotide sequence by a DNA-targeting sequence of the subject polynucleotide (e.g., gRNA), as long as it retains the ability to interact with the Cas-binding sequence of the subject polynucleotide (e.g., gRNA).

Inactivation of Cas endonuclease activity can create a catalytically deactivated Cas (dCas, e.g., dCas9). dCas can bind but not cleave DNA, thus preventing the transcription of the target gene by creating a physical barrier to the action of transcription factors. This rendition of CRISPR works at the transcription level in a reversible fashion. This strategy has been termed CRISPR interference, or CRISPRi. In CRISPR interference (CRISPRi), dCas fusion proteins (e.g., dCas fused to another protein or portion thereof) may be used in the present method for gene repression. In some embodiments, dCas is fused to a (transcriptional) repressor domain or a transcriptional silencer. Non-limiting examples of transcriptional repression domains include a Krüppel-associated Box (KRAB) domain, an ERF repressor domain (ERD), a mSin3A interaction domain (SID) domain, concatemers of SID (e.g. SID4X), or a homolog thereof. Non-limiting examples of transcriptional silencers include Heterochromatin Protein 1 (HP1). CRISPRi may be modified by fusing Cas (e.g., dCas) to the Kruppel-associated box repression domain (KRAB), which augments the repressive effects of Cas. Gilbert et al. CRISPR-mediated modular RNA-guided regulation of transcription in eukaryotes. Cell. 2013; 154(2):442-51.

Second generation CRISPRi strongly represses via PUF-KRAB repressors. PUF proteins (named after Drosophila Pumilio and C. elegans fern-3 binding factor) are known to be involved in mediating mRNA stability and translation. These proteins contain a unique RNA-binding domain known as the PUF domain. The RNA-binding PUF domain, such as that of the human Pumilio 1 protein (referred here also as PUM), contains 8 repeats (each repeat called a PUF motif or a PUF repeat) that bind consecutive bases in an anti-parallel fashion, with each repeat recognizing a single base, i.e., PUF repeats R1 to R8 recognize nucleotides N8 to N1, respectively. For example, PUM is composed of eight tandem repeats, each repeat consisting of 34 amino acids that folds into tightly packed domains composed of alpha helices. PUF and its derivatives or functional variants are programmable RNA-binding domains that can be used in the present methods and systems, as part of a PUF domain-fusion that brings any effector domain to a specific PUF-binding sequence on the subject polynucleotide (e.g., gRNA).

As used herein, "PUF domain" refers to a wildtype or naturally existing PUF domain, as well as a PUF homologue domain that is based on/derived from a natural or existing PUF domain, such as the prototype human Pumilio 1 PUF domain.

The PUF adaptor protein binds gRNA modified with a PUF-binding sequence. The gRNA may comprise one or more PUF-binding sequences. For example, the gRNA can be derived by inserting multiple copies of short PUF-binding sequences (e.g., 8-mer), e.g., downstream of gRNA stem loops or upstream of the target-matching region. In certain embodiments, each of the one or more copies of the PUF-binding sequence has about 8 nucleotides. The gRNA may have more than one copies of the PUF-binding sequence. In certain embodiments, the gRNA comprises about 1, 2, 3, 4, 5, 10, 15, 20, 25, 30, 35, 40, 45, 46, 47, 48, 49, 50, 5-15 copies, about 5-14 copies, about 5-13 copies, about 5-12 copies, about 5-11 copies, about 5-10 copies, or about 5-9 copies of the PUF-binding sequence, such as 5, 6, 7, 8, 9, 10, 11, 12, 13, 14, or 15 copies of PUF-binding sequence. In certain embodiments, the range of the PUF-binding sequence copy number is L to H, wherein L is any one of 1, 2, 3, 4, 5, 6, 7, 8, 9, 10, 11, 12, 13, 14, 15, 20, 25, 30, 35, or 40, and wherein H is any one of 2, 3, 4, 5, 6, 7, 8, 9, 10, 11, 12, 13, 14, 15, 20, 25, 30, 35, 40, 45, 50, 55, 60, 65, 70, 80, 90, or 100, so long as H is greater than L. Each PUF-binding sequence may be the same or different. The PUF-binding sequence may be those disclosed in U.S. Patent Publication No. 20180094257, the content of which is incorporated herein by reference in its entirety. The gRNA may comprise one or more tandem sequences, each of which can be specifically recognized and bound by a specific PUF domain. Since a PUF domain can be engineered to bind virtually any PUF-binding sequence based on the nucleotide-specific interaction between the individual PUF motifs of PUF domain and the single RNA nucleotide they recognize, the PUF-binding sequence sequences can be any designed sequence that bind their corresponding PUF domain. In certain embodiments, a PUF-binding sequence of the invention has 8-mer. In other embodiments, a PUF-binding sequence has 5, 6, 7, 8, 9, 10, 11, 12, 13, 14, 15, 16 or more RNA nucleotides. The PUF-binding sequence may bind the human Pumilio 1 PUF domain (wildtype or mutant).

In certain embodiments, the PUF domain comprises PUF motifs from different PUF domains from different proteins. For example, a PUF domain may be constructed with PUF motifs from the human Pumilio 1 protein and one or more other PUF motifs from one or more other PUF proteins, such as PuDp or FBF. In certain embodiments, the PUF domain is a Pumilio homology domain (PU-HUD). In a particular embodiment, the PU-HUD is a human Pumilio 1 domain. In certain embodiments, the PUF domain is any PUF protein family member with a Pum-HD domain. Non-limiting examples of a PUF family member include FBF in C. elegans, Ds pum in Drosophila, and PUF proteins in plants such as Arabidopsis and rice. Tam et al., The Puf family of RNA-binding proteins in plants: phylogeny, structural modeling, activity and subcellular localization, BMC Plant Biol. 10:44, 2010, the entire contents of which are incorporated by reference herein.

The present methods and systems may use CRISPR deletion (CRISPRd). CRISPRd capitalizes on the tendency of DNA repair strategies to default towards NHEJ, and does not require a donor template to repair the cleaved strand. Instead, Cas creates a DSB in the gene harboring a mutation first, then NHEJ occurs, and insertions and/or deletions (INDELs) are introduced that corrupt the sequence, thus either preventing the gene from being expressed or proper protein folding from occurring. This strategy may be particularly applicable for dominant conditions, in which case knocking out the mutated, dominant allele and leaving the wild type allele intact may be sufficient to restore the phenotype to wild type.

In certain embodiments, the Cas enzyme may be a catalytically defective Cas (e.g., Cas9) or dCas, or a Cas nickase or nickase.

The Cas enzyme (e.g., Cas9) may be modified to function as a nickase, named as such because it "nicks" the DNA by inducing single-strand breaks instead of DSBs. The term "Cas nickase" or "nickase", as used herein, refers to a Cas protein that is capable of cleaving only one strand of a duplexed nucleic acid molecule (e.g., a duplexed DNA molecule). In some embodiments, a Cas nickase may be any of the nickase disclosed in U.S. Patent No. 10,167,457, the content of which is incorporated herein by reference in its entirety. In one embodiment, a Cas (e.g., Cas9) nickase has an active HNH nuclease domain and is able to cleave the non-targeted strand of DNA, i.e., the strand bound by the gRNA. In one embodiment, a Cas (e.g., Cas9) nickase has an inactive RuvC nuclease domain and is not able to cleave the targeted strand of the DNA, i.e., the strand where base editing is desired. In some embodiments the Cas nickase cleaves the target strand of a duplexed nucleic acid molecule, meaning that the Cas nickase cleaves the strand that is base paired to (complementary to) a gRNA (e.g., an sgRNA) that is bound to the Cas. In some embodiments, the Cas nickase cleaves the non-target, non-base-edited strand of a duplexed nucleic acid molecule, meaning that the Cas nickase cleaves the strand that is not base paired to a gRNA (e.g., an sgRNA) that is bound to the Cas. In one embodiment, the Cas9 nickase is a Cas9 D10A nickase bearing a mutation in the RuvC endonuclease domain. Additional suitable Cas9 nickases will be apparent to those of skill in the art based on this disclosure and knowledge in the field, and are within the scope of this disclosure.

For example, the nickase can be a Cas9 nickase with a mutation at a position corresponding to D10A of S. *pyogenes* Cas9; or the nickase can be a Cas9 nickase with a mutation at a position corresponding to H840A of *S. pyogenes* Cas9. In some cases, the Cas nickase can cleave the complementary strand of the target DNA but has reduced ability to cleave the non-complementary strand of the target DNA. For example, the Cas9 nickase can have a mutation (e.g., an amino acid substitution) that reduces the function of the RuvC domain. As a non-limiting example, the Cas9 nickase is a D10A mutation of the amino acid sequence of *S*. *pyogenes* Cas9. In some cases, the Cas9 nickase can cleave the non-complementary strand of the target DNA but has reduced ability to cleave the complementary strand of the target DNA. For example, the Cas9 nickase can have a mutation (e.g., an amino acid substitution) that reduces the function of the HNH domain (RuvC/HNH/RuvC domain motifs). As a non-limiting example, the Cas9 nickase is a H840A of *S*. *pyogenes* Cas9. U.S. Patent Publication No. 20180094257, the content of which is incorporated herein by reference in its entirety.

In one embodiment, two gRNAs targeting sites that are close together can direct separate Cas nickases to induce breaks on each DNA strand. By requiring two different cleavage events to induce recombination, this strategy can decrease the likelihood of off-targeting effects.

In CRISPR activation (CRISPRa), dCas may be fused to an activator domain, such as VP64 or VPR. Such dCas fusion proteins may be used with the constructs described herein for gene activation. In some embodiments, dCas is fused to an epigenetic modulating domain, such as a histone demethylase domain or a histone acetyltransferase domain. In some embodiments, dCas is fused to a LSD1 or p300, or a portion thereof. In some embodiments, the dCas fusion is used for CRISPR-based epigenetic modulation. In some embodiments, dCas or Cas is fused to a Fok1 nuclease domain. In some embodiments, Cas or dCas fused to a Fok1 nuclease domain is used for genome editing. In some embodiments, Cas or dCas is fused to a fluorescent protein *(e.g.,* GFP, RFP, mCherry, etc.). In some embodiments, Cas/dCas proteins fused to fluorescent proteins are used for labeling and/or visualization of genomic loci or identifying cells expressing the Cas endonuclease.

The present disclosure provides for gene editing methods that can ablate the disease-causing mutant alleles, which in turn can be used for *in vivo* gene therapy for patients afflicted with autosomal dominant diseases.

The present disclosure takes advantage of the CRISPR gene-editing system. In certain embodiments, the method uses a gene-editing enzyme with one or multiple unique guide RNA (gRNA, such as single guide RNA or sgRNA) sequences that target mutant allele(s) specifically, or that target both the mutant and wild type alleles, of a gene carrying an autosomal dominant mutation for destruction. This targeting may or may not be followed by supplying the wild type gene cDNA, that may or may not be codon modified in order to evade recognition, by the sgRNA(s).

In certain embodiments, the present disclosure provides a gene-editing system referred to as a "ChopStick" system. The "Chop" step involves partial or complete disruption of the i) mutant copy of a gene that is to be corrected; and/or ii) the wild-type copy of said gene in a patient afflicted with autosomal dominant disease. Thus, the "Chop" step results in partial or complete loss of mutant and/or wild-type activity of the said gene. The "Stick" step encompasses the introduction of a codon-modified cDNA of a gene of interest or fragment thereof (characterized by the autosomal dominant mutation), which is intended to restore, correct, supplement, or augment the gene or gene product function in the cells. Deletion of both the mutant and/or wild-type forms of the gene, followed by supplying the wild type gene cDNA that is codon modified and resistant to recognition by the guide RNAs results in the correction of the mutation, and thus, restoration of a phenotype found in the autosomal dominant diseases.

In one embodiment, the "Stick" step results in integration of a codon-modified donor template of a gene of interest or fragment (characterized by the autosomal dominant mutation) into the endogenous autosomal disease-related gene. Such targeted integration is accomplished by homologous recombination. In general, a Cas-family nuclease makes a DNA double-strand break at a defined site in the genome, which can then be repaired by homologous recombination or non-homologous end joining.

Alternatively, the "Stick" step does not result in integration of a codon-modified donor template of a gene of interest or fragment (characterized by the autosomal dominant mutation) into the endogenous autosomal disease-related gene. For example, extrachromosomal, or episomal (episomally), vectors persist in the nucleus in an extrachromosomal state, and offer transgene delivery and expression without integration into the host genome. Among such vectors are AAV vectors, which are particularly efficient in transduction of nondividing cells, and where the vector genome persists predominantly in an episomal form.

The present disclosure provides a method that is the modification of the CRISPR/Cas9 gene editing system to treat autosomal dominant retinitis pigmentosa (RP) caused by rhodopsin mutations. The approach is to use a gene editing enzyme with a pair of unique guide RNA sequences that targets both mutant and wildtype forms of rhodopsin for destruction, and then supplying with wildtype rhodopsin cDNA that is codon modified to evade recognition by the guide RNAs. This codon modified engineered human rhodopsin sequence, which can be driven by the CMV promoter and is resistant to the effects of the gene editing enzyme, will rescue the phenotype of the patient. In general, patients with null rhodopsin have a milder phenotype than those with dominant rhodopsin mutations.

A codon-modified donor template can be delivered to cells or a patient via episomal vectors. Because episomal vectors persist in multiple copies per cell, the resulting expression of the gene of interest may be comparatively high at both the RNA as well as protein level. In non-dividing cells, the presence of the AAV vector as an episomal replicating element may be sufficient for stable expression of the gene, RNA, and/or protein.

Any suitable nuclease may be used in the present methods and systems. Nucleases are enzymes that hydrolyze nucleic acids. Nucleases may be classified as endonucleases or exonucleases. An endonuclease is any of a group of enzymes that catalyze the hydrolysis of bonds between nucleic acids in the interior of a DNA or RNA molecule. An exonuclease is any of a group of enzymes that catalyze the hydrolysis of single nucleotides from the end of a DNA or RNA chain. Nucleases may also be classified based on whether they specifically digest DNA or RNA. A nuclease that specifically catalyzes the hydrolysis of DNA may be referred to as a deoxyribonuclease or DNase, whereas a nuclease that specifically catalyses the hydrolysis of RNA may be referred to as a ribonuclease or an RNase. Some nucleases are specific to either single-stranded or double-stranded nucleic acid sequences. Some enzymes have both exonuclease and endonuclease properties. In addition, some enzymes are able to digest both DNA and RNA sequences.

Non-limiting examples of the endonucleases include a zinc finger nuclease (ZFN), a ZFN dimer, a ZFNickase, a transcription activator-like effector nuclease (TALEN), or a RNA-guided DNA endonuclease (e.g., CRISPR/Cas). Meganucleases are endonucleases characterized by their capacity to recognize and cut large DNA sequences (12 base pairs or greater). Any suitable meganuclease may be used in the present methods to create double-strand breaks in the host genome, including endonucleases in the LAGLIDADG and PI-Sce family.

One example of a sequence-specific nuclease system that can be used with the methods and compositions described herein includes the CRISPR system (Wiedenheft, B. et al. Nature 482, 331-338 (2012); Jinek, M. et al. Science 337, 816-821 (2012); Mali, P. et al. Science 339, 823-826 (2013); Cong, L. et al. Science 339, 819-823 (2013)). The CRISPR (Clustered Regularly interspaced Short Palindromic Repeats) system exploits RNA-guided DNA-binding and sequence-specific cleavage of target DNA. The guide RNA/Cas combination confers site specificity to the nuclease. A single guide RNA (sgRNA) contains about 20 nucleotides that are complementary to a target genomic DNA sequence upstream of a genomic PAM (protospacer adjacent motifs) site (e.g., NGG) and a constant RNA scaffold region. The Cas (CRISPR-associated) protein binds to the sgRNA and the target DNA to which the sgRNA binds and introduces a double-strand break in a defined location upstream of the PAM site. Cas9 harbors two independent nuclease domains homologous to HNH and RuvC endonucleases, and by mutating either of the two domains, the Cas9 protein can be converted to a nickase that introduces single-strand breaks (Cong, L. et al. Science 339, 819-823 (2013)). It is specifically contemplated that the methods and compositions of the present disclosure can be used with the single- or double-strand-inducing version of Cas9, as well as with other RNA-guided DNA nucleases, such as other bacterial Cas9-like systems. The sequence-specific nuclease of the present methods and compositions described herein can be engineered, chimeric, or isolated from an organism. The nuclease can be introduced into the cell in form of a DNA, mRNA and protein.

In one embodiment, the methods of the present disclosure comprise using one or more sgRNAs to "Chop", remove, or suppress an autosomal dominant disease-related gene. In another embodiment, one sgRNA(s) is used to "Chop", remove, or suppress an autosomal dominant disease-related gene. In yet further embodiment, two or more sgRNA(s) are used to "Chop", remove, or suppress an autosomal dominant disease- related gene.

In one embodiment, the DNA digesting agent can be a site-specific nuclease. In another embodiment, the site-specific nuclease may be a Cas-family nuclease. In a more specific embodiment, the Cas nuclease may be a Cas9 nuclease.

In one embodiment, Cas protein may be a functional derivative of a naturally occurring Cas protein.

In addition to well characterized CRISPR-Cas system, a new CRISPR enzyme, called Cpfl (Cas protein 1 of PreFran subtype) may be used in the present methods and systems (Zetsche et al. Cell. pii: S0092-8674(15)01200-3. doi: 10.1016/j.cell.2015.09.038 (2015)). Cpfl is a single RNA-guided endonuclease that lacks tracrRNA, and utilizes a T-rich protospacer-adjacent motif. The authors demonstrated that Cpfl mediates strong DNA interference with characteristics distinct from those of Cas9. Thus, in one embodiment of the present invention, CRISPR-Cpfl system can be used to cleave a desired region within the targeted gene.

In further embodiment, the nuclease is a transcription activator-like effector nuclease (TALEN). TALENs contains a TAL effector domain that binds to a specific nucleotide sequence and an endonuclease domain that catalyzes a double strand break at the target site (PCT Patent Publication No. WO2011072246; Miller et al., Nat. Biotechnol. 29, 143-148 (2011); Cermak et al., Nucleic Acid Res. 39, e82 (2011)). Sequence-specific endonucleases may be modular in nature, and DNA binding specificity is obtained by arranging one or more modules. Bibikova et al., Mol. Cell. Biol. 21, 289-297 (2001). Boch et al., Science 326, 1509-1512 (2009).

ZFNs can contain two or more (e.g., 2 - 8, 3 - 6, 6 - 8, or more) sequence-specific DNA binding domains (e.g., zinc finger domains) fused to an effector endonuclease domain (e.g., the FokI endonuclease). Porteus et al., Nat. Biotechnol. 23, 967-973 (2005). Kim et al. (2007) Hybrid restriction enzymes: Zinc finger fusions to Fok I cleavage domain, Proceedings of the National Academy of Sciences of USA, 93: 1156-1160. U.S. Patent No. 6,824,978. PCT Publication Nos. WO1995/09233 and WO1994018313.

In one embodiment, the nuclease is a site-specific nuclease of the group or selected from the group consisting of omega, zinc finger, TALE, and CRISPR/Cas.

The sequence-specific endonuclease of the methods and compositions described here can be engineered, chimeric, or isolated from an organism. Endonucleases can be engineered to recognize a specific DNA sequence, by, e.g., mutagenesis. Seligman et al. (2002) Mutations altering the cleavage specificity of a homing endonuclease, Nucleic Acids Research 30: 3870-3879. Combinatorial assembly is a method where protein subunits form different enzymes can be associated or fused. Amould et al. (2006) Engineering of large numbers of highly specific homing endonucleases that induce recombination to novel DNA targets, Journal of Molecular Biology 355: 443-458. In certain embodiments, these two approaches, mutagenesis and combinatorial assembly, can be combined to produce an engineered endonuclease with desired DNA recognition sequence.

The sequence-specific nuclease can be introduced into the cell in the form of a protein or in the form of a nucleic acid encoding the sequence-specific nuclease, such as an mRNA or a cDNA. Nucleic acids can be delivered as part of a larger construct, such as a plasmid or viral vector, or directly, e.g., by electroporation, lipid vesicles, viral transporters, microinjection, and biolistics. Similarly, the construct containing the one or more transgenes can be delivered by any method appropriate for introducing nucleic acids into a cell.

Single guide RNA(s) used in the methods of the present disclosure can be designed so that they direct binding of the Cas-sgRNA complexes to pre-determined cleavage sites in a genome. In one embodiment, the cleavage sites may be chosen so as to release a fragment or sequence that contains a region of autosomal dominant disease-related gene. In further embodiment, the cleavage sites may be chosen so as to release a fragment or sequence that contains a region of an autosomal dominant disease-related gene.

For Cas family enzyme (such as Cas9) to successfully bind to DNA, the target sequence in the genomic DNA can be complementary to the sgRNA sequence and may be immediately followed by the correct protospacer adjacent motif or "PAM" sequence. "Complementarity" refers to the ability of a nucleic acid to form hydrogen bond(s) with another nucleic acid sequence by either traditional Watson-Crick or other non-traditional types. A percent complementarity indicates the percentage of residues in a nucleic acid molecule, which can form hydrogen bonds (e.g., Watson-Crick base pairing) with a second nucleic acid sequence. Full complementarity is not necessarily required, provided there is sufficient complementarity to cause hybridization and promote formation of a CRISPR complex. A target sequence may comprise any polynucleotide, such as DNA or RNA polynucleotides. The Cas9 protein can tolerate mismatches distal from the PAM. The PAM sequence varies by the species of the bacteria from which Cas9 was derived. The most widely used CRISPR system is derived from S. *pyogenes* and the PAM sequence is NGG located on the immediate 3' end of the sgRNA recognition sequence. The PAM sequences of CRISPR systems from exemplary bacterial species include: *Streptococcus pyogenes* (NGG), *Neisseria meningitidis* (NNNNGATT), *Streptococcus thermophilus* (NNAGAA) and *Treponema denticola* (NAAAAC).

sgRNA(s) used in the present disclosure can be between about 5 and 100 nucleotides long, or longer (e.g., 5, 6, 7, 8, 9, 10, 11, 12, 13, 14, 15, 16, 17, 18, 19, 20, 21, 22, 23, 24, 25, 26, 27, 28, 29, 30, 31 , 32, 33, 34, 35, 36, 37, 38, 39, 40, 41, 42, 43, 44, 45, 46, 47, 48, 49, 50, 51 , 52, 53, 54, 55, 56, 57, 58, 59 60, 61, 62, 63, 63, 64, 65, 66, 67, 68, 69, 70, 71, 72, 73, 74, 75, 76, 77, 78, 79, 80, 81 , 82, 83, 84, 85, 86, 87, 88, 89, 90, 91 92, 93, 94, 95, 96, 97, 98, 99, or 100 nucleotides in length, or longer). In one embodiment, sgRNA(s) can be between about 15 and about 30 nucleotides in length (e.g., about 15-29, 15-26, 15-25; 16-30, 16-29, 16-26, 16-25; or about 18-30, 18-29, 18-26, or 18-25 nucleotides in length).

To facilitate sgRNA design, many computational tools have been developed (See Prykhozhij et al. (PLoS ONE, 10(3): (2015)); Zhu et al. (PLoS ONE, 9(9) (2014)); Xiao et al. (Bioinformatics. Jan 21 (2014)); Heigwer et al. (Nat Methods, 11(2): 122-123 (2014)). Methods and tools for guide RNA design are discussed by Zhu (Frontiers in Biology, 10 (4) pp 289-296 (2015)), which is incorporated by reference herein. Additionally, there is a publicly available software tool that can be used to facilitate the design of sgRNA(s)
(http://www.genscript.com/gRNA-design-tool.html).

A modified autosomal dominant disease-related gene or fragment sequence is a donor sequence that has been codon modified to be unrecognizable by sgRNA(s) used for targeting or recognition of the mutated autosomal dominant disease-related gene and resistant to sgRNA targeting. Such modified autosomal dominant disease-related gene sequence is a donor sequence encoding at least a functional fragment of the protein lacking or deficient in the subject with autosomal dominant disease.

As previously mentioned, the codon-modified cDNA (donor-template) may be modified in such a way as to render it unrecognizable by the sgRNA(s) used to target either mutant and wildtype disease-related gene(s). To achieve this, mutations need to be introduced into a donor-template gene or fragment to render donor-template gene or fragment unrecognizable by sgRNA(s) and consequently resistant to degradation by Cas enzyme (such as Cas9 nuclease) which has been introduced in cells. The donor-template gene may be modified by introducing a wobble base(s) into donor-template. Introduction of wobble base(s) in DNA results in a silent mutation, leaving the expression product of wt gene intact, but if nucleotide sequence has been sufficiently changed, it will render donor-template sequence unrecognizable by sgRNA(s) used to target either mutant and wt disease-related gene(s), ultimately resistant to Cas nuclease cleavage. The number of wobble bases that needs to be introduced into a donor-template may vary, but needs to be sufficient to prevent sgRNA hybridization and formation of a CRISPR complex.

In one embodiment, the donor template sequence may be delivered using the same gene transfer system as used to deliver the Cas nuclease (included on the same vector) or may be delivered using a different delivery system. In another embodiment, the donor template sequence may be delivered using the same transfer system as used to deliver sgRNA(s). In specific embodiments, the donor is delivered using a viral vector (e.g., AAV).

In one embodiment, the present disclosure comprises integration of codon-modified autosomal dominant disease-related gene sequence (donor template sequence) into the endogenous autosomal disease-related gene.

In another embodiment, the donor sequence or modified autosomal dominant disease-related gene sequence is integrated into endogenous gene by homologous recombination (HR).

In further embodiments, the donor sequence or modified autosomal dominant disease-related gene sequence is flanked by an upstream and a downstream homology arm. The homology arms, which flank the donor sequence or modified autosomal dominant disease-related gene sequence, correspond to regions within the targeted locus of autosomal dominant disease-related gene. For example, the corresponding regions within the targeted locus are referred to herein as "target sites". Thus, in one example, a vector that carries a donor or modified autosomal dominant disease-related gene sequence can comprise a donor or modified autosomal dominant disease-related gene sequence flanked by a first and a second homology arm.

A homology arm of the vector that carries a donor or modified autosomal dominant disease-related gene sequence can be of any length that is sufficient to promote a homologous recombination event with a corresponding target site, including for example, 50-100 base pairs, 100-1000 base pairs or at least 5-10, 5-15, 5-20, 5-25, 5-30, 5-35, 5-40, 5-45, 5-50, 5-55, 5-60, 5-65, 5-70, 5-75, 5-80, 5-85, 5-90, 5-95, 5-100, 100-200, or 200-300 base pairs in length or greater.

In one embodiment, the donor template is delivered as a double-stranded DNA. Under such circumstances, the homologous arm may comprise 15-4000 base pairs of each arm. In other embodiments, the donor template is delivered as a single-stranded DNA format. Under such circumstances, the homologous arm may comprise 8-1000 bps of each arm.

A homology arm and a target site "correspond" or are "corresponding" to one another when the two regions share a sufficient level of sequence identity to one another to act as substrates for a homologous recombination reaction. By "homology" is meant DNA sequences that are either identical or share sequence identity to a corresponding sequence. The sequence identity between a given target site and the corresponding homology arm found on the vector that carries a donor or modified autosomal dominant disease-related gene sequence can be any degree of sequence identity that allows for homologous recombination to occur. For example, the amount of sequence identity shared by the homology arm of the vector that carries a donor or modified autosomal dominant disease-related gene sequence (or a fragment thereof) and the target site (or a fragment thereof) should be 100% sequence identity, except the codon-modified region, such that the sequences undergo homologous recombination. Less than 100% sequence identity may be tolerated, provided that the Cas enzyme (Cas 9) cuts only the patient DNA and not the donor template or the patient DNA which is repaired/replaced by the donor template.

Alternatively, donor template (whether codon-modified or not) of a gene of interest or fragment is not integrated into the endogenous disease-related gene. Donor-template may be packaged into an extrachromosomal, or episomal vector (such as AAV vector), which persists in the nucleus in an extrachromosomal state, and offers donor-template delivery and expression without integration into the host genome. Use of extrachromosomal gene vector technologies has been discussed in detail by Wade-Martins R (Methods Mol Biol. 2011; 738:1-17).

The methods and systems of the present disclosure provide for a method for modifying an autosomal dominant disease-related gene in a cell, the method comprising: contacting the cell with at least one type of vector encoding a CRISPR-Cas system directed to a mutant allele of the autosomal dominant disease-related gene, wherein the autosomal dominant disease-related gene relates to an ocular disease, wherein the at least one type of vector comprises: (i) a first sequence encoding a first guide RNA that hybridizes to the autosomal dominant disease-related gene that comprises a single nucleotide polymorphism (SNP); (ii) a second sequence encoding a second guide RNA that hybridizes to an intron of the autosomal dominant disease-related gene; and, (iii) a third sequence encoding a Cas nuclease, wherein the Cas nuclease cleaves or targets only the mutant allele that encodes the autosomal dominant disease-related gene that comprises a single nucleotide polymorphism (SNP). The methods also provide for a method for modifying an autosomal dominant disease-related gene in a cell, the method comprising: contacting the cell with at least one type of vector encoding a CRISPR-Cas system directed to a mutant allele of the autosomal dominant disease-related gene, wherein the autosomal dominant disease-related gene relates to an ocular disease, wherein the at least one type of vector comprises: (i) a first sequence encoding a first guide RNA that hybridizes to the autosomal dominant disease-related gene that comprises a single nucleotide polymorphism (SNP); and, (ii) a second sequence encoding a catalytically defective Cas nuclease (dCas); wherein the Cas nuclease cleaves or targets only the mutant allele that encodes the autosomal dominant disease-related gene comprising a single nucleotide polymorphism (SNP). These methods and systems are referred to herein as CRISPR 3.0.

The methods and systems of the present disclosure also provide for a method for treating an autosomal dominant ocular disease in a subject, the method comprising: administering to the subject a therapeutically effective amount of at least one type of vector encoding a CRISPR-Cas system directed to a mutant allele of an autosomal dominant disease-related gene in the subject, wherein the at least one type of vector comprises: (i) a first sequence encoding a first guide RNA that hybridizes to the autosomal dominant disease-related gene that comprises a single nucleotide polymorphism (SNP); (ii) a second sequence encoding a second guide RNA that hybridizes to an intron of the autosomal dominant disease-related gene; and, (iii) a third sequence encoding a Cas nuclease, wherein the Cas nuclease cleaves or targets only the mutant allele that encodes the autosomal dominant disease-related gene that comprises a single nucleotide polymorphism (SNP). The methods and systems of the present disclosure also provide for a method of treating an autosomal dominant ocular disease in a subject, the method comprising: administering to the subject a therapeutically effective amount of at least one type of vector encoding a CRISPR-Cas system directed to a mutant allele of an autosomal dominant disease-related gene in the subject, wherein the at least one type of vector comprises: (i) a first sequence encoding a first guide RNA that hybridizes to the autosomal dominant disease-related gene that comprises a single nucleotide polymorphism (SNP); and, (ii) a second sequence encoding a catalytically defective Cas nuclease (dCas), wherein the Cas nuclease cleaves or targets only the mutant allele that encodes the autosomal dominant disease-related gene that comprises a single nucleotide polymorphism (SNP). These methods and systems are referred to as CRISPR 3.0.

### Conditions to be Treated and Disease-related Genes

The present methods and systems may be used to prevent, correct, or treat ocular diseases that arise due to the presence of autosomal dominant mutation. Non-limiting examples of ocular diseases include vitelliform macular dystrophy (VMD), such as Best vitelliform macular dystrophy (BVMD) or juvenile-onset vitelliform macular dystrophy, and adult-onset vitelliform macular dystrophy (AVMD); autosomal recessive bestrophinopathy; autosomal dominant vitreoretinochoroidopathy and retinitis pigmentosa (RP). Non-limiting examples of ocular diseases include retinopathies, retinal dystrophies, and retinal degenerative diseases.

The present systems and methods can be used as a gene-editing tool for the correction of the mutation(s) found in any autosomal dominant disease. Thus, the methods of the present disclosure can be used to treat any autosomal dominant disease, including, but not limited to, Acropectoral syndrome, Acute intermittent porphyria, Adermatoglyphia, Albright's hereditary osteodystrophy, Arakawa's syndrome II, Aromatase excess syndrome, Autosomal dominant cerebellar ataxia, Autosomal dominant retinitis pigmentosa, Axenfeld syndrome, Bethlem myopathy, Birt-Hogg-Dubé syndrome, Boomerang dysplasia, Branchio-oto-renal syndrome, Buschke-Ollendorff syndrome, Camurati-Engelmann disease, Central core disease, Collagen disease, Collagenopathy, types II and XI, Congenital distal spinal muscular atrophy, Congenital stromal corneal dystrophy, Costello syndrome, Currarino syndrome, Darier's disease, De Vivo disease, Dentatorubral-pallidoluysian atrophy, Dermatopathia pigmentosa reticularis, DiGeorge syndrome, Doyne honeycomb disease, Dysfibrinogenemia, Familial amyloid polyneuropathy, Familial atrial fibrillation, Familial hypercholesterolemia, Familial male-limited precocious puberty, Feingold syndrome, Felty's syndrome, Flynn-Aird syndrome, Gardner's syndrome, Gillespie syndrome, Gray platelet syndrome, Greig cephalopolysyndactyly syndrome, Hajdu-Cheney syndrome, Hawkinsinuria, Hay-Wells syndrome, Hereditary elliptocytosis, Hereditary hemorrhagic telangiectasia, Hereditary mucoepithelial dysplasia, Hereditary spherocytosis, Holt-Oram syndrome, Huntington's disease, Hypertrophic cardiomyopathy, Hypoalphalipoproteinemia, Hypochondroplasia, Jackson-Weiss syndrome, Keratolytic winter erythema, Kniest dysplasia, Kostmann syndrome, Langer-Giedion syndrome, Larsen syndrome, Liddle's syndrome, Marfan syndrome, Marshall syndrome, Medullary cystic kidney disease, Metachondromatosis, Miller-Dieker syndrome, MOMO syndrome, Monilethrix, Multiple endocrine neoplasia, Multiple endocrine neoplasia type 1, Multiple endocrine neoplasia type 2, Multiple endocrine neoplasia type 2b, Myelokathexis, Myotonic dystrophy, Naegeli-Franceschetti-Jadassohn syndrome, Nail-patella syndrome, Noonan syndrome, Oculopharyngeal muscular dystrophy, Pachyonychia congenital, Pallister-Hall syndrome, PAPA syndrome, Papillorenal syndrome, Parastremmatic dwarfism, Pelger-Huet anomaly, Peutz-Jeghers syndrome, Polydactyly, Popliteal pterygium syndrome, Porphyria cutanea tarda, Pseudoachondroplasia, RASopathy, Reis-Bucklers corneal dystrophy, Romano-Ward syndrome, Rosselli-Gulienetti syndrome, Roussy-Lévy syndrome, Rubinstein-Taybi syndrome, Saethre-Chotzen syndrome, Schmitt Gillenwater Kelly syndrome, Short QT syndrome, Singleton Merten syndrome, Spinal muscular atrophy with lower extremity predominance, Spinocerebellar ataxia, Spinocerebellar ataxia type 6, Spondyloepiphyseal dysplasia congenital, Spondyloperipheral dysplasia, Stickler syndrome, Tietz syndrome, Timothy syndrome, Treacher Collins syndrome, Tuberous sclerosis, Upington disease, Variegate porphyria, Vitelliform macular dystrophy, Von Hippel-Lindau disease, Von Willebrand disease, Wallis-Zieff-Goldblatt syndrome, WHIM syndrome, White sponge nevus, Worth syndrome, Zaspopathy, Zimmermann-Laband syndrome, and Zori-Stalker-Williams syndrome. For example, in the Examples provided in the present disclosure, the inventors present the data performing "Chop" on human kidney cells and iPS cells. These findings confirm the potential of the methods of the present disclosure to be used to prevent, correct, or treat autosomal dominant kidney diseases such as renal angiomyolipomas, medullary cystic kidney disease, or autosomal dominant polycystic kidney disease.

Examples of such ocular diseases also include, but are not limited, autosomal dominant chorioretinal atrophy or degeneration, autosomal dominant cone or cone-rod dystrophy, autosomal dominant congenital stationary night blindness, autosomal dominant Leber congenital amaurosis, autosomal dominant macular degeneration, autosomal dominant ocular-retinal developmental disease, autosomal dominant optic atrophy, autosomal dominant retinitis pigmentosa, autosomal dominant syndromic/systemic diseases with retinopathy, sorsby macular dystrophy, age-related macular degeneration, doyne honeycomb macular disease, and juvenile macular degeneration.

Generally, in the case of retinitis pigmentosa, patients with null rhodopsin mutations have a milder phenotype than those with severe dominant rhodopsin mutations. Even if the normal rhodopsin gene is destroyed together with the mutant one, the supply of the wild-type exon or cDNA (i.e., Stick) is still expected to improve retinal function in the recipient.

The methods of the present disclosure can be used for arresting progression of or ameliorating vision loss associated with retinitis pigmentosa (RP) in the subject.

Vision loss may include decrease in peripheral vision, central (reading) vision, night vision, day vision, loss of color perception, loss of contrast sensitivity, or reduction in visual acuity. The methods of the present disclosure can also be used to prevent, or arrest photoreceptor function loss, or increase photoreceptor function in the subject.

RP is diagnosed in part, through an examination of the retina. The eye exam usually reveals abnormal, dark pigment deposits that streak the retina. Additional tests for diagnosing RP include electroretinogram (ERG) and visual field testing.

Methods for measuring or assessing visual function, retinal function (such as responsiveness to light stimulation), or retinal structure in a subject are well known to one of skill in the art. See, e.g. Kanski's Clinical Ophthalmology: A Systematic Approach, Edition 8, Elsevier Health Sciences, 2015. Methods for measuring or assessing retinal response to light include may include detecting an electrical response of the retina to a light stimulus. This response can be detected by measuring an electroretinogram (ERG; for example, full-field ERG, multifocal ERG, or ERG photostress test), visual evoked potential, or optokinetic nystagmus (see, e.g., Wester et al., Invest. Ophthalmol. Vis. Sci. 48:4542-4548, 2007). Furthermore, retinal response to light may be measured by directly detecting retinal response (for example by use of a microelectrode at the retinal surface). ERG has been extensively described by Vincent et al. Retina, 2013; 33(1):5-12. Thus, methods of the present disclosure can be used to improve visual function, retinal function (such as responsiveness to light stimulation), retinal structure, or any other clinical symptoms or phenotypic changes associated with ocular diseases in subjects afflicted with ocular disease.

In one embodiment, the methods of the present disclosure can be used to prevent the development and progression of autosomal dominant disease. For example, a patient may be a carrier of autosomal dominant mutation, but the phenotypic expression of a disease has not been yet manifested, although the genomic defect has been identified by screening. The methods of the present disclosure may be applied to such patient to prevent the onset of disease.

In addition to being used for the prevention, correctness, or treatment of autosomal dominant diseases, the methods of the present disclosure can be used to prevent, correct, or treat any autosomal recessive diseases. Thus, all the methods described here as applicable to autosomal dominant diseases and autosomal dominant genes or fragments can be adopted for use in the treatment of autosomal recessive diseases.

In further embodiments, the methods of the present disclosure can be used to prevent, correct, or treat ocular diseases that arise due to the presence of autosomal recessive mutation. Examples of such diseases include, but are not limited to, autosomal recessive congenital stationary night, autosomal recessive deafness alone or syndromic, autosomal recessive Leber congenital amaurosis, autosomal recessive optic atrophy, autosomal recessive retinitis pigmentosa, autosomal recessive syndromic/systemic diseases with retinopathy, autosomal recessive usher syndrome, other autosomal recessive retinopathy, autosomal recessive cone or cone-rod dystrophy, autosomal recessive macular degeneration, and autosomal recessive bardet-biedl syndrome.

Mutations in various genes have been identified to give rise to autosomal dominant diseases (such genes are also referred to as autosomal dominant disease-related genes). The methods of the present disclosure can be used to fully or partially correct mutations in such autosomal dominant disease-related genes, resulting in partial or full restoration of wild type.

In all cases where accession numbers are used, the accession numbers refer to one embodiment of the gene which may be used with the methods of the present disclosure. In one embodiment, the accession numbers are NCBI (National Center for Biotechnology Information) reference sequence (RefSeq) numbers.

For example, the autosomal dominant disease-related gene in retinitis pigmentosa may include, but are not limited to, ARL3(NC_000010.11 (102673727..102714433, complement)), BEST1 (e.g., NG_009033.1), CA4 (NG_012050.1), CRX (NG_008605.1), FSCN2 (NG_015964.1), GUCA1B (NG_016216.1), HK1 (NG_012077.1), IMPDH1 (NG_009194.1), KLHL7 (NG_016983.1), NR2E3 (NG_009113.2), NRL (NG_011697.1), PRPF3 (NG_008245.1), PRPF4 (NG_034225.1), PRPF6 (NG_029719.1), PRPF8 (NG_009118.1), PRPF31 (NG_009759.1), PRPH2 (NG_009176.1), RDH12 (NG_008321.1), RHO (NG_009115.1), ROM1 (NG_009845.1), RP1 (NG_009840.1), RP9 (NG_012968.1), RPE65 (NG_008472.1), SEMA4A (NG_027683.1), SNRNP200 (NG_016973.1), SPP2 (NG_008668.1), and TOPORS (NG_017050.1). Genes and mutations causing autosomal dominant retinitis pigmentosa are in detail discussed by Daiger et al. (Cold Spring Harb Perspect Med. 2014 Oct 10;5(10)).

Another type of the autosomal dominant disease-related gene is autosomal dominant chorioretinal atrophy or degeneration-related gene, which may include: PRDM13 (NC_000006.12 (99606774..99615578)), RGR(NG_009106.1), and TEAD1 (NG_021302.1).

Another example of the autosomal dominant disease- related gene is autosomal dominant cone or cone-rod dystrophy-related gene, which can include: AIPL1 (NG_008474.1), CRX (NG_008605.1), GUCA1A (NG_009938.1), GUCY2D (NG_009092.1), PITPNM3 (NG_016020.1), PROM1 (NG_011696.1), PRPH2 (NG_009176.1), RIMS1 (NG_016209.1), SEMA4A (NG_027683.1), and UNC119 (NG_012302.1).

In one embodiment, the autosomal dominant disease-related gene is autosomal dominant congenital stationary night blindness-related gene, including: GNAT1 (NG_009831.1), PDE6B (NG_009839.1), and RHO (NG_009115.1).

In another embodiment, the autosomal dominant disease- related gene is autosomal dominant deafness (alone or syndromic)-related gene such as WSF1(NC_000004.12 (6269850..6303265)).

Another type of the autosomal dominant disease- related gene is autosomal dominant Leber congenital amaurosis-related gene, which may include: CRX(NG_008605.1), (NG_009194.1), and GTX2(NG_008204.1).

Another example of the autosomal dominant disease- related gene is autosomal dominant macular degeneration-related gene, which can include: BEST1(NG_009033.1), C1QTNF5 (NG_012235.1), CTNNA1 (NC_000005.10 (138753396..138935034)), EFEMP1 (NG_009098.1), ELOVL4 (NG_009108.1), FSCN2 (NG_015964.1), GUCA1B (NG_016216.1), HMCN1 (NG_011841.1), IMPG1 (NG_041812.1), OTX2 (NG_008204.1), PRDM13 (NC_000006.12 (99606774..99615578)), PROM1 (NG_011696.1), PRPH2 (NG_009176.1), RP1L1 (NG_028035.1), and TIMP3(NG_009117.1).

In one embodiment, the autosomal dominant disease- related gene is autosomal dominant ocular retinal developmental disease-related gene such as VCAN(NG_012682.1). The accession numbers are provided as specific examples of each gene which may be used with the methods of the disclosure.

In another embodiment, the autosomal dominant disease-related gene is autosomal dominant optic atrophy-related gene, including: MFN2 (NG_007945.1), NR2F1 (NG_034119.1), and OPA1 (NG_011605.1).

In one embodiment, the autosomal dominant disease-related gene is autosomal dominant syndromic/systemic disease with retinopathy-related gene, including: ABCC6 (NG_007558.2), ATXN7 (NG_008227.1), COL11A1 (NG_008033.1), COL2A1 (NG_008072.1), JAG1 (NG_007496.1), KCNJ13 (NG_016742.1), KIF11 (NG_032580.1), MFN2 (NG_007945.1), OPA3 (NG_013332.1), PAX2 (NG_008680.2), TREX1 (NG_009820.1), and VCAN (NG_012682.1).

Another example of the autosomal dominant disease-related gene is autosomal dominant retinopathy-related gene, including: BEST1 (NG_009033.1), CAPN5 (NG_033002.1), CRB1 (NG_008483.2), FZD4 (NG_011752.1), ITM2B (NG_013069.1), LRP5 (NG_015835.1), MAPKAPK3 (NC_000003.12(50611862..50649297)), MIR204 (NR_029621.1), OPN1SW (NG_009094.1), RB1 (NG_009009.1), TSPAN12 (NG_023203.1), and ZNF408 (NC_000011.10 (46700767..46705916).

According to the methods described here, autosomal recessive disease-related gene is corrected and can in-part or fully restore the function of a wild-type gene.

One type of the autosomal recessive disease- related gene is congenital stationary night -related gene, including: CABP4(NG_021211.1), GNAT1(NG_009831.1), GNB3 (NG_009100.1), GPR179(NG_032655.2), GRK1(NC_000013.11(113667279..113671659)), GR M6(NG_008105.1), LRIT3(NG _033249.1), RDH5(NG_008606.1),SAG(NG_009116.1), SLC24 A1(NG_031968.2), and TRPM1(NG_016453.2).

Another type of the autosomal recessive disease- related gene is bardet-biedl syndrome-related gene, including: ADIPOR1 (NC_000001.1 (202940825..202958572, complement)), ARL6 (NG_008119.2), BBIP1 (NG_041778.1), BBS1 (NG_009093.1), BBS2 (NG_009312.1), BBS4 (NG_009416.2), BBS5 (NG_011567.1), BBS7 (NG_009111.1), BBS9 (NG_009306.1), BBS10 (NG_016357.1), BBS12 (NG_021203.1), C8orf37 (NG_032804.1), CEP290 (NG_008417.1), IFT172 (NG_034068.1), IFT27 (NG_034205.1), INPP5E (NG_016126.1), KCNJ13 (NG_016742.1), LZTFL1 (NG_033917.1), MKKS (NG_009109.1), MKS1 (NG_013032.1), NPHP1 (NG_008287.1), SDCCAG8 (NG_027811.1), TRIM32 (NG_011619.1), and TTC8 (NG_008126.1).

One example of the autosomal recessive disease-related gene is cone or cone-rod dystrophy- related gene, including, but not limited to, ABCA4(NG_009073.1), ADAM9 (NG_016335.1), ATF6 (NG_029773.1), C21orf2 (NG_032952.1), C8orf37 (NG_032804.1), CACNA2D4 (NG_012663.1), CDHR1 (NG_028034.1),CERKL (NG_021178.1), CNGA3 (NG_009097.1), CNGB3 (NG_016980.1), CNNM4 (NG_016608.1), GNAT2 (NG_009099.1), KCNV2 (NG_012181.1), PDE6C (NG_016752.1),PDE6H (NG_016859.1), POC1B (NG_041783.1), RAB28 (NG_033891.1), RAX2 (NG_011565.1), RDH5 (NG_008606.1), RPGRIP1 (NG_008933.1), and TTLL5(NG_016974.1).

Another example of the autosomal recessive disease-related gene is deafness (alone or syndromic)-related gene including: CDH23(NG_008835.1), CIB2(NG_033006.1), DFNB31 (NG_016700.1), MYO7A (NG_009086.1), PCDH15 (NG_009191.2), PDZD7 (NG_028030.1), and USH1C(NG_011883.1).

In one embodiment, the autosomal recessive disease-related gene is Leber congenital amaurosis-related gene, including: AIPL1(NG_008474.1), CABP4(NG_021211.1), CEP290 (NG_008417.1), CLUAP1 (NC_000016.10(3500945..3539048)), CRB1 (NG_008483.2), CRX (NG_008605.1), DTHD1 (NG_032962.1), GDF6 (NG_008981.1), GUCY2D (NG_009092.1), IFT140 (NG_032783.1), IQCB1 (NG_015887.1), KCNJ13 (NG_016742.1), LCA5 (NG_016011.1), LRAT (NG_009110.1), NMNAT1 (NG_032954.1),PRPH2 (NG_009176.1), RD3 (NG_013042.1), RDH12 (NG_008321.1), RPE65 (NG_008472.1), RPGRIP1 (NG_008933.1), SPATA7 (NG_021183.1), and TULP1 (NG_009077.1).

In another embodiment, the autosomal recessive disease- related gene is optic atrophy-related gene, including: RTN4IP1(NC_000006.12 (106571028..106630500, complement)), SLC25A46 (NC_000005.10 (110738136..110765161)), and TMEM126A(NG_017157.1).

One example of the autosomal recessive disease- related gene is retinitis pigmentosa-related gene, including: ABCA4 (NG_009073.1), AGBL5 (NC_000002.12 (27051423..27070622)), ARL6 (NG_008119.2), ARL2BP (NG_033905.1), BBS1 (NG_009093.1), BBS2 (NG_009312.1), BEST1 (NG_009033.1), C2orf71 (NG_021427.1), C8orf37 (NG_032804.1), CERKL (NG_021178.1), CLRN1 (NG_009168.1), CNGA1 (NG_009193.1), CNGB1 (NG_016351.1), CRB1 (NG_008483.2), CYP4V2 (NG_007965.1), DHDDS (NG_029786.1), DHX38 (NG_034207.1), EMC1 (NG_032948.1), EYS (NG_023443.2), FAM161A (NG_028125.1), GPR125 (NC_000004.12 (22387374..22516058, complement)), HGSNAT(NG_009552.1), IDH3B (NG_012149.1), IFT140 (NG_032783.1), IFT172 (NG_034068.1), IMPG2 (NG_028284.1), KIAA1549 (NG_032965.1), KIZ (NG_033122.1), LRAT (NG_009110.1), MAK(NG_030040.1), MERTK (NG_011607.1), MVK (NG_007702.1), NEK2 (NG_029112.1), NEUROD1 (NG_011820.1), NR2E3 (NG_009113.2), NRL (NG_011697.1), PDE6A (NG_009102.1), PDE6B (NG_009839.1), PDE6G (NG_009834.1), POMGNT1 (NG_009205.2), PRCD (NG_016702.1), PROM1 (NG_011696.1), RBP3(NG_029718.1), RGR(NG_009106.1), RHO(NG_009115.1), RLBP1(NG_008116.1), RP1(NG_009840.1), RP1L1(NG_028035.1), RPE65(NG_008472.1), SAG(NG_009116.1), SLC7A14(NG_034121.1), SPATA7(NG_021183.1), TTC8(NG_008126.1), TULP1(NG_009077 .1), USH2A(NG_009497.1), ZNF408(NC_000011.10 (46700767..46705916)), and ZNF513 (NG_028219.1).

Another example of the autosomal recessive disease-related gene is syndromic/systemic disease with retinopathy- related gene, including: ABCC6(NG_007558.2), ABHD12 (NG _028119.1), ACBD5 (NG_032960.2), ADAMTS18(NG_031879.1), ADIPOR1 (NC_000001.11(202940825..202958572, complement)), AHI1(NG_008643.1), ALMS1 (NG_011690.1), CC2D2A(NG_013035.1), CEP164(NG_033032.1), CEP290 (NG_008417.1), CLN3(NG_008654.2), COL9A1(NG_011654.1), CSPP1(NG_034100.1), ELOVL4(NG_009108. 1), EXOSC2 (NC_000009.12 (130693760..130704894)), FLVCR1(NG_028131.1), FLVCR1 (NG_028131.1), GNPTG(NG_016985.1), HARS(NG_032158.1), HGSNAT(NG_009552.1), H MX1(NG_013062.2), IFT140(NG_032783.1),INPP5E(NG_016126.1), INVS(NG_008316.1), IQ CB1(NG_015887.1), LAMA1(NG_034251.1), LRP5(NG_015835.1), MKS1(NG_013032.1), M TTP(NG_011469.1), NPHP1(NG_008287.1),NPHP3(NG_008130.1), NPHP4(NG_011724.2), O PA3(NG_013332.1), PANK2(NG_008131.3), PCYT1A(NG_042817.1), PEX1(NG_008341.1), PEX2(NG_008371.1), PEX7(NG_008462.1),PHYH(NG_012862.1), PLK4(NG _041821.1), PNP LA6(NG_013374.1), POC1B(NG_041783.1), PRPS1(NG_008407.1), RDH11(NG_042282.1), RPGRIP1L(NG_008991.2),SDCCAG8(NG_027811.1),SLC25A46(NC_000005.10(110738136. .110765161)), TMEM237(NG_032049.1), TRNT1(NG_041800.1), TTPA(NG_016123.1), TUB( NG_029912.1),TUBGCP4(NG_042168.1), TUBGCP6(NG_032160.1), WDPCP(NG_028144.1), WDR19(NG_031813.1), WFS1(NG_011700.1), and ZNF423(NG_032972.2).

One type of the autosomal recessive disease- related gene is usher syndrome-related gene, including: ABHD12(NG_028119.1), CDH23(NG_008835.1), CEP250 (NC_000020.11 (35455139..35517531)), CIB2(NG_033006.1), CLRN1(NG_009168.1), DFNB31(NG_016700.1 ), GPR98(NG_007083.1), HARS(NG_032158.1),MYO7A(NG_009086.1), PCDH15(NG_00919 1.2), USH1C(NG_011883.1), USH1G(NG_007882.1), and USH2A(NG_009497.1).

Another type of the autosomal recessive disease- related gene is retinopathy-related gene, including: BEST1(NG_009033.1), C12orf65(NG_027517.1), CDH3(NG_009096.1), CNGA3NG_009097.1), CNGB3(NG_016980.1), CNNM4(NG_016608.1), CYP4V2(NG_00796 5.1), LRP5(NG_015835.1), MFRP(NG_012235.1), MVK(NG_007702.1), NBAS (NG_032964.1), NR2E3 (NG_009113.2), OAT(NG_008861.1), PLA2G5(NG_032045.1), PROM1(NG_011696.1), RBP4(NG_009104.1), RGS9(NG_013021.1), RGS9BP (NG_016751.1), and RLBP1 (NG_008116.1).

Yet another type of the autosomal recessive disease- related gene is macular degeneration-related gene, including: ABCA4(NG _009073.1), CFH(NG_007259.1), DRAM2 (NC_000001.11 (111117332..111140216, complement)), IMPG1(NG_041812.1), and MFSD8(NG_008657.1).

In addition to being used for the prevention, correctness, or treatment of autosomal dominant and recessive diseases, the methods of the present disclosure can be used to prevent, correct, or treat any X-linked diseases. Thus, all the methods described here as applicable to autosomal dominant diseases and autosomal dominant genes or fragments can be adopted for use in the treatment of X-linked diseases.

Furthermore, the methods of the present disclosure can be used to prevent, correct, or treat ocular diseases that arise due to the presence of X-linked mutation. Examples of such diseases include: X-linked cone or cone-rod dystrophy, X-linked congenital stationary night blindness, X-linked macular degeneration, X-linked retinitis pigmentosa, X-linked syndromic/systemic diseases with retinopathy, X-linked optic atrophy, and X-linked retinopathies. According to the methods described here, X-linked disease- related gene is corrected and can in part or fully restore the function of a wild-type gene.

One example of the X-linked disease-related gene is cone or cone-rod dystrophy-related gene, including: CACNA1F(NG_009095.2) and RPGR(NG_009553.1).

Another example of the X-linked disease-related gene is congenital stationary night blindness-related gene, including: CACNA1F(NG_009095.2) and NYX(NG_009112.1).

In one embodiment, the X-linked disease-related gene is macular degeneration-related gene, such as RPGR(NG_009553.1).

In another embodiment, the X-linked disease-related gene is optic atrophy-related gene, such as TIMM8A(NG_011734.1).

One type of the X-linked disease-related gene is retinitis pigmentosa-related gene, including: OFD1 (NG_008872.1), RP2 (NG_009107.1), and RPGR (NG_009553.1).

Another type of the X-linked disease-related gene is syndromic/systemic disease with retinopathy-related gene, including: OFD1(NG_008872.1) and TIMM8A(NG_011734.1).

Yet another example of the X-linked disease-related gene is retinopathy-related gene, including, CACNA1F (NG_009095.2), CHM (NG_009874.2), DMD (NG_012232.1), NDP (NG_009832.1), OPN1LW (NG_009105.2), OPN1MW(NG_011606.1), PGK1(NG_008862.1), andRS1(NG_008659.3).

The disease-related gene may be HTRA1.

In another embodiment, the methods of the present disclosure can be used to prevent, correct, or treat diseases that arise due to the presence of mutation in mitochondrial DNA. Such diseases may include, retinopathy caused by the gene mutations in mitochondrial DNA. Examples of genes that may be characterized by the mutation in mitochondrial DNA that causes the development of retinopathy include: MT-ATP6(NC_012920.1 (8527..9207)), MT-TH(NC_012920.1 (12138..12206)), MT-TL1(NC_012920.1 (3230..3304)), MT-TP(NC_012920.1 (15956..16023, complement), and MT-TS2(NC_012920.1 (12207..12265)).

Table 1 provides an exemplary list of diseases and disease-related genes (accompanied with corresponding accession numbers) that can be treated and/or corrected using methods of the present disclosure.

**Table 1: Disease Related Disorders and Genes**

| **Disease Category** | **Mapped and Identified Genes** |
|---|---|
| Bardet-Biedl syndrome, autosomal recessive | ADIPOR1(NC_000001.11 (202940825..202958572, complement)), ARL6(NG_008119.2), BBIP1(NG_041778.1), BBS1(NG _009093.1), BBS2(NG_009312.1), BBS4(NG_009416.2), BBS5(NG_01 1567.1), BBS7(NG_ 009111.1), BBS9(NG_009306.1),BBS10(NG_0163 57.1), BBS12(NG_021203.1), C8orf37(NG_032804.1), CEP290(NG_00 8417.1), IFT172(NG_034068.1), IFT27(NG_034205.1), INPP5E(NG_0 16126.1), KCNJ13(NG_016742.1),LZTFL1(NG_033917.1), MKKS(NG _009109.1), MKS1(NG_013032.1), NPHP1(NG_008287.1), SDCCAG8 (NG_027811.1), TRIM32(NG_011619.1), TTC8(NG_008126.1) |
| Chorioretinal atrophy or degeneration, autosomal dominant | PRDM13(NC_000006.12 (99606774..99615578)), RGR(NG_009106.1), TEAD1(NG_021302.1) |
| Cone or cone-rod dystrophy, autosomal dominant | AIPL1(NG_008474.1), CRX(NG_008605.1), GUCA1A(NG_009938.1), GUCY2D(NG_009092.1), PITPNM3(NG_016020.1), PROM1(NG_01 1696.1), PRPH2(NG_009176.1),RIMS1(NG_016209.1), SEMA4A(NG_ 027683.1), UNC119(NG_012302.1) |
| Cone or cone-rod dystrophy, autosomal recessive | ABCA4(NG_009073.1), ADAM9(NG_016335.1), ATF6(NG_029773.1 ), C21orf2(NG_032952.1), C8orf37(NG_032804.1), CACNA2D4(NG_0 12663.1), CDHR1(NG_028034.1),CERKL(NG_021178.1), CNGA3(NG _009097.1), CNGB3(NG_016980.1), CNNM4(NG_016608.1), GNAT2( NG_009099.1), KCNV2(NG_012181.1), PDE6C(NG_016752.1),PDE6 H(NG_016859.1), POC1B(NG_041783.1), RAB28(NG_033891.1), RA X2(NG_011565.1), RDH5(NG_008606.1), RPGRIP1(NG_008933.1), T TLL5(NG_016974.1) |
| Cone or cone-rod dystrophy, X-linked | CACNA1F(NG_009095.2), RPGR(NG_009553.1) |
| Congenital stationary night blindness, autosomal dominant | GNAT1(NG_009831.1), PDE6B(NG_009839.1), RHO(NG_009115.1) |
| Congenital stationary night blindness, autosomal recessive | CABP4(NG_021211.1), GNAT1(NG_009831.1), GNB3(NG_009100.1) GPR179(NG_032655.2), GRK1(NC_000013.11 (113667279..113671659)), GRM6(NG_008105.1), LRIT3(NG_033249. 1), RDH5(NG_008606.1),SAG(NG_009116.1), SLC24A1(NG_031968. 2), TRPM1(NG_ 016453.2) |
| Congenital stationary night blindness, X-linked | CACNA1F(NG_009095.2), NYX(NG_009112.1) |
| Deafness alone or syndromic, autosomal dominant | WSF1(NC_000004.12 (6269850..6303265)) |
| Deafness alone or syndromic, autosomal recessive | CDH23(NG_008835.1), CIB2(NG_033006.1), DFNB31(NG_016700.1), MYO7A(NG_009086.1), PCDH15(NG_009191.2), PDZD7(NG_02803 0.1), USH1C(NG_011883.1) |
| Leber congenital amaurosis, autosomal dominant | CRX(NG_008605.1), IMPDH1(NG_009194.1), OTX2(NG_008204.1) |
| Leber congenital amaurosis, autosomal recessive | AIPL1(NG_ 008474.1), CABP4(NG_021211.1), CEP290(NG_008417.1) CLUAP1(NC_000016.10 (3500945..3539048)), CRB1(NG_008483.2), CRX(NG_008605.1), DT HD1(NG_032962.1), GDF6(NG_008981.1),GUCY2D(NG_009092.1), I FT140(NG_032783.1), IQCB1(NG_015887.1), KCNJ13(NG_016742.1), LCA5(NG_016011.1), LRAT(NG_009110.1), NMNAT1(NG_032954. 1),PRPH2(NG_009176.1), RD3(NG_013042.1), RDH12(NG_008321.1) , RPE65(NG_008472.1), RPGRIP1(NG_008933.1), SPATA7(NG_0211 83.1), TULP1(NG_009077.1) |
| Macular degeneration, autosomal dominant | BEST1(NG_009033.1), C1QTNF5(NG_012235.1), CTNNA1(NC_0000 05.10 (138753396..138935034)), EFEMP1(NG_009098.1), ELOVL4(NG_009 108.1), FSCN2(NG_015964.1),GUCA1B(NG_016216.1), HMCN1(NG _011841.1), IMPG1(NG 041812.1), OTX2(NG_008204.1), PRDM13(N C_000006.12 (99606774..99615578)), PROM1(NG_011696.1), PRPH2(NG_009176.1 ),RP1L1(NG 028035.1), TIMP3(NG_009117.1) |
| Macular degeneration, autosomal recessive | ABCA4(NG_009073.1), CFH(NG_007259.1), DRAM2(NC_000001.11 (111117332..111140216, complement)), IMPG1(NG_041812.1), MFSD8(NG_008657.1) |
| Macular degeneration, X-linked | RPGR(NG_009553.1) |
| Ocular-retinal developmental disease, autosomal dominant | VCAN(NG_012682.1) |
| Optic atrophy, autosomal dominant | MFN2(NG_007945.1), NR2F1(NG_034119.1), OPA1(NG_011605.1) |
| Optic atrophy, autosomal recessive | RTN4IP1(NC_000006.12 (106571028..106630500, complement)), SLC25A46(NC_000005.10 (110738136..110765161)), TMEM126A(NG_017157.1) |
| Optic atrophy, X-linked | TIMM8A(NG_011734.1) |
| Retinitis pigmentosa, autosomal dominant | ARL3(NC_000010.11 (102673727..102714433, complement)), BEST1(NG_009033.1), CA4(NG_012050.1), CRX(NG_ 008605.1), FSCN2(NG_015964.1), GUCA1B(NG_016216.1), HK1(NG _012077.1), IMPDH1(NG_009194.1),KLHL7(NG_016983.1), NR2E3( NG_009113.2), NRL(NG_011697.1), PRPF3(NG_008245.1), PRPF4(N G_034225.1), PRPF6(NG_029719.1), PRPF8(NG_009118.1), PRPF31( NG_009759.1),PRPH2(NG_009176.1), RDH12(NG_008321.1), RHO(N G_009115.1), ROM1(NG_009845.1), RP1(NG_009840.1), RP9(NG_01 2968.1), RPE65(NG_008472.1), SEMA4A(NG_027683.1), SNRNP200(NG_016973.1), SPP2(NG_008668.1), TOPORS(NG_01705 0.1) |
| Retinitis pigmentosa, autosomal recessive | ABCA4(NG_009073.1), AGBL5(NC_000002.12 (27051423..27070622)), ARL6(NG_008119.2), ARL2BP(NG_033905.1 ), BBS1(NG_009093.1), BBS2(NG_009312.1), BEST1(NG_009033.1), C2orf71(NG_021427.1),C8orf37(NG_032804.1), CERKL(NG_021178. 1), CLRN1(NG_009168.1), CNGA1)NG_009193.1), CNGB1(NG_0163 51.1), CRB1(NG_008483.2), CYP4V2(NG_007965.1),DHDDS(NG _02 9786.1), DHX38(NG_034207.1), EMC1(NG_032948.1), EYS(NG_023 443.2), FAM161A(NG_028125.1), GPR125(NC_000004.12 (22387374..22516058, complement)), HGSNAT(NG_009552.1),IDH3B(NG_012149.1), IFT14 0(NG_032783.1), IFT172(NG_034068.1), IMPG2(NG_028284.1), KIA A1549(NG_032965.1), KIZ(NG_033122.1), LRAT(NG_009110.1), MA K(NG_030040.1),MERTK(NG _ 011607.1), MVK(NG_007702.1), NEK 2(NG_029112.1), NEUROD1(NG_011820.1), NR2E3(NG_009113.2), NRL(NG_011697.1), PDE6A(NG_009102.1), PDE6B(NG_009839.1),P DE6G(NG_009834.1), POMGNT1(NG_009205.2), PRCD(NG_016702. 1), PROM1(NG_011696.1), RBP3(NG_029718.1), RGR(NG_009106.1) RHO(NG_009115.1), RLBP1(NG_008116.1),RP1(NG_009840.1), RP 1L1(NG_028035.1), RPE65(NG_008472.1), SAG(NG_009116.1), SLC 7A14(NG_034121.1), SPATA7(NG_021183.1), TTC8(NG_008126.1), TULP1(NG_009077.1), USH2A(NG_009497.1), ZNF408(NC_000011.10 (46700767..46705916)), ZNF513(NG_028219.1) |
| Retinitis pigmentosa, X-linked | OFD1(NG_008872.1), RP2(NG_009107.1, RPGR(NG_009553.1) |
| Syndromic/systemic diseases with retinopathy, autosomal dominant | ABCC6(NG_007558.2), ATXN7(NG_008227.1), COL11A1(NG_00803 3.1), COL2A1(NG_008072.1), JAG1(NG_007496.1), KCNJ13(NG_016 742.1), KIF11(NG_032580.1),MFN2(NG_007945.1), OPA3(NG_01333 2.1), PAX2(NG_008680.2), TREX1(NG_009820.1), VCAN(NG_01268 2.1) |
| Syndromic/systemic diseases with retinopathy, autosomal recessive | ABCC6(NG_007558.2), ABHD12(NG_028119.1), ACBD5(NG_03296 0.2), ADAMTS18(NG_031879.1), ADIPOR1(NC_000001.11 (202940825..202958572, complement)), AHI1(NG_008643.1), ALMS1(NG_011690.1),CC2D2A( NG_013035.1), CEP164(NG_033032.1), CEP290(NG_008417.1), CLN 3(NG_008654.2), COL9A1(NG_011654.1), CSPP1(NG_034100.1), EL OVL4(NG _ 009108.1), EXOSC2(NC _ 000009.12 (130693760..130704894)), FLVCR1(NG_028131.1), GNPTG(NG_0169 85.1), HARS(NG_032158.1), HGSNAT(NG_009552.1), HMX1(NG_01 3062.2), IFT140(NG_032783.1), INPP5E(NG_016126.1), INVS(NG_008316.1), IQCB1(NG_015887.1), LAMA1(NG_034251.1), LRP5(NG_015835.1), MKS1(NG_013032.1), MTTP(NG_011469.1), NPHP1(NG_008287.1),NPHP3(NG_008130.1), NPHP4(NG_011724.2), OPA3(NG_013332.1), PANK2(NG_008131.3), PCYT1A(NG_042817.1), PEX1(NG_008341.1), PEX2(NG_008371.1), PEX7(NG_008462.1), PHYH(NG_012862.1), PLK4(NG_041821.1), PNPLA6(NG_013374.1), POC1B(NG_041783.1), PRPS1(NG_008407.1), RDH11(NG_042282.1), RPGRIP1L(NG_008991.2),SDCCAG8(NG_027811.1), SLC25A46(N C_000005.10 (110738136..110765161)), TMEM237(NG_032049.1), TRNT1(NG_041 800.1), TTPA(NG_016123.1), TUB(NG_029912.1), TUBGCP4(NG_042168.1), TUBGCP6(NG_032160.1), WDPCP(NG _ 0 28144.1), WDR19(NG_031813.1), WFS1(NG_011700.1), ZNF423(NG _032972.2) |
| Syndromic/systemic diseases with retinopathy, X-linked | OFD1(NG_008872.1), TIMM8A(NG _ 011734.1) |
| Usher syndrome, autosomal recessive | ABHD12(NG_028119.1), CDH23(NG_008835.1), CEP250(NC_000020 .11 (35455139..35517531)), CIB2(NG_033006.1), CLRN1(NG_009168.1), DFNB31(NG 016700.1), GPR98(NG_007083.1), HARS(NG_032158.1 ), MYO7A(NG_009086.1), PCDH15(NG_009191.2), USH1C(NG_01188 3.1), USH1G(NG_007882.1), USH2A(NG_009497.1) |
| Other retinopathy, autosomal dominant | BEST1(NG_009033.1), CAPN5(NG_033002.1), CRB1(NG_008483.2), FZD4(NG_011752.1), ITM2B(NG_013069.1), LRP5(NG_015835.1), M APKAPK3(NC_000003.12 (50611862..50649297)), MIR204(NR_029621.1), OPN1SW(NG_009094.1), RB1(NG_009009.1) , TSPAN12(NG_023203.1), ZNF408(NC_000011.10 (46700767..46705916)) |
| Other retinopathy, autosomal recessive | BEST1(NG_009033.1), C12orf65(NG_027517.1), CDH3(NG_009096.1 ), CNGA3(NG_009097.1), CNGB3(NG_016980.1), CNNM4(NG_0166 08.1), CYP4V2(NG_007965.1), LRP5(NG_015835.1), MFRP(NG_012235.1), MVK(NG_007702.1), N BAS(NG_032964.1), NR2E3(NG_009113.2), OAT(NG_008861.1), PL A2G5(NG_032045.1), PROM1(NG_011696.1), RBP4(NG_009104.1), RGS9(NG_013021.1),RGS9BP(NG_016751.1), RLBP1(NG_008116.1) |
| Other retinopathy, mitochondrial | MT-ATP6(NC_012920.1 (8527..9207)), MT-TH(NC_012920.1 (12138..12206)), MT-TL1(NC_012920.1 (3230..3304)), MT-TP(NC_012920.1 (15956..16023, complement)), MT-TS2(NC_012920.1 (12207..12265)) |
| Other retinopathy, X-linked | CACNA1F(NG_009095.2), CHM(NG_009874.2), DMD(NG_012232.1) , NDP(NG_009832.1), OPN1LW(NG_009105.2), OPN1MW(NG_0116 06.1), PGK1(NG_008862.1), RS1(NG_008659.3) |

The methods of the present disclosure can also be used to prevent, correct, or treat cancers that arise due to the presence of mutation in a tumor suppressor gene. Examples of tumor suppression genes include: retinoblastoma susceptibility gene (RB) gene, p53 gene, deleted in colon carcinoma (DCC) gene, adenomatous polyposis coli (APC) gene, p16, BRCA1, BRCA2, MSH2, and the neurofibromatosis type 1 (NF-1) tumor suppressor gene (Lee at al. Cold Spring Harb Perspect Biol. 2010 Oct; 2(10)).

Tumor suppressor genes are genes that, in their wild-type alleles, express proteins that suppress abnormal cellular proliferation. When the gene coding for a tumor suppressor protein is mutated or deleted, the resulting mutant protein or the complete lack of tumor suppressor protein expression may fail to correctly regulate cellular proliferation, and abnormal cellular proliferation may take place, particularly if there is already existing damage to the cellular regulatory mechanism. A number of well-studied human tumors and tumor cell lines have been shown to have missing or nonfunctional tumor suppressor genes. Thus, a loss of function or inactivation of tumor suppressor genes may play a central role in the initiation and/or progression of a significant number of human cancers.

The methods of the present disclosure may be used to treat patients at a different stage of the disease (e.g. early, middle or late). The present methods may be used to treat a patient once or multiple times. Thus, the length of treatment may vary and may include multiple treatments.

As discussed in the present disclosure, the methods or the present disclosure can be used for correcting or treating autosomal dominant ocular disease in a subject.

For example, the "Chop" step involves deletion of both the mutant copy of the autosomal dominant ocular disease-related gene that is to be corrected, and/or the endogenous wild-type copy of the same gene in a patient afflicted with autosomal dominant ocular disease. Thus, the "Chop" step results in complete or partial loss of both mutant and/or wild-type activity of a gene. The autosomal dominant ocular disease-related gene is then corrected using the "Stick" step, which involves the introduction of a sequence encoding a modified autosomal dominant ocular disease-related gene or fragment. The modified, autosomal dominant ocular disease-related gene sequence can be modified in such a way that it is not recognized (unrecognizable) by sgRNA, which targets the wild-type or mutant form of the gene (non-codon-modified form of the gene). This modification renders the codon-modified donor template resistant to the Cas-family nuclease.

### Vectors

The present system can be delivered to the subject or cell using one or more recombinant adeno-associated viral (AAV) vectors (e.g., 1, 2, 3, 4, 5, 6, 7, 8, 9, or more AAV vectors). One or more gRNAs (e.g., sgRNAs) can be packaged into single (one) recombinant AAV vector. The recombinant AAV vector may also include codon-modified autosomal dominant ocular disease-related gene sequence (donor template). A Cas-family nuclease can be packaged into the same, or alternatively separate recombinant AAV vectors.

The method described here also provides for correcting autosomal dominant ocular disease in a subject, comprising administering to said subject by injection a therapeutically effective amount of a recombinant AAV virus encoding the present system.

As the carrying capacity of AAV may pose challenges, two or more AAV vectors can be used simultaneously. For example, a Cas family nuclease may be packaged into a different AAV vectors. Furthermore, sequences encoding sgRNA(s), (codon-modified autosomal dominant disease-related gene or fragment,) and a Cas family nuclease can each be packaged into a separate AAV vector.

In the case of RP treatment, the methods of the present disclosure may comprise: administering to a subject by injection a therapeutically effective amount of a (1) recombinant AAV virus encoding a nucleic acid sequence comprising a CRISPR system polynucleotide sequence, wherein the polynucleotide sequence comprises: (i) two guide RNA sequences that hybridize to mutant and wild type RHO sequences; (ii) a second sequence encoding a codon-modified RHO gene or fragment, where the mutation(s) of the endogenous RHO gene has been corrected and the modified RHO gene or fragment cannot be recognized by one or more sgRNA sequences that hybridize to the mutant and wild type RHO gene sequence; and (2) a second recombinant AAV virus encoding a Cas family enzyme.

Generally, co-expression of a Cas-family enzyme and an autosomal dominant disease-related gene-specific gRNAs in ocular cells, may lead to truncation of the autosomal dominant disease-related gene, thereby preventing the expression of the disease-causing mutant allele (and/or the wild-type (wt) allele).

Codon-modified cDNA of the autosomal dominant disease-related gene may or may not be supplied to ocular cells. The coding sequence of the autosomal dominant disease-related gene may or may not modified in such a way that is resistant to the CRISPR/Cas system. This strategy results in the expression of the autosomal dominant disease-related gene, which can restore or correct the function of the autosomal dominant disease-related gene or fragment after the deletion of endogenous gene(s) or fragments.

The codon-modified cDNA (donor-template) may be modified in such a way as to render it unrecognizable by the gRNA(s) used to target either mutant and wt disease-related allele(s)/gene(s). Thus, mutations need to be introduced into a donor-template gene or fragment to avoid this donor-template gene or fragment being recognized by gRNA(s) and consequently degraded by Cas enzyme (for example a Cas9 nuclease) which has been introduced in cells. This can be accomplished by introducing a wobble base into donor-template, thus making sure that the change in DNA results in a silent mutation, leaving the expression product of wt gene intact. The term "wobble base" as used in the present disclosure refers to a change in a one or more nucleotide bases of a reference nucleotide sequence wherein the change does not change the sequence of the amino acid coded by the nucleotide relative to the reference sequence.

The number of wobble bases that need to be introduced into donor-template may range from about 1-30, about 1-20, about 2-19, about 3-18, about 4-17, about 5-16, about 6-15, about 7-14, about 8-13, about 9-12, about 10-11, about 9, about 8, about 7, about 6., or about 5. Additionally, given the numerous software applications available for in-silico predictive modeling of gRNAs, one can perform in-silico analysis to test whether codon-modified donor-template would be recognized by sgRNA. An example of publicly available CRISPR sgRNA tool can be found at http://www.genscript.com/gRNA-design-tool.html: retrieved April 30, 2016.

Alternatively, if the method deletes, destroys, or truncates only mutated form of a gene or a fragment (e.g., a mutant allele), and leave the wild type form (e.g., a wildtype allele) intact, donor template or wild type gene sequence that is supplemented to the cells or a patient may not be codon-modified. Under such circumstances, gRNA(s) used as part of the CRISPR components would be designed to recognize and target only the mutated form of a disease-related gene (and not recognize and target a wild type form of said gene).

The methods of the present disclosure have been applied to various genes, including PDE6A, EFEMP1, mouse Rhodopsin (RHO), and human RHO genes. RP can be caused by autosomal recessive mutations in the PDE6A gene, or autosomal dominant mutations in RHO gene. Mutations in EFEMP1 are responsible for autosomal dominant Malattia Leventinese (ML) and Doyne honeycomb retinal dystrophy (DHRD). Moreover, the methods have been applied to various cell types, including, but not limited to, mouse retina cells as well as human iPS cells. Additionally, the methods described here have also been applied *in vivo* using a mouse model of ocular disease. Thus, methods of the present disclosure can be applied to both animal as well as human subjects.

Furthermore, methods of the present disclosure that have been applied to specific gene-humanized mouse model as well as patient-derived cells allowing for determining the efficiency and efficacy of designed sgRNA and site-specific recombination frequency in human cells, which can be then used as a guide in a clinical setting.

In one embodiment, the "ChopStick" system comprises the following components: two recombinant AAV vectors: the first carrying a polynucleotide encoding the Cas9 enzyme to "Chop" the mutant and/or native rhodopsin genes, and the second carrying a nucleotide encoding the codon-modified human rhodopsin cDNA to "Stick" the normal rhodopsin back into the patient. The codon-modified or genetically engineered human rhodopsin sequence, which is driven by the CBh promoter is resistant to destruction by the gene-editing enzyme, rescues the patient's phenotype.

In one embodiment, the present method provides at least 50% rhodopsin levels from the CBh promoter-driven codon-modified RHO cDNA, which are sufficient to improve survival. In another embodiment, there is not an excessive amount of rhodopsin expressed using the codon-modified *RHO* donor sequence.

For studies using human and patient-derived cells, AAV2 vector may be used as a backbone vector for all the constructs, as it has been shown that AAV2 may transduce human iPS efficiently (Mitsui K et al. Biochem Biophys Res Commun. 2009 Oct 30;388(4):711-7; Deyle DR et al. Mol Ther. 2012 Jan;20(1):204-13; and Deyle DR et al. Nucleic Acids Res. 2014 Mar;42(5):3119-24). However, a variety of other AAV vectors may also be used to carry out the methods of the present disclosure.

The degree of improvement of the autosomal dominant disease by the present methods can vary. For example, the present methods may restore about 20%, about 30%, about 40%, greater than 10%, greater than 20%, greater than 30%, greater than 40%, greater than 50%, greater than 60%, greater than 70%, greater than 80%, or greater than 90%, of the autosomal dominant disease-related gene expression, of the normal levels of the gene product in a control subject, which may be age and sex matched.

In certain embodiments, expression of a wild-type gene (e.g., rhodopsin) can be observed in about 2 weeks following administration to a subject and/or cells. Expression may be maintained for unlimited period of time in nondividing somatic cells (e.g., photoreceptors, neuron cells, muscle cells, etc.). In one embodiment, expression of wild-type rhodopsin is observed in about 3 days, in about 1 week, in about 3 weeks, in about 1 month, in about 2 months, from about 1 week to about 2 weeks, or within different time-frames.

According to the various embodiments of the present disclosure, a variety of known viral constructs may be used to deliver the present system (such as Cas-family nuclease, sgRNA(s), codon-modified wild-type gene (also referred to as codon-modified donor template), donor template, etc.) to the targeted cells and/or a subject. Nonlimiting examples of such recombinant viruses include recombinant adeno-associated virus (AAV), recombinant adenoviruses, recombinant lentiviruses, recombinant retroviruses, recombinant poxviruses, and other known viruses in the art, as well as plasmids, cosmids, and phages. Options for gene delivery viral constructs are well known (see, e.g., Ausubel et al., Current Protocols in Molecular Biology, John Wiley & Sons, New York, 1989; Kay, M. A., et al., 2001 Nat. Medic. 7(1):33-40; and Walther W. and Stein U., 2000 Drugs, 60(2): 249-71).

Additionally, delivery vehicles such as nanoparticle- and lipid-based mRNA or protein delivery systems can be used as an alternative to AAV vectors. Further examples of alternative delivery vehicles include lentiviral vectors, ribonucleoprotein (RNP) complexes, lipid-based delivery system, gene gun, hydrodynamic, electroporation or nucleofection microinjection, and biolistics. Various gene delivery methods are discussed in detail by Nayerossadat et al. (Adv Biomed Res. 2012; 1: 27) and Ibraheem et al. (Int J Pharm. 2014 Jan 1;459(1-2):70-83).

The present methods may utilize adeno-associated virus (AAV) mediated genome engineering. AAV vectors possess a broad host range; transduce both dividing and non-dividing cells *in vitro* and *in vivo* and maintain high levels of expression of the transduced genes. Viral particles are heat stable, resistant to solvents, detergents, changes in pH, temperature, and can be concentrated on CsCl gradients. AAV is not associated with any pathogenic event, and transduction with AAV vectors has not been found to induce any lasting negative effects on cell growth or differentiation. In contrast to other vectors, such as lentiviral vectors, AAVs lack integration machinery and have been approved for clinical use (Wirth et al. Gene. 2013 Aug 10;525(2): 162-9).

The single-stranded DNA AAV viral vectors have high transduction rates in many different types of cells and tissues. Upon entering the host cells, the AAV genome is converted into double-stranded DNA by host cell DNA polymerase complexes and exist as an episome. In non-dividing host cells, the episomal AAV genome can persist and maintain long-term expression of a therapeutic transgene. (J Virol. 2008 Aug; 82(16): 7875-7885).

AAV vectors and viral particles of the present disclosure may be employed in various methods and uses. In one embodiment, a method encompasses delivering or transferring a heterologous polynucleotide sequence into a patient or a cell from a patient and includes administering a viral AAV particle, a plurality of AAV viral particles, or a pharmaceutical composition of a AAV viral particle or plurality of AAV viral particles to a patient or a cell of the patient, thereby delivering or transferring a heterologous polynucleotide sequence into the patient or cell of the patient.

In another embodiment, the method is for treating a patient deficient or in need of protein expression or function, or in need of reduced expression or function of an endogenous protein (e.g., an undesirable, aberrant or dysfunctional protein), that includes providing a recombinant AAV viral particle, a plurality of recombinant AAV viral particles, or a pharmaceutical composition of a recombinant AAV viral particle or plurality of AAV viral particles; and administering the recombinant AAV viral particle, plurality of recombinant AAV viral particles, or pharmaceutical composition of AAV viral particle or plurality of AV viral particles to the patient, where the heterologous polynucleotide sequence is expressed in the patient, or wherein the heterologous polynucleotide sequence encodes one or more sgRNA(s) that reduces and or deletes endogenous DNA segment (e.g. , an undesirable, aberrant or dysfunctional DNA segment) in the patient, and where the heterologous polynucleotide sequence encodes a codon modified gene or fragment thereof that is not recognizable by one or more sgRNA(s) used to reduce and or delete endogenous DNA segment.

The characterization of new AAV serotypes has revealed that they have different patterns of transduction in diverse tissues. AAV viral vectors may be selected from among any AAV serotype, including, without limitation, AAV1, AAV2, AAV3, AAV4, AAV5, AAV6, AAV7, AAV8, AAV9, AAV10 or other known and unknown AAV serotypes. In certain embodiment, AAV2 and/or AAV8 are used.

The term AAV covers all subtypes, serotypes and pseudotypes, and both naturally occurring and recombinant forms, except where required otherwise. Pseudotyped AAV refers to an AAV that contains capsid proteins from one serotype and a viral genome of a second serotype.

To minimize the intensity and duration of Cas9 expression and potential off-targeting effects, self-excisional AAV-Cas9 vectors have also been generated, which have the ability to self-inactivate Cas9 expression shortly after Cas9 production. This approach comprises flanking the Cas9 gene with two sgRNA-Y1 target sites (similar to loxP sites in Cre recombinase system) to terminate Cas9 own expression (as shown in FIG. 9). It is anticipated that the amount of Cas9 enzyme present (before it terminates itself) is still sufficient to cut the desired locus (such as Rho or PDE6A locus for example). The design of self-inactivating recombinant AAV vectors (see FIG. 9) enables the inventors to control the amount and duration of Cas9 expression in target cells, and can prevent the unwanted off-target effects due to excessive expression of Cas9 protein.

Vectors of the present disclosure can comprise any of a number of promoters known to the art, wherein the promoter is constitutive, regulatable or inducible, cell type specific, tissue-specific, or species specific. In addition to the sequence sufficient to direct transcription, a promoter sequence of the invention can also include sequences of other regulatory elements that are involved in modulating transcription (e.g., enhancers, kozak sequences and introns). Many promoter/regulatory sequences useful for driving constitutive expression of a gene are available in the art and include, but are not limited to, for example, CMV (cytomegalovirus promoter), EF1a (human elongation factor 1 alpha promoter), SV40 (simian vacuolating virus 40 promoter), PGK (mammalian phosphoglycerate kinase promoter), Ubc (human ubiquitin C promoter), human beta-actin promoter, rodent beta-actin promoter, CBh (chicken beta-actin promoter), CAG (hybrid promoter contains CMV enhancer, chicken beta actin promoter, and rabbit beta-globin splice acceptor), TRE (Tetracycline response element promoter), H1 (human polymerase III RNA promoter), U6 (human U6 small nuclear promoter), and the like. Moreover, inducible and tissue specific expression of a RNA, transmembrane proteins, or other proteins can be accomplished by placing the nucleic acid encoding such a molecule under the control of an inducible or tissue specific promoter/regulatory sequence. Examples of tissue specific or inducible promoter/regulatory sequences which are useful for this purpose include, but are not limited to, the rhodopsin promoter, the MMTV LTR inducible promoter, the SV40 late enhancer/promoter, synapsin 1 promoter, ET hepatocyte promoter, GS glutamine synthase promoter and many others. Various commercially available ubiquitous as well as tissue-specific promoters can be found at http://www.invivogen.com/prom-a-list. In addition, promoters which are well known in the art can be induced in response to inducing agents such as metals, glucocorticoids, tetracycline, hormones, and the like, are also contemplated for use with the invention. Thus, it will be appreciated that the present disclosure includes the use of any promoter/regulatory sequence known in the art that is capable of driving expression of the desired protein operably linked thereto.

Vectors according to the present disclosure can be transformed, transfected or otherwise introduced into a wide variety of host cells. Transfection refers to the taking up of a vector by a host cell whether or not any coding sequences are in fact expressed. Numerous methods of transfection are known to the ordinarily skilled artisan, for example, lipofectamine, calcium phosphate co-precipitation, electroporation, DEAE-dextran treatment, microinjection, viral infection, and other methods known in the art. Transduction refers to entry of a virus into the cell and expression (e.g., transcription and/or translation) of sequences delivered by the viral vector genome. In the case of a recombinant vector, "transduction" generally refers to entry of the recombinant viral vector into the cell and expression of a nucleic acid of interest delivered by the vector genome.

The method of treating an autosomal dominant ocular disease in a patient can comprise administering to the patient an effective concentration of a composition comprising any of the recombinant AAVs described herein and a pharmaceutically acceptable carrier. In one embodiment, an effective concentration of virus is 1×10⁶ - 11×10¹³ GC/ml (genome copies/ml). The range of viral concentration effective for the treatment can vary depending on factors including, but not limited to, the specific mutation, patient's age, and other clinical parameters.

Recombinant AAV vectors(s) can be produced *in vitro,* prior to administration into a patient. Production of recombinant AAV vectors and their use in *in vitro* and *in vivo* administration has been discussed in detail by Gray et al. (Curr. Protoc. Neurosci. 2011 Oct, Chapter: Unit 4.17).

The recombinant AAV containing the desired recombinant DNA can be formulated into a pharmaceutical composition intended for subretinal or intravitreal injection. Such formulation involves the use of a pharmaceutically and/or physiologically acceptable vehicle or carrier, particularly one suitable for administration to the eye, e.g., by subretinal injection, such as buffered saline or other buffers, e.g., HEPES, to maintain pH at appropriate physiological levels, and, optionally, other medicinal agents, pharmaceutical agents, stabilizing agents, buffers, carriers, adjuvants, diluents, etc. For injection, the carrier will typically be a liquid. Exemplary physiologically acceptable carriers include sterile, pyrogen-free water and sterile, pyrogen-free, phosphate buffered saline.

In one embodiment, the carrier is an isotonic sodium chloride solution. In another embodiment, the carrier is balanced salt solution. In one embodiment, the carrier includes tween. If the virus is to be stored long-term, it may be frozen in the presence of glycerol or Tween-20.

In another embodiment, the pharmaceutically acceptable carrier comprises a surfactant, such as perfluorooctane (Perfluoron liquid). In certain embodiments, the pharmaceutical composition described above is administered to the subject by subretinal injection. In other embodiments, the pharmaceutical composition is administered by intravitreal injection. Other forms of administration that may be useful in the methods described herein include, but are not limited to, direct delivery to a desired organ (e.g., the eye), oral, inhalation, intranasal, intratracheal, intravenous, intramuscular, subcutaneous, intradermal, and other parental routes of administration. Additionally, routes of administration may be combined, if desired.

In certain embodiments, the route of administration is subretinal injection or intravitreal injection.

### EXAMPLES

### Surveyor assay

Surveyor mutation detection assay provides a simple and robust method to detect mutations and polymorphisms in DNA mixture. The key component of the kit is a Surveyor Nuclease, a member of the CEL family of mismatch-specific nucleases derived from celery. The Surveyor Nuclease recognizes and cleaves mismatches due to the presence of single nucleotide polymorphisms (SNPs) or small insertions or deletions.

The Surveyor nuclease cleaves with high specificity at the 3' side of any mismatch site in both DNA strands, including all base substitutions and insertion/deletions up to at least 12 nucleotides. The Surveyor nuclease technology involves four steps: (i) PCR to amplify target DNA from the cell or tissue samples underwent Cas9 nuclease-mediated cleavage; (ii) hybridization to form heteroduplexes between affected and unaffected DNA (Because the affected DNA sequence is different from the affected, a bulge structure resulted from the mismatch can form after denature and renature); (iii) treatment of annealed DNA with a Surveyor nuclease to cleave heteroduplexes (*i.e.,* cut the bulges); and (iv) analysis of digested DNA products using the detection/separation platform of choice, for instance, agarose gel electrophoresis. The Cas9 nuclease-mediated cleavage efficacy can be estimated by the ratio of Surveyor nuclease-digested DNA to undigested DNA. The technology is highly sensitive, capable of detecting rare mutants present at as low as 1 in 32 copies. Surveyor mutation assay kits are commercially available from Integrated DNA Technologies (IDT), Coraville, IA.

### RFLP analysis

Restriction fragment length polymorphism (RFLP) analysis is a technique well-known to those skilled in the art. RFLP exploits variations in homologous DNA sequences. The basic technique for detecting RFLPs involves fragmenting a sample of DNA by a restriction enzyme, which can recognize and cut DNA wherever a specific short sequence is present, in a process known as a restriction digestion. The resulting DNA fragments are then separated by length with agarose gel electrophoresis for analysis. For the detection of donor-template replacement (also known as gene-correction), one or multiple kinds of additional restriction enzyme sites are introduced into the donor template by codon-modification, without affecting the overall length. After using PCR to amplify the target DNA sequence from tissue samples, the PCR amplicon can be evaluated by the aforementioned restriction enzyme(s) for the detection of the samples that have undergone gene correction.

The following examples of specific aspects for carrying out the present invention are offered for illustrative purposes only and are not intended to limit the scope of the present invention in any way.

### Example 1. ChopStick AAV Gene Therapy Strategy Evaluation

The present Example outlines the strategy behind ChopStick AAV gene therapy. The ChopStick Strategy is also referred to herein as CRISPR 2.0. The approach is based on using a gene-editing enzyme with one or more unique single guide RNA (sgRNA) sequences that target both mutant and wild type forms of rhodopsin for destruction. This initial step is then followed by supplying a wild-type codon modified rhodopsin cDNA to the cells. A significant advantage of this system is that the codon modified rhodopsin cDNA is not recognizable by the sgRNA(s) and thus is resistant to the cleavage by the nuclease.

As shown in FIG. 1, the "ChopStick" system described here is packaged into two recombinant AAV vectors (Fig. 1A). The first vector carries the polynucleotide sequence encoding the Cas9 enzyme (SEQ ID NO: 17), which is able to "chop" the mutant and native rhodopsin genes, while the second vector contains a polynucleotide encoding the codon-modified human rhodopsin to "stick" the normal rhodopsin back into the patient. The codon-modified engineered human rhodopsin sequence, which in this example is driven by the CBh promoter (SEQ ID NO: 10), is resistant to destruction by the gene-editing enzyme (Cas9 in this instance), and allows for the rescue of patient's phenotype. In addition to carrying a codon-modified engineered human rhodopsin sequence, the second vector carries a two single guide RNAs (sgRNA1 and sgRNA2) which act as a guide to define the target site to introduce DNA double-stranded break and thus act as a homing device for directing the Cas9 nuclease. Each pair of recombinant AAV vectors can be used to target rhodopsin genes. The codon-modified sequence is shown in FIG. 1B section II. Each sgRNA targeting site comprises four mismatches which are underlined.

AAV has a packaging capacity of 4.5-4.9Kb. Since the coding sequence of spCas9 is ~4.2Kb and the two inverted terminal repeat (ITRs) of AAV is ~0.3 Kb, there is about 0.4 Kb of space for promoter and poly-adenine termination signal. The inventors of the present disclosure used a 173 bp short CMV promoter and a 50 bp synthetic poly-adenine signal to construct the Cas AAV vector. The detailed sequence is listed and all of the components of the recombinant AAV vector are shown below: **Underline and bold:** ITR (SEQ ID NO: 13); Underlined: short CMV promoter; **Bold:** Flag tag (SEQ ID NO: 15) and SV40 NLS (SEQ ID NO: 16); UPPERCASE: spCas9 CDS (SEQ ID NO: 17); Framed : synthetic poly A site

(SPA)ggccttaattaggctgcgcgctcgctcgctcactgaggccgcccgggcaaagcccgggcgtcgggcgacctttggtcgccc
ggcctcagtgagcgagcgagcgcgcagagagggagtggccaactccatcactaggggttccttgtagttaatgattaacccgccatg
ctacttatctacgtagccatgctctaggaagatccactcacggggatttccaagtctccaccccattgacgtcaatgggagtttgttttggcacc
aaaatcaacgggactttccaaaatgtcgtaataaccccgccccgttgacgcaaatgggcggtaggcgtgtacggtgggaggtctatataag
cagagctcgtttagtgaaccgtgctagcatggactataaggaccacgacggagactacaaggatcatgatattgattacaaagacga
tgacgataagatggccccaaagaagaagcggaaggtcggtATCCACGGAGTCCCAGCAGCCGACAAGAA
GTACAGCATCGGCCTGGACATCGGCACCAACTCTGTGGGCTGGGCCGTGATCACCG
ACGAGTACAAGGTGCCCAGCAAGAAATTCAAGGTGCTGGGCAACACCGACCGGCAC
AGCATCAAGAAGAACCTGATCGGAGCCCTGCTGTTCGACAGCGGCGAAACAGCCGA
GGCCACCCGGCTGAAGAGAACCGCCAGAAGAAGATACACCAGACGGAAGAACCGG
ATCTGCTATCTGCAAGAGATCTTCAGCAACGAGATGGCCAAGGTGGACGACAGCTT
CTTCCACAGACTGGAAGAGTCCTTCCTGGTGGAAGAGGATAAGAAGCACGAGCGGC
ACCCCATCTTCGGCAACATCGTGGACGAGGTGGCCTACCACGAGAAGTACCCCACC
ATCTACCACCTGAGAAAGAAACTGGTGGACAGCACCGACAAGGCCGACCTGCGGCT
GATCTATCTGGCCCTGGCCCACATGATCAAGTTCCGGGGCCACTTCCTGATCGAGGG
CGACCTGAACCCCGACAACAGCGACGTGGACAAGCTGTTCATCCAGCTGGTGCAGA
CCTACAACCAGCTGTTCGAGGAAAACCCCATCAACGCCAGCGGCGTGGACGCCAAG
GCCATCCTGTCTGCCAGACTGAGCAAGAGCAGACGGCTGGAAAATCTGATCGCCCA
GCTGCCCGGCGAGAAGAAGAATGGCCTGTTCGGAAACCTGATTGCCCTGAGCCTGG
GCCTGACCCCCAACTTCAAGAGCAACTTCGACCTGGCCGAGGATGCCAAACTGCAG
CTGAGCAAGGACACCTACGACGACGACCTGGACAACCTGCTGGCCCAGATCGGCGA
CCAGTACGCCGACCTGTTTCTGGCCGCCAAGAACCTGTCCGACGCCATCCTGCTGAG

CGACATCCTGAGAGTGAACACCGAGATCACCAAGGCCCCCCTGAGCGCCTCTATGA
TCAAGAGATACGACGAGCACCACCAGGACCTGACCCTGCTGAAAGCTCTCGTGCGG
CAGCAGCTGCCTGAGAAGTACAAAGAGATTTTCTTCGACCAGAGCAAGAACGGCTA
CGCCGGCTACATTGACGGCGGAGCCAGCCAGGAAGAGTTCTACAAGTTCATCAAGC
CCATCCTGGAAAAGATGGACGGCACCGAGGAACTGCTCGTGAAGCTGAACAGAGAG
GACCTGCTGCGGAAGCAGCGGACCTTCGACAACGGCAGCATCCCCCACCAGATCCA
CCTGGGAGAGCTGCACGCCATTCTGCGGCGGCAGGAAGATTTTTACCCATTCCTGAA
GGACAACCGGGAAAAGATCGAGAAGATCCTGACCTTCCGCATCCCCTACTACGTGG
GCCCTCTGGCCAGGGGAAACAGCAGATTCGCCTGGATGACCAGAAAGAGCGAGGA
AACCATCACCCCCTGGAACTTCGAGGAAGTGGTGGACAAGGGCGCTTCCGCCCAGA
GCTTCATCGAGCGGATGACCAACTTCGATAAGAACCTGCCCAACGAGAAGGTGCTG
CCCAAGCACAGCCTGCTGTACGAGTACTTCACCGTGTATAACGAGCTGACCAAAGT
GAAATACGTGACCGAGGGAATGAGAAAGCCCGCCTTCCTGAGCGGCGAGCAGAAA
AAGGCCATCGTGGACCTGCTGTTCAAGACCAACCGGAAAGTGACCGTGAAGCAGCT
GAAAGAGGACTACTTCAAGAAAATCGAGTGCTTCGACTCCGTGGAAATCTCCGGCG
TGGAAGATCGGTTCAACGCCTCCCTGGGCACATACCACGATCTGCTGAAAATTATCA
AGGACAAGGACTTCCTGGACAATGAGGAAAACGAGGACATTCTGGAAGATATCGTG
CTGACCCTGACACTGTTTGAGGACAGAGAGATGATCGAGGAACGGCTGAAAACCTA
TGCCCACCTGTTCGACGACAAAGTGATGAAGCAGCTGAAGCGGCGGAGATACACCG
GCTGGGGCAGGCTGAGCCGGAAGCTGATCAACGGCATCCGGGACAAGCAGTCCGGC
AAGACAATCCTGGATTTCCTGAAGTCCGACGGCTTCGCCAACAGAAACTTCATGCAG
CTGATCCACGACGACAGCCTGACCTTTAAAGAGGACATCCAGAAAGCCCAGGTGTC
CGGCCAGGGCGATAGCCTGCACGAGCACATTGCCAATCTGGCCGGCAGCCCCGCCA
TTAAGAAGGGCATCCTGCAGACAGTGAAGGTGGTGGACGAGCTCGTGAAAGTGATG
GGCCGGCACAAGCCCGAGAACATCGTGATCGAAATGGCCAGAGAGAACCAGACCA
CCCAGAAGGGACAGAAGAACAGCCGCGAGAGAATGAAGCGGATCGAAGAGGGCAT
CAAAGAGCTGGGCAGCCAGATCCTGAAAGAACACCCCGTGGAAAACACCCAGCTGC
AGAACGAGAAGCTGTACCTGTACTACCTGCAGAATGGGCGGGATATGTACGTGGAC
CAGGAACTGGACATCAACCGGCTGTCCGACTACGATGTGGACCATATCGTGCCTCA
GAGCTTTCTGAAGGACGACTCCATCGACAACAAGGTGCTGACCAGAAGCGACAAGA
ACCGGGGCAAGAGCGACAACGTGCCCTCCGAAGAGGTCGTGAAGAAGATGAAGAA

CTACTGGCGGCAGCTGCTGAACGCCAAGCTGATTACCCAGAGAAAGTTCGACAATC
TGACCAAGGCCGAGAGAGGCGGCCTGAGCGAACTGGATAAGGCCGGCTTCATCAAG
AGACAGCTGGTGGAAACCCGGCAGATCACAAAGCACGTGGCACAGATCCTGGACTC
CCGGATGAACACTAAGTACGACGAGAATGACAAGCTGATCCGGGAAGTGAAAGTGA
TCACCCTGAAGTCCAAGCTGGTGTCCGATTTCCGGAAGGATTTCCAGTTTTACAAAG
TGCGCGAGATCAACAACTACCACCACGCCCACGACGCCTACCTGAACGCCGTCGTG
GGAACCGCCCTGATCAAAAAGTACCCTAAGCTGGAAAGCGAGTTCGTGTACGGCGA
CTACAAGGTGTACGACGTGCGGAAGATGATCGCCAAGAGCGAGCAGGAAATCGGCA
AGGCTACCGCCAAGTACTTCTTCTACAGCAACATCATGAACTTTTTCAAGACCGAGA
TTACCCTGGCCAACGGCGAGATCCGGAAGCGGCCTCTGATCGAGACAAACGGCGAA
ACCGGGGAGATCGTGTGGGATAAGGGCCGGGATTTTGCCACCGTGCGGAAAGTGCT
GAGCATGCCCCAAGTGAATATCGTGAAAAAGACCGAGGTGCAGACAGGCGGCTTCA
GCAAAGAGTCTATCCTGCCCAAGAGGAACAGCGATAAGCTGATCGCCAGAAAGAAG
GACTGGGACCCTAAGAAGTACGGCGGCTTCGACAGCCCCACCGTGGCCTATTCTGTG
CTGGTGGTGGCCAAAGTGGAAAAGGGCAAGTCCAAGAAACTGAAGAGTGTGAAAG
AGCTGCTGGGGATCACCATCATGGAAAGAAGCAGCTTCGAGAAGAATCCCATCGAC
TTTCTGGAAGCCAAGGGCTACAAAGAAGTGAAAAAGGACCTGATCATCAAGCTGCC
TAAGTACTCCCTGTTCGAGCTGGAAAACGGCCGGAAGAGAATGCTGGCCTCTGCCG
GCGAACTGCAGAAGGGAAACGAACTGGCCCTGCCCTCCAAATATGTGAACTTCCTG
TACCTGGCCAGCCACTATGAGAAGCTGAAGGGCTCCCCCGAGGATAATGAGCAGAA
ACAGCTGTTTGTGGAACAGCACAAGCACTACCTGGACGAGATCATCGAGCAGATCA
GCGAGTTCTCCAAGAGAGTGATCCTGGCCGACGCTAATCTGGACAAAGTGCTGTCCG
CCTACAACAAGCACCGGGATAAGCCCATCAGAGAGCAGGCCGAGAATATCATCCAC
CTGTTTACCCTGACCAATCTGGGAGCCCCTGCCGCCTTCAAGTACTTTGACACCACC
ATCGACCGGAAGAGGTACACCAGCACCAAAGAGGTGCTGGACGCCACCCTGATCCA
CCAGAGCATCACCGGCCTGTACGAGACACGGATCGACCTGTCTCAGCTGGGAGGCG
ACAAAAGGCCGGCGGCCACGAAAAAGGCCGGCCAGGCAAAAAAGAAAAAGTAAgtc
gacaataaaagatctttattttcattagatctgtgtgttggttttttgtgtgacgcgtgtggtaaaatcgataaggatcttcctagagcatggctacg
tagataagtagcatggcgggttaatcattaactacaaggaacccctagtgatggagttggccactccctctctgcgcgctcgctcgctca
ctgaggccgggcgaccaaaggtcgcccgacgcccgggctttgcccgggcggcctcagtgagcgagcgagcgcgcagccttaatta
acctaattcactggccgtcgttttacaacgtcgtgactgggaaaaccctggcgttacccaacttaatcgccttgcagcacatccccctttcgcc

agctggcgtaatagcgaagaggcccgcaccgatcgcccttcccaacagttgcgcagcctgaatggcgaatgggacgcgccctgtagcgg
cgcattaagcgcggcgggtgtggtggttacgcgcagcgtgaccgctacacttgccagcgccctagcgcccgctcctttcgctttcttcccttc
ctttctcgccacgttcgccggctttccccgtcaagctctaaatcgggggctccctttagggttccgatttagtgctttacggcacctcgacccca
aaaaacttgattagggtgatggttcacgtagtgggccatcgccctgatagacggtttttcgccctttgacgttggagtccacgttctttaatagtg
gactcttgttccaaactggaacaacactcaaccctatctcggtctattcttttgatttataagggattttgccgatttcggcctattggttaaaaaat
gagctgatttaacaaaaatttaacgcgaattttaacaaaatattaacgcttacaatttaggtggcacttttcggggaaatgtgcgcggaacccct
atttgtttatttttctaaatacattcaaatatgtatccgctcatgagacaataaccctgataaatgcttcaataatattgaaaaaggaagagtatgag
tattcaacatttccgtgtcgcccttattcccttttttgcggcattttgccttcctgtttttgctcacccagaaacgctggtgaaagtaaaagatgctga
agatcagttgggtgcacgagtgggttacatcgaactggatctcaacagcggtaagatccttgagagttttcgccccgaagaacgttttccaat
gatgagcacttttaaagttctgctatgtggcgcggtattatcccgtattgacgccgggcaagagcaactcggtcgccgcatacactattctcag
aatgacttggttgagtactcaccagtcacagaaaagcatcttacggatggcatgacagtaagagaattatgcagtgctgccataaccatgagt
gataacactgcggccaacttacttctgacaacgatcggaggaccgaaggagctaaccgcttttttgcacaacatgggggatcatgtaactcg
ccttgatcgttgggaaccggagctgaatgaagccataccaaacgacgagcgtgacaccacgatgcctgtagcaatggcaacaacgttgcg
caaactattaactggcgaactacttactctagcttcccggcaacaattaatagactggatggaggcggataaagttgcaggaccacttctgcg
ctcggcccttccggctggctggtttattgctgataaatctggagccggtgagcgtgggtctcgcggtatcattgcagcactggggccagatg
gtaagccctcccgtatcgtagttatctacacgacggggagtcaggcaactatggatgaacgaaatagacagatcgctgagataggtgcctc
actgattaagcattggtaactgtcagaccaagtttactcatatatactttagattgatttaaaacttcatttttaatttaaaaggatctaggtgaagat
cctttttgataatctcatgaccaaaatcccttaacgtgagttttcgttccactgagcgtcagaccccgtagaaaagatcaaaggatcttcttgaga
tcctttttttctgcgcgtaatctgctgcttgcaaacaaaaaaaccaccgctaccagcggtggtttgtttgccggatcaagagctaccaactcttttt
ccgaaggtaactggcttcagcagagcgcagataccaaatactgttcttctagtgtagccgtagttaggccaccacttcaagaactctgtagca
ccgcctacatacctcgctctgctaatcctgttaccagtggctgctgccagtggcgataagtcgtgtcttaccgggttggactcaagacgatagt
taccggataaggcgcagcggtcgggctgaacggggggttcgtgcacacagcccagcttggagcgaacgacctacaccgaactgagata
cctacagcgtgagctatgagaaagcgccacgcttcccgaagggagaaaggcggacaggtatccggtaagcggcagggtcggaacagg
agagcgcacgagggagcttccagggggaaacgcctggtatctttatagtcctgtcgggtttcgccacctctgacttgagcgtcgatttttgtg
atgctcgtcaggggggcggagcctatggaaaaacgccagcaacgcggcctttttacggttcctggccttttgctggccttttgctcacatgttc
tttcctgcgttatcccctgattctgtggataaccgtattaccgcctttgagtgagctgataccgctcgccgcagccgaacgaccgagcgcagc
gagtcagtgagcgaggaagcggaagagcgcccaatacgcaaaccgcctctccccgcgcgttggccgattcattaatgcagctggcacga
caggtttcccgactggaaagcgggcagtgagcgcaacgcaattaatgtgagttagctcactcattaggcaccccaggctttacactttatgct
tccggctcgtatgttgtgtggaattgtgagcggataacaatttcacacaggaaacagctatgaccatgattacgccagatttaattaa

In this Example, the inventors next tested above described CRISPR *RHO* strategy in human embryonic kidney cell line (HEK293FT). HEK293FT cells were transfected with Cas9 vector (pX459) (SEQ ID NO: 22) carrying no sgRNA, one sgRNA1 (SEQ ID NO: 1), one sgRNA2 (SEQ ID NO: 2), or both. Ninety-six hours later, DNA was extracted, and the RHO locus was amplified and analyzed by a mismatch detection SURVEYOR assay. Applying two sgRNAs together resulted in gene deletion (-30-40%), which indicated that "Chop" strategy works efficiently in mammalian cells (Fig. 2B, left, lane 4). Using one sgRNA (lanes 2 and 3) at a time does not result in a change in size. Approximately 30% of the genomic DNA underwent non-homologous end joining (NHEJ) by one sgRNA, and up to 80% was edited (deletion and NHEJ) when two sgRNAs were used. Equal amounts of plasmid DNA (1 µg / 1 × 10⁵ 293FT cells) were used in each group. These findings indicate that applying two sgRNAs can destroy, remove, or degrade endogenous human *RHO* sequence more efficiently.

The inventors further tested if the "Chop" can decrease wt *RHO* gene expression. In this experiment, a bicistronic construct was transfected into HEK293 cells to express wt *RHO* cDNA (SEQ ID NO: 8) and EGFP (Fig. 3A). Both *RHO* cDNA and EGFP (SEQ ID NO: 21) expressions were driven by a CMV promoter (SEQ ID NO: 20) independently and simultaneously, so that EGFP expression can be used as an internal control in western blot, which was used to normalize the difference in transfection efficiency and protein loading. FIG. 3A also illustrates the target sites of sgRNA1 (SEQ ID NO: 1) and sgRNA2 (SEQ ID NO: 2) on this *RHO* expression vector. When HEK293FT cells were co-transfected with RHO expression vector and another vector expressing Cas9 components (pX459) carrying sgRNA1 and sgRNA2, the *RHO* protein expression level was much lower (Fig. 3B). The sg3 group is a non-specific control sgRNA. These results were further normalized with an internal control, which is shown in FIG. 3C. These findings indicate that, applying two sgRNAs together lowers *RHO* expression about 70%, while using single sgRNA only reduced the expression by 0-30% compared to the control group (sg3). Together, these results indicate that "Chop" strategy can significantly abolish or inactivate wt *RHO* protein expression.

### Example 2. CRISPR/Cas9-Induced Gene Editing in a Mouse Model of Retinitis Pigmentosa Delays Disease Progression

Next, the inventors verified the feasibility and efficacy of the CRISPR/Cas9 endonuclease system as a gene-editing treatment modality in a mouse model of RP with the dominant D190N rhodopsin mutation. In these experiments, two AAV8 vectors containing the Cas9 coding sequence and the sgRNA (SEQ ID NO: 4)/donor template marked with an *Afl*II restriction site were used. Insertion of *Afl*II restriction site allows for the identification of cells that have undergone homologous recombination (Figs. 4A-4C). Briefly, heterozygous Rho^{D190N}/⁺ was transduced into the right eye before post-natal day 5 with above described recombinant AAV8 vectors. The sgRNA targeting frequency and recombination of donor template (SEQ ID # 23) were verified by TIDE indel tracking tool (Brinkman et al. Nucleic Acid Res. 2014 Dec 16, 42(22): e168) and *Afl*II enzyme digestion (Fig. 4). About 50% of cells underwent NHEJ (mostly are 1 bp insertion), and about 10% of cells incorporated donor template successfully. Structural preservation was assessed by H&E staining, and retinal function rescue was assessed by electroretinography (ERG) at 3 months of age (Figs. 5A and 5B). In the Rho^{D190N}/⁺ 3 month-old mice, treated eyes showed greater photoreceptor survival than did eyes that did not receive AAV injection (Fig. 5A). Furthermore, retinal function as measured by ERG was also increased in treated eyes compared with control eyes at 3 months of age (Fig. 5B). Collectively, these results demonstrate that CRISPR-Cas9 gene editing described in the present disclosure can be used to *in vivo* correct the phenotype of RP. The codon-modified donor sequence is shown as follows: **Underline and bold:** homologous arm; **Bold:** AflII site; Underlined: 5 codon-modified wobble nucleotides;

tcccttaaccaccgaaggcagggcagcaggctagtggagcagagctgcgtggtcaagtggcagggagcttaagaatcgtccaag
ggcggagaccagtaagtctcattaggtgatggggccagcaggtaaaagccattcatGcttatgtccagctgggcgtgtgttctcttc
ctgttttatcatcccttgcgctgaccatcaggtacatccctgagggcatgcaatgttcatgcgggattgactattatacccttaagccgg
aggtcaacaacgaatcctttgtcatctacatgttcgtggtccacttcaccattcctatgatcgtcatcttcttctgctatgggcagctggt
cttcacagtcaaggaggtatgagcaggg

### Example 3. CRISPR-Mediated Humanized Exon 1 at the Mouse Rho Locus

The inventors of the present disclosure next tested the ability to replace mouse Rho locus with wild-type (wt) (SEQ ID NO: 24) or mutant human (h) (SEQ ID NO: 25) *RHO* exon 1 in mouse embryonic stem (ES) cells (Fig. 6). Briefly, ES cells were co-transfected (via electroporation) with the Cas9 expression vector carrying a *Rho* exon 1-specific sgRNA (sgRNA-Rho Exon 1, SEQ ID NO: 5) targeting mouse *Rho* exon 1) and a targeting vector carrying with human RHO donor template, which contained a sequence of hRHO exon 1 flanked with ~750 bp homologous arm on each side (Fig. 6A). Human *RHO* donor template is expected to replace mouse exon 1 and confer resistance to sgRNA-Rho Exon 1. Seven days after electroporation, ES clones were picked and DNA was extracted and amplified with screening primers. Two out of 96 clones were detected with replacement of human exon 1 by RFLP analysis (Fig. 6B). As shown in Fig. 6C, sequence electropherograms of amplicons show perfect fused human and mouse sequence of one targeted ES clone (lane 2, Fig 6B). The correct targeted clones can be further used to produce the humanized RHO exon 1 mouse model. This patient-specific humanized mouse system enables the inventors to test various sgRNAs that may be used for targeting human genomic sequence for ChopStick strategy in vivo. The advantages of using these mouse models also enables the validation of the "ChopStick" efficacy and safety via functional evaluation methods like visual function, imaging of rescued tissue in live animals for long term observations. The sgRNA sequence is listed above and the donor template sequence is listed below: Underline and bold: homologous arm; UPPERCASE: human *RHO* exon 1

atgctcacctgaataacctggcagcctgctccctcatgcagggaccacgtcctgctgcacccagcaggccatcccgtctccatagcc
catggtcatccctccctggacaggaatgtgtctcctccccgggctgagtcttgctcaagctagaagcactccgaacagggttatggg
cgcctcctccatctcccaagtggctggcttatgaatgtttaatgtacatgtgagtgaacaaattccaattgaacgcaacaaatagtta
tcgagccgctgagccggggggcggggggtgtgagactggaggcgatggacggagctgacggcacacacagctcagatctgtca
agtgagccattgtcagggcttggggactggataagtcagggggtctcctgggaagagatgggataggtgagttcaggaggagac
attgtcaactggagccatgtggagaagtgaatttagggcccaaaggttccagtcgcagcctgaggccaccagactgacatgggga
ggaattcccagaggactctggggcagacaagatgagacaccctttcctttctttacctaagggcctccacccgatgtcaccttggcc
cctctgcaagccaattaggccccggtggcagcagtgggattagcgttagtatgatatctcgcggatgctgaatcagcctctggctta
gggagagaaggtcactttataagggtctggggggggtcagtgcctggagttgcgctgtgggagcGAGTCATCCAGCTG
GAGCCCTGAGTGGCTGAGCTCAGGCCTTCGCAGCATTCTTGGGTGGGAGCAGCCAC
GGGTCAGCCACAAGGGCCACAGCCATGAATGGCACAGAAGGCCCTAACTTCTACGT
GCCCTTCTCCAATGCGACGGGTGTGGTACGCAGCCCCTTCGAGTACCCACAGTACTA
CCTGGCTGAGCCATGGCAGTTCTCCATGCTGGCCGCCTACATGTTTCTGCTGATCGTG
CTGGGCTTCCCCATCAACTTCCTCACGCTCTACGTCACCGTCCAGCACAAGAAGCTG
CGCACGCCTCTCAACTACATCCTGCTCAACCTAGCCGTGGCTGACCTCTTCATGGTC
CTAGGTGGCTTCACCAGCACCCTCTACACCTCTCTGCATGGATACTTCGTCTTCGGGC
CCACAGGATGCAATTTGGAGGGCTTCTTTGCCACCCTGGGCGgtatgagcagagagactggggc

gggggggtgtagcatgggagccaaggggccacgaaagggcctgggagggtctgcagcttacttgagtctctttaattggtctcatc
taaaggcccagcttattcattggcaaacactgtgaccctgagctaggctgctgttgagagcaggcacggaacattcatctatctcat
cttgagcaatgcaagaaacatgggttcagagaggccaaggactcaccgaggagtcacagagtgtgggggtgtcctctgaggcag
ctgagctggggcacacacagactgagcaccaggagtgagctctagcttttttttttctatgtgtcttttctaaaagacacataggttta
ggactgtccctggtccaggtaagaactggttcagtaaacttgtacatctcactgcctggccagccctgtcagcttccaccagagtgcg
tgcactacacacccggcatctcaaaggattcattcctatctttcctatctttggagtgaggcacagtctcacgtagtccagtccagact
ggccttaaattctgcagctgaggatgtacttaaacttgtcatcctcctgccccagcctctcaagtgctgtgatcacaggcacggacca
ctatgctacgccaggtgtttccaaacattttctctcccttaactggaaggtcaatgaggctctttcgagaagcaacagagcc

### Example 4. Repair of Mouse Pde6a D670G Allele in Stem Cells

Mutations in genes encoding subunits of the rod-specific enzyme, cyclic guanosine monophosphate (cGMP) phosphodiesterase 6 (PDE6A and PDE6B), are responsible for approximately 72,000 cases of RP worldwide each year, making therapeutic modeling highly relevant to developing mechanisms based therapies. In the present Example, the inventors used the CRISPR/ Cas9 gene editing system to correct photoreceptor gene mutations in mouse ES cells.

For these studies, the inventors used ES cells isolated from Pde6a^{D670G/D670G} mouse model (Wert KJ et al. Hum Mol Genet. 2013 Feb 1;22(3):558-67; Wert KJ et al. J Vis Exp. 2012 Nov 25;(69)). As shown in Fig. 7A, a donor construct (SEQ ID NO: 26) used in this Example contains two modifications: 1) a Pde6a-codon modification which creates an additional SphI site upstream from the D670G codon, where SphI enzyme-digestion can identify ES cells that underwent CRISPR-mediated homologous recombination; and 2) eight wobble base pairs were introduced, which make the donor template unrecognizable to sgRNA (SEQ ID NO: 6) and thus resistant to Cas9, and which resulted in the change of the mutant amino acid sequence to that of the wt amino acid sequence. Thus, upon Cas9 cutting and homologous recombination, the endogenous mutant allele is replaced (i.e., repaired). In FIG. 7A, triangle indicates the sgRNA target site while the two arrows represent the primer pairs used for PCR amplification. As shown in FIG. 7B, amplicons generated from recombined cells were 303 bp and 402 bp while the unedited amplicon is 705 bp. Moreover, using direct sequencing of genomic DNA from a target clone, the inventors confirmed predicted replacement of the D670G exon with donor template (Fig. 7C). This is an example where there is no sgRNA target site on the mutation site, but researchers can still design sgRNA nearby and successfully replace mutant allele through homologous recombination.

Thus, in this Example, the inventors verified the ability of the CRISPR/Cas9 system to edit the mouse *PDE6a* locus and rescue photoreceptors.

### Example 5. CRISPR/Cas9-Induced In Vivo Gene Editing in Pde6a^{D670G} /Pde6a^{D670G} Mouse Model

The inventors have verified the use of CRISPR/Cas9 system to edit the mouse *Rho* locus and rescue photoreceptors (Example 2). Furthermore, as shown in Example 4, the inventors were able to repair mouse Pde6a D670G allele in ES cells. Next, *in vivo* experiments, where post-natal day (P) 5 *Pde6a^{D670G}* /*Pde6a^{D670G}* mice receive subretinal transductions of both recombinant AAV8-Cas9 and AAV8-sgRNA with the codon-optimized Cas9 resistant donor DNA (validated in Example 4) into one eye can be conducted. In control animals, one eye is transduced with an empty AAV8 vector or AAV8-Cas9 as a negative control.

The inventors next perform quantitative validation of recombination and correction of one of the *Pde6a^{D670G}* alleles in homozygous mutant. Briefly, one month after injection (before degeneration onset), inventors dissect retinas , isolate the DNA, perform the PCR, and verify the SphI restriction site (using RFLP). PCR samples are run in triplicate. At 3 weeks of age, retinas from 3 mice are collected, and Pde6a levels are quantified by immunoblotting.

To quantitatively assess photoreceptor function and survival, the inventors perform quantitative AF of outer segment thinning on SD-OCT, ERG, and rod-cone density at 8, 16, and 24 weeks (n = 36 total animals) using previously published techniques (Woodruff et al._J Neurosci. 2008 Feb 27;28(9):2064-74; Janisch et al. Biochem Biophys Res Commun 390, 1149-1153 (2009); Tsang et al. Science. 1996 May 17;272(5264):1026-9; Davis et al. Invest Ophthalmol Vis Sci. 2008 Nov;49(11):5067-76.) All measurements are performed on both eyes.

To determine the efficacy of PDE function, as a key biochemical indicator of rescue, the inventors measure whether total cGMP levels and PDE activity from light- and dark-adapted retinas are restored. Three additional sample right eyes, treated at P18, P21, P28, and P35, and control fellow left eyes of Pde6a^{D670G/D670G} are assayed. GUCY2E (guanylate cyclase) should remain stable for all experiments and is determined as previously described (Tsang et al. Science. 1996 May 17; 272(5264): 1026-1029; Science. 1998 Oct 2;282(5386):117-21).

### Example 6. Efficacy and frequency of homologous recombination vs. non-homologous end joining (NEHJ) for editing PD6A in patient specific stem cells

In this Example, the inventors study if the AAV2-Cas9 system can edit the human *PDE6A* locus. Here, 0.25×10⁶ patient iPSs in a 6-well matrigel coated plate (in NutriStem XF/FF Culture media, Stemgent, Cambridge) are co-transduced with AAV2-Cas9 and AAV2 vector-donor template mix (MOI: 2000) to repair *PDE6A.* After 48 hours, iPSs are passaged with Accutase onto regular 10-cm matrigel coated culture dishes.

Next, 1000 *PDE6A^{R102C}*/*PDE6A^{S303C}* patient iPS clones are picked, and DNA isolated for PCR; (Primers: forward: GCAGACTGCAAAACTGCCAT; reverse:
TGTCACCAGCCTTGTCTTGG). PCR products are cut with *BsiWI* to identify clones that have undergone homologous recombination. After *BsiWI* digestion, the amplicon generated from iPS that underwent homologous recombination gives bands at 271 bp and 380 bp, compared to the parental sequence, which gives only one band at 651 bp.

To determine the percentage of clones that have undergone NHEJ (as opposed to those that underwent homology-directed repair), DNA is analyzed from clones without the *BsiWI* site (i.e., not transduced, transduced off-target or NHEJ), and the frequency of the disruption of the PDE6A allele determined. DNA is analyzed by SURVEYOR mismatch detection assay and positive DNA samples are subjected to subcloned into plasmid vectors such as pCR^{™}4Blunt-TOPO^{®} vector and then send for Sanger sequencing. In addition, the assessment of off-targeting in iPS and live mice are prerequisites before application to humans. Off-target sites are analyzed by full-genome sequencing using Illumina next-generation sequencing.

The inventors anticipate a much higher rate of homologous recombination mediated by AAV8 in photoreceptors *in vivo,* compared to patient iPS. This is because AAV8 introduces sgRNA into photoreceptors at a much higher frequency than transduction into iPS. AAV8 introduces into photoreceptors ~10,000 copies of the sgRNA and Cas9, as opposed to both lipofection and electroporation, which generally introduce a single-copy DNA into each cell.

The percentage of transduced clones that undergo NHEJ is likely to be higher than those undergoing homologous recombination-approximately 10% vs. 1%, respectively, with a 90-bp donor template in human- induced pluripotent stem cells.

### Example 7. Use of CRISPR system to replace mutant allele R345W in iPS cells isolated from Doyne Honeycomb patient

Doyne Honeycomb retinal dystrophy (DHRD) is an inherited disease that affects the eyes and causes vision loss. It is characterized by small, round, white spots (drusen) that accumulate around the retinal pigment epithelium. Over time, drusen may grow and come together, creating a honeycomb pattern. It usually begins in early adulthood, but the age of onset varies. The degree of vision loss also varies. DHRD is caused by R345W mutations in the EFEMP1 gene, which are inherited in an autosomal dominant manner.

In this Example, the inventors used CRISPR components and a donor template (SEQ ID NO: SEQ ID 27) to correct the R345W mutation in the iPS cells derived from Doyne Honeycomb patient fibroblast (FIG. 8A). The resulting iPS cells comprise wild type EFEMP1 sequence with codon-modification, which confers resistance to further cutting by the Cas9. These cells can be used for autologous transplantation after the differentiation into RPE cells for the cure of DHRD.

Briefly, the inventors collected the iPS derived from the DHRD patient. Cas9 protein and sgRNA-EFEMP1 (SEQ ID NO: 3) (FIG. 8B) were mixed and co-transduced with donor template in the form of single strand oligodeoxynucleotide (ssODN) (sequence: tagttagtaaactctttgaccctacatctctacagatataaatgagtgtgagaccacaaaCgaGtgcCgggaggatgaaatgtgttggaatt atcatggcggcttccgttgttatccacgaaatcctt) into iPS cells by nucleofection. The donor template is codon-modified to prevent repeating recognizing and cutting by the CRISPR components. The colony with corrected sequence was confirmed by RFLP assay with the additional ScrFI restriction site. The genotype of the iPS cell is further confirmed by sequencing (FIG. 8C).

This experiment provides the evidence that the "Chop" strategy has potential to treat autosomal dominant diseases other than autosomal dominant retinitis pigmentosa.

### Example 8. SNP Editing for a Juvenile Macular Degeneration

Patient-specific cells are the most clinically relevant *in vitro* genomic target for mediated gene editing, and patient-derived fibroblasts (and iPS cells) have served as *in vitro* recipients of gene therapy in retinal degenerative disorders (Li, Tsai et al. 2012, Li, Wu et al. 2014). FDA approval of a previous Phase I/II clinical trial was based on efficacy shown in patient-derived fibroblasts (and iPS cells) (Vasireddy, Mills et al. 2013). To assess the efficacy of our allele-specific CRISPR strategy, we will use patients' iPS cells (harboring BEST1 pR218H, pL234P or pA243T alleles (Moshfegh, Velez et al. 2016)) to test the efficiency of different CRISPR approaches.

Inventors address these obstacles with allele-specific genome editing using CRISPR, a genome editing technology. We test three different CRISPR systems (FIG. 11): Left) CRISPRr (r = repair), which relies on homologous recombination to correct a mutation to wild type (WT) sequence; Middle) CRISPRd (d = destruction), which truncates the mutant allele through nonhomologous end-joining (NHEJ); and Right) CRISPRi (i = interference), which uses deactivated Cas9 (dCas9) to bind to (but not cleave) the transcription start site (TSS), specifically preventing transcription of the mutant protein. Inventors: i) compare the efficiency of the three CRISPR systems; and ii) validate morphology and function of CRISPR-treated, patient-derived RPE ("iRPE").

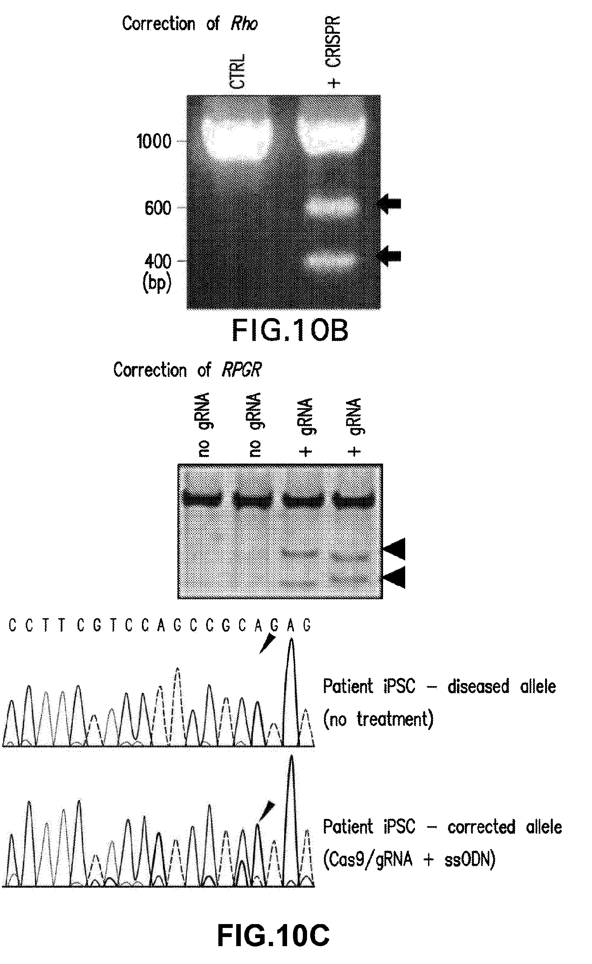

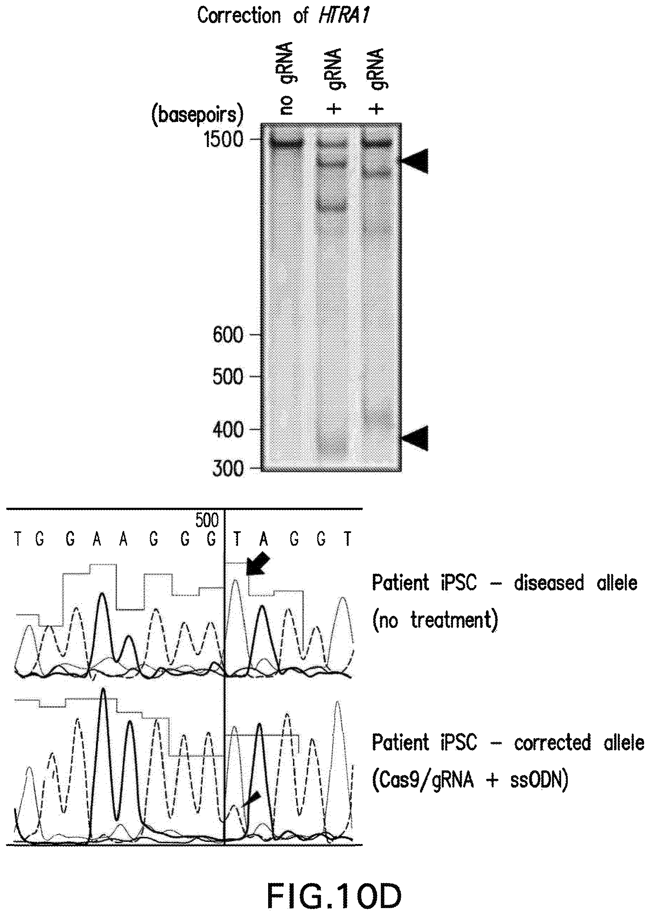

Inventors previously reported on four mouse strains which Inventors created via CRISPR/Cas9 homologous recombination (Ortega, Ittmann et al. 1998, Sancho-Pelluz and Paquet-Durand 2012, Davis, Hsu et al. 2013, Singh, Shen et al. 2013, Bassuk, Zheng et al. 2016). Retinal disease progression may be quantified using multimodular imaging and cell counting (Tsang, Gouras et al. 1996, Tsang, Burns et al. 1998, Davis, Tosi et al. 2008, Tsang, Tsui et al. 2008, Woodruff, Janisch et al. 2008, Janisch, Kasanuki et al. 2009, Wang, Tosi et al. 2010, Tosi, Davis et al. 2011, Sancho-Pelluz and Paquet-Durand 2012, Wert, Davis et al. 2012, Yang, Naumann et al. 2012). In one embodiment, AAV2 vectors (FIG. 10) may be used. Viral-mediated gene transfer and/or footprint-free gene-editing technology (Tsang, Chen et al. 1996, Tsang, Gouras et al. 1996, Ortega, Ittmann et al. 1998, Tsang, Burns et al. 1998, Davis, Tosi et al. 2008, Tsang, Tsui et al. 2008, Woodruff, Janisch et al. 2008, Janisch, Kasanuki et al. 2009, Wang, Tosi et al. 2010, Tosi, Davis et al. 2011, Sancho-Pelluz, Tosi et al. 2012, Wert, Davis et al. 2012, Yang, Naumann et al. 2012) may be used in the present method.

Compare efficiency between CRISPRr, CRISPRd, and CRISPRi in iPS cells.

Patient-specific cells are the clinically relevant *in vitro* genomic target for mediated gene editing, and patient-derived fibroblasts (and iPS cells) have served as *in vitro* recipients of gene therapy in retinal degenerative disorders (Li, Tsai et al. 2012, Li, Wu et al. 2014). FDA approval of a previous Phase I/II clinical trial was based on efficacy shown in patient-derived fibroblasts (and iPS cells) (Vasireddy, Mills et al. 2013). To assess the efficacy of our novel, allele-specific CRISPR strategy, inventors use patients' iPS cells (harboring BEST1 pR218H, pL234P or pA243T alleles (Moshfegh, Velez et al. 2016)) to test the efficiency of different CRISPR approaches.

CRISPRr can repair single mutations via homology-directed repair (HDR, a form of intrinsic DNA repair mechanism). CRISPRd may specifically truncates the mutant allele by targeting sequences, e.g., upstream of the gene (such as an allele-specific SNP in TSS) and destroying the mutant allele without disrupting the healthy allele. CRISPRi uses an interference approach that prevents transcription of the mutant allele, and may yield few off-targeting effects.

Validate functionality/morphology and mutation repair of CRISPR-edited, patient-derived RPE *in vitro.*

This experiment is designed to correct dominant forms of BEST1-VMD and confirm that manipulation by CRISPR technology will not negatively affect iRPE maturation and function.

Without being bound by theory, the inventors hypothesize that BEST1 haplosufficiency allows for normal vision in humans (Fung, Yzer et al. 2015, Li, Zhang et al. 2017) and that iPS reprogramming or application of CRISPR protocols may impair RPE maturation, proper RPE polarization, RPE65 expression, normal trans-retinal metabolism, and phagocytic activity (Gouras, Kong et al. 2002, Nguyen, Li et al. 2015, Li, Chan et al. 2016, Moshfegh, Velez et al. 2016).

The iRPE cells after CRISPRr, CRISPRd or CRISPRi editing express proper RPE markers and possess normal functions especially in maintaining Cl⁻ ion homeostasis.

40 existing BEST1 patients of different ages are studied to characterize BEST1 markers to detect progression. BEST1 is a disease that typically does not irreversibly affect vision until patients reach their 30's. This experiment tracks patients with disease alleles, to determine the clinical disease course and the optimal window for CRISPR intervention. 40 BEST1 patients are tracked clinically by imaging and biomarkers analysis for 5 years.

The experiment can identify biomarkers and clinical tests that can identify patients ready for intervention and identify BEST1 biomarkers for patients.

### Experimental Methods

Efficiency between CRISPRr and CRISPRd in iPS cells.

Establishment of patient iPS cells: Inventors use the CytoTune-iPS Sendai Reprogramming Kit (Life Technologies) (Li, Tsai et al. 2012) to generate iPS cells and leave no genomic viral remnants behind. For quality control, iPS cells are subjected to karyotyping, genome sequencing, testing for lack of Yamanaka transgenes, and a pluripotency assay (Li, Tsai et al. 2012).

AAV carrying capacity: AAV carrying capacity may be 4.7 kb. Inventors use 2 AAV vectors. For CRISPRr, both pZac 2.1-based constructs are packaged into AAV2 (Khan, Hirata et al. 2010, Asuri, Bartel et al. 2012, Deyle, Khan et al. 2012). Cells are transduced both *in vitro* and *in vivo* (viral titer of 1 × 10¹³ genome copies/ml for each vector) with AAV2-sgRNA and AAV2-Cas9 vectors (FIG. 10A), and with the Cas9-resistant donor template containing WT BEST1. The AAV2 serotype is efficient in transducing RPE and inner retinal cells (in mouse models), and iPS cells (Pang, Lauramore et al. 2008, Guziewicz, Zangerl et al. 2013, Rapti, Stillitano et al. 2015). To make isogenic BEST1, inventors pick 1,000 CRISPRr-edited patient stem cell clones, screen for restriction fragment length polymorphisms (RFLPs), and confirm them by Sanger sequencing.

gRNAs: Two gRNAs are needed for CRISPRd; one upstream of the TSS (gRNA1 or gRNA2) and the other downstream in the intron (gRNA3, FIG. 13A). gRNA3 targeting the intron can target both the mutant and WT allele. However, merely a single cut in the intron may not harm the WT allele. Only when the chromosome is simultaneously targeted by both upstream and downstream gRNAs (gRNA1+gRNA3 or gRNA2+gRNA3) for Cas9 cutting can the large-scale deletion of BEST 1 be achieved. This gene truncation can prevent expression of the mutant BEST1 allele (FIG. 13A-13C) while sparing the WT allele.

Quantitative validation of recombination and correction of one BEST1 allele for CRISPRr: One month after AAV transduction, HDR efficiency is assayed by PCR for the new restriction fragment length polymorphism (RFLP) site in CRISPRr-treated cells, which are cut in DNA from HDR-derived cells. Clones lacking the new RFLP site (which would indicate that they are either not transduced, transduced off-target, or altered via NHEJ) (FIG. 10A) will also be analyzed. DNA will be subjected to direct Sanger sequencing.

Functionality of CRISPR-edited iRPE lines (from patients carrying the pR218H, pL234P, and pA243T alleles (Moshfegh, Velez et al. 2016)) harboring the Cl⁻ biosensor is tested as above. For isogeneic controls generated by CRISPRr as above, the mutant BEST1 genomic sequence is replaced by a codon-modified donor template with an additional RFLP site (similar to the example in FIG. 10A), allowing identification of cells that have undergone homologous recombination.

It is unlikely that CRISPRd might disrupt the WT allele by NHEJ, as the *in vitro* assays using PCR-generated substrates show excellent specificity (FIG. 13B). Also, to minimize off-targeting effects, particularly in targeted exons, inventors shortened the gRNA complementarity region (Fu, Foden et al. 2013, Ran, Hsu et al. 2013, Pyzocha, Ran et al. 2014, Smith, Gore et al. 2014, Tsai, Wyvekens et al. 2014, Smith, Abalde-Atristain et al. 2015). In some instance, inventors could use a second gRNA and AAV::nickase instead of AAV::Cas9 (Mali, Aach et al. 2013, Ran, Hsu et al. 2013, Shen, Zhang et al. 2014, Tsai, Wyvekens et al. 2014). In another embodiment, inventors could design monomeric transcription activator-like effector nucleases (TALENs, 2.7 kb) against the mutant locus for AAV-mediated, targeted CRISPRr (Beurdeley, Bietz et al. 2013, M Scharenberg, Duchateau et al. 2013). Dual AAV vectors can transduce >55% of photoreceptors in live mice (Millington-Ward, Chadderton et al. 2011, Trapani, Colella et al. 2014, Cabral, DiCarlo et al. 2017). If the dual AAV::CRISPR vector does not ablate the BEST1 MT allele, inventors test AAV::SaCas9; U6::gRNA in a single AAV2 vector (Kleinstiver, Prew et al. 2015, Ran, Cong et al. 2015). Minimizing the level and duration of Cas9 expression (and thus the potential for off-targeting) is achieved by the AAV::Cas9 vector, which self-inactivates shortly after Cas9 production. Applicants can alternatively apply conventional Cre-lox knock-in technology (Kleinstiver, Prew et al. 2015, Ran, Cong et al. 2015), although footprint-free gene targeting cannot be achieved using that system. Persistence of donor DNA in cells could also yield a PCR artifact that may appear as low-frequency repair. A single-stranded donor (rather than a plasmid) should prevent this.

BEST1 targeting by CRISPRi using patient-derived RPE.

CRISPRi SNP Editing: CRISPRi (FIG. 14A-14B) inhibits transcription effectively due to the use of PUF-KRAB. The adaptor protein PUF binds to modified gRNA containing one or more PUF-binding sequences. The components of the 2nd generation CRISPRi can be packaged into two AAVs, allowing the CRISPRi machinery's smooth delivery into iRPE cells.

AAV2 construction and delivery: Inventors have the AAV2::PUF-KRAB plus AAV2::dCas9;U6::gRNA1-PUF-binding site constructs in hand and have tested the U6::gRNA1 in iRPE (FIG. 15B) (Millington-Ward, Chadderton et al. 2011, Trapani, Colella et al. 2014). Parental lines for CRISPR will be pL234P, pR218H, and pA243T patient-derived iRPE, engineered to harbor the YFP-H148Q/I152L fluorescent Cl⁻ biosensor (FIG.s 17A-17B). The gRNA with 5 PUF-binding sequences/sites added (sgRNA-PBS) acts as an RNA scaffold to seed assembly of a multi-silencer complex (FIG.s 17A-17B). After 2 to 3 passages, individual "polyclonal" populations (carrying different gRNAs) are tested for BEST1 knockdown by qPCR and immunoblotting.

RNA sequencing (RNA-Seq) for analyzing gene expression profile of AAV-transduced BEST1^{MT/+}RPE. To confirm BEST1^{MT} suppression by CRISPRi, inventors perform RNA-Seq of iRPE transduced with dual AAV2::PUF-KRAB; AAV2::dCas9 versus control single AAV2::dCas9 alone (30 million sequence reads).

To analyze off-targeting effects that are possibly produced by CRISPR, inventors perform WGS, digenome-seq, and GUIDE-seq.

CRISPRi should have few off-target effects, as it does not cut DNA. Consequently, this should reduce oncogene activation. In one embodiment, CRISPRi may have a higher efficacy than CRISPRr, because around one-third of NHEJ disruptions mediated by CRISPRr result in additional in-frame mutations. SSilencing the MT (mutant) BEST1 allele using CRISPRi results in negligible perturbation of genome-wide transcription. WT BEST1 mRNA levels should not be altered by CRISPRi. RNA-seq shows how effectively CRISPRi inhibits the MT BEST1 allele in iRPE.

The inventors also test if allele-specific editing is feasible or not by targeting SNPs rs972353 and rs972355. In certain embodiments, patients who are homozygous at these SNPs are not treated by CRISPRi or CRISPRd. These patients may be treated by targeting other SNPs within BEST1 gene. Following AAV2 delivery, catalytically-defective Cas9 (dCas9) may persist in non-dividing RPE cells and may prolong potential dCas9 toxicity (Platt, Chen et al. 2014). If the dual AAV2::dCas9 plus AAV2::gRNAPBS/PUF-KRAB does not promote sufficient suppression, EIAV::dSaCas9-KRAB can be administered in a single vector (Kleinstiver, Prew et al. 2015, Ran, Cong et al. 2015). Lentiviral equine infectious anemia virus (EIAV) is FDA-approved, and hence Lenti::CRISPRi can be easily adapted for clinical trials.

Determine whether RPE derived from CRISPR-edited cells is structurally normal and expresses appropriate biomarkers.

To set up the positive control for the test, inventors include a WT (wildtype) iRPE from a healthy donor; each of the CRISPRr, CRISPRd and CRISPRi experiments also includes respective scramble gRNA controls as negative treatment controls. In total, seven groups of cells are analyzed.

Isogenic CRISPRr-repaired iPS cells as the current standard serve as positive control for comparative effectiveness of CRISPRd and CRISPRi.

Like parental lines, CRISPR-edited clones can differentiate into RPE monolayers exhibiting normal phenotypes (Gouras, Kong et al. 2002, Wang, Tosi et al. 2010, Li, Tsai et al. 2012, Li, Wu et al. 2014; Bassuk, Zheng et al. 2016, Wu, Tsai et al. 2016).

Possible mosaic results in cell culture may be created by CRISPR technology. If the characterization by immunostaining and immunoblotting is not sufficient, inventors perform mass-spectrometry to analyze the proteomic profile for cells after CRISPR-editing. To translate the strategy of long-term expression of Cas9 using RPE cells into human trials, an intraocular Cyclosporin A implant may be used (Lai, Gouras et al. 2000). No immunosuppression may be used for the AAV::Cas9 trials (Long, McAnally et al. 2014, Long, Amoasii et al. 2016, Nelson, Hakim et al. 2016, Tabebordbar, Zhu et al. 2016, Bengtsson, Hall et al. 2017).

Quantify restoration of Cl⁻ efflux in CRISPR-edited RPE.

Biosensor to measure Cl⁻ efflux in iRPE: Inventors include a novel Cl⁻ biosensor as a breakthrough of previous challenges in testing function restoration and assessing possible perturbation of cellular functions after CRISPR editing. Using Cl⁻ concentration biosensor, inventors can directly test both possibilities using on CRISPR-edited iPS cells. inventors published our Cl⁻ efflux assay using iRPE harboring a single copy of a yellow fluorescent protein (YFP)-H148Q/I152L anion biosensor (FIG.s 17A-17B). This assay permits qualification of CRISPR-mediated functional rescue of BEST 1 channel activity.

Biosensor imaging: inventors observe Cl⁻ efflux in BEST^{MT} (heterozygous SNP rs972355) iRPE harboring YFPH148Q/I152L (Galietta, Haggie et al. 2001, Moshfegh, Velez et al. 2016) in CRISPRd- and CRISPRi-edited cells. This biosensor is sensitive to Cl⁻: when intracellular Cl⁻ increases, YFP intensity drops, and vice versa. Treatment with the Ca²⁺ ionophore A23187 induces Ca²⁺ release from endoplasmic reticulum stores to activate BEST1 (Moshfegh, Velez et al. 2016). Previously, within two minutes of stimulation, biosensor-expressing WT RPE cells exhibited decreased intracellular Cl⁻ concentrations, followed by recovery within 15 min (Galietta, Haggie et al. 2001, Moshfegh, Velez et al. 2016).

For specific Ca²⁺ concentrations, inventors estimate mean steady state Cl⁻ currents, their 95% confidence interval, and compare between groups at different voltage values. At 100 mV, Cl⁻ current density will also be compared between groups at different Ca²⁺ concentration levels. Using fluorescence biosensor imaging (Moshfegh, Velez et al. 2016), inventors also examine the trajectory of Cl⁻ conductance over time and compare groups at each of the time points.

The experiment can be repeated 20 different single cells per group. Comparing mean differences within a group (e.g., between different time points within a group), 20 cells would have 80% power to detect a standardized effect size (i.e., a mean difference in units of standard deviation) of 0.66 at a significance level of 0.05. Comparing mean differences between groups (e.g., comparing mean Cl⁻ current between groups at a specific Ca²⁺ concentration), 20 cells per group would have 80% power to detect a standardized effect size (i.e., a mean difference in units of sd) of 0.91 at a significance level of 0.05. From previous experiments, we would be able todetect such differences.

To confirm biosensor findings (Galietta, Haggie et al. 2001, Moshfegh, Velez et al. 2016), whole-cell recordings of iRPE are conducted for 48 to 72 h, using an EPC10 patch clamp amplifier (HEKA Electronics) (Li, Zhang et al. 2017).

Inventors expect the CRISPR methods to restore calcium-dependent Cl⁻ efflux and anticipate no experimental difficulties in differentiating cells to RPE or imaging Cl⁻ conductance (Li, Tsai et al. 2012, Li, Wu et al. 2014, Yang, Li et al. 2014, Yang, Li et al. 2014, Moshfegh, Velez et al. 2016, Li, Zhang et al. 2017). The performance of our 2nd generation of CRISPRi should be good, since the ability of transcriptional inhibition has been reinforced by the PUF-KRAB system.

If differentiated RPE do not exhibit normal polarity, biomarker expression, or phagocytic ability, inventors revise RPE differentiation protocols or employ a non-viral CRISPR delivery system using ribonucleoprotein (RNP) complexes.

Study VMD patients to characterize the natural history of the condition.

Imaging modalities such as short-wavelength autofluorescence (SW-AF), NIR-AF, and OCT can determine the optimal window for preventing VMD using our CRISPR intervention.

Progress in understanding the natural history of VMD has been a challenge because mice do not approximate human VMD phenotypes (i.e., no appropriate mouse model) (Marmorstein, Wu et al. 2006, Zhang, Stanton et al. 2010), and histopathological material is limited. Also unanswered is how VMD disproportionately affects the macula more than the rest of the retina (Boon, Klevering et al. 2007, Boon, Theelen et al. 2009) and whether increased RPE lipofuscin formation contributes to the disease pathogenesis.

Fundus abnormalities are typically visible within the first two decades of life, but cases of late onset (e.g., age 75) have been reported (Mullins, Oh et al. 2005). The early stage of VMD is characterized by a dome-shaped fluid-filled lesion (vitelliform lesion) in the central macula that is well delineated and appears markedly intense by SW-AF imaging (Spaide, Noble et al. 2006). A decreased electrooculogram (EOG) light peak to dark trough ratio is a hallmark of bestrophinopathies and is caused by malfunctions in BEST1. Multiple anion channel functions have been attributed to BEST1, including an outward Ca²⁺-dependent Cl⁻ conductance and bicarbonate efflux (Sun, Tsunenari et al. 2002, Rosenthal, Bakall et al. 2006, Qu and Hartzell 2008, Marmorstein, Cross et al. 2009). Inventors also track the impact of the fluid-filled lesion on photoreceptor cell health.

VMD progressively worsens with age, but the rate of decline is typically slow and provides a long therapeutic window, as central photoreceptors remain treatable despite morphological and functional changes caused by the persistence of subretinal fluid and serous detachment (Ponjavic, Eksandh et al. 1999, North, Gelman et al. 2014, Yang, Justus et al. 2015). The rate of progression and prognosis of patients with VMD can be predicted by combining observations of genotypic and phenotypic features. Currently, there are no published guidelines to prognosticate VMD. Therefore, stratification and counseling based on fundoscopic examination have been challenging.

This data suggests that OCT segmentation, qAF, NIR-AF imaging and functional testing may be used for classifying and predicting future visual function of patients (Ferrara, Costa et al. 2010, Duncker, Greenberg et al. 2014, Fung, Yzer et al. 2014, North, Gelman et al. 2014). Non-invasive retinal imaging methods are widely available and child friendly, and they rapidly provide a direct and sensitive measure of retinal degeneration and disease progression. An expansion of current protocols previously applied to the characterization of VMD (Ferrara, Costa et al. 2010, Duncker, Marsiglia et al. 2014, Fung, Yzer et al. 2014, North, Gelman et al. 2014) is expected to provide meaningful endpoints for monitoring efficacy in treatment trials for these conditions.

To assess the rate of progression and prognosis of VMD patients, a combination of validated methodologies can be applied (Duncker, Marsiglia et al. 2014, Fung, Yzer et al. 2014, North, Gelman et al. 2014): genotyping, OCT segmentation, qAF, NIR-AF, and functional measurements. A precise OCT-qAF classification system will reveal the best stages for treatment and identify any milestones or points of "no return" for disease progression. Therapeutic editing may not benefit patients who have progressed beyond the *vitelloruptive* stage (Ponjavic, Eksandh et al. 1999). Findings can validate the finding that OCT thickening is the earliest pathological marker (Ferrara, Costa et al. 2010, Querques, Zerbib et al. 2011). This pre-symptomatic stage is yet to be characterized and may be the best window for CRISPR-based intervention in VMD.

Natural history data can inform the design of outcome measures used in clinical trials. Confirmed genotype-phenotype correlations and a precise classification system will assist in patient stratification for trial selection criteria. Results from all clinical modalities, OCT segmentation, areas of increase in qAF and NIR-AF imaging, combined with measures of rod and cone function with fundus-guided mesopic and dark-adapted microperimetry (Nidek MP-1S and MAIA), and automated light and dark-adapted perimetry in a sufficiently large cohort, facilitate understanding of genotype-phenotype correlations and clinical classification. OCT segmentation may allow for earlier detection of progression than other currently available imaging tools. Progression rates will exhibit bilateral concordance so as to allow for contralateral control eyes. Inventors' registry serves as a benchmark for basic, clinical and pharmacogenomic studies.

Compare the sensitivity of SW-AF, NIR-AF and SD-OCT imaging vs visual function testing (fundus perimetry and automated light and dark-adapted perimetry) as reliable endpoints in monitoring disease progression in VMD patients over a 4-year period.

Quantifiable changes in the fundus, ascribed to disease progression, can be precisely documented with current imaging modalities that include SW-AF (488 nm) and NIR-AF (787 nm) imaging and OCT.

The sensitivity and comparability of structural and functional data can be evaluated as outcome measurements over a 4-year period. Inventors also assess the utility of OCT, qAF, NIR-AF and fundus perimetry to detect progression and determine prognosis.

### Experimental

*Genotyping and clinical characterization.* All patients are screened for mutations in *BEST1.* Diagnosis is typically made based on fundus appearance, family history and a decreased Arden ratio in the EOG recorded according to International Society for Clinical Electrophysiology of Vision (ISCEV) standards (Marmor, Brigell et al. 2011). VMD patients are staged (subclinical, pseudohypopyon, vitelliruptive, atrophic) based on fundus photographs and OCT findings (Gass, Chuang et al. 1988, Ferrara, Costa et al. 2010).

*SW-AF.* For qualitative and quantitative qAF, SW-AF images (30°; 488 nm excitation) are acquired using a cSLO (Spectralis HRA+OCT; Heidelberg Engineering, Heidelberg, Germany) modified by the insertion of an internal fluorescent reference to account for variable laser power and detector gain as described (Delori, Greenberg et al. 2011). Before image acquisition, pupils are dilated to at least 7 mm with topical 1% tropicamide and 2.5% phenylephrine. During focusing and alignment, the fundus is exposed for 20-30 seconds to bleach rhodopsin (Delori, Greenberg et al. 2011). Detector sensitivity is adjusted so that grey levels (GL) do not exceed the linear range of the detector (GL < 175) (Delori, Greenberg et al. 2011).

*NIR-AF.* The NIR-AF originates primarily from RPE melanin with a smaller contribution from the choroid (Keilhauer and Delori 2006, Duncker, Tabacaru et al. 2013, Duncker, Greenberg et al. 2014, Duncker, Marsiglia et al. 2014, Sparrow, Marsiglia et al. 2015, Lee, Xie et al. 2016, Schuerch, Marsiglia et al. 2016). Inventors document areas of NIR-AF in fundus AF and correlate them with changes in SW-AF, qAF, retinal structure evaluated with OCT, visual sensitivity quantified by fundus perimetry (MAIA-Nidek-MP-1S) and automated light and dark-adapted perimetry at yearly intervals over a four-year period.

*SD-OCT.* High resolution single foveal scans and volume thickness maps are utilized. Assignment of reflectivity bands in OCT scans is based on Staurenghi et al. (Staurenghi, Sadda et al. 2014). The thickness of photoreceptor-attributable layers is evaluated using a computer aided manual segmentation technique. Progression of photoreceptor outer segment-equivalent thickening will be evaluated (Querques, Zerbib et al. 2011, Duncker, Greenberg et al. 2014); the area of ellipsoid zone (EZ) loss in the SD-OCT images (Duncker, Greenberg et al. 2014) is measured and inventors validate the finding that thickening of the zone of interdigitation (IZ) between outer segments and RPE apical processes (IZ) is the earliest pathological marker (Ferrara, Costa et al. 2010, Querques, Zerbib et al. 2011). This pre-symptomatic stage is yet to be characterized and may be the best window for *BEST1* -preventive CRISPR. Scan tilt and Henle fiber obstructions are corrected during both image acquisition and analysis. Changes in structures (e.g. width of EZ loss) are compared to microperimetry.

Fluid resolution on OCT thickness measurements (in microns) provides sensitive therapeutic endpoints and can reduce the required sample size in a gene therapy trial.

Track qAF levels in the vitelliform lesion at each stage of the disease to determine whether qAF intensities are greater than would be expected at the same location in age-matched healthy eyes.

The formation of bisretinoid in the POS is accelerated because photoreceptor cells are disabled and thus unable to expend the energy to detoxify retinaldehyde.

Background. The natural SW-AF that is excited by SW 488 nm light and imaged by confocal scanning laser ophthalmoscopy exhibits the spectral features, location, and an age-relationship indicative of a principle origin from RPE bisretinoid lipofuscin. Although It has been assumed (Frangieh, Green et al. 1982, Weingeist, Kobrin et al. 1982, O'Gorman, Flaherty et al. 1988, Delori, Dorey et al. 1995, Vedantham and Ramasamy 2005, Spaide, Noble et al. 2006, Bakall, Radu et al. 2007, Zhang, Bregman et al. 2011, Singh, Shen et al. 2013) that RPE lipofuscin is increased in non-lesion areas of VMD, inventors demonstrated by non-invasive qAF that SW-AF intensities outside the lesion are within normal limits for age (Duncker, Marsiglia et al. 2014). The experimental is to understand the elevated SW-AF of the vitelliform lesion. We have previously reported that the emission spectra recorded within vitelliform lesions of VMD patients and RPE lipofuscin of healthy eyes have similar shapes, thus indicating that the fluorophores are the same (Duncker, Marsiglia et al. 2014). Since RPE lipofuscin is well known to originate from POS (Sparrow and Yamamoto 2012), the auto-fluorescent material in the vitelliform lesion likely originates from accumulating outer segment debris within the lesion. Indeed, the formation of bisretinoid in the POS is accelerated because the photoreceptor cells are disabled and thus unable to expend the energy to detoxify retinaldehyde. Thus, the intense AF is indicative of dysfunctional photoreceptor cells. This view is consistent with the thinning of the ONL apparent in OCT (Duncker, Marsiglia et al. 2014). This issue is important since heightened bisretinoid levels in photoreceptor cells would be toxic and thus would further accelerate photoreceptor degeneration.

Successful completion of the prior described work will serve to identify patients that could benefit from CRISPR and perhaps from future small molecule therapeutics having potential to combat over-production of bisretinoid formation (Sparrow, Duncker et al. 2016). Targeting the visual cycle to reduce bisretinoid formation is currently under preclinical and clinical study (Sparrow, Duncker et al. 2016).

### Experimental design

*Imaging techniques.* The vitelliform lesions are identified in color fundus photographs, NIR-reflectance, NIR-AF and in SW-AF images. Lesion areas in SW-AF images are be measured (Image J) and compared to the lesion defined by perimetry. For patients in the vitelliform and pseudohypopyon stages, within-lesion qAF levels will be measured in rectangular areas (region of interest, ROI) at a minimum of 3 locations (26 X 26 pixels) in both eyes of VMD patients. Measurements are acquired from 2 images (each eye) and averaged to obtain a single value/eye. qAF in VMD patients are compared to values acquired from our healthy eye database. The control values are from 5 age-similar healthy eyes at the same eccentricity (pixels) and from subjects at the same race/ethnicity within our database (374 eyes of 277 subjects; white, Asian, Hispanic, female and male) (Greenberg, Duncker et al. 2013).

SW-AF images for qAF analysis are acquired according to an established protocol (Delori, Greenberg et al. 2011). Briefly SW-AF images (30°; 486 nm excitation) are acquired using a confocal scanning laser ophthalmoscope (Spectralis HRA+OCT; Heidelberg Engineering, Heidelberg, Germany) modified by the insertion of an internal fluorescent reference to account for variations in laser power and detector gain. Detector sensitivity is adjusted so that grey levels (GLs) do not exceed the linear range of the detector (GL < 175) in the high-speed mode (8.9 frames/s) within a 30x30° field (768x768 pixels). Frames are aligned and averages are saved in "non-normalized mode" (no histogram stretching). GL are calibrated to the GL of the internal reference after accounting for the zero GL, magnification (refraction), and ocular media absorption.

For statistical analysis, a mixed-effects model will be fit to the data with adjustment for location, eccentricity, age, gender and race-ethnicity, from which normalized scores were calculated for each location in each eye, and those z-scores outside the 95% confidence interval were noted.

The within-lesion qAF is many folds higher than in non-lesion parafoveal regions of retina. These extremely high levels of AF reflect accelerated bisretinoid in photoreceptor cells impaired by the milieu of the vitelliform lesion. As an alternative or in addition to the ROIqAF approach, inventors measure qAF with our standard qAF segmental analysis (Delori, Greenberg et al. 2011). In this analysis, mean qAF in each of 8 pre-set circularly arranged segments at an eccentricity of approximately 5-9° is computed and averaged to yield a single qAF value for each image. Segments can be excluded if they do not overlap with the lesion. Inventorsuse the Bland-Altmann approach to assess repeatability of the measurements and concordance between eyes of the same patient.

Validate whether the untreated contralateral eye is an appropriate internal control.

Without being bound by theory, it is the inventor's hypothesis that there is a bilateral concordance in phenotypic severity and rates of VMD progression.

### Methods.

In individuals with VMD, inventors use the datasets to longitudinally calculate extent of bilateral concordance in terms of disease progression in eyes of the same patient. Inventors evaluate variability and symmetry of left and right vitelliform lesions in OCT, NIR-AF and SW-AF images and kinetic perimetry outcomes at baseline and after 4 years. Inventors also compare vitelliform lesion area and qAF.

Interocular agreement/correlation are tested by calculating intraclass correlation coefficients (ICC) and Pearson's coefficients. Based on the 95% confidence interval of the ICC estimate, values less than 0.5, 0.5 to 0.75, 0.75 to 0.90, and greater than 0.90 will be interpreted as poor, moderate, good, and excellent, respectively.

**Table 2. Demographics of current iPS donors and status of BEST1 testing results**

| **Subject ID** | **AGE** | **SEX** | **BES1 MUTATION** |
|---|---|---|---|
| 1 | 52 | M | p.Arg218His |
| 2 | 5 | F | p.Ala10Thr |
| 3 | 6 | M | p.Leu234Pro |
| 4 | 62 | M | p.Ala243Thr |
| 5 | 8 | F | Results Pending |
| 6 | 55 | M | p.Asn296Ser |
| 7 | 20 | M | p.Asp302Ala |
| 8 | 58 | F | p.Asp302Ala |
| 9 | 53 | F | p.Leu294Val |
| 10 | 15 | M | p.Pro274Arg Homo |
| 11 | 31 | F | p.Cys211Trp |
| 12 | 43 | F | IVS1+5G>A homo |
| 13 | 41 | M | p.Ala243Thr |
| 14 | 13 | M | p.Arg218His |
| 15 | 69 | M | p.lle201Thr |
| 16 | 54 | M | Results Pending |
| 17 | 39 | M | p.Asn73Thr |
| 18 | 45 | M | p.Glu300Asp |
| 19 | 42 | M | p.Arg218His |
| 20 | 15 | M | Results Pending |
| 21 | 24 | M | p.Phe17Leu |
| 22 | 30 | F | p.R218H; R158H |
| 23 | 32 | F | Results Pending |
| 24 | 33 | M | Results Pending |
| 25 | 44 | M | p.Gln293Lys |
| 26 | 43 | M | p.lle295Thr |
| 27 | 72 | M | p.Gly299Glu |
| 28 | 55 | M | Results Pending |
| 29 | 54 | M | Results Pending |

The tests shows a similar rate of disease progression between eyes of patients, which can provide support for using the contralateral, untreated eye as a control for treatment outcomes. If there is a significant difference in disease progression between a patient's two eyes, then inventors need to use the data we collect on the natural history of VMD progression to compare any progression in photoreceptor disease following gene therapy.

Inventors generated fibroblasts or iPS cell lines from 29 out of 40 VMD patients and recruit a total of at least 52 patients.

Additional patients can be recruited. The following are a few inclusion and exclusion criteria.ecruit additional patients.

### Inclusion Criteria

1. Willing and able to provide a completed/signed informed consent or assent that includes parental consent.
2. Ability to follow-up at all required visits over the 4- month period.
3. Age ≥ 4 years.
4. Clinical diagnosis of Best Disease with molecular confirmation of a pathogenic BEST1 mutation(s) or a characteristic electroretinogram (ERG) and electrooculogram (EOG) with features consistent with Best Disease on OCT.
5. Baseline visual acuity of 20/40 or worse.
6. Ocular media suitable for multimodal retinal imaging. Pupil dilation and fixation that permits quality photographic imaging.
7. Good general health without significant physical examination findings or clinically significant abnormal laboratory results.

### Exclusion Criteria

1. Pre-existing AAV immunity.
2. Current or past retina disease that may confound longitudinal assessment of disease progression. This includes but is not limited to vitreous hemorrhage, history of rhegmatogenous retinal detachment, and glaucoma.
3. History or evidence of previous intraocular surgery which includes but is not limited to: vitrectomy, cataract extraction, and LASIK which were performed within 6 months.
4. Patient expects to have cataract removal at any time during the study duration.
5. Mutations in genes that cause dominant or X-linked inherited retinal dystrophies, or the presence of biallelic mutations known to cause recessively inherited retinal disease.
6. Prior receipt of any experimental therapies.
7. Received chemotherapy or biological therapy in the past that may confound results.

Conventional gene supplementation therapy involving the delivery of a WT cDNA (Cideciyan, Jacobson et al. 2013, Bainbridge, Mehat et al. 2015) is unlikely to ameliorate autosomal-dominant or dominant-negative conditions due to the fact that the endogenous mutant (MT) allele is left intact. CRISPR-based gene-editing technology, which involves the Cas9 nuclease directed by an engineered guide RNA (gRNA) to specifically edit a sequence at the designated location in the genome, has been proposed to destroy the mutant gene (Fu, Foden et al. 2013, Ran, Hsu et al. 2013, Pyzocha, Ran et al. 2014, Smith, Gore et al. 2014, Tsai, Wyvekens et al. 2014, Smith, Abalde-Atristain et al. 2015, Wu, Tsai et al. 2016). The approach brings this technology to the next level and enables allele-specific (or parental chromosome-specific) targeting rather than conventional mutation-specific targeting for such dominantly-inherited conditions. In addressing this hurdle, the approach ablates mutant genes in a manner that is allele-specific so that the WT allele would be left intact to support normal function (FIG. 11). The present method may be used to treat other macular degenerative and dominantly-inherited conditions.

Justification of iPS cells as allele-specific gene editing targets: In humans, BEST1 functions as a Ca²⁺-activated Cl⁻ channel, and RPE cells of VMD patients show abnormal Clefflux (Moshfegh, Velez et al. 2016, Li, Zhang et al. 2017). However, BEST1 may have a less essential role in Cl⁻ efflux in mice, as RPE cells from heterozygous Best1^{+/W93C} mice exhibit normal Cl⁻ conductance (Zhang, Stanton et al. 2010). This same mutation (+/W93C) would cause VMD in humans. Given this lack of an appropriate animal model, inventors have developed a human model to test restoration of BEST1 function by establishing CRISPR-edited RPE derived from patients' own iPS cells ("iRPE"). Inventors also created a novel Cl⁻ biosensor to readily evaluate whether CRISPR-edited BEST1 protein effuxes Cl⁻ ions appropriately.

Although many CRISPR-based gene therapy approaches focus on a mutation-specific gene repair strategy, the present method employs a more generalizable approach. Otherwise, more than 200 different gRNAs and respective clinical trials would be required to encompass all the mutations found in BEST1. Thus, inventors have devised a novel method that capitalizes on SNP heterozygosity to create a system applicable to multiple mutations. SNPs in the BEST1 transcription start site (TSS) can be used to enable a CRISPR gRNA to discriminate between MT and WT alleles. The approach involves mapping these SNPs to a pathological mutation and designing a gRNA to direct Cas9 to cleave only the diseased allele. This innovative approach allows targeting and editing of all BEST1 mutations, rather than just one.

The 2nd generation application of CRISPRi enables strong gene repression activity due to the use of PUF-KRAB (Pumilio mRNA binding factor-Kruppel-associated Box Domain) repressors (FIG.s 15A-15B). The CRISPRi design overcomes the oversizing hurdle to fitting KRAB system (FIG. 15B) into AAV vectors and provides a more stable gene inhibition to the designated gene (Cheng, Jillette et al. 2016). CRISPRi is also suggested to be a safer technology since no genomic DNA modification is involved.

The proposed studies use an innovative outcome measurement. Inventors have developed a genetically-encoded fluorescent chloride sensor to assess changes in intracellular Cl⁻concentration in normal versus VMD patient-derived iPS cells that have been differentiated into RPE. Thus, we can test for restored BEST1 function not only in animal models, but also directly in patients' RPE. The present methods can use stem cell therapy to cure currently non-treatable autosomal dominant diseases.

### Example 9. CRISPR3.0: A treatment for patients with Best VMD patients carrying different dominant mutations.

The methods and systems of the disclosure provide for treatment of certain types of SNP genotypes. First, Sanger sequencing will be used to determine the SNP genotype of patients with BEST VMD. Approximately, 50% of the patients are expected to be heterozygous for one of the SNPs and thus qualified for the treatment using CRISPR3.0. Next, the eligible patients will be further haplotyped by pedigree analysis (or abridged linkage genotyping) to confirm the allelic linkage between each of the SNP variants and the mutation. Finally, the patient will be treated with one of the two sets of treatment materials for selective ablation of the mutant allele.

In CRISPR3.0, a pair of closely-linked SNPs are utilized in CRISPR3.0 to mark the identity of each homologue in a pair of homologous. In one embodiment, the closely-linked SNPs comprise r972355 and rs972353; however, any closely-linked set of SNPs for BEST1 is encompassed in the treatment scheme set forth herein. CRISPR3.0 involves the use of at least three gRNAs. Three gRNAs are designed in order to achieve CRISPR3.0: gRNA-a targets SNP variant 1; gRNA-b targets SNP variant 2; and, gRNA-c targets a downstream intron region. When mutation is present in homologue 1 (e.g., Genotype 1), gRNA-a + gRNA-c will be used to specifically destroy expression of the mutant *BEST1* gene from homologue 1. If the mutation is instead on homologue 2 (e.g., Genotype 2), gRNA-b + gRNA-c will be used.

As shown in Fig. 22A - (A) A pair of closely-linked SNPs, r972355 and rs972353, are utilized in "CRISPR3.0" to mark the identity of each homologue in a pair of homologous. All four possible combinations of nucleotide variants of both SNPs on chromosomes are shown. Three gRNAs were designed in order to achieve CRISPR3.0: gRNA-a targets rs972355 nucleotide variant G (and not A); gRNA-b targets rs972353 nucleotide variant G (and not C); gRNA-c targets a downstream intron region. When mutation is present in homologue 1 (Genotype 1), gRNA-a + gRNA-c is used to specifically destroy expression of the mutant *BEST1* gene from homologue 1. If the mutation is instead on homologue 2 (Genotype 2), gRNA-b + gRNA-c are used. Because these two SNPs do not distinguish between the two homologues in genotypes 3 and 4, these genotypes would require the use of other SNPs and gRNAs.

FIG. 22B - (B) shows the gRNA sets designed based on rs972355 and rs972353 can treat ~50% of the Best VMD patient population (Genotypes 1 + 2, based on TOPMED database). DNA from each patient is analyzed to determine the genotype before treatment (See FIG.. 22Dz, workflow).

FIG. 23A shows that in CRISPR3.0, a pair of closely-linked SNPs, r972355 and rs972353, are utilized to mark the identity of each homologue in a pair of homologous. All four possible combinations of nucleotide variants of both SNPs on chromosomes are shown. Three gRNAs were designed in order to achieve CRISPR3.0: gRNA-a targets rs972355 nucleotide variant G (and not A); gRNA-b targets rs972353 nucleotide variant G (and not C); gRNA-c targets a downstream intron region. When a mutation is present in homologue 1 (Genotype 1), gRNA-a + gRNA-c is used to specifically destroy expression of the *BEST1* gene from homologue 1. If the mutation is instead on homologue 2 (Genotype 2), gRNA-b + gRNA-c is used. Because these two SNPs do not distinguish between the two homologues in genotypes 3 and 4, these genotypes would require the use of other SNPs and gRNAs.

**FIG. 23B** - **(B)** The gRNA sets designed based on rs972355 and rs972353 can treat -50% of the total Best VMD patient population (Genotypes 1 + 2, based on TOPMED database). DNA from each patient will be analyzed to determine the genotype before treatment (See Fig. 22D, workflow).

**FIG. 24** is an overview of CRISPR 1.0, CRIPSR 2.0 and CRIPSR 3.0 Strategies.

**FIG. 25** is a workflow for CRISPR 3.0 Treatment. First, Sanger sequencing can be used to determine the SNP genotype of patients with BEST VMD; 50% of the patients are expected to be heterozygous for one of the SNPs (49.9%, see Fig. 25) and thus qualified for the treatment. Next, the eligible patients are further haplotyped by pedigree analysis (or abridged linkage genotyping, see **FIG.**24) to confirm the allelic linkage between each of the SNP variants and the mutation. Finally, the patient is treated with one of the two sets of treatment materials (see, **FIG.**. **24**) for selective ablation of the mutant allele.

### Example 10. Treatment of Best VMD Patients

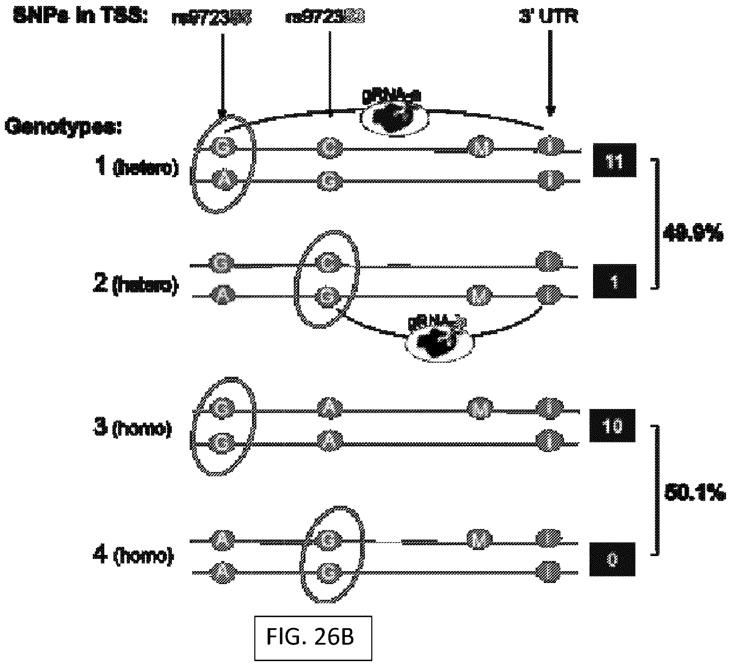

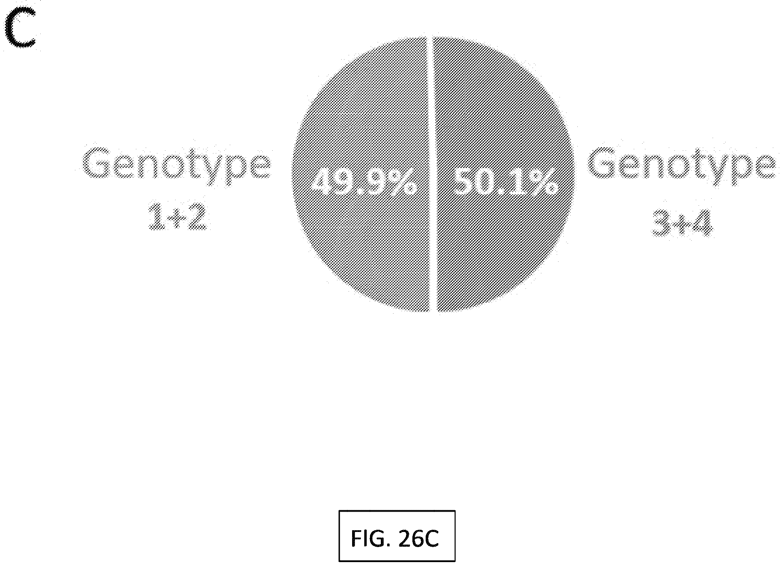

CSGT (CRISPR3.0) is a treatment for roughly half of Best VMD patients. The BEST1 gene was sequenced using blood samples from my 35 Best VMD patients to reveal all four possible combinations of nucleotide variants of both upstream SNPs, rs972355 and rs972353 (note that these two SNPs are genetically linked). CRISPR3.0, which exploits differences in TSS SNPs between homologous chromosomes, mediates selective gene truncation when the targeted TSS SNP is heterozygous. Thus, genotypes 1 and 2 (SNP heterozygotes in cis with the mutation), which represent 49.9% of the Best VMD patient population, are treatable by CRISPR3.0, and genotypes 3 and 4 (SNP homozygotes) are can be treated by CRISPR2.0. FIGS. 26 A, B and C.

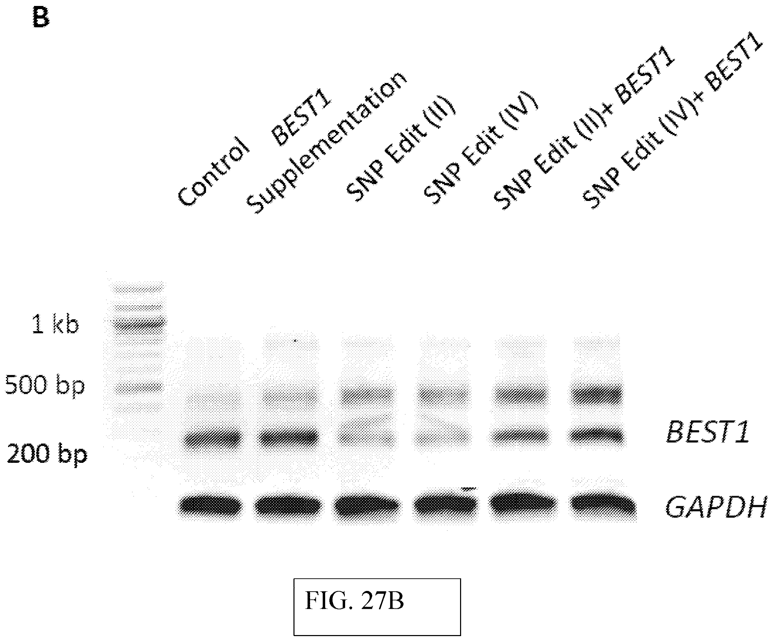

In FIG. 27B, the allele-specific excision of genomic *BEST1* by dual gRNA and measurement of *BEST1* expression upon SNP editing in RPE cells *in vitro.* (A) Genomic *BEST1* was truncated in 293FT cells in vitro by different dual-gRNA systems (CRISPR 3.0). Of the four groups. the truncated *BEST1* alleles of different sizes (arrows) were detected in three groups (I, II and IV). FIG. 27A. (B) *BEST1* expression is robust in CRISPR2.0- and 3.0-edited VMD iRPE. iRPE derived from VMD patient fibroblasts was transduced with lentiviral dual-gRNA vectors for CRISPR2.0 or 3.0 editing of BEST1 alleles; vectors carried WT BEST1 cDNA (+ WT) or not; WT iRPE was not transduced. After two weeks, RNA was collected, cDNA generated, and expression of BEST1 transcript determined by qPCR. PCR product of *BEST1* is ~260 bp. WT, WT RPE homozygous for SNP rs972355; Control, dual vector carrying WT *BEST1* cDNA only (no gRNA); GAPDH, internal control. No lentiviral vector was employed in the control group; GAPDH was used as the internal control. FIG. 27 B.

### Equivalents

Unless otherwise defined, all technical and scientific terms used herein have the same meaning as commonly understood by one of ordinary skill in the art to which this technology belongs.

The present technology illustratively described herein may suitably be practiced in the absence of any element or elements, limitation or limitations, not specifically disclosed herein. Thus, for example, the terms "comprising," "including," "containing," etc. shall be read expansively and without limitation. Additionally, the terms and expressions employed herein have been used as terms of description and not of limitation, and there is no intention in the use of such terms and expressions of excluding any equivalents of the features shown and described or portions thereof, but it is recognized that various modifications are possible within the scope of the present technology claimed.

Thus, it should be understood that the materials, methods, and examples provided here are representative of preferred aspects, are exemplary, and are not intended as limitations on the scope of the present technology.

The present technology has been described broadly and generically herein. Each of the narrower species and sub-generic groupings falling within the generic disclosure also form part of the present technology. This includes the generic description of the present technology with a proviso or negative limitation removing any subject matter from the genus, regardless of whether or not the excised material is specifically recited herein.

In addition, where features or aspects of the present technology are described in terms of Markush groups, those skilled in the art will recognize that the present technology is also thereby described in terms of any individual member or subgroup of members of the Markush group.

All publications, patent applications, patents, and other references mentioned herein are expressly incorporated by reference in their entirety, to the same extent as if each were incorporated by reference individually. In case of conflict, the present specification, including definitions, will control.

Other aspects are set forth within the following claims.

### Reference

Asuri, P., M. A. Bartel, T. Vazin, J.-H. Jang, T. B. Wong and D. V. Schaffer (2012). "Directed evolution of adeno-associated virus for enhanced gene delivery and gene targeting in human pluripotent stem cells." Molecular Therapy 20(2): 329-338.
Bainbridge, J. W., M. S. Mehat, V. Sundaram, S. J. Robbie, S. E. Barker, C. Ripamonti, A. Georgiadis, F. M. Mowat, S. G. Beattie and P. J. Gardner (2015). "Long-term effect of gene therapy on Leber's congenital amaurosis." New England Journal of Medicine 372(20): 1887-1897.
Bakall, B., R. A. Radu, J. B. Stanton, J. M. Burke, B. S. McKay, C. Wadelius, R. F. Mullins, E. M. Stone, G. H. Travis and A. D. Marmorstein (2007). "Enhanced accumulation of A2E in individuals homozygous or heterozygous for mutations in BEST1 (VMD2)." Exp Eye Res 85(1): 34-43.
Bassuk, A. G., A. Zheng, Y. Li, S. H. Tsang and V. B. Mahajan (2016). "Precision Medicine: Genetic Repair of Retinitis Pigmentosa in Patient-Derived Stem Cells." Sci Rep 6: 19969.
Bassuk, A. G., A. Zheng, Y. Li, S. H. Tsang and V. B. Mahajan (2016). "Precision medicine: genetic repair of retinitis pigmentosa in patient-derived stem cells." Scientific reports 6.
Bengtsson, N. E., J. K. Hall, G. L. Odom, M. P. Phelps, C. R. Andrus, R. D. Hawkins, S. D. Hauschka, J. R. Chamberlain and J. S. Chamberlain (2017). "Muscle-specific CRISPR/Cas9 dystrophin gene editing ameliorates pathophysiology in a mouse model for Duchenne muscular dystrophy." Nature Communications 8.
Beurdeley, M., F. Bietz, J. Li, S. Thomas, T. Stoddard, A. Juillerat, F. Zhang, D. F. Voytas, P. Duchateau and G. H. Silva (2013). "Compact designer TALENs for efficient genome engineering." Nature communications 4: 1762.
Boon, C. J., B. J. Klevering, A. I. den Hollander, M. N. Zonneveld, T. Theelen, F. P. Cremers and C. B. Hoyng (2007). "Clinical and genetic heterogeneity in multifocal vitelliform dystrophy." Arch Ophthalmol 125(8): 1100-1106.
Boon, C. J., T. Theelen, E. H. Hoefsloot, M. J. van Schooneveld, J. E. Keunen, F. P. Cremers, B. J. Klevering and C. B. Hoyng (2009). "Clinical and molecular genetic analysis of best vitelliform macular dystrophy." Retina 29(6): 835-847.
Cabral, T., J. E. DiCarlo, S. Justus, J. D. Sengillo, Y. Xu and S. H. Tsang (2017). "CRISPR applications in ophthalmologic genome surgery." Current opinion in ophthalmology 28(3): 252-259.
Cheng, A. W., N. Jillette, P. Lee, D. Plaskon, Y. Fujiwara, W. Wang, A. Taghbalout and H. Wang (2016). "Casilio: a versatile CRISPR-Cas9-Pumilio hybrid for gene regulation and genomic labeling." Cell research 26(2): 254.
Cho, G. Y., Y. Abdulla, J. D. Sengillo, S. Justus, K. A. Schaefer, A. G. Bassuk, S. H. Tsang and V. B. Mahajan (2017). "CRISPR-mediated Ophthalmic Genome Surgery." Curr Ophthalmol Rep 5(3): 199-206.
Cho, G. Y., S. Justus, J. D. Sengillo and S. H. Tsang (2017). "CRISPR in the Retina: Evaluation of Future Potential." Adv Exp Med Biol 1016: 147-155.
Cideciyan, A. V., S. G. Jacobson, W. A. Beltran, A. Sumaroka, M. Swider, S. Iwabe, A. J. Roman, M. B. Olivares, S. B. Schwartz and A. M. Komdromy (2013). "Human retinal gene therapy for Leber congenital amaurosis shows advancing retinal degeneration despite enduring visual improvement." Proceedings of the National Academy of Sciences 110(6): E517-E525.
Davis, R. J., C.-W. Hsu, Y.-T. Tsai, K. J. Wert, J. Sancho-Pelluz, C.-S. Lin and S. H. Tsang (2013). "Therapeutic margins in a novel preclinical model of retinitis pigmentosa." Journal of Neuroscience 33(33): 13475-13483.
Davis, R. J., J. Tosi, K. M. Janisch, J. M. Kasanuki, N.-K. Wang, J. Kong, I. Tsui, M. Cilluffo, M. L. Woodruff and G. L. Fain (2008). "Functional rescue of degenerating photoreceptors in mice homozygous for a hypomorphic cGMP phosphodiesterase 6 b allele (Pde6b H620Q)." Investigative ophthalmology & visual science 49(11): 5067-5076.
Davis, R. J., J. Tosi, K. M. Janisch, J. M. Kasanuki, N. K. Wang, J. Kong, I. Tsui, M. Cilluffo, M. L. Woodruff, G. L. Fain, C. S. Lin and S. H. Tsang (2008). "Functional rescue of degenerating photoreceptors in mice homozygous for a hypomorphic cGMP phosphodiesterase 6 b allele (Pde6bH620Q)." Invest Ophthalmol Vis Sci 49(11): 5067-5076.
Delori, F., J. P. Greenberg, R. L. Woods, J. Fischer, T. Duncker, J. Sparrow and R. T. Smith (2011). "Quantitative measurements of autofluorescence with the scanning laser ophthalmoscope." Invest Ophthalmol Vis Sci 52(13): 9379-9390.
Delori, F. C., C. K. Dorey, G. Staurenghi, O. Arend, D. G. Goger and J. J. Weiter (1995). "In vivo fluorescence of the ocular fundus exhibits retinal pigment epithelium lipofuscin characteristics." Invest Ophthalmol Vis Sci 36(3): 718-729.
Deyle, D. R., I. F. Khan, G. Ren, P.-R. Wang, J. Kho, U. Schwarze and D. W. Russell (2012). "Normal collagen and bone production by gene-targeted human osteogenesis imperfecta iPSCs." Molecular Therapy 20(1): 204-213.
DiCarlo, J. E., A. Deeconda and S. H. Tsang (2017). "Viral Vectors, Engineered Cells and the CRISPR Revolution." Adv Exp Med Biol 1016: 3-27.
Duncker, T., J. P. Greenberg, R. Ramachandran, D. C. Hood, R. T. Smith, T. Hirose, R. L. Woods, S. H. Tsang, F. C. Delori and J. R. Sparrow (2014). "Quantitative fundus autofluorescence and optical coherence tomography in best vitelliform macular dystrophy." Invest Ophthalmol Vis Sci 55(3): 1471-1482.
Duncker, T., M. Marsiglia, W. Lee, J. Zernant, S. H. Tsang, R. Allikmets, V. C. Greenstein and J. R. Sparrow (2014). "Correlations among near-infrared and short-wavelength autofluorescence and spectral-domain optical coherence tomography in recessive Stargardt disease." Invest Ophthalmol Vis Sci 55(12): 8134-8143.
Duncker, T., M. R. Tabacaru, W. Lee, S. H. Tsang, J. R. Sparrow and V. C. Greenstein (2013). "Comparison of near-infrared and short-wavelength autofluorescence in retinitis pigmentosa." Invest Ophthalmol Vis Sci 54(1): 585-591.
Ferrara, D. C., R. A. Costa, S. Tsang, D. Calucci, R. Jorge and K. B. Freund (2010). "Multimodal fundus imaging in Best vitelliform macular dystrophy." Graefes Arch Clin Exp Ophthalmol 248(10): 1377-1386.
Frangieh, G. T., W. R. Green and S. L. Fine (1982). "A histopathologic study of Best's macular dystrophy." Arch Ophthalmol 100(7): 1115-1121.
Fu, Y., J. A. Foden, C. Khayter, M. L. Maeder, D. Reyon, J. K. Joung and J. D. Sander (2013). "Highfrequency off-target mutagenesis induced by CRISPR-Cas nucleases in human cells." Nature biotechnology 31(9): 822-826.
Fung, A. T., S. Yzer, N. Goldberg, H. Wang, M. Nissen, A. Giovannini, J. E. Merriam, E. N. Bukanova, C. Cai and L. A. Yannuzzi (2015). "New best1 mutations in autosomal recessive bestrophinopathy." Retina (Philadelphia, Pa.) 35(4): 773.
Galietta, L. J., P. M. Haggie and A. Verkman (2001). "Green fluorescent protein-based halide indicators with improved chloride and iodide affinities." FEBS letters 499(3): 220-224.
Gass, J. D., E. L. Chuang and H. Granek (1988). "Acute exudative polymorphous vitelliform maculopathy." Trans Am Ophthalmol Soc 86: 354-366.
Gouras, P., J. Kong and S. H. Tsang (2002). "Retinal degeneration and RPE transplantation in RPE65-/- mice." Investigative ophthalmology & visual science 43(10): 3307-3311.
Greenberg, J. P., T. Duncker, R. L. Woods, R. T. Smith, J. R. Sparrow and F. C. Delori (2013). "Quantitative fundus autofluorescence in healthy eyes." Invest Ophthalmol Vis Sci 54(8): 5684-5693.
Guziewicz, K. E., B. Zangerl, A. M. Komáromy, S. Iwabe, V. A. Chiodo, S. L. Boye, W. W. Hauswirth, W. A. Beltran and G. D. Aguirre (2013). "Recombinant AAV-mediated BEST1 transfer to the retinal pigment epithelium: analysis of serotype-dependent retinal effects." PLoS One 8(10): e75666.
Janisch, K. M., J. M. Kasanuki, M. C. Naumann, R. J. Davis, C.-S. Lin, S. Semple-Rowland and S. H. Tsang (2009). "Light-dependent phosphorylation of the gamma subunit of cGMP-phophodiesterase (PDE6γ) at residue threonine 22 in intact photoreceptor neurons." Biochemical and biophysical research communications 390(4): 1149-1153.
Justus, S., Y.-T. Tsai, W. H. Wu, C. W. Hsu, W.-P. Wu, A. G. Bassuk, V. B. Mahajan and S. H. Tsang (2016). "Genome Editing in the Retina: a Case Study in CRISPR for a Patient-Specific Autosomal Dominant Retinitis Pigmentosa Model." Turksken K, ed. Genome Editing. New York, NY: Springer 00.
Keilhauer, C. N. and F. C. Delori (2006). "Near-infrared autofluorescence imaging of the fundus: visualization of ocular melanin." Invest Ophthalmol Vis Sci 47(8): 3556-3564.
Khan, I. F., R. K. Hirata, P.-R. Wang, Y. Li, J. Kho, A. Nelson, Y. Huo, M. Zavaljevski, C. Ware and D. W. Russell (2010). "Engineering of human pluripotent stem cells by AAV-mediated gene targeting." Molecular Therapy 18(6): 1192-1199.
Kleinstiver, B. P., M. S. Prew, S. Q. Tsai, N. T. Nguyen, V. V. Topkar, Z. Zheng and J. K. Joung (2015). "Broadening the targeting range of Staphylococcus aureus CRISPR-Cas9 by modifying PAM recognition." Nature biotechnology 33(12): 1293-1298.
Koch, S. F., Y. T. Tsai, J. K. Duong, W. H. Wu, C. W. Hsu, W. P. Wu, L. Bonet-Ponce, C. S. Lin and S. H. Tsang (2015). "Halting progressive neurodegeneration in advanced retinitis pigmentosa." J Clin Invest 125(9): 3704-3713.
Lai, C. C., P. Gouras, K. Doi, F. Lu, H. Kjeldbye, S. P. Goff, R. Pawliuk, P. Leboulch and S. H. Tsang (1999). "Tracking RPE transplants labeled by retroviral gene transfer with green fluorescent protein." Invest Ophthalmol Vis Sci 40(9): 2141-2146.
Lai, C. C., P. Gouras, K. Doi, S. H. Tsang, S. P. Goff and P. Ashton (2000). "Local immunosuppression prolongs survival of RPE xenografts labeled by retroviral gene transfer." Investigative ophthalmology & visual science 41(10): 3134-3141.
Lee, W., Y. Xie, J. Zernant, B. Yuan, S. Bearelly, S. H. Tsang, J. R. Lupski and R. Allikmets (2016). "Complex inheritance of ABCA4 disease: four mutations in a family with multiple macular phenotypes." Hum Genet 135(1): 9-19.
Li, Y., L. Chan, H. V. Nguyen and S. H. Tsang (2016). "Personalized Medicine: Cell and Gene Therapy Based on Patient-Specific iPSC-Derived Retinal Pigment Epithelium Cells." Adv Exp Med Biol 854: 549-555.
Li, Y., Y.-T. Tsai, C.-W. Hsu, D. Erol, J. Yang, W.-H. Wu, R. J. Davis, D. Egli and S. H. Tsang (2012). "Long-term safety and efficacy of human-induced pluripotent stem cell (iPS) grafts in a preclinical model of retinitis pigmentosa." Molecular medicine 18(1): 1312.
Li, Y., Y. T. Tsai, C. W. Hsu, D. Erol, J. Yang, W. H. Wu, R. J. Davis, D. Egli and S. H. Tsang (2012). "Long-term safety and efficacy of human induced pluripotent stem cell (iPS) grafts in a preclinical model of retinitis pigmentosa." Molecular medicine 18: 1312-1319.
Li, Y., W.-H. Wu, C.-W. Hsu, H. V. Nguyen, Y.-T. Tsai, L. Chan, T. Nagasaki, I. H. Maumenee, L. A. Yannuzzi and Q. V. Hoang (2014). "Gene therapy in patient-specific stem cell lines and a preclinical model of retinitis pigmentosa with membrane frizzled-related protein defects." Molecular Therapy 22(9): 1688-1697.
Li, Y., W. H. Wu, C. W. Hsu, H. V. Nguyen, Y. T. Tsai, L. Chan, T. Nagasaki, I. H. Maumenee, L. A. Yannuzzi, Q. V. Hoang, H. Hua, D. Egli and S. H. Tsang (2014). "Gene therapy in patient-specific stem cell lines and a preclinical model of retinitis pigmentosa with membrane frizzled-related protein defects." Mol Ther 22(9): 1688-1697.
Li, Y., Y. Zhang, Y. Xu, A. Kittredge, N. Ward, S. Chen, S. H. Tsang and T. Yang (2017). "Patientspecific mutations impair BESTROPHIN1's essential role in mediating Ca2+-dependent Cl-currents in human RPE." eLife 6.
Long, C., L. Amoasii, A. A. Mireault, J. R. McAnally, H. Li, E. Sanchez-Ortiz, S. Bhattacharyya, J. M. Shelton, R. Bassel-Duby and E. N. Olson (2016). "Postnatal genome editing partially restores dystrophin expression in a mouse model of muscular dystrophy." Science 351(6271): 400-403.
Long, C., J. R. McAnally, J. M. Shelton, A. A. Mireault, R. Bassel-Duby and E. N. Olson (2014). "Prevention of muscular dystrophy in mice by CRISPR/Cas9-mediated editing of germline DNA." Science 345(6201): 1184-1188.
M Scharenberg, A., P. Duchateau and J. Smith (2013). "Genome engineering with TAL-effector nucleases and alternative modular nuclease technologies." Current gene therapy 13(4): 291-303. Mali, P., J. Aach, P. B. Stranges, K. M. Esvelt, M. Moosburner, S. Kosuri, L. Yang and G. M. Church (2013). "CAS9 transcriptional activators for target specificity screening and paired nickases for cooperative genome engineering." Nature biotechnology 31(9): 833-838.
Marmor, M. F., M. G. Brigell, D. L. McCulloch, C. A. Westall, M. Bach and V. International Society for Clinical Electrophysiology of (2011). "ISCEV standard for clinical electro-oculography (2010 update)." Doc Ophthalmol 122(1): 1-7.
Marmorstein, A. D., H. E. Cross and N. S. Peachey (2009). "Functional roles of bestrophins in ocular epithelia." Prog Retin Eye Res 28(3): 206-226.
Marmorstein, L. Y., J. Wu, P. McLaughlin, J. Yocom, M. O. Karl, R. Neussert, S. Wimmers, J. B. Stanton, R. G. Gregg, O. Strauss, N. S. Peachey and A. D. Marmorstein (2006). "The light peak of the electroretinogram is dependent on voltage-gated calcium channels and antagonized by bestrophin (best-1)." J Gen Physiol 127(5): 577-589.
Millington-Ward, S., N. Chadderton, M. O'reilly, A. Palfi, T. Goldmann, C. Kilty, M. Humphries, U. Wolfrum, J. Bennett and P. Humphries (2011). "Suppression and replacement gene therapy for autosomal dominant disease in a murine model of dominant retinitis pigmentosa." Molecular therapy 19(4): 642-649.
Moshfegh, Y., G. Velez, Y. Li, A. G. Bassuk, V. B. Mahajan and S. H. Tsang (2016). "BESTROPHIN1 mutations cause defective chloride conductance in patient stem cell-derived RPE." Human molecular genetics 25(13): 2672-2680.
Mullins, R. F., K. T. Oh, E. Heffron, G. S. Hageman and E. M. Stone (2005). "Late development of vitelliform lesions and flecks in a patient with best disease: clinicopathologic correlation." Arch Ophthalmol 123(11): 1588-1594.
Nelson, C. E., C. H. Hakim, D. G. Ousterout, P. I. Thakore, E. A. Moreb, R. M. C. Rivera, S. Madhavan, X. Pan, F. A. Ran and W. X. Yan (2016). "In vivo genome editing improves muscle function in a mouse model of Duchenne muscular dystrophy." Science 351(6271): 403-407. Nguyen, H. V., Y. Li and S. H. Tsang (2015). "Patient-specific iPSC-derived RPE for modeling of retinal diseases." Journal of clinical medicine 4(4): 567-578.
North, V., R. Gelman and S. H. Tsang (2014). "Juvenile-onset macular degeneration and allied disorders." Dev Ophthalmol 53: 44-52.
O'Gorman, S., W. A. Flaherty, G. A. Fishman and E. L. Berson (1988). "Histopathologic findings in Best's vitelliform macular dystrophy." Arch Ophthalmol 106(9): 1261-1268.
Ortega, S., M. Ittmann, S. H. Tsang, M. Ehrlich and C. Basilico (1998). "Neuronal defects and delayed wound healing in mice lacking fibroblast growth factor 2." Proceedings of the National Academy of Sciences 95 (10): 5672-5677.
Pang, J.-j., A. Lauramore, W.-t. Deng, Q. Li, T. J. Doyle, V. Chiodo, J. Li and W. W. Hauswirth (2008). "Comparative analysis of in vivo and in vitro AAV vector transduction in the neonatal mouse retina: effects of serotype and site of administration." Vision research 48(3): 377-385.
Petersen-Jones, S. M., L. M. Occelli, P. A. Winkler, W. Lee, J. R. Sparrow, M. Tsukikawa, S. L. Boye, V. Chiodo, J. E. Capasso, E. Becirovic, C. Schon, M. W. Seeliger, A. V. Levin, S. Michalakis, W. W. Hauswirth and S. H. Tsang (2018). "Patients and animal models of CNGbeta1-deficient retinitis pigmentosa support gene augmentation approach." J Clin Invest 128(1): 190-206.
Platt, R. J., S. Chen, Y. Zhou, M. J. Yim, L. Swiech, H. R. Kempton, J. E. Dahlman, O. Parnas, T. M. Eisenhaure and M. Jovanovic (2014). "CRISPR-Cas9 knockin mice for genome editing and cancer modeling." Cell 159(2): 440-455.
Ponjavic, V., L. Eksandh, S. Andreasson, K. Sjostrom, B. Bakall, S. Ingvast, C. Wadelius and B. Ehinger (1999). "Clinical expression of Best's vitelliform macular dystrophy in Swedish families with mutations in the bestrophin gene." Ophthalmic Genet 20(4): 251-257.
Pyzocha, N. K., F. A. Ran, P. D. Hsu and F. Zhang (2014). "RNA-guided genome editing of mammalian cells." Gene Correction: Methods and Protocols: 269-277.
Qu, Z. and H. C. Hartzell (2008). "Bestrophin Cl- channels are highly permeable to HCO3." Am J Physiol Cell Physiol 294(6): C1371-1377.
Querques, G., J. Zerbib, R. Santacroce, M. Margaglione, N. Delphin, L. Querques, J. M. Rozet, J. Kaplan and E. H. Souied (2011). "The spectrum of subclinical Best vitelliform macular dystrophy in subjects with mutations in BEST1 gene." Invest Ophthalmol Vis Sci 52(7): 4678-4684.
Ran, F. A., L. Cong, W. X. Yan, D. A. Scott, J. S. Gootenberg, A. J. Kriz, B. Zetsche, O. Shalem, X. Wu and K. S. Makarova (2015). "In vivo genome editing using Staphylococcus aureus Cas9." Nature 520(7546): 186-191.
Ran, F. A., P. D. Hsu, C.-Y. Lin, J. S. Gootenberg, S. Konermann, A. E. Trevino, D. A. Scott, A. Inoue, S. Matoba and Y. Zhang (2013). "Double nicking by RNA-guided CRISPR Cas9 for enhanced genome editing specificity." Cell 154(6): 1380-1389.
Rapti, K., F. Stillitano, I. Karakikes, M. Nonnenmacher, T. Weber, J.-S. Hulot and R. J. Hajjar (2015). "Effectiveness of gene delivery systems for pluripotent and differentiated cells." Molecular Therapy-Methods & Clinical Development 2: 14067.
Rosenthal, R., B. Bakall, T. Kinnick, N. Peachey, S. Wimmers, C. Wadelius, A. Marmorstein and O. Strauss (2006). "Expression of bestrophin-1, the product of the VMD2 gene, modulates voltage-dependent Ca2+ channels in retinal pigment epithelial cells." FASEB J 20(1): 178-180. Sancho-Pelluz, J., J. Tosi, C.-W. Hsu, F. Lee, K. Wolpert, M. R. Tabacaru, J. P. Greenberg, S. H. Tsang and C.-S. Lin (2012). "Mice with a D190N mutation in the gene encoding rhodopsin: a model for human autosomal-dominant retinitis pigmentosa." Molecular medicine 18(1): 549. Sancho-Pelluz, J., J. Tosi, C. W. Hsu, F. Lee, K. Wolpert, M. R. Tabacaru, J. P. Greenberg, S. H.
Tsang and C. S. Lin (2012). "Mice with a D190N mutation in the gene encoding rhodopsin: a model for human autosomal-dominant retinitis pigmentosa." Molecular medicine 18(1): 549-555.
Schuerch, K., M. Marsiglia, W. Lee, S. H. Tsang and J. R. Sparrow (2016). "Multimodal Imaging of Disease-Associated Pigmentary Changes in Retinitis Pigmentosa." Retina.
Shen, B., W. Zhang, J. Zhang, J. Zhou, J. Wang, L. Chen, L. Wang, A. Hodgkins, V. Iyer and X. Huang (2014). "Efficient genome modification by CRISPR-Cas9 nickase with minimal off target effects." Nature methods 11(4): 399-402.
Singh, R., W. Shen, D. Kuai, J. M. Martin, X. Guo, M. A. Smith, E. T. Perez, M. J. Phillips, J.
M. Simonett, K. A. Wallace, A. D. Verhoeven, E. E. Capowski, X. Zhang, Y. Yin, P. J. Halbach, G. A. Fishman, L. S. Wright, B. R. Pattnaik and D. M. Gamm (2013). "iPS cell modeling of Best disease: insights into the pathophysiology of an inherited macular degeneration." Hum Mol Genet 22(3): 593-607.
Smith, C., L. Abalde-Atristain, C. He, B. R. Brodsky, E. M. Braunstein, P. Chaudhari, Y.-Y. Jang, L. Cheng and Z. Ye (2015). "Efficient and allele-specific genome editing of disease loci in human iPSCs." Molecular Therapy 23(3): 570-577.
Smith, C., A. Gore, W. Yan, L. Abalde-Atristain, Z. Li, C. He, Y. Wang, R. A. Brodsky, K. Zhang and L. Cheng (2014). "Whole-genome sequencing analysis reveals high specificity of CRISPR/Cas9 and TALEN-based genome editing in human iPSCs." Cell stem cell 15(1): 12-13. Spaide, R. F., K. Noble, A. Morgan and K. B. Freund (2006). "Vitelliform macular dystrophy." Ophthalmology 113(8): 1392-1400.
Sparrow, J. R., T. Duncker, R. Woods and F. C. Delori (2016). "Quantitative Fundus Autofluorescence in Best Vitelliform Macular Dystrophy: RPE Lipofuscin is not Increased in Non-Lesion Areas of Retina." Adv Exp Med Biol 854: 285-290.
Sparrow, J. R., M. Marsiglia, R. Allikmets, S. Tsang, W. Lee, T. Duncker and J. Zernant (2015). "Flecks in Recessive Stargardt Disease: Short-Wavelength Autofluorescence, Near-Infrared Autofluorescence, and Optical Coherence Tomography." Invest Ophthalmol Vis Sci 56(8): 5029-5039.
Sparrow, J. R. and K. Yamamoto (2012). "The bisretinoids of RPE lipofuscin: a complex mixture." Adv Exp Med Biol 723: 761-767.
Staurenghi, G., S. Sadda, U. Chakravarthy, R. F. Spaide and P. International Nomenclature for Optical Coherence Tomography (2014). "Proposed lexicon for anatomic landmarks in normal posterior segment spectral-domain optical coherence tomography: the IN*OCT consensus." Ophthalmology 121(8): 1572-1578.
Sun, H., T. Tsunenari, K. W. Yau and J. Nathans (2002). "The vitelliform macular dystrophy protein defines a new family of chloride channels." Proc Natl Acad Sci U S A 99(6): 4008-4013.
Tabebordbar, M., K. Zhu, J. K. Cheng, W. L. Chew, J. J. Widrick, W. X. Yan, C. Maesner, E. Y. Wu, R. Xiao and F. A. Ran (2016). "In vivo gene editing in dystrophic mouse muscle and muscle stem cells." Science 351(6271): 407-411.
Tosi, J., R. J. Davis, N. K. Wang, M. Naumann, C. S. Lin and S. H. Tsang (2011). "shRNA knockdown of guanylate cyclase 2e or cyclic nucleotide gated channel alpha 1 increases photoreceptor survival in a cGMP phosphodiesterase mouse model of retinitis pigmentosa." Journal of cellular and molecular medicine 15(8): 1778-1787.
Tosi, J., J. Sancho-Pelluz, R. J. Davis, C. W. Hsu, K. V. Wolpert, J. D. Sengillo, C. S. Lin and S. H. Tsang (2011). "Lentivirus-mediated expression of cDNA and shRNA slows degeneration in retinitis pigmentosa." Exp Biol Med (Maywood).
Trapani, I., P. Colella, A. Sommella, C. Iodice, G. Cesi, S. de Simone, E. Marrocco, S. Rossi, M. Giunti and A. Palfi (2014). "Effective delivery of large genes to the retina by dual AAV vectors." EMBO molecular medicine 6(2): 194-211.
Tsai, S. Q., N. Wyvekens, C. Khayter, J. A. Foden, V. Thapar, D. Reyon, M. J. Goodwin, M. J. Aryee and J. K. Joung (2014). "Dimeric CRISPR RNA-guided FokI nucleases for highly specific genome editing." Nature biotechnology 32(6): 569-576.
Tsang, S. H., M. E. Burns, P. D. Calvert, P. Gouras, D. A. Baylor, S. P. Goff and V. Y. Arshavsky (1998). "Role for the target enzyme in deactivation of photoreceptor G protein in vivo." Science 282(5386): 117-121.
Tsang, S. H., L. Chan, Y. T. Tsai, W. H. Wu, C. W. Hsu, J. Yang, J. Tosi, K. J. Wert, R. J. Davis and V. B. Mahajan (2014). "Silencing of tuberin enhances photoreceptor survival and function in a preclinical model of retinitis pigmentosa (an american ophthalmological society thesis)." Trans Am Ophthalmol Soc 112: 103-115.
Tsang, S. H., J. Chen, C. K. Yamashita, M. I. Simon, P. Gouras, D. B. Farber and S. P. Goff (1996). "Apoptotic photoreceptor death in gene targeted mutant mice and its deceleration by gene therapy." The American Journal of Human Genetics 59(Oct.): A54.
Tsang, S. H., P. Gouras, C. K. Yamashita, H. Kjeldbye, J. Fisher, D. B. Farber and S. P. Goff (1996). "Retinal degeneration in mice lacking the gamma subunit of the rod cGMP phosphodiesterase." Science 272(5264): 1026-1029.
Tsang, S. H., P. Gouras, C. K. Yamashita, H. Kjeldbye, J. Fisher, D. B. Farber and S. P. Gofft (1996). "Retinal degeneration in mice lacking the γ subunit of the rod cGMP phosphodiesterase." Science (New York, NY) 272(5264): 1026.
Tsang, S. H., I. Tsui, C. L. Chou, J. Zernant, E. Haamer, R. Iranmanesh, J. Tosi and R. Allikmets (2008). "A novel mutation and phenotypes in phosphodiesterase 6 deficiency." American journal of ophthalmology 146(5): 780-788. e781.
Tsang, S. H., I. Tsui, C. L. Chou, J. Zernant, E. Haamer, R. Iranmanesh, J. Tosi and R. Allikmets (2008). "A novel mutation and phenotypes in phosphodiesterase 6 deficiency." Am J Ophthalmol 146(5): 780-788.
Vasireddy, V., J. A. Mills, R. Gaddameedi, E. Basner-Tschakarjan, M. Kohnke, A. D. Black, K. Alexandrov, S. Zhou, A. M. Maguire and D. C. Chung (2013). "AAV-mediated gene therapy for choroideremia: preclinical studies in personalized models." PLoS One 8(5): e61396.
Vedantham, V. and K. Ramasamy (2005). "Optical coherence tomography in Best's disease: an observational case report." Am J Ophthalmol 139(2): 351-353.
Velez, G., S. H. Tsang, Y. T. Tsai, C. W. Hsu, A. Gore, A. H. Abdelhakim, M. Mahajan, R. H. Silverman, J. R. Sparrow, A. G. Bassuk and V. B. Mahajan (2017). "Gene Therapy Restores Mfrp and Corrects Axial Eye Length." Sci Rep 7(1): 16151.
Wang, N. K., J. Tosi, J. M. Kasanuki, C. L. Chou, J. Kong, N. Parmalee, K. J. Wert, R. Allikmets, C. C. Lai, C. L. Chien, T. Nagasaki, C. S. Lin and S. H. Tsang (2010). "Transplantation of reprogrammed embryonic stem cells improves visual function in a mouse model for retinitis pigmentosa." Transplantation 89(8): 911-919.
Weingeist, T. A., J. L. Kobrin and R. C. Watzke (1982). "Histopathology of Best's macular dystrophy." Arch Ophthalmol 100(7): 1108-1114.
Wert, K. J., R. J. Davis, J. Sancho-Pelluz, P. M. Nishina and S. H. Tsang (2012). "Gene therapy provides long-term visual function in a pre-clinical model of retinitis pigmentosa." Human molecular genetics 22(3): 558-567.
Wert, K. J., R. J. Davis, J. Sancho-Pelluz, P. M. Nishina and S. H. Tsang (2013). "Gene therapy provides long-term visual function in a pre-clinical model of retinitis pigmentosa." Human molecular genetics 22(3): 558-567.
Wert, K. J., J. M. Skeie, A. G. Bassuk, A. K. Olivier, S. H. Tsang and V. B. Mahajan (2014). "Functional validation of a human CAPN5 exome variant by lentiviral transduction into mouse retina." Hum Mol Genet 23(10): 2665-2677.
Woodruff, M. L., K. M. Janisch, I. V. Peshenko, A. M. Dizhoor, S. H. Tsang and G. L. Fain (2008). "Modulation of phosphodiesterase6 turnoff during background illumination in mouse rod photoreceptors." J Neurosci 28(9): 2064-2074.
Wu, W.-H., Y.-T. Tsai, S. Justus, T.-T. Lee, L. Zhang, C.-S. Lin, A. G. Bassuk, V. B. Mahajan and S. H. Tsang (2016). "CRISPR repair reveals causative mutation in a preclinical model of retinitis pigmentosa." Molecular Therapy.
Yang, J., Y. Li, L. Chan, Y. T. Tsai, W. H. Wu, H. V. Nguyen, C. W. Hsu, X. Li, L. M. Brown, D. Egli, J. R. Sparrow and S. H. Tsang (2014). "Validation of genome-wide association study (GWAS)-identified disease risk alleles with patient-specific stem cell lines." Hum Mol Genet 23(13): 3445-3455.
Yang, J., Y. Li, D. Erol, W.-H. Wu, Y.-T. Tsai, X.-R. Li, R. J. Davis and S. H. Tsang (2014). "Generation of induced pluripotent stem cells from conjunctiva." Graefe's Archive for Clinical and Experimental Ophthalmology 252(3): 423-431.
Yang, J., M. C. Naumann, Y.-T. Tsai, J. Tosi, D. Erol, C.-S. Lin, R. J. Davis and S. H. Tsang (2012). "Vigabatrin-induced retinal toxicity is partially mediated by signaling in rod and cone photoreceptors." PloS one 7(8): e43889.
Yang, J., M. C. Naumann, Y. T. Tsai, J. Tosi, D. Erol, C. S. Lin, R. J. Davis and S. H. Tsang (2012). "Vigabatrin-induced retinal toxicity is partially mediated by signaling in rod and cone photoreceptors." Plos ONE 7(8): e43889. doi:10.1371/journal.pone.0043889.
Yang, T., S. Justus, Y. Li and S. H. Tsang (2015). "BEST1: the Best Target for Gene and Cell Therapies." Mol Ther 23(12): 1805-1809.
Zhang, L., J. Du, S. Justus, C. W. Hsu, L. Bonet-Ponce, W. H. Wu, Y. T. Tsai, W. P. Wu, Y. Jia, J. K. Duong, V. B. Mahajan, C. S. Lin, S. Wang, J. B. Hurley and S. H. Tsang (2016). "Reprogramming metabolism by targeting sirtuin 6 attenuates retinal degeneration." J Clin Invest 126(12): 4659-4673.
Zhang, X., C. J. Bregman, A. S. Raza, G. De Moraes and D. C. Hood (2011). "Deriving visual field loss based upon OCT of inner retinal thicknesses of the macula." Biomed Opt Express 2(6): 1734-1742.
Zhang, Y., J. B. Stanton, J. Wu, K. Yu, H. C. Hartzell, N. S. Peachey, L. Y. Marmorstein and A. D. Marmorstein (2010). "Suppression of Ca 2+ signaling in a mouse model of Best disease." Human molecular genetics 19(6): 1108-1118.

Table 3 provides gRNA/sgRNA sequences, donor-template modified sequences, and additional sequences used in the Examples of the present disclosure.

**Table 3**

| **Sequence ID Number** | **Sequence** | **Species** |
|---|---|---|
| SEQ ID NO: 1 (sgRNA1) | GGACGGTGACGTAGAGCGTG | *Homo sapiens* |
| SEQ ID NO: 2 (sgRNA2) | GACGAAGTATCCATGCAGAG | *Homo sapiens* |
| SEQ ID NO: 3 (sgRNA-EFEMP1) | TGAGACCACAAATGAATGCT | *Homo sapiens* |
| SEQ ID NO: 4 (sgRNA-D 190N) | AACTACTACACACTCAAGCCTG | *Mus musculus* |
| SEQ ID NO: 5 (sgRNA-Rho Exon 1) | GTAGTACTGCGGCTGCTCGA | *Mus musculus* |
| SEQ ID NO: 6 (sgRNA-*Pde6a*^{D670G}) | GCTCATGCTGCCGGCGATTC | *Mus musculus* |
| SEQ ID NO: 7 (sgRNA-Y1) | GGTTTTGGACAATGGAACCG | *Drosophila melanogaste r* |
| SEQ ID NO: 8 (wt *RHO* cDNA) | atgaatggcacagaaggccctaacttctacgtgcccttctccaatgcgac gggtgtggtacgcagccccttcgagtacccacagtactacctggctgag ccatggcagttctccatgctggccgcctacatgtttctgctgatcgtgctg | *Homo sapiens* |
| | ggcttccccatcaacttcctcacgctctacgtcaccgtccagcacaagaa gctgcgcacgcctctcaactacatcctgctcaacctagccgtggctgac ctcttcatggtcctaggtggcttcaccagcaccctctacacctctctgcat ggatacttcgtcttcgggcccacaggatgcaatttggagggcttctttgc caccctgggcggtgaaattgccctgtggtccttggtggtcctggccatc gagcggtacgtggtggtgtgtaagcccatgagcaacttccgcttcggg gagaaccatgccatcatgggcgttgccttcacctgggtcatggcgctgg cctgcgccgcacccccactcgccggctggtccaggtacatccccgag ggcctgcagtgctcgtgtggaatcgactactacacgctcaagccggag gtcaacaacgagtcttttgtcatctacatgttcgtggtccacttcaccatcc ccatgattatcatctttttctgctatgggcagctcgtcttcaccgtcaagga ggccgctgcccagcagcaggagtcagccaccacacagaaggcagag aaggaggtcacccgcatggtcatcatcatggtcatcgctttcctgatctg ctgggtgccctacgccagcgtggcattctacatcttcacccaccagggc tccaacttcggtcccatcttcatgaccatcccagcgttctttgccaagagc gccgccatctacaaccctgtcatctatatcatgatgaacaagcagttccg gaactgcatgctcaccaccatctgctgcggcaagaacccactgggtga cgatgaggcctctgctaccgtgtccaagacggagacgagccaggtgg ccccggcctaa | |
| SEQ ID NO: 9 (cm*RHO* cDNA) | atgaatggcacagaaggccctaacttctacgtgcccttctccaatgcgac gggtgtggtacgcagccccttcgagtacccacagtactacctggctgag ccatggcagttctccatgctggccgcctacatgtttctgctgatcgtgctg ggcttccccatcaactttcttacactgtacgtcaccgtccagcacaagaa gctgcgcacgcctctcaactacatcctgctcaacctagccgtggctgac ctcttcatggtcctaggtggcttcaccagcaccctctacacgtcgcttcac ggatatttcgtcttcgggcccacaggatgcaatttggagggcttctttgcc accctgggcggtgaaattgccctgtggtccttggtggtcctggccatcg agcggtacgtggtggtgtgtaagcccatgagcaacttccgcttcgggga gaaccatgccatcatgggcgttgccttcacctgggtcatggcgctggcc tgcgccgcacccccactcgccggctggtccaggtacatccccgaggg | *Homo sapiens* |
| | cctgcagtgctcgtgtggaatcgactactacacgctcaagccggaggtc aacaacgagtcttttgtcatctacatgttcgtggtccacttcaccatcccca tgattatcatctttttctgctatgggcagctcgtcttcaccgtcaaggaggc cgctgcccagcagcaggagtcagccaccacacagaaggcagagaag gaggtcacccgcatggtcatcatcatggtcatcgctttcctgatctgctgg gtgccctacgccagcgtggcattctacatcttcacccaccagggctcca acttcggtcccatcttcatgaccatcccagcgttctttgccaagagcgcc gccatctacaaccctgtcatctatatcatgatgaacaagcagttccggaa ctgcatgctcaccaccatctgctgcggcaagaacccactgggtgacgat gaggcctctgctaccgtgtccaagacggagacgagccaggtggcccc ggcc | |
| SEQ ID NO: 10 (CBh promoter) | cgttacataacttacggtaaatggcccgcctggctgaccgcccaacgac ccccgcccattgacgtcaatagtaacgccaatagggactttccattgacg tcaatgggtggagtatttacggtaaactgcccacttggcagtacatcaag tgtatcatatgccaagtacgccccctattgacgtcaatgacggtaaatgg cccgcctggcattgtgcccagtacatgaccttatgggactttcctacttgg cagtacatctacgtattagtcatcgctattaccatggtcgaggtgagcccc acgttctgcttcactctccccatctcccccccctccccacccccaattttgt atttatttattttttaattattttgtgcagcgatgggggcgggggggggg ggggggcgcgcgccaggcggggcggggcggggcgaggggcgggg cggggcgaggcggagaggtgcggcggcagccaatcagagcggcgc gctccgaaagtttccttttatggcgaggcggcggcggcggcggccctat aaaaagcgaagcgcgcggcgggcgggagtcgctgcgacgctgcctt cgccccgtgccccgctccgccgccgcctcgcgccgcccgccccggct ctgactgaccgcgttactcccacaggtgagcgggcgggacggcccttc tcctccgggctgtaattagctgagcaagaggtaagggtttaagggatgg ttggttggtggggtattaatgtttaattacctggagcacctgcctgaaatca ctttttttcaggtt | *Gallus gallus* |
| SEQ ID NO: 11 (BGH poly-A) | taagagctcgctgatcagcctcgactgtgccttctagttgccagccatct gttgtttgcccctcccccgtgccttccttgaccctggaaggtgccactccc | *Bos taurus* |
| | actgtcctttcctaataaaatgaggaaattgcatcgcattgtctgagtaggt gtcattctattctggggggtggggtggggcaggacagcaagggggag gattgggaagagaatagcaggcatgctgggga | |
| SEQ ID NO: 12 (U6 promoter) | gagggcctatttcccatgattccttcatatttgcatatacgatacaaggctg ttagagagataattggaattaatttgactgtaaacacaaagatattagtaca aaatacgtgacgtagaaagtaataatttcttgggtagtttgcagttttaaaa ttatgttttaaaatggactatcatatgcttaccgtaacttgaaagtatttcgat ttcttggctttatatatcttgtggaaaggacgaaacacc | *Homo sapiens* |
| SEQ ID NO: 13 (ITR) | cgcgctcgctcgctcactgaggccgcccgggcaaagcccgggcgtc gggcgacctttggtcgcccggcctcagtgagcgagcgagcgcgcaga gagggagtggccaactccatcactaggggttccttgt | *Adeno-associated virus-2* |
| SEQ ID NO: 14 (sCMV promoter) | actcacggggatttccaagtctccaccccattgacgtcaatgggagtttg ttttggcaccaaaatcaacgggactttccaaaatgtcgtaataaccccgc cccgttgacgcaaatgggcggtaggcgtgtacggtgggaggtctatat aagcagagctcgtttagtgaaccgt | *Human herpesvirus 5* |
| SEQ ID NO: 15 (3xFlag) | GACTATAAGGACCACGACGGAGACTACAA GGATCATGATATTGATTACAAAGACGATGA CGATAAG | |
| SEQ ID NO: 16 (SV40-NLS) | CCAAAGAAGAAGCGGAAGGTC | *Simian virus 40* |
| SEQ ID NO: 17 (Cas9) | gacaagaagtacagcatcggcctggacatcggcaccaactctgtgggc tgggccgtgatcaccgacgagtacaaggtgcccagcaagaaattcaag gtgctgggcaacaccgaccggcacagcatcaagaagaacctgatcgg agccctgctgttcgacagcggcgaaacagccgaggccacccggctga agagaaccgccagaagaagatacaccagacggaagaaccggatctg ctatctgcaagagatcttcagcaacgagatggccaaggtggacgacag cttcttccacagactggaagagtccttcctggtggaagaggataagaag cacgagcggcaccccatcttcggcaacatcgtggacgaggtggcctac cacgagaagtaccccaccatctaccacctgagaaagaaactggtggac | *Streptococcu s pyogenes* |
| | agcaccgacaaggccgacctgcggctgatctatctggccctggcccac atgatcaagttccggggccacttcctgatcgagggcgacctgaacccc gacaacagcgacgtggacaagctgttcatccagctggtgcagacctac aaccagctgttcgaggaaaaccccatcaacgccagcggcgtggacgc caaggccatcctgtctgccagactgagcaagagcagacggctggaaa atctgatcgcccagctgcccggcgagaagaagaatggcctgttcggaa acctgattgccctgagcctgggcctgacccccaacttcaagagcaactt cgacctggccgaggatgccaaactgcagctgagcaaggacacctacg acgacgacctggacaacctgctggcccagatcggcgaccagtacgcc gacctgtttctggccgccaagaacctgtccgacgccatcctgctgagcg acatcctgagagtgaacaccgagatcaccaaggcccccctgagcgcct ctatgatcaagagatacgacgagcaccaccaggacctgaccctgctga aagctctcgtgcggcagcagctgcctgagaagtacaaagagattttcttc gaccagagcaagaacggctacgccggctacattgacggcggagcca gccaggaagagttctacaagttcatcaagcccatcctggaaaagatgga cggcaccgaggaactgctcgtgaagctgaacagagaggacctgctgc ggaagcagcggaccttcgacaacggcagcatcccccaccagatccac ctgggagagctgcacgccattctgcggcggcaggaagatttttacccat tcctgaaggacaaccgggaaaagatcgagaagatcctgaccttccgca tcccctactacgtgggccctctggccaggggaaacagcagattcgcct ggatgaccagaaagagcgaggaaaccatcaccccctggaacttcgag gaagtggtggacaagggcgcttccgcccagagcttcatcgagcggat gaccaacttcgataagaacctgcccaacgagaaggtgctgcccaagca cagcctgctgtacgagtacttcaccgtgtataacgagctgaccaaagtg aaatacgtgaccgagggaatgagaaagcccgccttcctgagcggcga gcagaaaaaggccatcgtggacctgctgttcaagaccaaccggaaagt gaccgtgaagcagctgaaagaggactacttcaagaaaatcgagtgctt cgactccgtggaaatctccggcgtggaagatcggttcaacgcctccctg ggcacataccacgatctgctgaaaattatcaaggacaaggacttcctgg acaatgaggaaaacgaggacattctggaagatatcgtgctgaccctga cactgtttgaggacagagagatgatcgaggaacggctgaaaacctatg | |
| | cccacctgttcgacgacaaagtgatgaagcagctgaagcggcggaga tacaccggctggggcaggctgagccggaagctgatcaacggcatccg ggacaagcagtccggcaagacaatcctggatttcctgaagtccgacgg cttcgccaacagaaacttcatgcagctgatccacgacgacagcctgacc tttaaagaggacatccagaaagcccaggtgtccggccagggcgatag cctgcacgagcacattgccaatctggccggcagccccgccattaagaa gggcatcctgcagacagtgaaggtggtggacgagctcgtgaaagtgat gggccggcacaagcccgagaacatcgtgatcgaaatggccagagag aaccagaccacccagaagggacagaagaacagccgcgagagaatg aagcggatcgaagagggcatcaaagagctgggcagccagatcctgaa agaacaccccgtggaaaacacccagctgcagaacgagaagctgtacc tgtactacctgcagaatgggcgggatatgtacgtggaccaggaactgg acatcaaccggctgtccgactacgatgtggaccatatcgtgcctcagag ctttctgaaggacgactccatcgacaacaaggtgctgaccagaagcga caagaaccggggcaagagcgacaacgtgccctccgaagaggtcgtg aagaagatgaagaactactggcggcagctgctgaacgccaagctgatt acccagagaaagttcgacaatctgaccaaggccgagagaggcggcct gagcgaactggataaggccggcttcatcaagagacagctggtggaaa cccggcagatcacaaagcacgtggcacagatcctggactcccggatg aacactaagtacgacgagaatgacaagctgatccgggaagtgaaagtg atcaccctgaagtccaagctggtgtccgatttccggaaggatttccagttt tacaaagtgcgcgagatcaacaactaccaccacgcccacgacgcctac ctgaacgccgtcgtgggaaccgccctgatcaaaaagtaccctaagctg gaaagcgagttcgtgtacggcgactacaaggtgtacgacgtgcggaa gatgatcgccaagagcgagcaggaaatcggcaaggctaccgccaagt acttcttctacagcaacatcatgaactttttcaagaccgagattaccctgg ccaacggcgagatccggaagcggcctctgatcgagacaaacggcga aaccggggagatcgtgtgggataagggccgggattttgccaccgtgc ggaaagtgctgagcatgccccaagtgaatatcgtgaaaaagaccgag gtgcagacaggcggcttcagcaaagagtctatcctgcccaagaggaac agcgataagctgatcgccagaaagaaggactgggaccctaagaagta | |
| | cggcggcttcgacagccccaccgtggcctattctgtgctggtggtggcc aaagtggaaaagggcaagtccaagaaactgaagagtgtgaaagagct gctggggatcaccatcatggaaagaagcagcttcgagaagaatcccat cgactttctggaagccaagggctacaaagaagtgaaaaaggacctgat catcaagctgcctaagtactccctgttcgagctggaaaacggccggaa gagaatgctggcctctgccggcgaactgcagaagggaaacgaactgg ccctgccctccaaatatgtgaacttcctgtacctggccagccactatgag aagctgaagggctcccccgaggataatgagcagaaacagctgtttgtg gaacagcacaagcactacctggacgagatcatcgagcagatcagcga gttctccaagagagtgatcctggccgacgctaatctggacaaagtgctg tccgcctacaacaagcaccgggataagcccatcagagagcaggccga gaatatcatccacctgtttaccctgaccaatctgggagcccctgccgcct tcaagtactttgacaccaccatcgaccggaagaggtacaccagcacca aagaggtgctggacgccaccctgatccaccagagcatcaccggcctgt acgagacacggatcgacctgtctcagctgggaggcgac | |
| SEQ ID NO: 18 (nucleoplasmin NLS) | aaaaggccggcggccacgaaaaaggccggccaggcaaaaaagaaa aag | *Xenopus laevis* |
| SEQ ID NO: 19 (synthetic poly A) | aataaaagatctttattttcattagatctgtgtgttggttttttgtgtg | *Oryctolagus cuniculus* |
| SEQ ID NO: 20 (CMV promoter) | cgttacataacttacggtaaatggcccgcctggctgaccgcccaacgac ccccgcccattgacgtcaataatgacgtatgttcccatagtaacgccaat agggactttccattgacgtcaatgggtggagtatttacggtaaactgccc acttggcagtacatcaagtgtatcatatgccaagtacgccccctattgac gtcaatgacggtaaatggcccgcctggcattatgcccagtacatgacctt atgggactttcctacttggcagtacatctacgtattagtcatcgctattacc atggtgatgcggttttggcagtacatcaatgggcgtggatagcggtttga ctcacggggatttccaagtctccaccccattgacgtcaatgggagtttgtt ttggcaccaaaatcaacgggactttccaaaatgtcgtaacaactccgcc ccattgacgcaaatgggcggtaggcgtgtacggtgggaggtctatataa gcagagct | *Human herpesvirus 5* |
| SEQ ID NO: 21 (EGFP) | atggtgagcaagggcgaggagctgttcaccggggtggtgcccatcct ggtcgagctggacggcgacgtaaacggccacaagttcagcgtgtccg gcgagggcgagggcgatgccacctacggcaagctgaccctgaagttc atctgcaccaccggcaagctgcccgtgccctggcccaccctcgtgacc accctgacctacggcgtgcagtgcttcagccgctaccccgaccacatg aagcagcacgacttcttcaagtccgccatgcccgaaggctacgtccag gagcgcaccatcttcttcaaggacgacggcaactacaagacccgcgcc gaggtgaagttcgagggcgacaccctggtgaaccgcatcgagctgaa gggcatcgacttcaaggaggacggcaacatcctggggcacaagctgg agtacaactacaacagccacaacgtctatatcatggccgacaagcaga agaacggcatcaaggtgaacttcaagatccgccacaacatcgaggac ggcagcgtgcagctcgccgaccactaccagcagaacacccccatcgg cgacggccccgtgctgctgcccgacaaccactacctgagcacccagtc cgccctgagcaaagaccccaacgagaagcgcgatcacatggtcctgc tggagttcgtgaccgccgccgggatcactctcggcatggacgagctgt acaagtaa | *Aequorea victoria* |
| SEQ ID NO: 22 [pSpCas9(BB)-2A-puro(pX459)] | gagggcctatttcccatgattccttcatatttgcatatacgatacaaggctg ttagagagataattggaattaatttgactgtaaacacaaagatattagtaca aaatacgtgacgtagaaagtaataatttcttgggtagtttgcagttttaaaa ttatgttttaaaatggactatcatatgcttaccgtaacttgaaagtatttcgat ttcttggctttatatatcttGTGGAAAGGACGAAACACCg gGTCTTCgaGAAGACctgttttagagctaGAAAtagcaa gttaaaataaggctagtccgttatcaacttgaaaaagtggcaccgagtcg gtgcTTTTTTgttttagagctagaaatagcaagttaaaataaggcta gtccgtTTTTagcgcgtgcgccaattctgcagacaaatggctctaga ggtacccgttacataacttacggtaaatggcccgcctggctgaccgccc aacgacccccgcccattgacgtcaatagtaacgccaatagggactttcc attgacgtcaatgggtggagtatttacggtaaactgcccacttggcagta catcaagtgtatcatatgccaagtacgccccctattgacgtcaatgacgg taaatggcccgcctggcattGtgcccagtacatgaccttatgggactttc | *Streptococcu s pyogenes* |
| | ctacttggcagtacatctacgtattagtcatcgctattaccatggtcgaggt gagccccacgttctgcttcactctccccatctcccccccctccccacccc caattttgtatttatttattttttaattattttgtgcagcgatgggggcggggg gggggggggggcgcgcgccaggcggggcggggcggggcgaggg gcggggcggggcgaggcggagaggtgcggcggcagccaatcaga gcggcgcgctccgaaagtttccttttatggcgaggcggcggcggcgg cggccctataaaaagcgaagcgcgcggcgggcgggagtcgctgcga cgctgccttcgccccgtgccccgctccgccgccgcctcgcgccgccc gccccggctctgactgaccgcgttactcccacaggtgagcgggcggg acggcccttctcctccgggctgtaattagctgagcaagaggtaagggttt aagggatggttggttggtggggtattaatgtttaattacctggagcacctg cctgaaatcactttttttcaggttGGaccggtgccaccATGGACT ATAAGGACCACGACGGAGACTACAAGGAT CATGATATTGATTACAAAGACGATGACGAT AAGATGGCCCCAAAGAAGAAGCGGAAGGT CGGTATCCACGGAGTCCCAGCAGCCGACAA GAAGTACAGCATCGGCCTGGACATCGGCAC CAACTCTGTGGGCTGGGCCGTGATCACCGA CGAGTACAAGGTGCCCAGCAAGAAATTCAA GGTGCTGGGCAACACCGACCGGCACAGCAT CAAGAAGAACCTGATCGGAGCCCTGCTGTT CGACAGCGGCGAAACAGCCGAGGCCACCC GGCTGAAGAGAACCGCCAGAAGAAGATAC ACCAGACGGAAGAACCGGATCTGCTATCTG CAAGAGATCTTCAGCAACGAGATGGCCAAG GTGGACGACAGCTTCTTCCACAGACTGGAA GAGTCCTTCCTGGTGGAAGAGGATAAGAAG CACGAGCGGCACCCCATCTTCGGCAACATC GTGGACGAGGTGGCCTACCACGAGAAGTAC CCCACCATCTACCACCTGAGAAAGAAACTG GTGGACAGCACCGACAAGGCCGACCTGCGG | |
| | CTGATCTATCTGGCCCTGGCCCACATGATCA AGTTCCGGGGCCACTTCCTGATCGAGGGCG ACCTGAACCCCGACAACAGCGACGTGGACA AGCTGTTCATCCAGCTGGTGCAGACCTACA ACCAGCTGTTCGAGGAAAACCCCATCAACG CCAGCGGCGTGGACGCCAAGGCCATCCTGT CTGCCAGACTGAGCAAGAGCAGACGGCTGG AAAATCTGATCGCCCAGCTGCCCGGCGAGA AGAAGAATGGCCTGTTCGGAAACCTGATTG CCCTGAGCCTGGGCCTGACCCCCAACTTCA AGAGCAACTTCGACCTGGCCGAGGATGCCA AACTGCAGCTGAGCAAGGACACCTACGACG ACGACCTGGACAACCTGCTGGCCCAGATCG GCGACCAGTACGCCGACCTGTTTCTGGCCG CCAAGAACCTGTCCGACGCCATCCTGCTGA GCGACATCCTGAGAGTGAACACCGAGATCA CCAAGGCCCCCCTGAGCGCCTCTATGATCA AGAGATACGACGAGCACCACCAGGACCTG ACCCTGCTGAAAGCTCTCGTGCGGCAGCAG CTGCCTGAGAAGTACAAAGAGATTTTCTTC GACCAGAGCAAGAACGGCTACGCCGGCTAC ATTGACGGCGGAGCCAGCCAGGAAGAGTTC TACAAGTTCATCAAGCCCATCCTGGAAAAG ATGGACGGCACCGAGGAACTGCTCGTGAAG CTGAACAGAGAGGACCTGCTGCGGAAGCA GCGGACCTTCGACAACGGCAGCATCCCCCA CCAGATCCACCTGGGAGAGCTGCACGCCAT TCTGCGGCGGCAGGAAGATTTTTACCCATT CCTGAAGGACAACCGGGAAAAGATCGAGA AGATCCTGACCTTCCGCATCCCCTACTACGT GGGCCCTCTGGCCAGGGGAAACAGCAGATT | |
| | CGCCTGGATGACCAGAAAGAGCGAGGAAA CCATCACCCCCTGGAACTTCGAGGAAGTGG TGGACAAGGGCGCTTCCGCCCAGAGCTTCA TCGAGCGGATGACCAACTTCGATAAGAACC TGCCCAACGAGAAGGTGCTGCCCAAGCACA GCCTGCTGTACGAGTACTTCACCGTGTATA ACGAGCTGACCAAAGTGAAATACGTGACCG AGGGAATGAGAAAGCCCGCCTTCCTGAGCG GCGAGCAGAAAAAGGCCATCGTGGACCTGC TGTTCAAGACCAACCGGAAAGTGACCGTGA AGCAGCTGAAAGAGGACTACTTCAAGAAA ATCGAGTGCTTCGACTCCGTGGAAATCTCC GGCGTGGAAGATCGGTTCAACGCCTCCCTG GGCACATACCACGATCTGCTGAAAATTATC AAGGACAAGGACTTCCTGGACAATGAGGA AAACGAGGACATTCTGGAAGATATCGTGCT GACCCTGACACTGTTTGAGGACAGAGAGAT GATCGAGGAACGGCTGAAAACCTATGCCCA CCTGTTCGACGACAAAGTGATGAAGCAGCT GAAGCGGCGGAGATACACCGGCTGGGGCA GGCTGAGCCGGAAGCTGATCAACGGCATCC GGGACAAGCAGTCCGGCAAGACAATCCTGG ATTTCCTGAAGTCCGACGGCTTCGCCAACA GAAACTTCATGCAGCTGATCCACGACGACA GCCTGACCTTTAAAGAGGACATCCAGAAAG CCCAGGTGTCCGGCCAGGGCGATAGCCTGC ACGAGCACATTGCCAATCTGGCCGGCAGCC CCGCCATTAAGAAGGGCATCCTGCAGACAG TGAAGGTGGTGGACGAGCTCGTGAAAGTGA TGGGCCGGCACAAGCCCGAGAACATCGTGA TCGAAATGGCCAGAGAGAACCAGACCACCC | |
| | AGAAGGGACAGAAGAACAGCCGCGAGAGA ATGAAGCGGATCGAAGAGGGCATCAAAGA GCTGGGCAGCCAGATCCTGAAAGAACACCC CGTGGAAAACACCCAGCTGCAGAACGAGA AGCTGTACCTGTACTACCTGCAGAATGGGC GGGATATGTACGTGGACCAGGAACTGGACA TCAACCGGCTGTCCGACTACGATGTGGACC ATATCGTGCCTCAGAGCTTTCTGAAGGACG ACTCCATCGACAACAAGGTGCTGACCAGAA GCGACAAGAACCGGGGCAAGAGCGACAAC GTGCCCTCCGAAGAGGTCGTGAAGAAGATG AAGAACTACTGGCGGCAGCTGCTGAACGCC AAGCTGATTACCCAGAGAAAGTTCGACAAT CTGACCAAGGCCGAGAGAGGCGGCCTGAG CGAACTGGATAAGGCCGGCTTCATCAAGAG ACAGCTGGTGGAAACCCGGCAGATCACAAA GCACGTGGCACAGATCCTGGACTCCCGGAT GAACACTAAGTACGACGAGAATGACAAGCT GATCCGGGAAGTGAAAGTGATCACCCTGAA GTCCAAGCTGGTGTCCGATTTCCGGAAGGA TTTCCAGTTTTACAAAGTGCGCGAGATCAA CAACTACCACCACGCCCACGACGCCTACCT GAACGCCGTCGTGGGAACCGCCCTGATCAA AAAGTACCCTAAGCTGGAAAGCGAGTTCGT GTACGGCGACTACAAGGTGTACGACGTGCG GAAGATGATCGCCAAGAGCGAGCAGGAAA TCGGCAAGGCTACCGCCAAGTACTTCTTCT ACAGCAACATCATGAACTTTTTCAAGACCG AGATTACCCTGGCCAACGGCGAGATCCGGA AGCGGCCTCTGATCGAGACAAACGGCGAAA CCGGGGAGATCGTGTGGGATAAGGGCCGG | |
| | GATTTTGCCACCGTGCGGAAAGTGCTGAGC ATGCCCCAAGTGAATATCGTGAAAAAGACC GAGGTGCAGACAGGCGGCTTCAGCAAAGA GTCTATCCTGCCCAAGAGGAACAGCGATAA GCTGATCGCCAGAAAGAAGGACTGGGACCC TAAGAAGTACGGCGGCTTCGACAGCCCCAC CGTGGCCTATTCTGTGCTGGTGGTGGCCAA AGTGGAAAAGGGCAAGTCCAAGAAACTGA AGAGTGTGAAAGAGCTGCTGGGGATCACCA TCATGGAAAGAAGCAGCTTCGAGAAGAATC CCATCGACTTTCTGGAAGCCAAGGGCTACA AAGAAGTGAAAAAGGACCTGATCATCAAG CTGCCTAAGTACTCCCTGTTCGAGCTGGAA AACGGCCGGAAGAGAATGCTGGCCTCTGCC GGCGAACTGCAGAAGGGAAACGAACTGGC CCTGCCCTCCAAATATGTGAACTTCCTGTAC CTGGCCAGCCACTATGAGAAGCTGAAGGGC TCCCCCGAGGATAATGAGCAGAAACAGCTG TTTGTGGAACAGCACAAGCACTACCTGGAC GAGATCATCGAGCAGATCAGCGAGTTCTCC AAGAGAGTGATCCTGGCCGACGCTAATCTG GACAAAGTGCTGTCCGCCTACAACAAGCAC CGGGATAAGCCCATCAGAGAGCAGGCCGA GAATATCATCCACCTGTTTACCCTGACCAAT CTGGGAGCCCCTGCCGCCTTCAAGTACTTTG ACACCACCATCGACCGGAAGAGGTACACCA GCACCAAAGAGGTGCTGGACGCCACCCTGA TCCACCAGAGCATCACCGGCCTGTACGAGA CACGGATCGACCTGTCTCAGCTGGGAGGCG ACAAAAGGCCGGCGGCCACGAAAAAGGCC GGCCAGGCAAAAAAGAAAAAGgaattcGGCA | |
| | GTGGAGAGGGCAGAGGAAGTCTGCTAACAT GCGGTGACGTCGAGGAGAATCCTGGCCCAA TGACCGAGTACAAGCCCACGGTGCGCCTCG CCACCCGCGACGACGTCCCCAGGGCCGTAC GCACCCTCGCCGCCGCGTTCGCCGACTACC CCGCCACGCGCCACACCGTCGATCCGGACC GCCACATCGAGCGGGTCACCGAGCTGCAAG AACTCTTCCTCACGCGCGTCGGGCTCGACA TCGGCAAGGTGTGGGTCGCGGACGACGGCG CCGCGGTGGCGGTCTGGACCACGCCGGAGA GCGTCGAAGCGGGGGCGGTGTTCGCCGAGA TCGGCCCGCGCATGGCCGAGTTGAGCGGTT CCCGGCTGGCCGCGCAGCAACAGATGGAAG GCCTCCTGGCGCCGCACCGGCCCAAGGAGC CCGCGTGGTTCCTGGCCACCGTCGGAGTCT CGCCCGACCACCAGGGCAAGGGTCTGGGCA GCGCCGTCGTGCTCCCCGGAGTGGAGGCGG CCGAGCGCGCCGGGGTGCCCGCCTTCCTGG AGACCTCCGCGCCCCGCAACCTCCCCTTCTA CGAGCGGCTCGGCTTCACCGTCACCGCCGA CGTCGAGGTGCCCGAAGGACCGCGCACCTG GTGCATGACCCGCAAGCCCGGTGCCTGAgaat tctaaCTAGAGCTCGCTGATCAGCCTCGACTGT GCCTTCTAGTTGCCAGCCATCTGTTGTTTGC CCCTCCCCCGTGCCTTCCTTGACCCTGGAAG GTGCCACTCCCACTGTCCTTTCCTAATAAAA TGAGGAAATTGCATCGCATTGTCTGAGTAG GTGTCATTCTATTCTGGGGGGTGGGGTGGG GCAGGACAGCAAGGGGGAGGATTGGGAAG AgAATAGCAGGCATGCTGGGGAgcggccgcagga acccctagtgatggagttggccactccctctctgcgcgctcgctcgctca | |
| | ctgaggccgggcgaccaaaggtcgcccgacgcccgggctttgcccg ggcggcctcagtgagcgagcgagcgcgcagctgcctgcaggggcgc ctgatgcggtattttctccttacgcatctgtgcggtatttcacaccgcatac gtcaaagcaaccatagtacgcgccctgtagcggcgcattaagcgcggc gggtgtggtggttacgcgcagcgtgaccgctacacttgccagcgccct agcgcccgctcctttcgctttcttcccttcctttctcgccacgttcgccggc tttccccgtcaagctctaaatcgggggctccctttagggttccgatttagt gctttacggcacctcgaccccaaaaaacttgatttgggtgatggttcacg tagtgggccatcgccctgatagacggtttttcgccctttgacgttggagtc cacgttctttaatagtggactcttgttccaaactggaacaacactcaaccc tatctcgggctattcttttgatttataagggattttgccgatttcggcctattg gttaaaaaatgagctgatttaacaaaaatttaacgcgaattttaacaaaata ttaacgtttacaattttatggtgcactctcagtacaatctgctctgatgccgc atagttaagccagccccgacacccgccaacacccgctgacgcgccct gacgggcttgtctgctcccggcatccgcttacagacaagctgtgaccgt ctccgggagctgcatgtgtcagaggttttcaccgtcatcaccgaaacgc gcgagacgaaagggcctcgtgatacgcctatttttataggttaatgtcatg ataataatggtttcttagacgtcaggtggcacttttcggggaaatgtgcgc ggaacccctatttgtttatttttctaaatacattcaaatatgtatccgctcatg agacaataaccctgataaatgcttcaataatattgaaaaaggaagagtat gagtattcaacatttccgtgtcgcccttattcccttttttgcggcattttgcctt cctgtttttgctcacccagaaacgctggtgaaagtaaaagatgctgaaga tcagttgggtgcacgagtgggttacatcgaactggatctcaacagcggt aagatccttgagagttttcgccccgaagaacgttttccaatgatgagcact tttaaagttctgctatgtggcgcggtattatcccgtattgacgccgggcaa gagcaactcggtcgccgcatacactattctcagaatgacttggttgagta ctcaccagtcacagaaaagcatcttacggatggcatgacagtaagaga attatgcagtgctgccataaccatgagtgataacactgcggccaacttac ttctgacaacgatcggaggaccgaaggagctaaccgcttttttgcacaa catgggggatcatgtaactcgccttgatcgttgggaaccggagctgaat gaagccataccaaacgacgagcgtgacaccacgatgcctgtagcaat | |
| | ggcaacaacgttgcgcaaactattaactggcgaactacttactctagctt cccggcaacaattaatagactggatggaggcggataaagttgcaggac cacttctgcgctcggcccttccggctggctggtttattgctgataaatctg gagccggtgagcgtggaagccgcggtatcattgcagcactggggcca gatggtaagccctcccgtatcgtagttatctacacgacggggagtcagg caactatggatgaacgaaatagacagatcgctgagataggtgcctcact gattaagcattggtaactgtcagaccaagtttactcatatatactttagattg atttaaaacttcatttttaatttaaaaggatctaggtgaagatcctttttgata atctcatgaccaaaatcccttaacgtgagttttcgttccactgagcgtcag accccgtagaaaagatcaaaggatcttcttgagatcctttttttctgcgcgt aatctgctgcttgcaaacaaaaaaaccaccgctaccagcggtggtttgtt tgccggatcaagagctaccaactctttttccgaaggtaactggcttcagc agagcgcagataccaaatactgtccttctagtgtagccgtagttaggcca ccacttcaagaactctgtagcaccgcctacatacctcgctctgctaatcct gttaccagtggctgctgccagtggcgataagtcgtgtcttaccgggttgg actcaagacgatagttaccggataaggcgcagcggtcgggctgaacg gggggttcgtgcacacagcccagcttggagcgaacgacctacaccga actgagatacctacagcgtgagctatgagaaagcgccacgcttcccga agggagaaaggcggacaggtatccggtaagcggcagggtcggaaca ggagagcgcacgagggagcttccagggggaaacgcctggtatctttat agtcctgtcgggtttcgccacctctgacttgagcgtcgatttttgtgatgct cgtcaggggggcggagcctatggaaaaacgccagcaacgcggccttt ttacggttcctggccttttgctggccttttgctcacatgt | |
| SEQ ID NO: 23 (*Rho*^{D190N} donor template) | tcccttaaccaccgaaggcagggcagcaggctagtggagcagagctg cgtggtcaagtggcagggagcttaagaatcgtccaagggcggagacc agtaagtctcattaggtgatggggccagcaggtaaaagccattcatgctt atgtccagctgggcgtgtgttctcttcctgttttatcatcccttgcgctgacc atcaggtacatccctgagggcatgcaatgttcatgcgggattgactattat acccttaagccggaggtcaacaacgaatcctttgtcatctacatgttcgtg gtccacttcaccattcctatgatcgtcatcttcttctgctatgggcagctggt | *Mus musculus* |
| | cttcacagtcaaggaggtatgagcaggg | |
| SEQ ID NO: 24 (wt *RHO* exon1 donor template) | atgctcacctgaataacctggcagcctgctccctcatgcagggaccacg tcctgctgcacccagcaggccatcccgtctccatagcccatggtcatcc ctccctggacaggaatgtgtctcctccccgggctgagtcttgctcaagct agaagcactccgaacagggttatgggcgcctcctccatctcccaagtg gctggcttatgaatgtttaatgtacatgtgagtgaacaaattccaattgaac gcaacaaatagttatcgagccgctgagccggggggcggggggtgtga gactggaggcgatggacggagctgacggcacacacagctcagatctg tcaagtgagccattgtcagggcttggggactggataagtcagggggtct cctgggaagagatgggataggtgagttcaggaggagacattgtcaact ggagccatgtggagaagtgaatttagggcccaaaggttccagtcgcag cctgaggccaccagactgacatggggaggaattcccagaggactctg gggcagacaagatgagacaccctttcctttctttacctaagggcctccac ccgatgtcaccttggcccctctgcaagccaattaggccccggtggcag cagtgggattagcgttagtatgatatctcgcggatgctgaatcagcctct ggcttagggagagaaggtcactttataagggtctggggggggtcagtg cctggagttgcgctgtgggagcgagtcatccagctggagccctgagtg gctgagctcaggccttcgcagcattcttgggtgggagcagccacgggt cagccacaagggccacagccatgaatggcacagaaggccctaacttct acgtgcccttctccaatgcgacgggtgtggtacgcagccccttcgagta cccacagtactacctggctgagccatggcagttctccatgctggccgcc tacatgtttctgctgatcgtgctgggcttccccatcaacttcctcacgctct acgtcaccgtccagcacaagaagctgcgcacgcctctcaactacatcct gctcaacctagccgtggctgacctcttcatggtcctaggtggcttcacca gcaccctctacacctctctgcatggatacttcgtcttcgggcccacagga tgcaatttggagggcttctttgccaccctgggcggtatgagcagagaga ctggggcgggggggtgtagcatgggagccaaggggccacgaaagg gcctgggagggtctgcagcttacttgagtctctttaattggtctcatctaaa ggcccagcttattcattggcaaacactgtgaccctgagctaggctgctgt tgagagcaggcacggaacattcatctatctcatcttgagcaatgcaaga | *Mus musculus & Homo sapiens* |
| | aacatgggttcagagaggccaaggactcaccgaggagtcacagagtg tgggggtgtcctctgaggcagctgagctggggcacacacagactgag caccaggagtgagctctagcttttttttttctatgtgtcttttctaaaagacac ataggtttaggactgtccctggtccaggtaagaactggttcagtaaacttg tacatctcactgcctggccagccctgtcagcttccaccagagtgcgtgc actacacacccggcatctcaaaggattcattcctatctttcctatctttgga gtgaggcacagtctcacgtagtccagtccagactggccttaaattctgca gctgaggatgtacttaaacttgtcatcctcctgccccagcctctcaagtgc tgtgatcacaggcacggaccactatgctacgccaggtgtttccaaacatt ttctctcccttaactggaaggtcaatgaggctctttcgagaagcaacaga gcctgtttagctgagaaaactgaggcagggagcaggcaa | |
| SEQ ID NO: 25 (mutant *RHO* exon1 donor template) | atgctcacctgaataacctggcagcctgctccctcatgcagggaccacg tcctgctgcacccagcaggccatcccgtctccatagcccatggtcatcc ctccctggacaggaatgtgtctcctccccgggctgagtcttgctcaagct agaagcactccgaacagggttatgggcgcctcctccatctcccaagtg gctggcttatgaatgtttaatgtacatgtgagtgaacaaattccaattgaac gcaacaaatagttatcgagccgctgagccggggggcggggggtgtga gactggaggcgatggacggagctgacggcacacacagctcagatctg tcaagtgagccattgtcagggcttggggactggataagtcagggggtct cctgggaagagatgggataggtgagttcaggaggagacattgtcaact ggagccatgtggagaagtgaatttagggcccaaaggttccagtcgcag cctgaggccaccagactgacatggggaggaattcccagaggactctg gggcagacaagatgagacaccctttcctttctttacctaagggcctccac ccgatgtcaccttggcccctctgcaagccaattaggccccggtggcag cagtgggattagcgttagtatgatatctcgcggatgctgaatcagcctct ggcttagggagagaaggtcactttataagggtctggggggggtcagtg cctggagttgcgctgtgggagcgagtcatccagctggagccctgagtg gctgagctcaggccttcgcagcattcttgggtgggagcagccacgggt cagccacaagggccacagccatgaatggcacagaaggccctaacttct acgtgcccttctccaatgcgacgggtgtggtacgcagccccttcgagta | *Mus musculus & Homo sapiens* |
| | cccacagtactacctggctgagccatggcagttctccatgctggccgcc tacatgtttctgctgatcgtgctgggcttccccatcaacttcctcacgctct acgtcaccgtccagcacaagaagctgcgcacgcctctcaactacatcct gctcaacctagccgtggctgacctcttcatggtcctaggtggcttcacca gcaccctctacacctctctgcatggatacttcgtcttcgggcccacagga cgcaatttggagggcttctttgccaccctgggcggtatgagcagagaga ctggggcgggggggtgtagcatgggagccaaggggccacgaaagg gcctgggagggtctgcagcttacttgagtctctttaattggtctcatctaaa ggcccagcttattcattggcaaacactgtgaccctgagctaggctgctgt tgagagcaggcacggaacattcatctatctcatcttgagcaatgcaaga aacatgggttcagagaggccaaggactcaccgaggagtcacagagtg tgggggtgtcctctgaggcagctgagctggggcacacacagactgag caccaggagtgagctctagcttttttttttctatgtgtcttttctaaaagacac ataggtttaggactgtccctggtccaggtaagaactggttcagtaaacttg tacatctcactgcctggccagccctgtcagcttccaccagagtgcgtgc actacacacccggcatctcaaaggattcattcctatctttcctatctttgga gtgaggcacagtctcacgtagtccagtccagactggccttaaattctgca gctgaggatgtacttaaacttgtcatcctcctgccccagcctctcaagtgc tgtgatcacaggcacggaccactatgctacgccaggtgtttccaaacatt ttctctcccttaactggaaggtcaatgaggctctttcgagaagcaacaga gcctgtttagctgagaaaactgaggcagggagcaggcaa | |
| SEQ ID NO: 26 (*Pde6a*^{D670G} donor template) | tgagagatgaggtagggtggcgcccatctcgagggcagcttgcgtga gcacaggcagccttcttgccattggctgaggctgtcattgccgtcaccac ttcgggtgggcaccggaagaagagtgaccttattgccagcaccatttct caaacgttgtctaattcttttctctagagcctgaatatcttccagaatctcaa ccgacgtcaacacgagcatgcgatccacatgatggacatcgcgatcatt gccacagaccttgccttgtatttcaagtgggtatttctcctcactttaatagt agcagtgtgggggctggagagatggttcagtggttaacagcactgact gctcttccagaggtcctgagttcaaatcccagcaaccacatggtggctc acaactatctgtaatgggatctgataccctcttctggtgtgtgtctgaagac | *Mus musculus* |
| | agcgatggagtactcacat | |
| SEQ ID NO: 27 (*EFEMP1*^{R345W} donor template) | tagttagtaaactctttgaccctacatctctacagatataaatgagtgtgag accacaaaCgaGtgcCgggaggatgaaatgtgttggaattatcatgg cggcttccgttgttatccacgaaatcctt | *Homo sapiens* |
| SEQ ID NO: 28 (partial sequence of RPGR showing mutation) | ccttcgtcca gccgcagag | *Homo sapiens* |
| SEQ ID NO: 29 (partial sequence of HTRA1 showing mutation) | tggaagggta ggt | *Homo sapiens* |
| SEQ ID NO: 30 (partial sequence of BEST1 after deletion) | gaggagctga aacctactgt agggaaaggt gcggac | *Homo sapiens* |
| SEQ ID NO: 31 (gRNA1-BEST1) | agaggagctg aaacctaccc g | *Homo sapiens* |
| SEQ ID NO: 32 (partial sequence of BEST1) | gagaggagct gaaacctacc cggggtca | *Homo sapiens* |
| SEQ ID NO: 33 (partial sequence of BEST1) | ctctcctcga ctttggatgg gccccagt | *Homo sapiens* |
| SEQ ID NO: 34 gRNA for BEST1 (intron) | cacagccaggaatggaccat | *Synthetic* |
| SEQ ID NO: 35 gRNA for BEST1 (intron) | atggtccattcctggctgtg | *Synthetic* |
| SEQ ID NO: 36 gRNA for BEST1 (intron) | taagtgtgcaagtcagaaca | *Synthetic* |
| SEQ ID NO: 37 gRNA for BEST1 (intron) | cagcagctgaggtttaaagg | *Synthetic* |
| SEQ ID NO: 38 gRNA for BEST1 (intron) | gtctgaggctgagtatcggg | *Synthetic* |
| SEQ ID NO: 39 gRNA for BEST1 (intron) | ggtctcgctatgttgctcag | *Synthetic* |
| SEQ ID NO: 40 gRNA for BEST1 (SNP rs972355) | agaggagctgaaacctaccc | *Synthetic* |
| SEQ ID NO: 41 gRNA for BEST1 (SNP rs972355) | gaggagctgaaacctacccg | *Synthetic* |
| SEQ ID NO: 42 gRNA for BEST1 (SNP rs972353) | ccacaaaggagtccttgtct | *Synthetic* |
| SEQ ID NO: 43 gRNA for BEST1 (SNP rs2668899) | ttagagtagttttaggttca | *Synthetic* |
| SEQ ID NO: 44 gRNA against RHO (SNP rs_7984 (A/G)) | TTCTTGGGTGGGAGCAGCCA | *Synthetic* |
| SEQ ID NO: 45 gRNA against RHO (SNP rs_7984 (A/G)) | TTCTTGGGTGGGAGCAGCCG | *Synthetic* |
| SEQ ID NO: 46 gRNA against RHO (SNP rs_2855558 | TGAAGGCCAAGTTCCCAATG | *Synthetic* |
| (A/G)) | | |
| SEQ ID NO: 47 gRNA against RHO (SNP rs_2855558 (A/G)) | TGAAGGCCAAGTTCCCAGTG | *Synthetic* |
| SEQ ID NO: 48 gRNA against RHO Exon 1 (ablate/replace) | GGACGGTGACGTAGAGCGTG | *Synthetic* |
| SEQ ID NO: 49 gRNA against RHO Exon 1 (ablate/replace) | GACGAAGTATCCATGCAGAG | *Synthetic* |

The scope of the present invention is not limited by what has been specifically shown and described hereinabove. Those skilled in the art will recognize that there are suitable alternatives to the depicted examples of materials, configurations, constructions and dimensions. Numerous references, including patents and various publications, are cited and discussed in the description of this invention. The citation and discussion of such references is provided merely to clarify the description of the present invention and is not an admission that any reference is prior art to the invention described herein. All references cited and discussed in this specification are incorporated herein by reference in their entirety. Variations, modifications and other implementations of what is described herein will occur to those of ordinary skill in the art without departing from the spirit and scope of the invention. While certain embodiments of the present invention have been shown and described, it will be obvious to those skilled in the art that changes and modifications may be made without departing from the spirit and scope of the invention. The matter set forth in the foregoing description is offered by way of illustration only and not as a limitation.

### Embodiments of the invention

1. A method for modifying an autosomal dominant disease-related gene in a cell, the method comprising:
   contacting the cell with at least one type of vector encoding a CRISPR-Cas system directed to a mutant allele of the autosomal dominant disease-related gene, wherein the autosomal dominant disease-related gene relates to an ocular disease, wherein the at least one type of vector comprises:
      (i) a first sequence encoding a first guide RNA that hybridizes to a variant of a single nucleotide polymorphism (SNP) in the autosomal dominant disease-related gene;
      (ii) a second sequence encoding a second guide RNA that hybridizes to an intron of the autosomal dominant disease-related gene; and,
      (iii) a third sequence encoding a Cas nuclease,
   wherein the Cas nuclease cleaves or targets only the mutant allele that encodes the autosomal dominant disease-related gene that comprises a single nucleotide polymorphism (SNP).
2. The method of embodiment 1, wherein the first guide RNA comprises a nucleotide sequence selected from SEQ ID NO: 40, SEQ. ID NO. 41, SEQ ID NO: 42, SEQ ID NO: 43, SEQ ID NO: 44, SEQ ID NO: 45, SEQ ID NO: 46, SEQ ID NO: 47, SEQ ID NO: 48, or SEQ ID NO: 49 or an equivalent of each thereof.
3. A method for modifying an autosomal dominant disease-related gene in a cell, the method comprising: contacting the cell with at least one type of vector encoding a CRISPR-Cas system directed to a mutant allele of the autosomal dominant disease-related gene, wherein the autosomal dominant disease-related gene relates to an ocular disease, wherein the at least one type of vector comprises: (i) a first sequence encoding a first guide RNA that hybridizes to a variant of a single nucleotide polymorphism (SNP) in the autosomal dominant disease-related; and, (ii) a second sequence encoding a catalytically defective Cas nuclease (dCas);
   wherein the Cas nuclease cleaves or targets only the mutant allele that encodes the autosomal dominant disease-related gene comprising a single nucleotide polymorphism (SNP).
4. The method of embodiment 3, wherein the second guide RNA comprises a nucleotide sequence selected from SEQ ID NO: 34, SEQ ID NO: 35, SEQ ID NO: 36, SEQ ID NO: 37, SEQ ID NO: 38 or SEQ ID NO: 39 or an equivalent of each thereof.
5. The method of embodiments 1 or 3, wherein the cell is an induced pluripotent stem cell (iPSC).
6. The method of embodiment 5, wherein the iPSC is derived from a fibroblast of a subject.
7. The method of embodiment 5, further comprising culturing the iPSC to differentiate into a retinal pigment epithelium (RPE) cell.
8. The method of embodiment 7, further comprising administering the RPE cell to a subject.
9. The method of embodiment 8, wherein the RPE cell is autologous to the subject.
10. The method of embodiment 8, wherein the RPE cell is administered via subretinal transplantation.
11. The method of embodiments 1 or 3, wherein the cell is heterozygous for the SNP.
12. The method of embodiments 1 or 3, wherein the cell is heterozygous for the autosomal dominant disease-related gene having a mutant allele and a wildtype allele.
13. The method of any one of embodiments 1-12, wherein the autosomal dominant disease-related gene comprises a sequence selected from SEQ ID NO: 28, SEQ ID NO: 29, SEQ ID NO: 30, SEQ ID NO: 32, or SEQ ID NO: 33, or an equivalent of each thereof.
14. The method of any one of embodiments 1 or 3-13, wherein the first guide RNA that hybridizes to the autosomal dominant disease-related gene comprises a sequence of SEQ ID NO: 31.
15. The method of any one of embodiments 1-14, wherein the Cas nuclease does not cleave or target the wildtype allele that encodes the autosomal dominant disease-related gene.
16. A method for treating an autosomal dominant ocular disease in a subject, the method comprising: administering to the subject a therapeutically effective amount of at least one type of vector encoding a CRISPR-Cas system directed to a mutant allele of an autosomal dominant disease-related gene in the subject, wherein the at least one type of vector comprises:
   (i) a first sequence encoding a first guide RNA that hybridizes to a variant of a single nucleotide polymorphism (SNP) in the autosomal dominant disease-related gene;
   (ii) a second sequence encoding a second guide RNA that hybridizes to an intron of the autosomal dominant disease-related gene; and,
   (iii) a third sequence encoding a Cas nuclease,
   wherein the Cas nuclease cleaves or targets only the mutant allele that encodes the autosomal dominant disease-related gene that comprises a single nucleotide polymorphism (SNP).
17. A method for treating an autosomal dominant ocular disease in a subject, the method comprising: administering to the subject a therapeutically effective amount of at least one type of vector encoding a CRISPR-Cas system directed to a mutant allele of an autosomal dominant disease-related gene in the subject, wherein the at least one type of vector comprises:
   (i) a first sequence encoding a first guide RNA that hybridizes to a variant of a single nucleotide polymorphism (SNP) in the autosomal dominant disease-related gene; and,
   (ii) a second sequence encoding a catalytically defective Cas nuclease (dCas),
   wherein the Cas nuclease cleaves or targets only the mutant allele that encodes the autosomal dominant disease-related gene that comprises a single nucleotide polymorphism (SNP).
18. The method of embodiments 16 or 17, wherein the subject is heterozygous for the SNP.
19. The method of embodiments 16 or 17, wherein the subject is heterozygous for the autosomal dominant disease-related gene having a mutant allele and a wildtype allele.
20. The method of any of embodiments 1, 3, 16 or 17, wherein the variant of the SNP and the mutant allele are on a same chromosome.
21. The method of any of embodiments 1, 3, 16 or 17, wherein the SNP is in a non-coding region of the autosomal dominant disease-related gene.
22. The method of any of embodiments 1, 3, 16 or 17, wherein the SNP is in a transcription start site (TSS) region of the autosomal dominant disease-related gene.
23. The method of embodiments 1 or 16, wherein the Cas nuclease is a Cas nickase.
24. The method of any of embodiments 1, 3, 16 or 17, wherein the Cas nuclease is Cas9.
25. The method of embodiments 3 or 17, wherein the dCas is fused to a repressor domain.
26. The method of embodiment 25, wherein the repressor domain is a Krüppel-associated Box (KRAB) domain.
27. The method of embodiments 3 or 17, wherein the first guide RNA comprises at least one PUF (Pumilio mRNA binding factor) binding sequence.
28. The method of embodiment 27, wherein the at least one PUF binding sequence binds to PUF-KRAB.
29. The method of any of embodiments 1, 3, 16 or 17, wherein the autosomal dominant disease-related gene is *BEST1.*
30. The method of any of embodiments 1, 3, 16 or 17, wherein the variant of the SNP is rs972353:G SNP, rs972355:G SNP, or rs2668899 SNP.
31. The method of embodiments 1 or 16, wherein the second guide RNA hybridizes to intron 1 of *BEST1.*
32. The method of any of embodiments 1, 3, 16 or 17, wherein the at least one type of vector is at least one type of recombinant adeno-associated viral (AAV) vector.
33. The method of embodiment 32, wherein the at least one type of recombinant AAV vector is at least one type of AAV2 vector.
34. The method of embodiment 32, wherein the at least one type of recombinant AAV vector is at least one type of AAV8 vector.
35. The method of any of embodiments 1, 3, 16 or 17, wherein the autosomal dominant disease-related gene is selected from the group consisting of, PRDM13, RGR, TEAD1, AIPL1, CRX, GUCA1A, GUCY2D, PITPNM3, PROM1, PRPH2,RIMS1, SEMA4A, UNC119, GNAT1, PDE6B, RHO, WSF1, IMPDH1, OTX2, BEST1, C1QTNF5, CTNNA1, EFEMP1, ELOVL4, FSCN2,GUCA1B, HMCN1, IMPG1, RP1L1, TIMP3, VCAN, MFN2, NR2F1, OPA1, ARL3, CA4, HK1, KLHL7, NR2E3, NRL, PRPF3, PRPF4, PRPF6, PRPF8, PRPF31, RDH12, ROM1, RP1, RP9, RPE65, SNRNP200, SPP2, TOPORS, ABCC6, ATXN7, COL11A1, COL2A1, JAG1, KCNJ13, KIF11, OPA3, PAX2, TREX1, CAPN5, CRB1, FZD4, ITM2B, LRP5, MAPKAPK3, MIR204, OPN1SW, RB1, TSPAN12, and ZNF408.
36. The method of embodiments 1 or 16, wherein two types of recombinant AAV vectors are administered to the subject, wherein a first type of recombinant AAV vector comprises the first sequence and the second sequence, and wherein a second type of recombinant AAV vector comprises the third sequence.
37. The method of any of embodiments 1, 3, 16 or 17, wherein the ocular disease is selected from the group consisting of, vitelliform macular dystrophy (VMD), Best vitelliform macular dystrophy, autosomal dominant chorioretinal atrophy or degeneration, autosomal dominant cone or cone-rod dystrophy, autosomal dominant congenital stationary night blindness, autosomal dominant Leber congenital amaurosis, autosomal dominant macular degeneration, autosomal dominant ocular-retinal developmental disease, autosomal dominant optic atrophy, autosomal dominant retinitis pigmentosa, autosomal dominant syndromic/systemic diseases with retinopathy, sorsby macular dystrophy, age-related macular degeneration, doyne honeycomb macular disease, and juvenile macular degeneration.
38. The method of any of embodiments 1, 3, 16 or 17, wherein the ocular disease is age-related macular degeneration.
39. The method of any of embodiments 1, 3, 16 or 17, wherein the ocular disease is juvenile macular degeneration.
40. The method of embodiments 16 or 17, wherein the at least one type of vector is administered by injection into an eye of the subject.
41. The methods of any one of embodiments 16-40, wherein the autosomal dominant disease-related gene comprises a sequence selected from SEQ ID NO: 28, SEQ ID NO: 29, SEQ ID NO: 30, SEQ ID NO: 32, or SEQ ID NO: 33.
42. The methods of any one of embodiments 16-41, wherein the first guide RNA that hybridizes to the autosomal dominant disease-related gene comprises a sequence of SEQ ID NO: 31.
43. The methods of any one of embodiments 16-42, wherein the Cas nuclease does not cleave or target the wildtype allele that encodes the autosomal dominant disease-related gene.
44. A system comprising at least one type of vector: (i) a first sequence encoding a first guide RNA that hybridizes to a variant of a single nucleotide polymorphism (SNP) in an autosomal dominant disease-related gene; (ii) a second sequence encoding a second guide RNA that hybridizes to an intron of the autosomal dominant disease-related gene; and, (iii) a third sequence encoding a Cas nuclease, wherein the Cas nuclease cleaves or targets only the mutant allele that encodes the autosomal dominant disease-related gene that comprises a single nucleotide polymorphism (SNP).
45. The system of embodiment 44, wherein the first guide RNA comprises a nucleotide sequence selected from SEQ ID NO: 40, SEQ. ID NO. 41, SEQ ID NO: 42, SEQ ID NO: 43, SEQ ID NO: 44, SEQ ID NO: 45, SEQ ID NO: 46, SEQ ID NO: 47, SEQ ID NO: 48, or SEQ ID NO: 49 or an equivalent of each thereof.
46. The system of embodiment 44, wherein the Cas nuclease is a Cas nickase.
47. The system of embodiment 44, wherein the Cas nuclease is Cas9.
48. A system comprising at least one type of vector: (i) a first sequence encoding a first guide RNA that hybridizes to a variant of a single nucleotide polymorphism (SNP) in an autosomal dominant disease-related gene that comprises a single nucleotide polymorphism (SNP); and, (ii) a second sequence encoding a catalytically defective Cas nuclease (dCas); wherein the Cas nuclease cleaves or targets only the mutant allele that encodes the autosomal dominant disease-related gene comprising a single nucleotide polymorphism (SNP).
49. The system of embodiment 48, wherein the second guide RNA comprises a nucleotide sequence selected from SEQ ID NO: 34, SEQ ID NO: 35, SEQ ID NO: 36, SEQ ID NO: 37, SEQ ID NO: 38 or SEQ ID NO: 39 or an equivalent of each thereof.
50. The system of embodiment 48, wherein the dCas is fused to a repressor domain.
51. The system of embodiment 48, wherein the repressor domain is a Krüppel-associated Box (KRAB) domain.
52. The system of embodiment 48, wherein the first guide RNA comprises at least one PUF (Pumilio mRNA binding factor) binding sequence.
53. The system of embodiment 52, wherein the at least one PUF binding sequence binds to PUF-KRAB.
54. The method of embodiments 16 or 17 further comprising the step of genotyping the the subject before administering to the subject a therapeutically effective amount of at least one type of vector encoding a CRISPR-Cas system directed to a mutant allele of an autosomal dominant disease-related gene in the subject.
55. The method of any of embodiments 1, 3, 16 or 17, wherein the autosomal dominant disease-related gene is *RHO.*
56. The method of any of embodiments 1, 3, 16 or 17, wherein the variant of the SNP is rs7984:G SNP, rs7984:A SNP, rs2855558:G SNP, or rs2855558:A SNP.

## Claims

1. An *in vitro* method for modifying an autosomal dominant disease-related RHO gene in a cell, wherein the cell is heterozygous for the RHO gene having a mutant allele and a wildtype allele, the method comprising contacting the cell with at least one type of vector encoding a CRISPR-Cas system directed to the mutant allele of the RHO gene, wherein the at least one type of vector comprises:
(i) a first sequence encoding a first guide RNA that hybridizes to a single nucleotide polymorphism (SNP) in the mutant allele of the RHO gene selected from rs7984:G SNP, rs7984:A SNP, rs2855558:G SNP and rs2855558:A SNP;
(ii) a second sequence encoding a second guide RNA that hybridizes to an intron of the RHO gene; and
(iii) a third sequence encoding a Cas nuclease,
wherein the Cas nuclease cleaves or targets only the mutant allele of the RHO gene that comprises the SNP.

2. The method of claim 1, wherein the first guide RNA comprises a nucleotide sequence selected from the group consisting of SEQ ID NO: 44, SEQ ID NO: 45, SEQ ID NO: 46, SEQ ID NO: 47, SEQ ID NO: 48, and SEQ ID NO: 49, or a nucleotide sequence having at least 80% sequence identity of each thereof.

3. An *in vitro* method for modifying an autosomal dominant disease-related RHO gene in a cell, wherein the cell is heterozygous for the RHO gene having a mutant allele and a wildtype allele, the method comprising contacting the cell with at least one type of vector encoding a CRISPR-Cas system directed to the mutant allele of the RHO gene, wherein the at least one type of vector comprises:
(i) a first sequence encoding a first guide RNA that hybridizes to a single nucleotide polymorphism (SNP) in the mutant allele of the RHO gene selected from rs7984:G SNP, rs7984:A SNP, rs2855558:G SNP and rs2855558:A SNP; and
(ii) a second sequence encoding a catalytically defective Cas nuclease (dCas);
wherein the Cas nuclease cleaves or targets only the mutant allele of the RHO gene that comprises the SNP.

4. The method of claims 1 or 3, wherein the cell is an induced pluripotent stem cell (iPSC).

5. At least one type of vector encoding a CRISPR-Cas system for use in a method for treating a RHO-related autosomal dominant ocular disease in a subject, wherein the subject is heterozygous for the RHO gene having a mutant allele and a wildtype allele, the method comprising:
administering to the subject a therapeutically effective amount of the at least one type of vector, wherein the at least one type of vector comprises:
(i) a first sequence encoding a first guide RNA that hybridizes to a single nucleotide polymorphism (SNP) in the mutant allele of the RHO gene selected from rs7984:G SNP, rs7984:A SNP, rs2855558:G SNP and rs2855558:A SNP;
(ii) a second sequence encoding a second guide RNA that hybridizes to an intron of the RHO gene; and
(iii) a third sequence encoding a Cas nuclease,
wherein the Cas nuclease cleaves or targets only the mutant allele of the RHO gene that comprises the SNP.

6. At least one type of vector encoding a CRISPR-Cas system for use in a method for treating a RHO-related autosomal dominant ocular disease in a subject, wherein the subject is heterozygous for the RHO gene having a mutant allele and a wildtype allele, the method comprising:
administering to the subject a therapeutically effective amount of the at least one type of vector, wherein the at least one type of vector comprises:
(i) a first sequence encoding a first guide RNA that hybridizes to a single nucleotide polymorphism (SNP) in the mutant allele of the RHO gene selected from rs7984:G SNP, rs7984:A SNP, rs2855558:G SNP and rs2855558:A SNP; and
(ii) a second sequence encoding a catalytically defective Cas nuclease (dCas);
wherein the Cas nuclease cleaves or targets only the mutant allele of the RHO gene comprises the SNP.

7. The at least one type of vector for use according to claim 5 or 6, wherein the at least one type of vector is administered by injection into an eye of the subject.

8. A system comprising at least one type of vector: (i) a first sequence encoding a first guide RNA that hybridizes to a single nucleotide polymorphism (SNP) selected from rs7984:G SNP, rs7984:A SNP, rs2855558:G SNP and rs2855558:A SNP in a mutant allele of an autosomal dominant disease-related RHO gene; (ii) a second sequence encoding a second guide RNA that hybridizes to an intron of the RHO gene; and, (iii) a third sequence encoding a Cas nuclease, wherein the Cas nuclease cleaves or targets only the mutant allele of the RHO gene that comprises the SNP.

9. The method according to claim 1, the at least one vector for use according to claim 5, or the system according to claim 8, wherein the Cas nuclease is a Cas nickase.

10. A system comprising at least one type of vector: (i) a first sequence encoding a first guide RNA that hybridizes to a single nucleotide polymorphism (SNP) selected from rs7984:G SNP, rs7984:A SNP, rs2855558:G SNP and rs2855558:A SNP in a mutant allele of an autosomal dominant disease-related Rho gene; and, (ii) a second sequence encoding a catalytically defective Cas nuclease (dCas); wherein the Cas nuclease cleaves or targets only the mutant allele of the RHO gene that comprises the SNP.

11. The method according to claim 1 or 3, the at least one vector for use according to claim 5 or 6, or the system according to claim 8 or 10, wherein the Cas nuclease is Cas9.

12. The method according to claim 1 or 3, the at least one vector for use according to claim 5 or 6, or the system according to claim 8 or 10, wherein the at least one type of vector is at least one type of recombinant adeno-associated viral (AAV) vector.

13. The method according to claim 1, the at least one vector for use according to claim 5, or the system according to claim 8, wherein the at least one vector is two types of recombinant AAV vectors, wherein a first type of recombinant AAV vector comprises the first sequence and the second sequence, and wherein a second type of recombinant AAV vector comprises the third sequence.
